# EUROPÄISCHE PATENTSCHRIFT

(11) **EP 2 352 723 B1**
(45) Veröffentlichungstag und Bekanntmachung des Hinweises auf die Patenterteilung: **23.11.2016**
(21) Anmeldenummer: 09744338.6
(22) Anmeldetag: 27.10.2009
(51) Int. Cl.: C07D 213/81, C07D 231/14, C07D 239/28, C07D 239/34, A01N 43/40, A01N 43/54, A01N 43/56, A61K 31/415, A61K 31/44, A61K 31/505, A61P 33/00

(54) **PESTIZIDE N-ARYL- ODER N-HETEROARYL-PYRAZOLCARBOXAMIDVERBINDUNGEN**
PESTICIDAL N-ARYL- OR N-HETEROARYL PYRAZOLE CARBOXAMIDE COMPOUNDS
COMPOSÉS PESTICIDES DE PYRAZOLE CARBOXAMIDES À SUBSTITUTION N-ARYLE OU N-HÉTÉROARYLE

(30) Priorität: 05.11.2008 EP 08168405
(43) Veröffentlichungstag der Anmeldung: 10.08.2011
(73) Patentinhaber: Bayer Intellectual Property GmbH, 40789 Monheim am Rhein (DE)
(72) Erfinder: MAUE, Michael, 40764 Langenfeld (DE); ADELT, Isabelle, 42781 Haan (DE); GIENCKE, Wolfgang, 65719 Hofheim (DE); HEIL, Markus, 42799 Leichlingen (DE); JESCHKE, Peter, 51467 Bergisch Gladbach (DE); KRÜGER, Bernd-Wieland, 51467 Bergisch Gladbach (DE); MÜHLTHAU, Friedrich August, 65812 Bad Soden am Taunus (DE); SUDAU, Alexander, 42799 Leichlingen (DE); RAMING, Klaus, 51375 Leverkusen (DE); EBBINGHAUS-KINTSCHER, Ulrich, 44287 Dortmund (DE); ADAMCZEWSKI, Martin, 51067 Köln (DE); VOERSTE, Arnd, 50674 Köln (DE); GÖRGENS, Ulrich, 40882 Ratingen (DE); KAPFERER, Tobias, 40477 Düsseldorf (DE); DREWES, Mark Wilhelm, 40764 Langenfeld (DE); BECKER, Angela, 40235 Düsseldorf (DE); FRANKEN, Eva-Maria, 51381 Leverkusen (DE)
(74) Vertreter: BIP Patents
(86) Internationale Anmeldenummer: PCT/EP2009/007668
(87) Internationale Veröffentlichungsnummer: WO 2010/051926

(56) Entgegenhaltungen:
- EP-A- 1 714 958
- EP-A- 1 911 751
- WO-A-2004/099156
- WO-A-2004/106324
- WO-A-2007/052843
- WO-A-2008/059214
- WO-A1-2005/040152
- WO-A1-2006/067446
- WO-A1-2008/000438
- WO-A1-2008/029084
- WO-A2-2004/035545
- WO-A2-2009/080203
- DE-A1- 2 701 091
- DE-A1- 19 623 744
- PARLOW JOHN J ET AL: "Utility of Complementary Molecular Reactivity and Molecular Recognition (CMR/R) Technology and Polymer -Supported Reagents in the Solution-Phase Synthesis of Heterocyclic Carboxamides", JOURNAL OF ORGANIC CHEMISTRY, AMERICAN CHEMICAL SOCIETY, EASTON.; US, vol. 62, no. 17, 1 January 1997 (1997-01-01), pages 5908-5919, XP002213047, ISSN: 0022-3263, DOI: 10.1021/JO970571I
- SANTOS FUSTERO ET AL: 'Synthesis of New Fluorinated Tebufenpyrad Analogs with Acaricidal Activity Through Regioselective Pyrazole Formation' THE JOURNAL OF ORGANIC CHEMISTRY Bd. 73, Nr. 21, 15 Oktober 2008, Seiten 8545 - 8552, XP055020602 DOI: 10.1021/jo801729p ISSN: 0022-3263
- FUSTERO S ET AL: "Improved regioselectivity in pyrazole formation through the use of fluorinated alcohols as solvents: Synthesis and biological activity of fluorinated tebufenpyrad analogs", JOURNAL OF ORGANIC CHEMISTRY, AMERICAN CHEMICAL SOCIETY, EASTON.; US, vol. 73, no. 9, 2 May 2008 (2008-05-02), pages 3523-3529, XP002595196, ISSN: 0022-3263, DOI: 10.1021/JO800251G [retrieved on 2008-04-10]

## Beschreibung

Die vorliegende Anmeldung betrifft neue Halogen-substituierte Verbindungen, Verfahren zu ihrer Herstellung und ihre Verwendung zur Bekämpfung von tierischen Schädlingen, vor allem von Arthropoden und insbesondere von Insekten, Spinnentieren und Nematoden.

Es ist bekannt, dass bestimmte Halogen-substituierte Verbindungen herbizid wirksam sind (vgl. J. Org. Chem. 1997, 62(17), 5908-5919, J. Heterocycl. Chem. 1998, 35(6), 1493-1499, WO 2004/035545, WO 2004/106324, US 2006/069132, WO 2008/029084).

Ferner ist bekannt, dass bestimmte Halogen-substituierte Verbindungen Cytokin-inhibitorische Aktivitäten aufweisen (WO 00/07980).

Über die Verwendung solcher Halogen-substituierten Verbindungen zur Bekämpfung von tierischen Schädlingen, insbesondere als Pflanzenschutzmittel, ist jedoch nichts bekannt.

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Es wurde nun überraschenderweise gefunden, dass bestimmte Halogen-substituierte Verbindungen, sowie deren N-Oxide und Salze biologische Eigenschaften aufweisen und sich insbesondere zur Bekämpfung von tierischen Schädlingen eignen, und deshalb besonders gut im agrochemischen Bereich und im Bereich der Tiergesundheit einsetzbar sind.

Gegenstand dieser Erfindung ist die Verwendung der beschriebenen Verbindungen zur Bekämpfung tierischer Schädlinge in der Landwirtschaft und in der Tiergesundheit. Ferner sind Gegenstand dieser Erfindung auch neue Verbindungen, die die oben beschriebenen Verwendungen haben sowie Verfahren zu deren Herstellung.

Die erfindungsgemäßen Halogen-substituierten Verbindungen sind durch die allgemeine Formel (Ik), 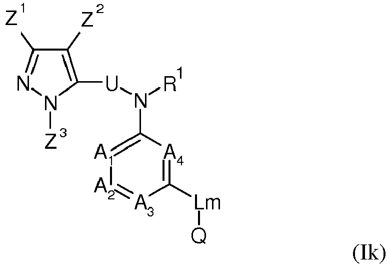 beschrieben worin
- Z¹: für 2,4-Dichlorphenyl, 2-Chlorphenyl, 3,5-Bis(trifluormethyl)phenyl, 4-(Trifluormethoxy)phenyl, 4-(Trifluormethyl)phenyl, 4-Fluorphenyl, C₂F₅, C₃F₇, C₄F₉, CF(CH₃)₂, CF₂CF₃, CF₃, CF₃CClF, CF₃CH₂O, CF₃OC₂F₄OCF₂, CHF₂, CHFCF₃, OCHF₂ steht,
- Z²: für H, 4-Fluor-phenyl, Cl, F, Br, I, CF₃, CH₃, Ethyl, Prop-2-enyl, Vinyl steht, Prop-2-enyl
- Z³: für H, Methyl, Ethyl, 1-Methylethyl, C(CH₃)₃, CH₂OCH₃, CF(CH₃)₂ steht,
- R¹: für H, Methyl, Ethyl, 1-Methylethyl, CH₂OCH₃ steht,
- A¹: für C-H, CCH₃, C-Cl, C-F, N steht,
- A²: für C-H, C-F, C-Cl, C-Br, C-OCH₃, CCONHcPr, C-Cyan, N steht,
- A³: für Bindung zu Q, C-H, C-F, C-Cl, C-Br, C-I, C-NO₂, C-CH₃, C-OCH3, COCHF₂, C-SCH₃, C-SO₂CH₃, N steht,
- A⁴: für C-H, C-F, C-Cl, N steht,
- Lm: für C(O)O, C(O)OCH₂C(O), C(O)OCH₂C(O)NH, C(O)OCH₂C(O)NHC(O), C(O)OCH₂C(O)NMe, CO, CON(CH₂CH₃), CON(CH₃), CON(Cyclopropyl), CONCH₃, CONH, steht und
- Q: für einen der Reste H, Methyl, Ethyl, NH₂, 3-Chlor-prop-2-enyl, (1R,2R)-2-Methylcyclopropyl, (1R,2S)-2-Methylcyclopropyl, (1S,2R)-2-Fluorcyclopropyl, (2R)-1,1,1-Trifluorpropan-2-yl, (2S)-1,1,1-Trifluor-3-methylbutan-2-yl, (2S)-1,1,1-Trifluorpropan-2-yl, (2Z)-3-Chlorbut-2-en-1-yl, [4-(Trifluormethyl)-1,3-thiazol-2-yl]methyl, 1-(1-Chlorcyclopropyl)ethyl, 1-(2-Fluorphenyl)ethyl, 1-(3-Fluorphenyl)ethyl, 1-(Pyridin-3-yl)ethyl, 1-(Trifluormethyl)cyclopropyl, 1,1,1-Trifluorbutan-2-yl, 1,1,1-Trifluorpentan-2-yl, 1,1-Dimethylbut-2-inyl, 1,1-Dimethylethyl, 1,1-Dioxido-2,3-dihydrothiophen-3-yl, 1,2,4-Triazol-3-ylmethyl, 1-CH₃-2-(ethylsulfanyl)ethyl, 1-Cyanethyl, 1-Cyclopropylethyl, 1-Fluorpropan-2-yl, 1-Methylethyl, 1-Methylpropyl, 1-Phenylethyl, 1-Pyridin-2-ylethyl, 1-Trifluormethylethyl, 2-(2,2,2-Trifluorethoxy)ethyl, 2-(Methylsulfanyl)ethyl, 2,2,2-Trifluor-1-(4-methyl-1,3thiazol-2-yl)ethyl, 2-Methyl-1-(methylsulfanyl)propan-2-yl 2,2,2-Trifluor-1-[1-methyl-4-(trifluormethyl)-1H-imidazol-2-yl]ethyl, 2,2,2-Trilfuorethyl, 2,2,3,3,3-Pentafluorpropyl, 2,2-Difluorcyclopropyl, 2,2-Difluorethyl, 2,2-Difluorpropyl, 2,2-Dimethyl-3-fluorpropyl, 2,2-Dimethylcyclopropyl-methyl, 2,4-Difluorbenzyl, 2,5-Difluorphenylmethyl, 2,6-Difluorphenylmethyl, 2-Chlorbenzyl, 2-Cyan-1-methoxypropan-2-yl, 2-Cyanoethyl, 2-Cyanpropan-2-yl, 2-Ethoxyethyl, 2-Ethylcyclopropyl, 2-Fluorcyclopropyl, 2-Fluorphenylmethyl, 2-Hydroxypropyl, 2-Methyl-1-(methylsulfanyl)propan-2-yl, 2-Methyl-1-(methylsulfonyl)propan-2-yl, 2-Methylbenzyl, 2-Methylbutyl, 2-Methylcyclopropyl, 2-Methylprop-2-enyl, 2-Methylpropyl, 2-Phenylcyclopropyl, 2-Trifluormethylphenylethyl, 3,3,3-Trifluorpropyl, 3,3-Dichlor-1,1-dimethylprop-2-enyl, 3,3-Dichlorprop-2-en-1-yl, 3,4-Difluorbenzyl, 3-Chlor-prop-2-enyl, 3-Fluorphenylmethyl, 3-Methylbenzyl, 3-Methyloxetan-3-ylmethyl, 4-(Trifluormethyl)piperidin-1-yl, 4,4,4-Trifluor-2-methylbutan-2-yl, 4,4,4-Trifluorbutan-2-yl, 4-Chlorbenzyl, 4-Chlorphenylethyl, 4-Fluorbenzyl, 4-Methylbenzyl, 4-Trifluormethylcyclohexyl, 5-Fluorpyridin-2-ylmethyl, Benzyl, But-3-in-2-yl, CH(CH₃)CF₃, -CH₂-[Bindung zu A³], cis-2-Fluorcyclopropyl, Cyanomethyl, Cyclobutyl, Cyclopent-3-en-1-yl, Cyclopentyl, Cyclopropyl, Cyclopropyl(tetrahydro-2H-pyran-4-yl)amino, Dicyclopropylmethyl, Hydroxymethyl, Isoxazol-3-ylmethyl, Methoxy, Methoxycarbonyl, Methoxycarbonylmethyl, Methylsulfonyl, N,N-Diethylamino, N,N-Dimethylamino, N-Allylamino, N-Ethylamino, Oxetan-3-yl, Prop-2-enyl, Prop-2-inyl, Propyl, Pyridin-2-ylmethyl, Pyridin-4-ylmethyl, Pyrimidin-2-ylmethyl, Tetrahydro-2H-pyran-4-yl, trans-2-Fluorcyclopropyl, trans-4-Hydroxycyclohexyl steht, und
- U: für C(=O) steht.

Beschrieben sind hier weiterhin Halogen-substituierten Verbindungen der Formel (I) 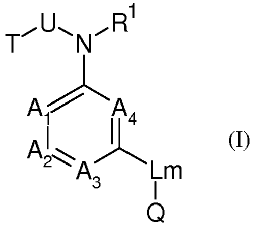 in denen
- R¹: für Wasserstoff, gegebenenfalls substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyan-C₁-C₂-alkyl, Aryl(C₁-C₃)-alkyl, Heteroaryl(C₁-C₃)-alkyl,

- die: chemische Gruppierung
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N*,*N*-Di-C₂-C₆alkylamino, *N*-C₂-C₇-Alkylaminocarbonyl, *N*-C₂-C₇-Cycloalkylaminocarbonyl oder C₂-C₄-Alkoxycarbonyl, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
- U: für eine Gruppierung C(=W), SO oder SO₂ steht,
wobei
- W: für Sauerstoff oder Schwefel steht;
- L: für eine bivalente chemische Gruppierung steht, die ausgewählt ist aus den Gruppierungen -NHC(=W)-, -NR⁶C(=W)-, -CH₂NHC(=W)-, -CH₂NR⁶C(=W)-, -C(=W)NH-, -C(=W)NR⁶, -C(=W)NHCH₂-, -C(=W)NR⁶CH₂-, -CH=N-OCH₂C(=W)NH-, -CH=N-OCH₂C(=W)NR⁶-, -CH₂NHC(=W)NH-, -CH₂NHC(=W)NR⁶-, -NH(C=W)NH-, -NH(=W)NR⁶-, -NR⁶(C=W)NH-,-NR⁶(=W)NR⁶-, -C(=W)-, -C(=W)O-, -C(=W)OCH₂C(=W)-, -C(=W)OCH₂C(=W)NR⁶-, -C(=W)OCH₂C(=W)NHC(=W)NH-, -C(=W)OCH2C(=W)NH-, -CH₂-, -(CH₂)₂-,-(CH₂)₃-, -Si-, -O-, -S(O)ₚ-, und -CH₂-S(O)ₚ-, -SO(=N-CN)- und -S(=N-CN)-,-C(=W)NHSO₂-, wobei
- p: die Werte 0, 1 oder 2 annehmen kann;
- R⁶: für Wasserstoff, gegebenenfalls substituiertes C₁-C₆-Alkyl, Aryl(C₁-C₃)-alkyl, Heteroaryl(C₁-C₃)-alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl, C₂-C₇-Alkylcarbonyl, C₂-C₇-Alkoxycarbonyl steht;
- m: die Werte 0 oder 1 annehmen kann;
- Q: für Wasserstoff oder eine der gegebenenfalls substituierten Gruppierungen C₁-C₆-Alkyl, C₂-C₆-Alkenyl, C₂-C₆-Alkinyl, C₃-C₆-Cycloalkyl, Cyano-C₁-C₂-alkyl, C₁-C₅-Heterocycloalkyl, C₁-C₄-Alkoxy, C₄-C₇-Alkylcycloalkyl, C₄-C₇-Cycloalkylalkyl, C₂-C₇-Alkylcarbonyl, C₁-C₆-Alhylaldehyd, C₁-C₆-Hydroxyalkyl, C₂-C₇-Alkoxycarbonyl, C₁-C₆-Halogenalkyl, für Formyl, Hydroxy, Halogen, Cyano, Aryl(C₁-C₃)-alkyl, Heteroaryl(C₁-C₃)-alkyl oder für eine Gruppierung OR⁷, NR⁶R⁸ steht;
- R⁷: ausgewählt ist aus den gegebenenfalls substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R⁸: ausgewählt ist aus Wasserstoff oder den gegebenenfalls mit R⁹ substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R⁹: ausgewählt ist aus Wasserstoff oder den gegebenenfalls mit R¹⁰ substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R¹⁰: ausgewählt ist aus Halogen, C₁-C₆-Alkyl, C₁-C₆-Alkoxy, C₁-C₆-Alkylthio, C₁-C₆-Alkylsulfinyl, C₁-C₆-Alkylsulfonyl, -CN, -NO₂;
- T: für einen gegebenenfalls mehrfach mit Z substituierten gesättigten oder ungesättigten 5-oder 6-gliedrigen Ring steht, oder für einen gegebenenfalls mehrfach mit Z substituierten 5- oder 6-gliedrigen heterozyklischen Ring steht;
- Z: für Wasserstoff, Halogen, Cyano, Nitro, gegebenenfalls substituiertes C₁-C₆-Alkyl, C₁-C₄-Alkenyl, C₁-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*,*N*-Di-(C₁-C₆)alkylamino, -CN, -NO₂, -C(=W)NR¹¹R⁵,-C(=W)OR¹², -S(O)₂NR¹³R¹⁴, -S(O)ₚR¹⁵, -S(O)(=NR¹⁶)R¹⁷ und gegebenenfalls mit R¹⁸ substituiertes Phenyl und Pyridinyl steht;
- R¹¹: ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, C₂-C₇-Alkylcarbonyl und C₂-C₇-Alkoxycarbonyl;
- R¹²: ausgewählt ist aus Wasserstoff oder den gegebenenfalls mit R⁶ substituierten Gruppierung C₁-C₆-Alkyl, C₁-C₆-Halogenlkyl, C₂-C₆-Alkenyl, C₂-C₆-Halogenalkenyl, C₂-C₆-Alkinyl, C₃-C₆-Cycloalkyl, C₃-C₆-Halogencycloalkyl, C₄-C₇-Alkylcycloalkyl und C₄-C₇-Cycloalkylalkyl;
- R¹³: ausgewählt ist aus Wasserstoff oder den gegebenenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl und C₂-C₇-Alkoxycarbonyl;
- R¹⁴: ausgewählt ist aus Wasserstoff oder den gegebenenfalls mit R¹⁹ substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R¹⁵: ausgewählt ist aus den gegebenenfalls mit R²⁰ substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl, C₁-C₄-Haloalkyl;
- R¹⁶: ausgewählt ist aus Wasserstoff, C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl und C₂-C₇-Alkoxycarbonyl;
- R¹⁷: ausgewählt ist aus Wasserstoff oder den gegebenenfalls mit R²⁰ substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R¹⁸: ausgewählt ist aus Halogen, -OH, -NH₂, -COOH, -CN, -NO₂, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N*,*N*-Di-(C₁-C₆)alkylamino, C₂-C₆-Alkylcarbonyl, C₂-C₆-Alkoxycarbonyl, C₂-C₇-Alkylaminocarbonyl und *N*,*N*-Di-(C₁-C₆)alkylaminocarbonyl;
- R¹⁹: ausgewählt ist aus Wasserstoff oder den gegebenenfalls mit R²¹ substituierten Gruppierungen C₁-C₆-Alkyl, C₁-C₆-Alkoxy, C₁-C₆-Alkylthio, C₁-C₆-Alkylsulfinyl, C₁-C₆-Alkylsulfonyl, -CN, -NO₂ sowie gegebenenfalls mit R²⁰ substituiertem Phenyl oder Pyridyl;
- R²⁰: ausgewählt ist aus Halogen, -CN, -NO₂, C₁-C₆-Alkyl, C₁-C₆-Alkoxy, C₁-C₆-Alkylthio, C₁-C₆-Alkylsulfinyl, C₁-C₆-Alkylsulfonyl, C₂-C₇-Alkylcarbonyl, C₂-C₇-Alkoxycarbonyl, C₂-C₇-Alkylaminocarbonyl, oder gegebenenfalls mit R²² substituiertem Phenyl oder Pyridyl;
- R²¹: ausgewählt ist aus Halogen, -OH, -NH₂, -COOH, -CN, -NO₂ oder den gegebenenfalls subsitutierten Gruppierungen C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N*,*N*-Di-(C₁-C₆)alkylamino, C₂-C₄-Alkylcarbonyl, C₂-C₄-Alkoxycarbonyl, C₂-C₇-Alkylaminocarbonyl, und *N*,*N*-Di-(C₁-C₆)alkylaminocarbonyl, wobei,

- R²²: ausgewählt ist unter Halogen, -OH, -NH₂, -COOH, -CN -NO₂, -CH=N-O-CH₃,-C(CH₃)=N-O-CH₃, C₁-C₆-Alkyl, C₂-C₆-Alkenyl, C₁-C₆-Halogenalkyl, C₂-C₆-Halogenalkenyl, C₂-C₃-Alkinyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N*,*N*-Di-(C₁-C₆)alkylamino, C₂-C₄-Alkylcarbonyl, C₂-C₄-Alkoxycarbonyl, C₂-C₇-Alkylaminocarbonyl, *N*,*N*-Di-(C₁-C₆)alkylaminocarbonyl; oder
- L, Q und R⁴: zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

Weiterhin beschrieben sind Verbindungen der Formel (I) 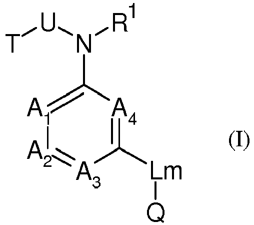 in denen
- R¹: für Wasserstoff oder den gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyan-C₁-C₂-alkyl, Aryl(C₁-C₃)-alkyl, Heteroaryl(C₁-C₃)-alkyl steht;
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber höchstens drei der chemischen Gruppierungen A₁ bis A₄ gleichzeitig für Stickstoff stehen, und wobei
- R², R³, R⁴ und R⁵: unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N*,*N*-Di-(C₂-C₆)alkylamino, *N*-C₂-C₇-Alkylaminocarbonyl, *N*-C₂-C₇-Cycloalkylaminocarbonyl oder C₂-C₄-Alkoxycarbonyl, stehen,
wenn keine der Gruppierungen A₂ und A₃ für Stickstoff steht, können R³ und R⁴ gemeinsam mit dem Kohlenstoff, an den sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoff, an den sie gebunden sind, einen 6-gliedrigen Ring bilden, der 0, 1 oder 2 Stickstoffatome enthält;
- U: für eine Gruppierung C(=W), SO oder SO₂, steht;
- W: für Sauerstoff oder Schwefel steht;,
- L: für eine bivalente chemische Gruppierung steht, die ausgewählt ist aus den Gruppierungen -NHC(=W)-, -NR⁶C(=W)-, -CH₂NHC(=W)-, -CH₂NR⁶C(=W)-, -C(=W)NH, -C(=W)NR⁶, -C(=W)NHCH₂-, -C(=W)NR⁶CH₂-, -CH₂NHC(=W)NH-, -CH₂NHC(=W)NR⁶-, -NH(C=W)NH-, -NH(=W)NR⁶-, -NR⁶(C=W)NH-,-NR⁶(=W)NR⁶-, -C(=W)-, -C(=W)O-, -C(=W)OCH₂C(=W)-, -C(=W)OCH₂C(=W)NR⁶-, -C(=W)OCH₂C(=W)NHC(=W)NH-, -C(=W)OCH₂C(=W)NH-, -CH₂-, -(CH₂)₂-,-(CH₂)₃-, -Si-, -O-, -S(O)ₚ-, und -CH₂-S(O)ₚ-, -SO(=N-CN)- und -S(=N-CN)-, -C(=W)NHSO₂-;
- p: die Werte 0, 1 oder 2 annehmen kann;
- R⁶: für Wasserstoff oder die gegebenenfalls substituierten Gruppierungen C₁-C₆-Alkyl, Aryl(C₁-C₃)-alkyl, Heteroaryl(C₁-C₃)-alkyl, C₂-C6-Alkenyl, C₂-C₆-Alkinyl, C₃-C₆-Cycloalkyl, C₄-C₇-Alkylcycloalkyl und C₄-C₇-Cycloalkylalkyl, C₂-C₇-Alkylcarbonyl, C₂-C₇-Alkoxycarbonyl steht;
- m: die Werte 0 oder 1 annehmen kann;
- Q: für Wasserstoff oder die gegebenenfalls substituierten Gruppierungen C₁-C₆-Alkyl, C₂-C₆-Alkenyl, C₂-C₆-Alkinyl, C₃-C₆-Cycloalkyl, Cyano-C₁-C₂-alkyl, C₁-C₅-Heterocycloalkyl, C₁-C₄-Alkoxy, C₄-C₇-Alkylcycloalkyl, C₄-C₇-Cycloalkylalkyl, C₂-C₇-Alkylcarbonyl, C₁-C₆-Alkylaldehyd, C₁-C₆-Hydroxyalkyl, C₂-C₇-Alkoxycarbonyl, C₁-C₆-Halogenalkyl, für Formyl, Hydroxy, Halogen, Cyano, Aryl(C₁-C₃)-alkyl, Heteroaryl(C₁-C₃)-alkyl oder für eine Gruppierung OR⁷, NR⁶R⁸ steht;
- R⁷: ausgewählt ist gegebenenfalls substituiertem C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R⁸: ausgewählt ist aus Wasserstoff oder gegebenenfalls mit R⁹ substituiertem C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R⁹: ausgewählt ist aus Wasserstoff oder gegebenenfalls mit R¹⁰ substituiertem C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R¹⁰: ausgewählt ist aus Halogen, C₁-C₆-Alkyl, C₁-C₆-Alkoxy, C₁-C₆-Alkylthio, C₁-C₆-Alkylsulfinyl, C₁-C₆-Alkylsulfonyl, -CN, -NO₂;
die chemische Gruppierung T für einen der unten dargestellten Reste (T-1) bis (T-90), die gegebenenfalls. mehrfach mit Z substituiert sein können: 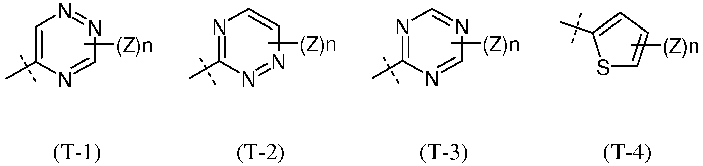 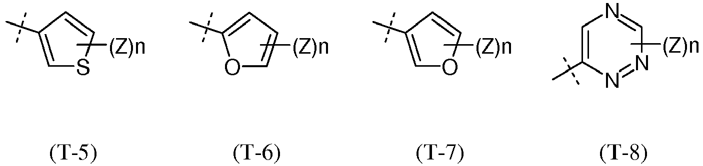 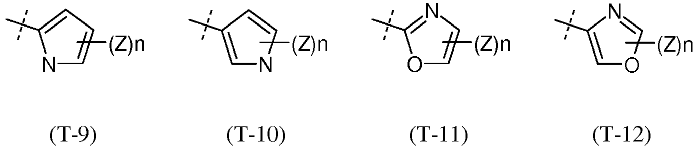 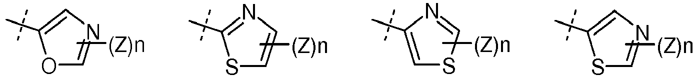 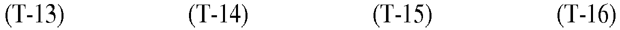 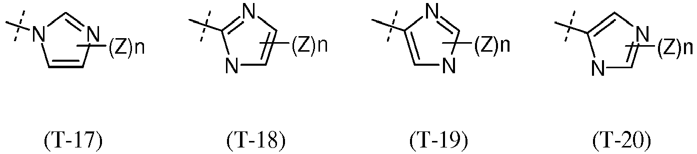 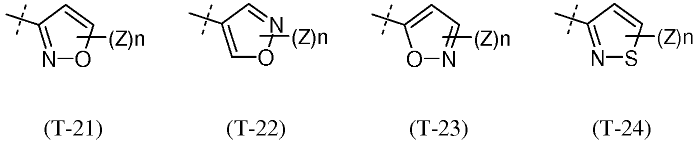 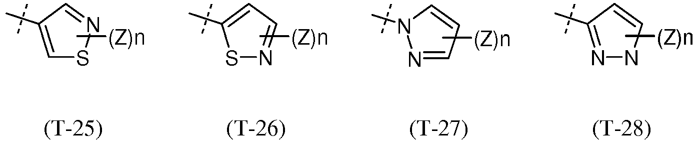 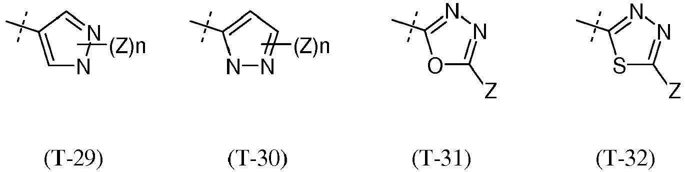 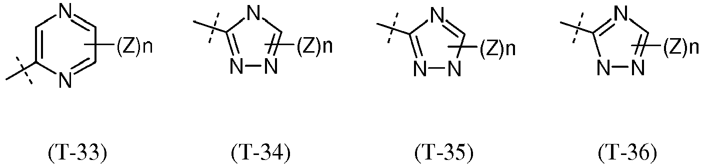 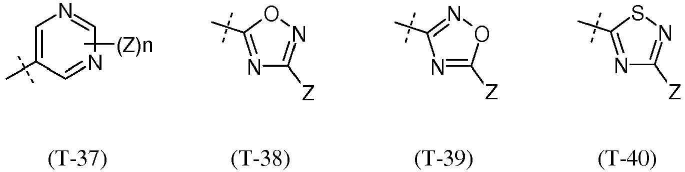 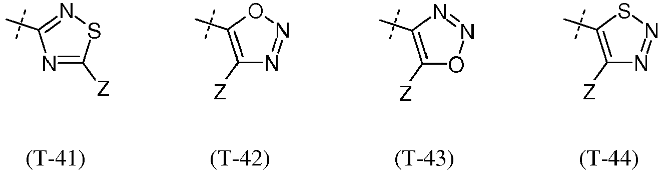 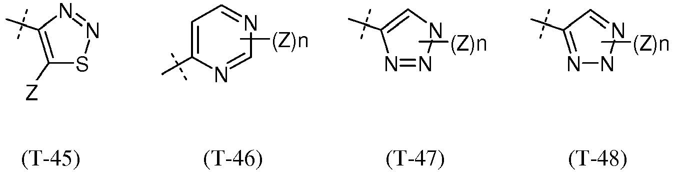 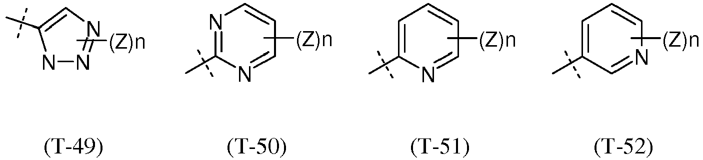 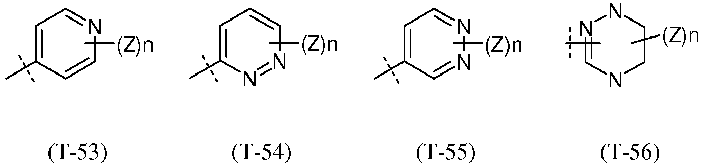 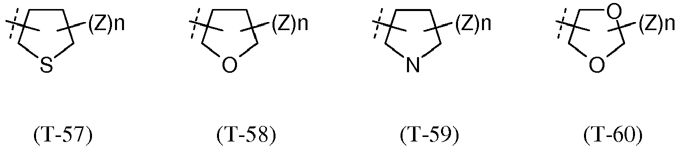 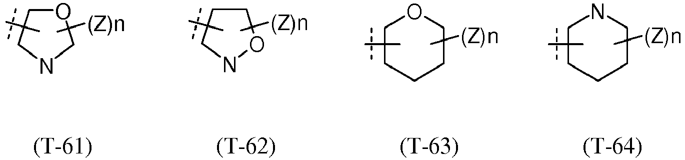 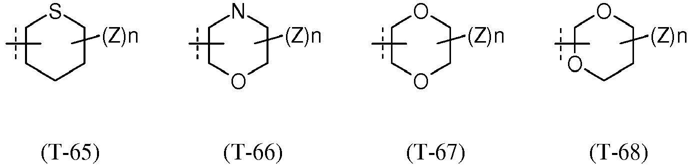 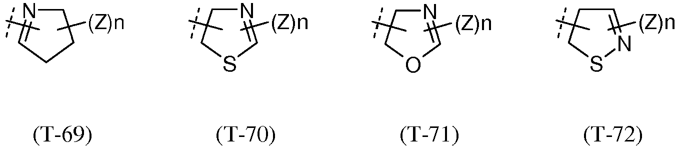 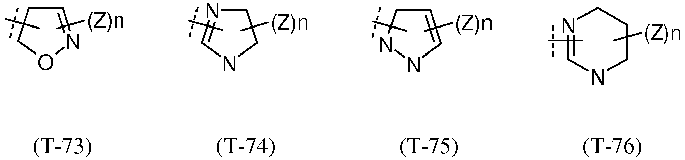 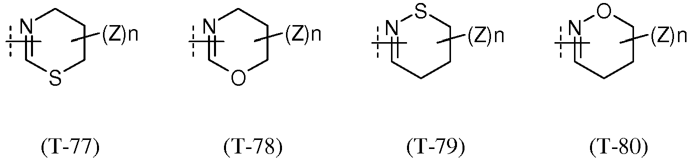 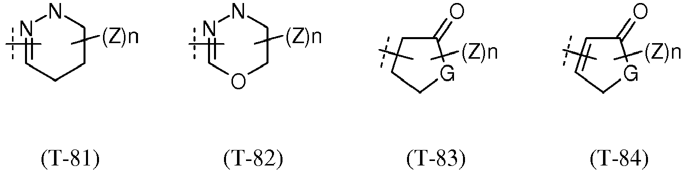 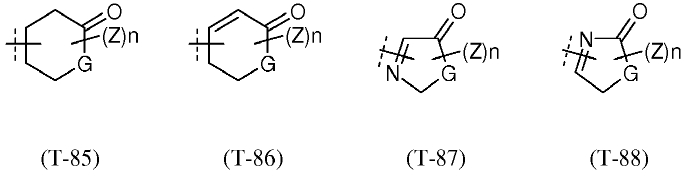 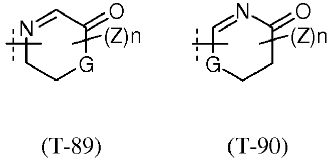 wobei
- G: für Sauerstoff, Schwefel oder mit Z substituierten Stickstoff steht,
- n: Werte von 0 bis 4 annehmen kann,
- Z: für Wasserstoff, Halogen, Cyano, Nitro oder die gegebenenfalls substituierten Gruppierungen C₁-C₆-Alkyl, C₁-C₄-Alkenyl, C₁-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆- Halogenalkylsulfinyl, C₁-C₆-Alkylsulfonyl, C₁-C₆-Halogenalkylsulfonyl, *N*,*N*-Di-(C₁-C₆)alkylamino, -CN, -NO₂,-S(O)₂NR¹³R¹⁴, -S(O)ₚR¹⁵, -S(O)(=NR¹⁶)R¹⁷ oder für gegebenenfalls mit R¹⁸ substituiertes Phenyl oder Pyridinyl steht;
- R¹³: ausgewählt ist aus Wasserstoff oder einer der gegebenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl und C₂-C₇-Alkoxycarbonyl;
- R¹⁴: ausgewählt ist aus Wasserstoff oder einer der gegebenenfalls mit R¹⁹ substituierten Gruppierungen C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- R¹⁵: ausgewählt ist aus gegebenenfalls mit R²⁰ substituiertem C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl, C₁-C₄-Haloalkyl;
- R¹⁶: ausgewählt ist aus Wasserstoff, C₁-C₆-Alkyl, C₁-C₆-Halogenalkyl, C₂-C₆-Alkenyl, C₂-C₆-Alkinyl, C₃-C₆-Cycloalkyl, C₄-C₇-Alkylcycloalkyl, C₄-C₇-Cycloalkylalkyl, C₂-C₇-Alkylcarbonyl und C₂-C₇-Alkoxycarbonyl;
- R¹⁷: ausgewählt ist aus Wasserstoff, gegebenenfalls mit R²⁰ substituiertem C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl,;
- R¹⁸: ausgewählt ist aus Halogen, -OH, -NH₂, -COOH, -CN, -NO₂, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N*,*N*-Di-(C₁-C₆)alkylamino, C₂-C₆-Alkylcarbonyl, C₂-C₆-Alkoxycarbonyl, C₂-C₇-Alkylaminocarbonyl und *N*,*N*-Di-(C₁-C₆)alkylaminocarbonyl;
- R¹⁹: ausgewählt ist aus Wasserstoff, gegebenenfalls mit R²¹ substituiertem C₁-C₆-Alkyl, C₁-C₆-Alkoxy, C₁-C₆-Alkylthio, C₁-C₆-Alkylsulfinyl, C₁-C₆-Alkylsulfonyl, -CN, -NO₂, gegebenenfalls mit R¹⁹ substituiertem Phenyl oder Pyridyl;
- R²⁰: ausgewählt ist aus Halogen, -CN, -NO₂, C₁-C₆-Alkyl, C₁-C₆-Alkoxy, C₁-C₆-Alkylthio, C₁-C₆-Alkylsulfinyl, C₁-C₆-Alkylsulfonyl, C₂-C₇-Alkylcarbonyl, C₂-C₇-Alkoxycarbonyl, C₂-C₇-Alkylaminocarbonyl oder gegebenenfalls mit R²² substituiertem Phenyl oder Pyridyl;
- R²¹: ausgewählt ist aus Halogen, -OH, -NH₂, -COOH, -CN, -NO₂, oder gegebenenfalls subsitutiertem C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆₋Alkylsulfonyl, C₁-C₆-Halogenalkylsulfonyl, C₁-C₆-Alkylamino, *N*,*N*-Di-(C₁-C₆)alkylamino, C₂-C₄-Alkylcarbonyl, C₂-C₄-Alkoxycarbonyl, C₂-C₇-Alkylaminocarbonyl, und *N*,*N*-Di-(C₁-C₆)alkylaminocarbonyl;
- R²²: ausgewählt ist aus Halogen, -OH, -NH₂, -COOH, -CN -NO₂, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₁-C₆-Alkylamino, *N*,*N*-Di-(C₁-C₆)alkylamino, C₂-C₄-Alkylcarbonyl, C₂-C₄-Alkoxycarbonyl, C₂-C₇-Alkylaminocarbonyl, *N*,*N*-Di-(C₁-C₆)alkylaminocarbonyl;

### Ferner können

L, Q und R⁴ zusammen mit den Kohlenstoffatomen, an die sie gebunden sind, einen gegenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

Weiterhin beschrieben sind Verbindungen der Formel (I) 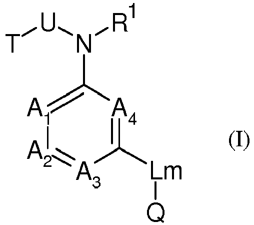 in denen
- R¹: für Wasserstoff oder die gegebenenfalls substituierten Gruppierungen C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Cyan-C₁-C₂-alkyl, Aryl(C₁-C₃)-alkyl, Heteroaryl(C₁-C₃)-alkyl steht;
- A₁: für CR² oder Stickstoff,
- A₂: für CR³ oder Stickstoff,
- A₃: für CR⁴ oder Stickstoff und
- A₄: für CR⁵ oder Stickstoff stehen, wobei aber höchstens drei der chemischen Gruppierungen A₁ bis A₄ gleichzeitig für Stickstoff stehen, und wobei
- R², R³, R⁴ und R⁵: unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-C₂-C₇-Alkylaminocarbonyl, *N*-C₂-C₇-Cycloalkylamino-carbonyl stehen,
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
- U: für C(=W), SO oder SO₂, steht;
- W: für Sauerstoff oder Schwefel steht;
- L: für eine bivalente chemische Gruppierung steht, die ausgewählt ist aus den Gruppierungen -CH₂NHC(=W)-, -CH₂NR⁶C(=W)-, -C(=W)NH, -C(=W)NR⁶,-NH(C=W)NH-, -NH(C=W)NR⁶-, -NR⁶(C=W)NH-, -NR⁶(=W)NR⁶-, -C(=W)-,-C(=W)O-, -C(=W)OCH₂C(=W)-, -C(=W)OCH₂C(=W)NR⁶-, -C(=W)OCH₂C(=W)NHC(=W)NH-, -C(=W)OCH₂C(=W)NH-, -O-, -S(O)ₚ-, und -CH₂-S(O)ₚ-, -SO(=N-CN)- und -S(=N-CN)-, -C(=W)NHSO₂-, wobei
- p: die Werte 0, 1 oder 2 annehmen kann und
- R⁶: für Wasserstoff, C₁-C₆-Alkyl, Aryl(C₁-C₃)-alkyl, Heteroaryl(C₁-C₃)-alkyl, C₂-C₇-Alkylcarbonyl, C₂-C₇-Alkoxycarbonyl steht;
- m: die Werte 0 oder 1 annehmen kann;
- Q: für Wasserstoff oder die gegebenenfalls substituierten Gruppierungen C₁-C₆-Alkyl, C₂-C₆-Alkenyl, C₂-C₆-Alkinyl, C₃-C₆-Cycloalkyl, Cyano-C₁-C₂-alkyl, C₁-C₅-Heterocycloalkyl, C₁-C₄-Alkoxy, C₄-C₇-Alkylcycloalkyl, C₄-C₇-Cycloalkylalkyl, C₂-C₇-Alkylcarbonyl, C₁-C₆-Alkylaldehyd, C₁-C₆-Hydroxyalkyl, C₂-C₇-Alkoxycarbonyl, C₁-C₆-Halogenalkyl, Cyano, Aryl-(C₁-C₃)-alkyl, Heteroaryl-(C₁-C₃)-alkyl, oder für eine Gruppierung NR⁶R⁸ steht, wobei
- R⁸: ausgewählt ist aus Wasserstoff, C₁-C₆-Alkyl, C₂-C₆-Alkenyl, C₂-C₆-Alkinyl, C₃-C₆-Cycloalkyl, C₄-C₇-Alkylcycloalkyl und C₄-C₇-Cycloalkylalkyl;
- T: für einen der gegebenenfalls ein- oder mehrfach mit Z substituierten Heterozyklen (T-5), (T-7), (T-9), (T-10), (T-12), (T-13), (T-15), (T-16), (T-19), (T-20), (T-23), (T-26), (T-28), (T-29), (T-34), (T-35), (T-36), (T-30), (T-33), (T-37), (T-46), (T-51), (T-52), (T-53) steht, wobei
- n: Werte von 0 bis 4 annehmen kann und
- Z: für Wasserstoff, Chlor, Brom, Iod, Cyano, Nitro oder die gegebenenfalls substituierten Gruppierungen C₁-C₄-Alkyl, C₁-C₄-Alkenyl, C₁-C₄-Alkinyl, C₁-C₄-Halogenalkyl, C₃-C₆-Cycloalkyl, C₃-C₆-Halogencycloalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, *N*,*N*-Di-(C₁-C₄)alkylamino, und gegebenenfalls mit R¹⁸ substituiertes Phenyl und Pyridinyl steht;
- R¹⁸: ausgewählt ist aus Halogen, -OH, -NH₂, -COOH, -CN, -NO₂, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, C₁-C₄-Alkylamino, *N*,*N*-Di-(C₁-C₄)alkylamino, C₁-C₄-Alkylcarbonyl, C₁-C₄-Alkoxycarbonyl, C₁-C₄-Alkylaminocarbonyl und *N*,*N*-Di-(C₁-C₄)alkylaminocarbonyl.

### Ferner können

L, Q und R⁴ zusammen mit den Kohlenstoffatomen, an die sie gebunden sind, einen gegenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

Weiterhin beschrieben sind Verbindungen der Formel (I) 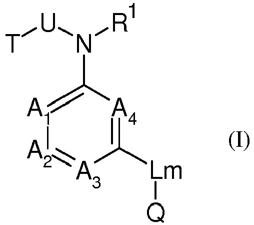 in denen
- R¹: für Wasserstoff, Methyl, Ethyl, n-Propyl, Isopropyl, n-Butyl, 2-Butinyl, Isobutyl, sec-Butyl, tert-Butyl, Methoxymethyl, Ethoxymethyl, Propoxymethyl, Methylcarbonyl, Ethylcarbonyl, n-Propylcarbonyl, Allyl, Propargyl, Isopropylcarbonyl, sec-Butylcarbonyl, tert-Butylcarbonyl, Methoxycarbonyl, Ethoxycarbonyl, n-Propoxycarbonyl, Isopropoxycarbonyl, sec-Butoxycarbonyl, tert-Butoxycarbonyl, Cyanomethyl, 2-Cyanoethyl steht;
- A₁: für CR² oder Stickstoff,
- A₂: für CR³ oder Stickstoff,
- A₃: für CR⁴ oder Stickstoff und
- A₄: für CR⁵ oder Stickstoff stehen, wobei aber höchstens drei der chemischen Gruppierungen A₁ bis A₄ gleichzeitig für Stickstoff stehen, und wobei
- R² und R⁵: unabhängig voneinander für Wasserstoff, Methyl, Fluor und Chlor stehen und
- R³ und R⁴: unabhängig voneinander für Wasserstoff, Fluor, Chlor, Brom, CN, NO₂, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl und *N*-Cyclopropylaminocarbonyl stehen; wobei
- U: für C(=W), SO₂ steht,
- W: für Sauerstoff steht,
- L: für eine bivalente chemische Gruppierung steht, die ausgewählt ist aus den Gruppierungen -C(=O)NH, -C(=O)NR⁶, -C(=O)O-, -C(=O)OCH₂C(=O)-, -C(=O)OCH₂C(=O)NR⁶-, -C(=O)OCH₂C(=O)NHC(=O)NH-, -C(=O)OCH₂C(=O)NH-, -C(=W)NHSO₂-, wobei
- R⁶: für Wasserstoff, Methyl, Ethyl, n-Propyl, Isopropyl, n-Butyl, Isobutyl, sec-Butyl, tert-Butyl, Methoxymethyl, Ethoxymethyl, Propoxymethyl, Methylcarbonyl, Ethylcarbonyl, n-Propylcarbonyl, Isopropylcarbonyl, sec-Butylcarbonyl, tert-Butylcarbonyl, Methoxycarbonyl, Ethoxycarbonyl, n-Propoxycarbonyl, Isopropoxycarbonyl, sec-Butoxycarbonyl, tert-Butoxycarbonyl, Cyanomethyl, 2-Cyanoethyl steht;
- m: den Wert 1 annimmt;
- Q: für Wasserstoff, Methyl, Ethyl, n-Propyl, 1-Methylethyl, 1,1-Dimethylethyl, 1-Methylpropyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 2,2-Dimethyl-3-fluorpropyl, Cyclopropyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl" 3,3-Dichlor-1,1-dimethylprop-2-enyl, Oxetan-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, Pyrimidin-2-ylmethyl, Methoxy, 2-Ethoxyethyl, 2-(Methylsulfanyl)ethyl, 1-Methyl-2-(ethylsulfanyl)ethyl, Methoxycarbonyl, Methoxycarbonylmethyl, NH₂, *N*-Ethylamino, *N*-Allylamino, *N,N-*Dimethylamino, *N*,*N*-Diethylamino steht; oder
- Q: für Wasserstoff, Methyl, Ethyl, n-Propyl, 1-Methylethyl, 1,1-Dimethylethyl, 1-Methylpropyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 2,2-Dimethyl-3-fluorpropyl, Cyclopropyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl" 3,3-Dichlor-prop-2-enyl, 3,3-Dichlor-1,1-dimethylprop-2-enyl, Oxetan-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N*,*N*-Dimethylamino, *N,N-*Diethylamino steht;
- T: für einen der gegebenenfalls mehrfach mit Z substituierten Heterozyklen (T-12), (T-13), (T-15), (T-16), (T-19), (T-20), (T-23), (T-26), (T-30), (T-33), (T-37), (T-46), (T-51), (T-52), (T-53) steht, wobei
- n: Werte von 0 bis 3 annehmen kann und
- Z: für Wasserstoff, Chlor, Brom, Iod, Cyano, Nitro, Methyl, Ethyl, n-Propyl, Isopropyl, n-Butyl, Isobutyl, sec-Butyl, tert-Butyl, Ethenyl, 1-Propenyl, 2-Propenyl, Ethinyl, 1-Propinyl, 1-Butinyl, Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n-butyl, Trifluormethoxy-1,1,2,2-tetrafluorethoxy-difluormethyl, Trifluormethylthio, Trifluormethylsulfinyl, Trifluormethylsulfonyl, Methoxy, Ethoxy, n-Propoxy, Trifluormethoxy, Difluormethoxy, Cyclopropyl, Cyclobutyl, 2,2,2-Trifluorethoxy, 1-Trifluormethylethoxy, 3,3,3,2,2-Pentafluorpropoxy, 4-Fluorphenyl, 4-Chlorphenyl, 4-Trifluormethylphenyl, 2,2,2-Trifluorethyl, 2,2-Difluor-1-methyl-cyclopropyl, steht; oder
- Z: für Wasserstoff, Chlor, Brom, Iod, Cyano, Nitro, Methyl, Ethyl, n-Propyl, Isopropyl, n-Butyl, Isobutyl, sec-Butyl, tert-Butyl, Ethenyl, 1-Propenyl, 2-Propenyl, Ethinyl, 1-Propinyl, 1-Butinyl, Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n-butyl, Trifluormethoxy-1,1,2,2-tetrafluorethoxy-difluormethyl, Trifluormethylthio, Trifluormethylsulfinyl, Trifluormethylsulfonyl, Methoxy, Ethoxy, n-Propoxy, Trifluormethoxy, Difluormethoxy, Cyclopropyl, Cyclobutyl, 2,2,2-Trifluorethoxy, 1-Trifluormethylethoxy, 3,3,3,2,2-Pentafluorpropoxy, 4-Fluorphenyl, 4-Chlorphenyl, 4-Trifluormethylphenyl, 2,2,2-Trifluorethyl, 2,2-Difluor-1-methyl-cyclopropyl, Phenyl, Methoxymethyl, Cyclopropyl(fluor)methyl, 2,4-Dichlorphenyl, 4-Trifluormethoxylphenyl, 2-Chlorphenyl, 3,5-Bis(trifluormethyl)phenyl, (1,1,1,3,3,3-Hexafluorpropan-2-yl)oxy steht

### Ferner können

L, Q und R⁴ zusammen mit den Kohlenstoffatomen, an die sie gebunden sind, einen gegenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

"Alkyl" steht - in Alleinstellung oder als Bestandteil einer chemischen Gruppe - für geradkettige oder verzweigte Kohlenstoffwasserstoffe, vorzugsweise mit 1 bis 6 Kohlenstoffatomen, wie beispielsweise Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl. Ferner bevorzugt für Alkyle mit 1 bis 4 Kohlenstoffatomen, wie unter anderem Methyl, Ethyl, Ethyl, n-Propyl, Isopropyl, n-Butyl, Isobutyl, sec-Butyl oder tert-Butyl. Die erfindungsgemäßen Alkyle können mit einem oder mehreren, gleichen oder verschiedenen Resten substituiert sein.

"Alkenyl" steht - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl. Ferner bevorzugt für Alkenyle mit 2 bis 4 Kohlenstoffatomen, wie unter anderem 2-Propenyl, 2-Butenyl oder 1-Methyl-2-propenyl. Die erfindungsgemäßen Alkenyle können mit einem oder mehreren, gleichen oder verschiedenen Resten substituiert sein.

"Alkinyl" steht - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Dreifachbindung wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl, 1-Ethyl-1-methyl-2-propinyl und 2,5-Hexadiynyl. Ferner bevorzugt für Alkinyle mit 2 bis 4 Kohlenstoffatomen wie unter anderem Ethinyl, 2-Propinyl oder 2-Butinyl-2-propenyl. Die erfindungsgemäßen Alkinyle können mit einem oder mehreren, gleichen oder verschiedenen Resten substituiert sein.

"Cycloalkyl" steht - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Cycloheptyl, Cyclooctyl, Bicyclo[2.2.1]heptyl, Bicyclo[2.2.2]octyl oder Adamantyl. Ferner bevorzugt für Cycloalkyle mit 3, 4, 5, 6 oder 7 Kohlenstoffatomen, wie unter anderem Cyclopropyl oder Cyclobutyl. Die erfindungsgemäßen Cycloalkyle können mit einem oder mehreren, gleichen oder verschiedenen Resten substituiert sein.

"Alkylcycloalkyl" steht für mono-, bi- oder tricyclisches Alkylcycloalkyl, vorzugsweise mit 4 bis 10 oder 4 bis 7 Kohlenstoffatomen, wie beispielsweise Ethylcyclopropyl, Isopropylcyclobutyl, 3-Methylcyclopentyl und 4-Methyl-cyclohexyl. Ferner bevorzugt für Alkylcycloalkyle mit 4, 5 oder 7 Kohlenstoffatomen wie unter anderen Ethylcyclopropyl oder 4-Methyl-cyclohexyl. Die erfindungsgemäßen Alkylcycloalkyle können mit einem oder mehreren, gleichen oder verschiedenen Resten substituiert sein.

"Cycloalkylalkyl" steht für mono, bi- oder tricyclisches Cycloalkylalkyl, vorzugsweise mit 4 bis 10 oder 4 bis 7 Kohlenstoffatomen, wie beispielsweise Cyclopropylmethyl, Cyclobutylmethyl, Cyclopentylmethyl, Cyclohexylmethyl und Cyclopentylethyl. Ferner bevorzugt für Cycloalkylalkyle mit 4, 5 oder 7 Kohlenstoffatomen wie unter anderen Cyclopropylmethyl oder Cyclobutylmethyl. Die erfindungsgemäßen Cycloalkylalkyle können mit einem oder mehreren, gleichen oder verschiedenen Resten substituiert sein.

"Halogen" steht für Fluor, Chlor, Brom oder Iod, insbesondere für Fluor, Chlor oder Brom.

Mit Halogen substituierten chemischen Gruppen, wie beispielsweise Halogenalkyl, Halogencycloalkyl, Halogenalkyloxy, Halogenalkylthio, Halogenalkylsunfinyl oder Halogenalkylsulfonyl sind einfach oder mehrfach bis zur maximal möglichen Substituentenzahl mit Halogen substituiert. Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor, Chlor oder Brom und besonders bevorzugt für Fluor.

"Halogencycloalkyl" steht für mono-, bi- oder tricyclisches Halogencycloalkyl, vorzugsweise mit 3 bis 10 Kohlenstoffatomen, wie unter anderen 1-Fluor-cyclopropyl, 2-Fluor-cyclopropyl oder 1-Fluor-cyclobutyl. Ferner bevorzugt für Halogencycloalkyl mit 3, 5 oder 7 Kohlenstoffatomen. Die erfindungsgemäßen Halogencycloalkyle können mit einem oder mehreren, gleichen oder verschiedenen Resten substituiert sein.

"Halogenalkyl" "Halogenalkenyl" oder "Halogenalkinyl" steht für mit Halogen substituierte Alkyle, Alkenyle oder Alkinyle mit vorzugsweise 1 bis 9 gleichen oder verschiedenen Halogenatomen, wie beispielsweise Monohaloalkyl (= Monohalogenalkyl) wie CH₂CH₂Cl, CH₂CH₂F, CHClCH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃. Entsprechendes gilt für Haloalkenyl und andere durch Halogen substituierten Reste. Haloalkoxy ist z.B. OCF₃, OCHF₂, OCH₂F, OCF₂CF₃, OCH₂CF₃ und OCH₂CH₂Cl;

Weitere Beispiele für Halogenalkyle sind Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl und Pentafluor-tert-butyl. Bevorzugt sind Halogenalkyle mit 1 bis 4 Kohlenstoffatomen und 1 bis 9, vorzugsweise 1 bis 5 gleichen oder verschiedenen Halogenatomen, die ausgewählt sind unter Fluor, Chlor oder Brom. Besonders bevorzugt sind Halogenalkyle mit 1 oder 2 Kohlenstoffatomen und mit 1 bis 5 gleichen oder verschiedenen Halogenatomen, die ausgewählt sind unter Fluor oder Chlor, wie unter anderen Difluormethyl, Trifluormethyl oder 2,2-Difluorethyl.

"Hydroxyalkyl" steht für geradkettigen oder verzweigten Alkohol, vorzugsweise mit 1 bis 6 Kohlenstoffatomen, wie beispielsweise Methanol, Ethanol, n-Propanol, Isopropanol, n-Butanol, Isobutanol, sec-Butanol und tert-Butanol. Ferner bevorzugt für Hydroxyalkylgruppen mit 1 bis 4 Kohlenstoffatomen. Die erfindungsgemäßen Hydroxyalkylgruppen können mit einem oder mehreren, gleichen oder verschiedenen Resten substituiert sein

"Alkoxy" steht für geradkettiges oder verzweigtes O-Alkyl, vorzugsweise mit 1 bis 6 Kohlenstoffatomen, wie beispielsweise Methoxy, Ethoxy, n-Propoxy, Isopropoxy, n-Butoxy, Isobutoxy, sec-Butoxy und tert-Butoxy. Ferner bevorzugt für Alkoxygruppen mit 1 bis 4 Kohlenstoffatomen. Die erfindungsgemäßen Alkoxygruppen können mit einem oder mehreren, gleichen oder verschiedenen Resten substituiert sein.

"Halogenalkoxy" steht für mit Halogen substituiertes geradkettiges oder verzweigtes O-Alkyl, vorzugsweise mit 1 bis 6 Kohlenstoffatomen , wie unter anderem Difluormethoxy, Trifluormethoxy, 2,2-Difluorethoxy, 1,1,2,2-Tetrafluorethoxy, 2,2,2-Trifluorethoxy und 2-Chlor-1,1,2-trifluorethoxy. Ferner bevorzugt für Halogenalkoxygruppen mit 1 bis 4 Kohlenstoffatomen.

Die erfindungsgemäßen Halogenalkoxygruppen können mit einem oder mehreren, gleichen oder verschiedenen Resten substituiert sein.

"Alkylthio" steht für geradkettiges oder verzweigtes S-Alkyl, vorzugsweise mit 1 bis 6 Kohlenstoffatomen, wie beispielsweise Methylthio, Ethylthio, n-Propylthio, Isopropylthio, n-Butylthio, Isobutylthio, sec-Butylthio und tert-Butylthio. Ferner bevorzugt für Alkylthiogruppen mit 1 bis 4 Kohlenstoffatomen. Die erfindungsgemäßen Alkylthiogruppen können mit einem oder mehreren, gleichen oder verschiedenen Resten substituiert sein.

Beispiele für Halogenalkylthioalkyle, d.h. mit Halogen substituierte Alkylthiogruppen, sind unter anderem Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio oder 2-Chlor-1,1,2-trifluorethylthio.

"Alkylsulfinyl" steht für geradkettiges oder verzweigtes Alkylsulfinyl, vorzugsweise mit 1 bis 6 Kohlenstoffatomen wie beispielsweise Methylsulfinyl, Ethylsulfinyl, n-Propylsulfinyl, Isopropylsulfinyl, n-Butylsulfinyl, Isobutylsulfinyl, sec-Butylsulfinyl und tert-Butylsulfinyl. Ferner bevorzugt für Alkylsulfinylgruppen mit 1 bis 4 Kohlenstoffatomen. Die erfindungsgemäßen Alkylsulfinylgruppen können mit einem oder mehreren, gleichen oder verschiedenen Resten substituiert sein.

Beispiele für Halogenalkylsulfinylgrupen, d.h. mit Halogen substituierte Alkylsulfinylgruppen, sind unter anderem Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl.

"Alkylsulfonyl" steht für geradkettiges oder verzweigtes Alkylsulfonyl, vorzugsweise mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, sec-Butylsulfonyl und tert-Butylsulfonyl. Ferner bevorzugt für Alkylsulfonylgruppen mit 1 bis 4 Kohlenstoffatomen. Die erfindungsgemäßen Alkylsulfonylgruppen können mit einem oder mehreren, gleichen oder verschiedenen Resten substituiert sein.

Beispiele für Halogenalkylsulfonylgrupen, d.h. mit Halogen substituierte Alkylsulfonylgruppen sind unter anderem Difluormethylsulfonyl, Trifluormethylsulfonyl, Trichlormethylsulfonyl, Chlordifluormethylsulfonyl, 1-Fluorethylsulfonyl, 2-Fluorethylsulfonyl, 2,2-Difluorethylsulfonyl, 1,1,2,2-Tetrafluorethylsulfonyl, 2,2,2-Trifluorethylsulfonyl und 2-Chlor-1,1,2-trifluorethylsulfonyl.

"Alkylcarbonyl" steht für geradkettiges oder verzweigtes Alkyl-C(=O), vorzugsweise mit 2 bis 7 Kohlenstoffatomen, wie Methylcarbonyl, Ethylcarbonyl, n-Propylcarbonyl, Isopropylcarbonyl, sec-Butylcarbonyl und tert-Butylcarbonyl. Ferner bevorzugt für Alkylcarbonyle mit 1 bis 4 Kohlenstoffatomen. Die erfindungsgemäßen Alkylcarbonyle können mit einem oder mehreren, gleichen oder verschiedenen Resten substituiert sein.

"Cycloalkylcarbonyl" steht für geradkettiges oder verzweigtes Cycloalkylcarbonyl, vorzugsweise mit 3 bis 10 Kohlenstoffatomen im Cycloalkylteil, wie beispielsweise Cyclopropylcarbonyl, Cyclobutylcarbonyl, Cyclopentylcarbonyl, Cyclohexyl-carbonyl, Cycloheptylcarbonyl, Cyclooctylcarbonyl, Bicyclo[2.2.1]heptyl, Bycyclo[2.2.2]octylcarbonyl und Adamantylcarbonyl. Ferner bevorzugt für Cycloalkylcarbonyl mit 3, 5 oder 7 Kohlenstoffatomen im Cycloalkylteil. Die erfindungsgemäßen Cycloalkylcarbonylgruppen können mit einem oder mehreren, gleichen oder verschiedenen Resten substituiert sein.

"Alkoxycarbonyl" steht - in Alleinstellung oder als Bestandteil einer chemischen Gruppe - für geradkettiges oder verzweigtes Alkoxycarbonyl, vorzugsweise mit 1 bis 6 Kohlenstoffatomen oder 1 bis 4 Kohlenstoffatomen im Alkoxyteil, wie beispielsweise Methoxycarbonyl, Ethoxycarbonyl, n-Propoxycarbonyl, Isopropoxycarbonyl, sec-Butoxycarbonyl und tert-Butoxycarbonyl. Die erfindungsgemäßen Alkoxycarbonylgruppen können mit einem oder mehreren, gleichen oder verschiedenen Resten substituiert sein.

"Alkylaminocarbonyl" steht für geradkettiges oder verzweigtes Alkylaminocarbonyl mit vorzugsweise 1 bis 6 Kohlenstoffatomen oder 1 bis 4 Kohlenstoffatomen im Alkylteil, wie beispielsweise Methylaminocarbonyl, Ethylaminocarbonyl, n-Proylaminocarbonyl, Isopropylaminocarbonyl, sec-Butylaminocarbonyl und tert-Butylaminocarbonyl. Die erfindungsgemäßen Alkylaminocarbonylgruppen können mit einem oder mehreren, gleichen oder verschiedenen Resten substituiert sein.

"*N*,*N*-Dialkylamino-carbonyl" steht für geradkettiges oder verzweigtes *N*,*N*-Dialkylaminocarbonyl mit vorzugsweise 1 bis 6 Kohlenstoffatomen oder 1 bis 4 Kohlenstoffatomen im Alkylteil, wie beispielsweise *N*,*N*-Dimethylamino-carbonyl, N.N-Diethylamino-carbonyl, N,N-Di(n-propylamino)-carbonyl, *N*,*N*-Di-(isopropylamino)-carbonyl und *N*,*N*-Di-(sec-butylamino)-carbonyl. Die erfindungsgemäßen *N*,*N*-Dialkylamino-carbonylgruppen können mit einem oder mehreren, gleichen oder verschiedenen Resten substituiert sein.

"Aryl" steht für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Die erfindungsgemäßen Arylgruppen können mit einem oder mehreren, gleichen oder verschiedenen Resten substituiert sein.

Beispiele substitutierter Aryle stellen die Arylalkyle dar, die gleichfalls mit einem oder mehreren, gleichen oder verschiedenen Resten im Alkyl- und/oder Arylteil substituiert sein können. Beispiele solcher Arylalkyle sind unter anderem Benzyl und 1-Phenylethyl.

"Heterocyclus", "heterocyclischer Ring" oder "heterocyclisches Ringsystem" steht für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder mit einem Substituenten Z substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl.

Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen. Vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furyl, Thienyl, Pyrazolyl, Imidazolyl, 1,2,3-und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten substituiert sein.

Substituierte Gruppen, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und *N*,*N*-Dialkylamino-carbonyl, substituiertes Amino, wie Acylamino, Mono- und *N*,*N*-Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfonylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylthio-alkyl, Alkoxy-alkyl, gegebenfalls substituiertes Mono- und *N*,*N*-Dialkyl-aminoalkyl und Hydroxyalkyl bedeutet.

Im Begriff "substituierte Gruppen" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und *N*,*N*-Dialkenylamino-carbonyl, Mono- und Dialkinylaminocarbonyl, Mono- und *N*,*N*-Dialkenylamino, Mono- und *N*,*N*-Dialkinylamino, Trialkenylsilyl, Trialkinylsilyl, gegebenenfalls substituiertes Cycloalkenyl, gegebenenfalls substituiertes Cycloalkinyl, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe sowie einer substituierten Iminogruppe.

Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich.

Vorzugsweise werden vom Begriff "substituierter Rest" nur ein oder zwei Substitutentenebenen umfasst.

### Bevorzugte Substituenten für die Substituentenebenen sind beispielsweise

Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, N-Monoalkyl-amino, *N*,*N*-Dialkylamino, *N*-Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylthio, Cycloalkylthio, Alkenylthio, Cycloalkenylthio, Alkinylthio, Alkylsulfenyl und Alkylsulfinyl, wobei beide Enantiomere der Alkylsulfinylgruppe umfasst sind, Alkylsulfonyl, N-Mono-alkylaminosulfonyl, *N*,*N*-Dialkyl-aminosulfonyl, Alkylphosphinyl, Alkylphosphonyl, wobei für Alkylphosphinyl bzw. Alkylphosphonyl beide Enantiomere umfasst sind, *N*-Alkyl-aminocarbonyl, *N*,*N*-Dialkyl-amino-carbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Substituenten, die aus mehreren Substituentenebenen zusammengesetzt sind, sind bevorzugt Alkoxyalkyl, Alkylthioalkyl, Alkylthioalkoxy, Alkoxyalkoxy, Phenethyl, Benzyloxy, Halogenalkyl, Halogencycloalkyl, Halogenalkoxy, Halogenalkylthio, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylthio, Halogenalkoxyalkanoyl, Halogenalkoxyalkyl.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl *N*-substituiert sind; vorzugsweise N-Mono- und *N*,*N*-Dialkylamino, (z.B. Methylamino, Ethylamino, *N,N-*Dimethylamino, *N*,*N*-Diethylamino, *N*,*N*-Di-n-propylamino, *N*,*N*-Diisopropylamino oder *N,N-*Dibutylamino), N-Mono- oder *N*,*N*-Dialkoxyalkylaminogruppen (z.B. *N*-Methoxymethylamino, N-Methoxyethylamino, *N*,*N*-Di-(methoxymethyl)-amino oder *N*,*N*-Di-(methoxyethyl)-amino), *N-*Mono- und *N*,*N*-Diarylamino, wie gegebenenfalls substituierte Aniline, Acylamino, *N,N-*diacylamino, *N*-Alkyl-*N*-arylamino, *N*-Alkyl-*N*-acylamino sowie gesättigte *N*-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Der Begriff "cyclische Aminogruppen" umfasst heteroaromatische oder aliphatische Ringsysteme mit einem oder mehreren Stickstoffatomen. Die Heterocyclen sind gesättigt oder ungesättigt, bestehen aus einem oder mehreren, gegebenenfalls kondensierten Ringsystemen und beinhalten gegebenenfalls weitere Heteroatome, wie beispielsweise ein oder zwei Stickstoff-, Sauerstoff-und/oder Schwefelatome. Ferner umfasst der Begriff auch solche Gruppen, die einen Spiroring oder verbrücktes Ringsystem aufweisen. Die Anzahl der Atome, die die cyclische Aminogruppe bilden, ist beliebig und kann z.B. im Falle eines Einringsystems aus 3 bis 8 Ringatomen und im Falle eines Zweiringsystems aus 7 bis 11 Atomen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolidin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-piperazin-1-yl, 1,2-Dihydro-pyrimidin-1-yl, Perhydropyrimidin-1-yl, 1,4-Diazacycloheptan-1-yl, genannt; beispielhaft für cyclische Aminogruppen mit gesättigten und ungesättigten monocyclischen Gruppen mit einem oder zwei Sauerstoffatomen und einem bis drei Stickstoffatomen als Heteroatome, wie beispielsweise Oxazolidin-3-yl, 2,3-Dihydroisoxazol-2-yl, Isoxazol-2-yl, 1,2,3-Oxadiazin-2-yl, Morpholino, beispielhaft für cyclische Aminogruppen mir gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl genannt.

Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, (C₁-C₄)Alkylthio, (C₁-C₄)Halogenalkylthio, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl-und -Trichlormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein-oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy , (C₁-C₄)Alkoxy-(C₁-C₄)alkyl, (C₁-C₄)Halogenalkyl und (C₁-C₄)Halogenalkoxy substituiert ist, insbesondere durch einen oder zwei (C₁-C₄)Alkylreste substituiert ist,

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Beispiele für Alkyl substituierte Heteroaryle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl,, Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Erfindungsgemäß geeignete Salze der erfindungsgemäßen Verbindungen, beispielsweise Salze mit Basen oder Säureadditionssalze, sind alle üblichen nicht toxischen Salze, vorzugsweise landwirtschaftlich und/oder physiologisch annehmbare Salze. Beispielsweise Salze mit Basen oder Säureadditionssalze. Bevorzugt werden Salze mit anorganischen Basen, wie beispielsweise Alkalimetallsalze (z.B. Natrium-, Kalium- oder Cäsiumsalze), Erdalkalimetallsalze (z.B. Calzium- oder Magnesiumsalze), Ammoniumsalze oder Salze mit organischen Basen, insbesondere mit organischen Aminen, wie beispielsweise Triethylammonium-, Dicyclohexylammonium-, *N,N'-*Dibenzylethylendiammonium-, Pyridinium-, Picolinium- oder Ethanolammoniumsalze, Salze mit anorganischen Säuren (z.B. Hydrochloride, Hydrobromide, Dihydrosulfate, Trihydrosulfate, oder Phosphate), Salze mit organischen Carbonsäuren oder organischen Sulfosäuren (z.B. Formiate, Acetate, Trifluoracetate, Maleate, Tartrate, Methansulfonate, Benzolsulfonate oder 4-Toluolsulfonate). Bekannterweise können tert- Amine, wie beispielsweise manche der erfindungsgemäßen Verbindungen, *N*-Oxide bilden, welche ebenfalls erfindungsgemäße Salze darstellen.

In den nachfolgenden allgemeinen Formeln (Ia) bis (Iv) haben die Gruppierungen und Substituenten A₁, A₂, A₃, A₄, U, L, m, Q und R¹ die oben angegebenen Bedeutungen. Die Reste Z¹, Z² und Z³ werden durch die oben angegebene Restedefinition von Z beschrieben.

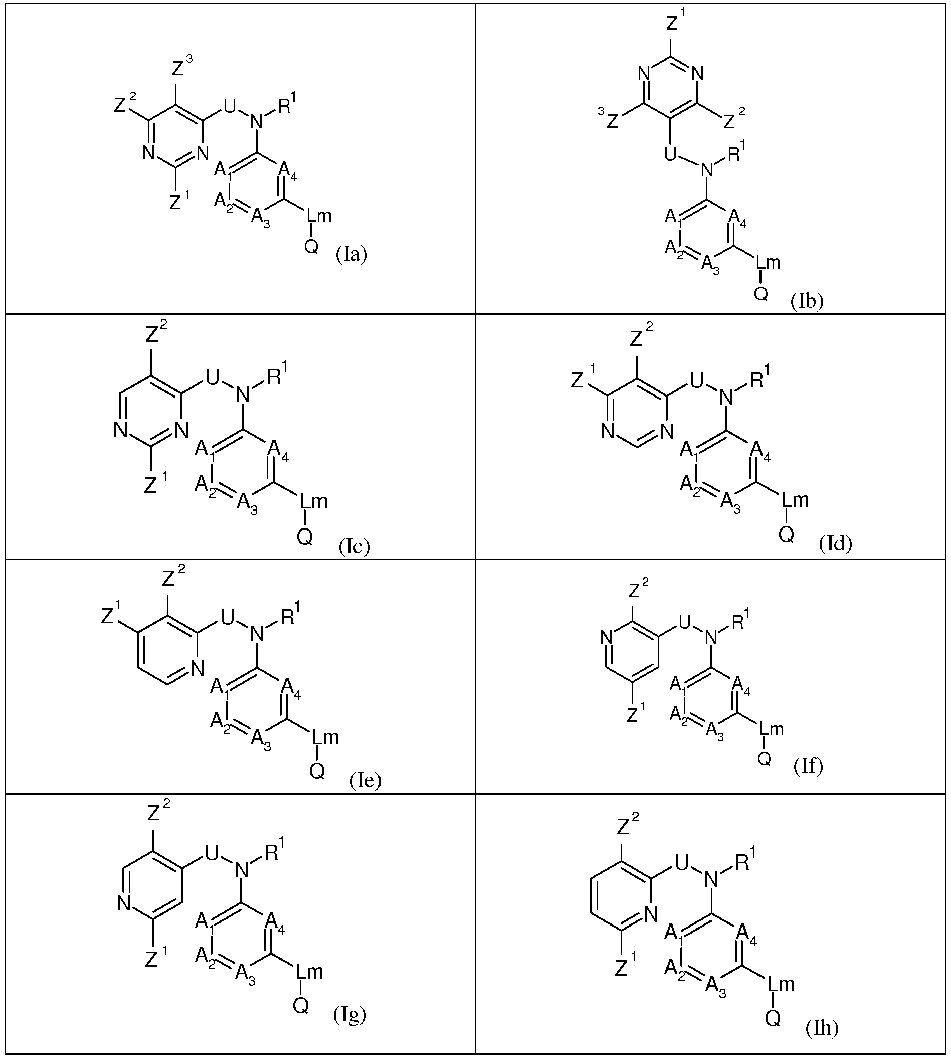

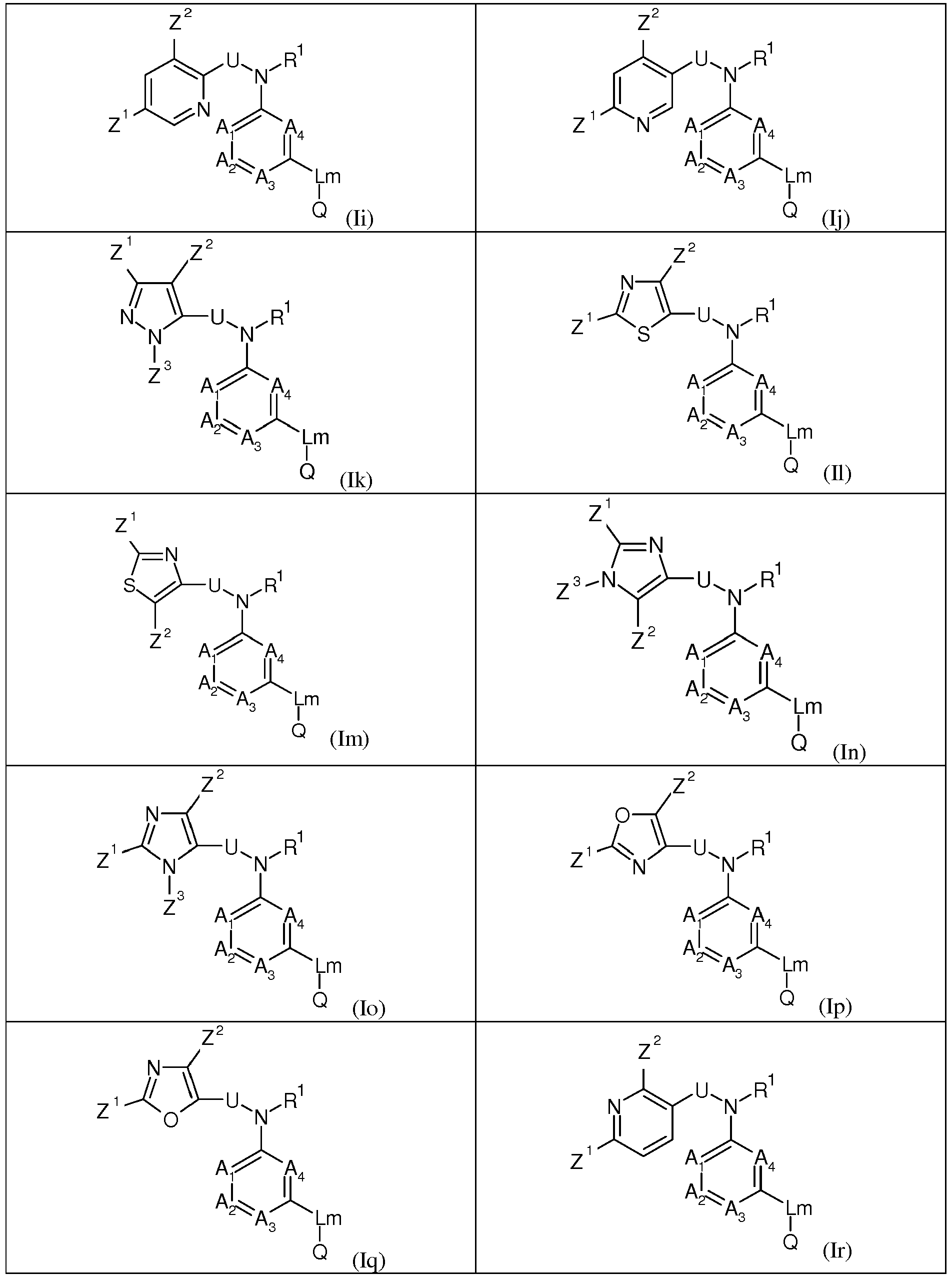

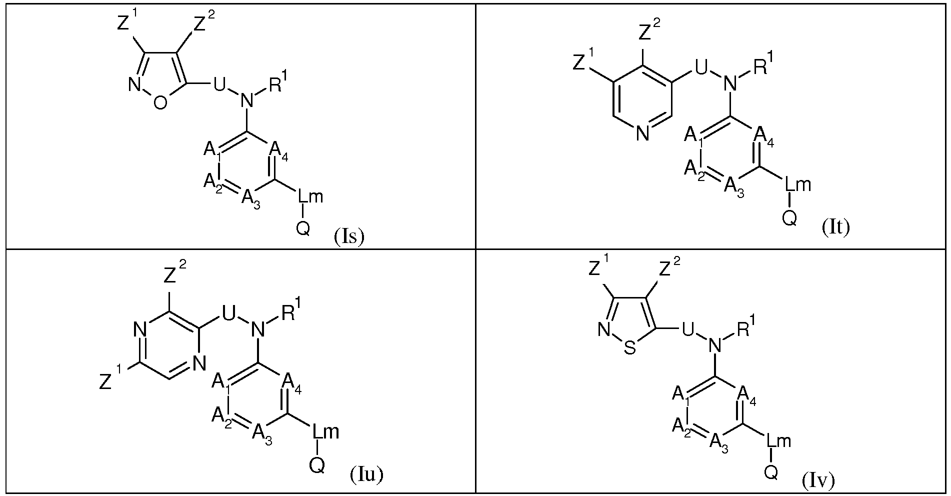

Die Verbindungen (Ia) bis (Iv) eignen sich zur Bekämpfung tierischer Schädlinge in der Landwirtschaft und in der Tiergesundheit.

Die erfindungsgemäßen Verbindungen können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die erfindungsgemäßen Verbindungen können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Schließlich wurde gefunden, dass die neuen Verbindungen der Formel (Ik) sich bei guter Pflanzenverträglichkeit, günstiger Warmblütertoxizität und guter Umweltverträglichkeit vor allem zur Bekämpfung von tierischen Schädlingen, insbesondere von Arthropoden, Insekten, Spinnentieren, Helminthen, Nematoden und Mollusken, die in der Landwirtschaft, in den Forsten, im Vorrats- und Materialschutz sowie auf dem Hygienesektor oder im Bereich der Tiergesundheit vorkommen, eignen. Gleichfalls können die erfindungsgemäßen Verbindungen im Bereich der Tiergesundheit verwendet werden, beispielsweise zur Bekämpfung von Endo- und/oder Ectoparasiten.

Die erfindungsgemäßen Verbindungen können als Mittel zur Bekämpfung tierischer Schädlinge, vorzugsweise als Pflanzenschutzmittel eingesetzt werden. Sie sind gegen normal sensible und resistente Arten sowie gegen alle oder einzelne Entwicklungsstadien wirksam.

Die erfindungsgemäßen Verbindungen können in allgemein bekannte Formulierungen überführt werden. Solche Formulierungen enthalten im Allgemeinen von 0,01 bis 98 Gew.-% Wirkstoff, vorzugsweise von 0,5 bis 90 Gew.-%.

Die erfindungsgemäßen Verbindungen können in ihren handelsüblichen Formulierungen sowie in den aus diesen Formulierungen bereiteten Anwendungsformen in Mischung mit anderen Wirkstoffen oder Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise von 0,00001 bis 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Coccidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Außerdem wurde gefunden, daß die erfindungsgemäßen Verbindungen eine hohe insektizide Wirkung gegen Insekten zeigen, die technische Materialien zerstören. Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam.

Unter Pflanzen werden alle Pflanzenarten, Pflanzensorten und Pflanzenpopulationen, wie erwünschte und unerwünschte Wildpflanzen oder Kulturpflanzen verstanden. Erfindungsgemäß zu behandelnde Kulturpflanzen sind Pflanzen, die natürlich vorkommen, oder solche, die durch konventionelle Züchtungs- und Optimierungsmethoden oder durch biotechnologische und gentechnologische Methoden oder durch Kombinationen der vorgenannten Methoden erhalten wurden. Der Begriff Kulturpflanze umfasst selbstverständlich auch transgene Pflanzen.

Unter Pflanzensorten versteht man Pflanzen mit neuen Eigenschaften, sogenannten Traits, die sowohl durch konventionelle Züchtung, durch Mutagenese oder durch rekombinante DNA-Techniken oder einer Kombination hieraus gezüchtet worden sind. Dies können Sorten, Rassen, Bio- und Genotypen sein.

Unter Pflanzenteilen werden alle oberirdischen und unterirdischen Teile und Organe der Pflanzen, wie Spross, Blatt, Blüte und Wurzel verstanden, insbesondere Blätter, Nadeln, Stängel, Stämme, Blüten, Fruchtkörper, Früchte, Samen, Wurzeln, Knollen und Rhizome. Der Begriff Pflanzenteilen umfasst weiterhin Erntegut sowie vegetatives und generatives Vermehrungsmaterial, wie z.B. Stecklinge, Knollen, Rhizome, Ableger und Samen bzw. Saatgut.

In einer erfindungsgemäßen Ausführungsform werden natürlich vorkommende oder durch konventionelle Züchtungs- und Optimierungsmethoden (z.B. Kreuzung oder Protoplastenfusion) erhaltene Pflanzenarten und Pflanzensorten sowie deren Pflanzenteile behandelt.

In einer weiteren erfindungsgemäßen Ausführungsform werden transgene Pflanzen, die durch gentechnologische Methoden gegebenenfalls in Kombination mit konventionellen Methoden erhalten wurden und deren Teile behandelt.

Das erfindungsgemäße Behandlungsverfahren wird vorzugsweise auf genetisch modifizierten Organismen, wie beispielsweise Pflanzen oder Pflanzenteile, verwendet.

Genetisch modifizierte Pflanzen, sogenannte transgene Pflanzen, sind Pflanzen, bei denen ein heterologes Gen stabil in das Genom integriert worden ist.

Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Pflanzen, die weiterhin vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Neben den vorgenannten Pflanzen und Pflanzensorten, können auch solche erfindungsgemäß behandelt werden, die gegen einen oder mehrere abiotische Streßfaktoren widerstandsfähig sind.

Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPDtolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insecticidal proteins, VIP),
   die unter http://www.lifesci.sussex.ac.uk/Home/Neil_Crickmore/Bt/vip.html
   angeführt sind, z. B. Proteine der Proteinklasse VIP3Aa; oder
6) ein sezerniertes Protein aus *Bacillus thuringiensis* oder *Bacillus cereus*, das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfindungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie *Bacillus, Rhizobium, Pseudomonas*, *Serratia, Trichoderma, Clavibacter, Glomus* oder *Gliocladium* stammen. Die vorliegende Erfindung eignet sich besonders für die Behandlung von transgenem Saatgut, das zumindest ein heterologes Gen enthält, das aus *Bacillus sp.* stammt und dessen Genprodukt Wirksamkeit gegen Maiszünsler und/oder Maiswurzel-Bohrer zeigt. Besonders bevorzugt handelt es sich dabei um ein heterologes Gen, das aus *Bacillus thuringiensis* stammt.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Darüber hinaus können die erfindungsgemäßen Verbindungen zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Die vorliegende Erfindung schließt somit auch ein Verfahren zur Bekämpfung von Schädlingen ein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Kozzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Zu diesen Parasiten gehören:
Aus der Ordnung der Anoplurida z.B. Haematopinus spp., Linognathus spp., Pediculus spp., Phtirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;
Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;
Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;
Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp. (z.B. Suppella longipalpa);
Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Sternostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroa jacobsoni;
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

Die erfindungsgemäßen Wirkstoffe eignen sich auch zur Bekämpfung von Arthropoden, Helminthen und Protozoen, die Tiere befallen. Zu den Tieren zählen landwirtschaftliche Nutztiere wie z.B. Rinder, Schafe, Ziegen, Pferde, Schweine, Esel, Kamele, Büffel, Kaninchen, Hühner, Puten, Enten, Gänse, Zuchtfische, Honigbienen. Zu den Tieren zählen außerdem Haustiere - die auch als Heimtiere bezeichnet werden - wie z.B. Hunde, Katzen, Stubenvögel, Aquarienfische sowie sogenannte Versuchstiere wie z.B. Hamster, Meerschweinchen, Ratten und Mäuse.

Durch die Bekämpfung dieser Arthropoden, Helminthen und/oder Protozoen sollen Todesfälle vermindert und die Leistung (bei Fleisch, Milch, Wolle, Häuten, Eiern, Honig usw.) und die Gesundheit des Wirtstieres verbessert werden, so dass durch den Einsatz der erfindungsgemäßen Wirkstoffe eine wirtschaftlichere und einfachere Tierhaltung möglich ist.

So ist es beispielsweise wünschenswert, die Aufnahme von Blut des Wirts durch die Parasiten (falls zutreffend) zu verhindern oder zu unterbrechen. Eine Bekämpfung der Parasiten kann außerdem dazu beitragen, die Übertragung infektiöser Substanzen zu verhindern.

Der Begriff "Bekämpfung", so wie er hier bezogen auf den Bereich Tiergesundheit verwendet wird, bedeutet, dass die Wirkstoffe wirken, indem sie das Vorkommen des betreffenden Parasiten in einem mit solchen Parasiten befallenen Tier auf unschädliche Niveaus reduzieren. Genauer gesagt bedeutet "Bekämpfung", wie hier verwendet, dass der Wirkstoff den betreffenden Parasiten tötet, sein Wachstum hemmt oder seine Proliferation inhibiert.

Im allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"]), die die Wirkstoffe in einer Menge von 1 bis 80 Gew.-% enthalten, direkt oder nach Verdünnung (z.B. 100-bis 10 000facher Verdünnung) anwenden oder sie als chemisches Bad verwenden.

Beim Einsatz im Bereich Tiergesundheit können die erfindungsgemäßen Wirkstoffe in Kombination mit geeigneten Synergisten oder anderen Wirkstoffen wie beispielsweise Akariziden, Insektiziden, Anthelmintika, Mittel gegen Protozoen, verwendet werden.

Die erfindungsgemäßen Verbindungen können nach üblichen, dem Fachmann bekannten Methoden hergestellt werden.

Im Reaktionsschema 1 ist das allgemeine Darstellungsverfahren A für die Verbindungen (**I**-**1**) abgebildet.

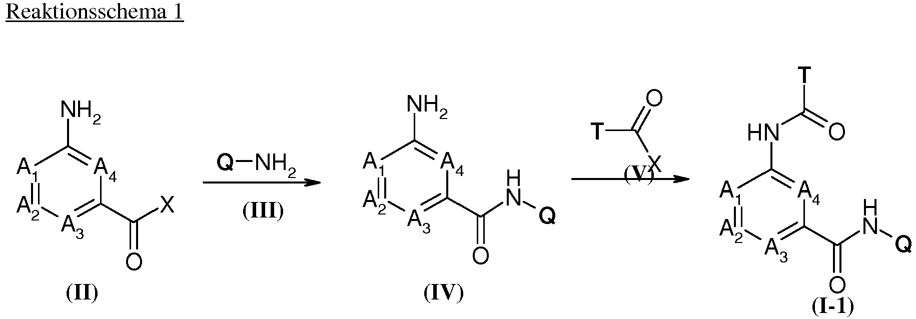

Die Reste A₁-A₄, Q und T haben die oben beschriebenen Bedeutungen. X steht für eine beliebige Abgangsgruppe.

Erfindungsgemäße Verbindungen des Typs (**I-1**) können durch die Umsetzung von Aminen der allgemeinen Struktur **(IV)** mit aktivierten Carbonsäurederivaten der allgemeinen Struktur **(V)** dargestellt werden. Die Reaktion kann mit oder ohne Lösungsmittel durchgeführt werden. In diesem Schritt kann ebenfalls eine geeignete Base zum Einsatz kommen.

Im Allgemeinen ist es vorteilhaft, den ersten Reaktionsschritt des erfindungsgemäßen Darstellungsverfahrens A gegebenenfalls in Gegenwart eines geeigneten Verdünnungsmittels und gegebenenfalls in Gegenwart eines geeigneten basischen Reaktionshilfsmittels durchzuführen.

Verdünnungsmittel werden vorteilhaft in einer solchen Menge eingesetzt, dass das Reaktionsgemisch während des ganzen Verfahrens gut rührbar bleibt.

Als Lösungsmittel kann jedes Lösungsmittel verwendet werden, das die Reaktion nicht beeinträchtigt, wie zum Beispiel Wasser. In Frage kommen aromatische Kohlenwasserstoffe wie Benzol oder Toluol; halogenierte Kohlenwasserstoffe wie Dichlormethan, Chloroform oder Tetrachlorkohlenwasserstoff, offenkettige oder zyklische Ether wie Diethylether, Dioxan, Tetrahydrofuran oder 1,2-Dimethoxyethan; Ester wie Ethylacetat und Butylacetat; Ketone wie zum Beispiel Aceton, Methyl-isobutylketon und Cyclohexanon; Amide wie Dimethylformamid und Dimethylacetamid; Nitrile wie Acetonitril; und andere inerte Lösungsmittel wie 1,3-Dimethyl-2-imidazolidinon; die Lösungsmittel können allein oder in Kombination von zwei oder mehr eingesetzt werden.

Als Base kann eine organische Base wie Triethylamin, Ethyl-diisopropylamin, Tri-n-butylamin, Pyridin und 4-Dimethylamino-pyridin verwendet werden; Des Weiteren können beispielsweise folgende Basen eingesetzt werden: Alkalimetallhydroxide wie z.B Natriumhydroxid und Kaliumhydroxid; Carbonate wie Natriumhydrogencarbonat und Kaliumcarbonat; Phosphate wie Dikalium-hydrogenphosphat und Tri-natriumphosphat; Alkalimetallhydride wie Natriumhydrid; Alkalimetallalkoholate wie Natriummethanolat und Natriumethanolat. Diese Basen können in Verhältnissen von 0.01 bis 5.0 Moläquivalenten in Bezug auf **(IV)** und **(V)** eingesetzt werden. Des Weiteren kann auch Silber(I)cyanid als Base und Aktivator eingesetzt werden [Journal of Organic Chemistry. 1992, 57, 4394-4400; Journal of Medicina Chemistry 1992, 35, 3905-3918; Journal of Organic Chemistry 2003, 68, 1843-1851]

Die geeignete Reaktionstemperatur liegt im Bereich von -20°C bis zum Siedepunkt des jeweiligen Lösungsmittels und die Reaktionsdauer liegt je nach Wahl der Reaktanden, Lösungsmittel und Reaktionstemperatur zwischen wenigen Minuten bis 96 Stunden.

Zyklische Carbonsäurehalogenide, wie sie durch die allgemeine Struktur **(V)** repräsentiert werden, können einfach durch die Umsetzung einer heterozyklischen Carbonsäure mit Halogenierungsreagenzien wie Thionylchlorid, Thionylbromid, Phosphorylchlorid, Oxalylchlorid, Phosphortrichlorid, etc. hergestellt werden. [Houben-Weyl, 1952, Bd. VIII, S.463 ff.]

Die Darstellung von Carboxamiden repräsentiert durch die Formel (**I-1**) kann aber auch unter der Verwendung von Kupplungsreagenzien wie Dicyclohexylcarbodiimid und Additiven wie 1-Hydroxybenzotriazol durchgeführt werden.[Chem. Ber. 1970, 788] Verwendbar sind ferner Kupplungsreagenzien wie 1-Ethyl-3-(3-dimethylaminopropyl)carbodiimid, 1,1'-Carbonyl-1H-imidazol und ähnliche Verbindungen.

Als Kupplungsreagenzien zur Durchführung des Dartellungsverfahrens finden alle, die zur Herstellung einer Ester- oder Amidbindung geeignet sind (vgl. z. B. Bodansky et al., Peptide Synthesis, 2nd ed., Wiley & Sons, New York, 1976; Gross, Meienhofer, The Peptide: Analysis, Synthesis, Biology (Academic Press, New York, 1979), Verwendung.

Des Weiteren können auch gemischte Anhydride zur Darstellung von (**I-1**) verwendet werden. [J. Am. Chem. Soc 1967, 5012] Bei diesem Verfahren können verschiedene Chlorameisensäureester zum Einsatz kommen, wie z.B. Chlorameisensäureisobutylester, Chlorameisensäureisopropylester. Ebenfalls können dafür Diethylacetylchlorid, Trimethylacetylchlorid und ähnliche verwendet werden.

Verbindungen der allgemeinen Struktur **(IV)** können durch die Umsetzung eines Amins der allgemeinen Struktur (**III**) mit aktivierten Carbonsäurederivaten der allgemeinen Struktur **(II)** dargestellt werden. Dabei gelten dieselben Bedingungen für die Wahl des Lösungsmittels, der Reaktionsbedingungen, der Reaktionsdauer und der Reagenzien wie bei der oben beschriebenen Darstellung von (**I-1**).

Das Reaktionsschema 2 zeigt das allgemeine Darstellungsverfahren B für die Synthese der Verbindungen (**I-1**).

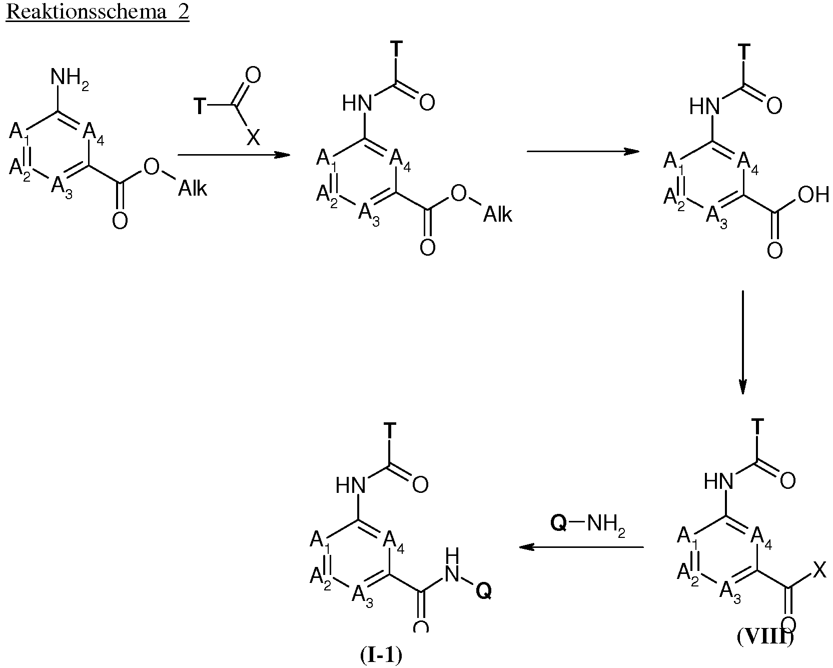

Die Reste A₁-A₄, Q und T haben die oben beschriebenen Bedeutungen. X steht für eine beliebige Abgangsgruppe und Alk für einen Alkylrest, wie z.B. Methyl oder Ethyl.

Verbindungen des Typs **(I-1)** können durch die Umsetzung eines Amins der allgemeinen Struktur **(III)** mit aktivierten Carbonsäurederivaten der allgemeinen Struktur **(VIII)** dargestellt werden. Dabei gelten dieselben Bedingungen für die Wahl des Lösungsmittels, der Reaktionsbedingungen, der Reaktionsdauer und der Reagenzien wie bei der im Darstellungsverfahren A beschriebenen Umsetzung von **(IV)** und **(V)** zu (**I-1**).

Die Darstellung von aktivierten Carbonsäurederivaten der allgemeinen Struktur **(VIII)** kann durch eine zweistufige Synthese aus den entsprechenden Carbonsäureestern der allgemeinen Struktur **(VII)** erfolgen. Im ersten Schritt wird die in Form eines Esters geschützte Carbonsäurefunktion (O-Alk) der Verbindung **(VII)** in Abhängigkeit des verwendeten Alkylesters mit einem passenden Reagenz entschützt [Greene's protective groups in organic synthesis, 4. Edition, P. G. M. Wuts, T. W. Greene, John Wiley & Sons, Inc., Hoboken, New Jersey] und die daraus resultierende freie Hydroxygruppe der Säurefunktion von (**VIII-1**) in eine Abgangsgruppe X überführt. Dabei können dieselben Verfahren eingesetzt werden, die bereits in der Darsellung von **(V)** beschrieben wurden. Verbindungen der allgemeinen Struktur **(VII)** können durch die Umsetzung von Aminen der allgemeinen Struktur **(VI)** mit aktivierten Carbonsäurederivaten der allgemeinen Struktur **(V)** dargestellt werden. Dabei gelten dieselben Bedingungen für die Wahl des Lösungsmittels, der Reaktionsbedingungen, der Reaktionsdauer, der Reagenzien wie bei der im Darstellungsverfahren A beschriebenen Synthese von (**I-1**).

Wenn es sich bei den erfindungsgemäßen Verbindungen **(I)** um Verbindungen der allgemeinen Strukturen (I-**2**) und (I-**3**) handelt, kann die Synthese über das Darstellungsverfahren C (Reaktionsschema 3) erfolgen.

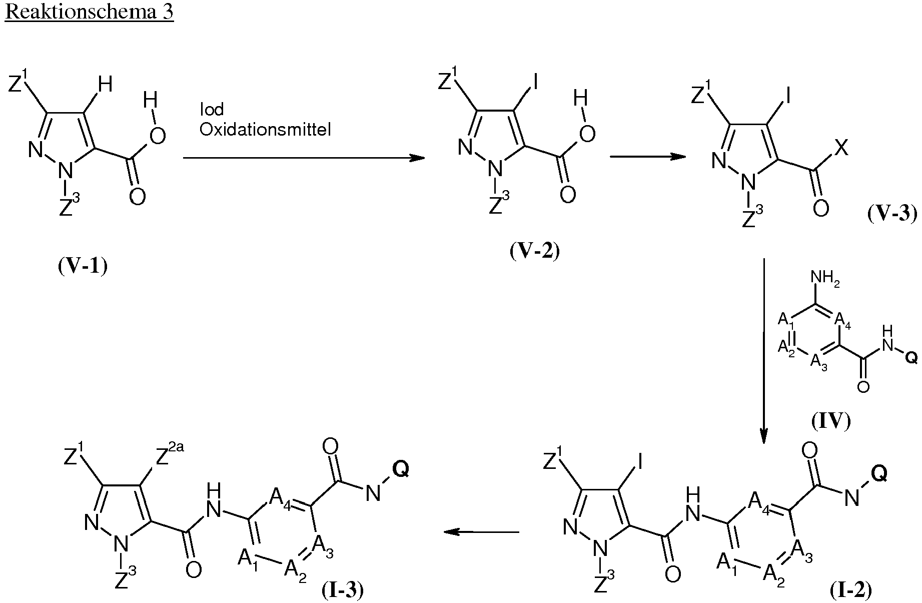

Die Reste Z¹, Z³ und Q haben die oben beschriebenen Bedeutungen. Z^{2a} steht für gegebenenfalls substituiertes Aryl oder Heteroaryl, gegebenenfalls substituiertes Alkenyl oder Akinyl oder für Perfluoralkyl X steht für eine beliebige Abgangsgruppe.

Verbindungen der allgemeinen Struktur **(I-3)** können aus den erfindungsgemäßen Verbindungen der allgemeinen Struktur **(I-2)** dargestellt werden. Dabei wird das Iodatom der Verbindungen des Typs **(I-2)** metallvermittelt unter anderem durch Perfluoralkyl [PCT Int. Appl., 2005095351], Aryl [Journal of Organic Chemistry 2007, 72(9), 3589-3591; Synthesis 1997, (5), 563-566;; Heterocycles 2006, 68(11), 2247-2252] und Alkenyl [PCT Int. Appl., 2005060749; Organic Process Research & Development 2005, 9(5), 694-696; Chemical & Pharmaceutical Bulletin 2005, 53(2), 153-163; Journal of Organic Chemistry 1986, 51(26), 5286-90] ersetzt.

Die Verbindungen der allgemeinen Struktur **(I-2)** können durch Umsetzung von aktivierten Carbonsäurederivaten der allgemeinen Struktur **(V-3)** mit Aminen der allgemeinen Struktur **(IV)** dargestellt werden. Die Reaktionsbedingungen für diese Umsetzung sind bereits im Darstellungsverfahren A bei der Synthese von **(I-1)** beschrieben worden.

Die Verbindungen der allgemeinen Struktur (**V**-**3**) können in einem zweistufigen Verfahren ausgehend von Pyrazolcarbonsäuren des Typs **(V-1)**, die nach literaturbekannten Verfahren hergestellt werden können [Journal of Organic Chemistry 2008, 73(9), 3523-3529; Bioorganic & Medicinal Chemistry Letters 2005, 15(22), 4898-4906; US2006069270], dargestellt werden. Dabei werden im ersten Schritt Verbindungen der Formel **(V-1)** in der 4-Position mit Iod und einem Oxidationsmittel, wie z.B. Cer(IV)ammoniumnitrat iodiert. Die Hydroxyfunktion von **(V-2)** wird anschließend nach denen bereits im Darstellungsverfahren A zur Herstellung von **(V)** beschriebenen Methoden in die Abgangsgruppe X überführt.

Das Reaktionsschema 4 zeigt das allgemeine Darstellungsverfahren D für die Synthese der Verbindungen **(I-4)**.

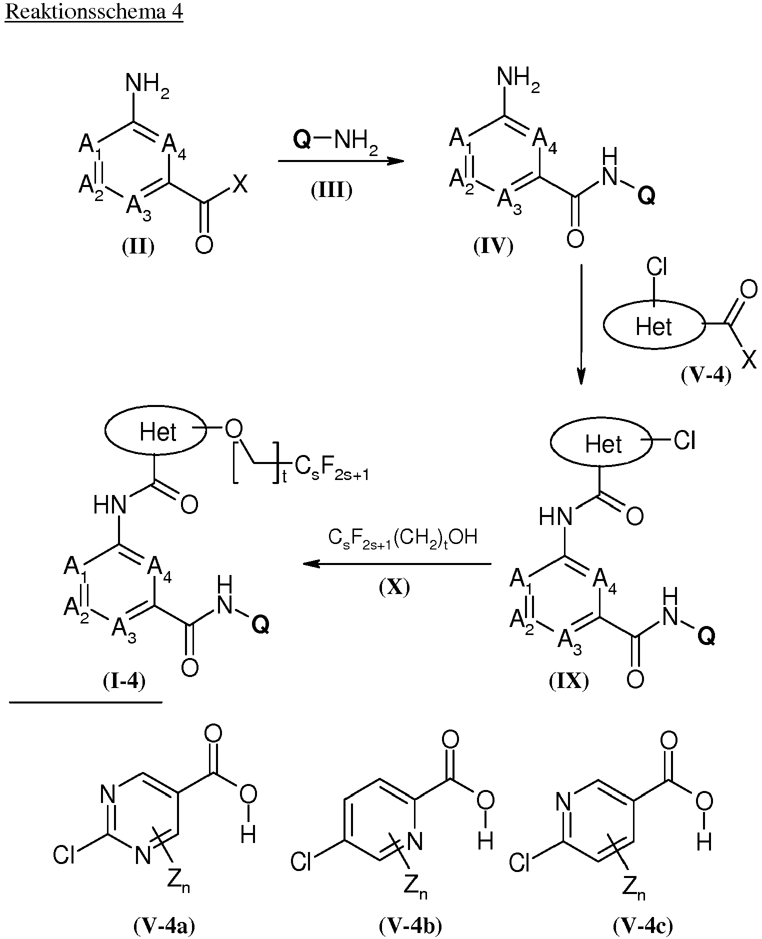

Verbindungen der allgemeinen Formel (**V**-**4**) repräsentieren dabei spezielle *N*-heteroyklische Carbonsäuren, beispielsweise kann es sich dabei um die Carbonsäuren des Typs (**V**-**4a**) - (**V**-**4ac**) handeln. Allen diesen Carbonsäuren ist gemeinsam, dass das Chloratom aufgrund seiner Position zu den Stickstoffatomen für eine nucleophile Substitution aktiviert ist. Die Reste A₁-A₄. und Q haben die oben beschriebenen Bedeutungen. X steht für eine beliebige Abgangsgruppe. "n", "t" und "s" stehen unabhängig voneinander für 0-3, wobei die Summe aus "s" und "t" ≤ 4 ist.

Die Verbindungen der allgemeinen Struktur **(I-4)** können durch die Umsetzung von perfluorierten Alkoholen der allgemeinen Struktur **(X)** mit Chlorverbindungen der allgemeinen Struktur **(IX)** dargestellt werden. Diese Substitutionsreaktion kann nach literaturbekannten Verfahren durchgeführt werden [Pest Management Science 2001; 57(9), 844-851; Canadian Journal of Chemistry 1985, 63(11), 3037-42].

Die Verbindungen der allgemeinen Struktur **(IX)** können durch die Umsetzung von Aminen der allgemeinen Struktur **(IV)** mit aktivierten heterozyklischen Carbonsäurederivaten der allgemeinen Struktur (**V**-**4**) dargestellt werden. Die Reaktionsbedingungen für diese Umsetzung sind bereits im Darstellungsverfahren A bei der Synthese von **(I-1)** beschrieben worden.

Die Herstellung von Aminen der allgemeinen Strutktur **(IV)** erfolgt nach der im Darstellungsverfahren A beschriebenen Methode durch Umsetzung von Aminen der allgemeinen Struktur **(III)** mit aktivierten Carbonsäurederivaten der allgemeinen Struktur **(II)**.

Das Reaktionsschema 5 zeigt das allgemeine Darstellungsverfahren E für die Synthese der erfindungsgemäßen Verbindungen der allgemeinen Struktur **(I**-**5)**.

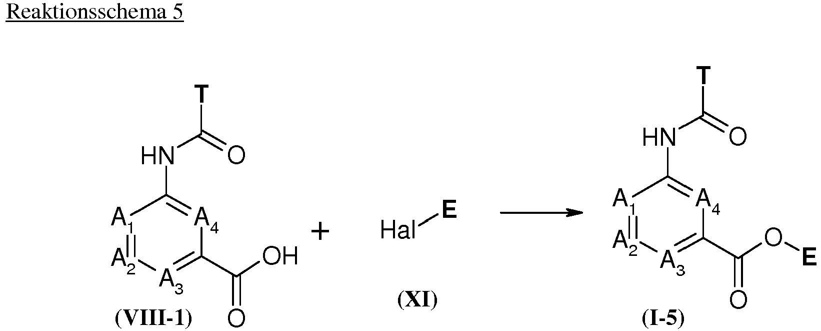

Die Reste A₁-A₄ und T haben die oben beschriebenen Bedeutungen. Bei E handelt es sich um gegebenenfalls substituierte primäre und sekundäre Alkylreste. Hal repräsentiert ein Halogenatom, vorzugsweise Chlor oder Brom.

Die Verbindungen der allgemeinen Struktur **(I-5)** können durch eine Substitutionsreaktion zwischen Carbonsäure der allgemeinen Struktur **(VIII-1)** und Halogenverbindungen der allgemeinen Struktur **(XI)** erfolgen. Die Reaktion kann analog zu bekannten Literaturvorschriften durchgeführt werden [Tetrahedron Letters 2007, 48 (39), 6974-6976; International Journal of Pharmaceutics 1987, 39 (1), 75-85].

Das Reaktionsschema 6 zeigt das allgemeine Darstellungsverfahren F für die Synthese der Verbindungen der allgemeinen Struktur (I-**6**).

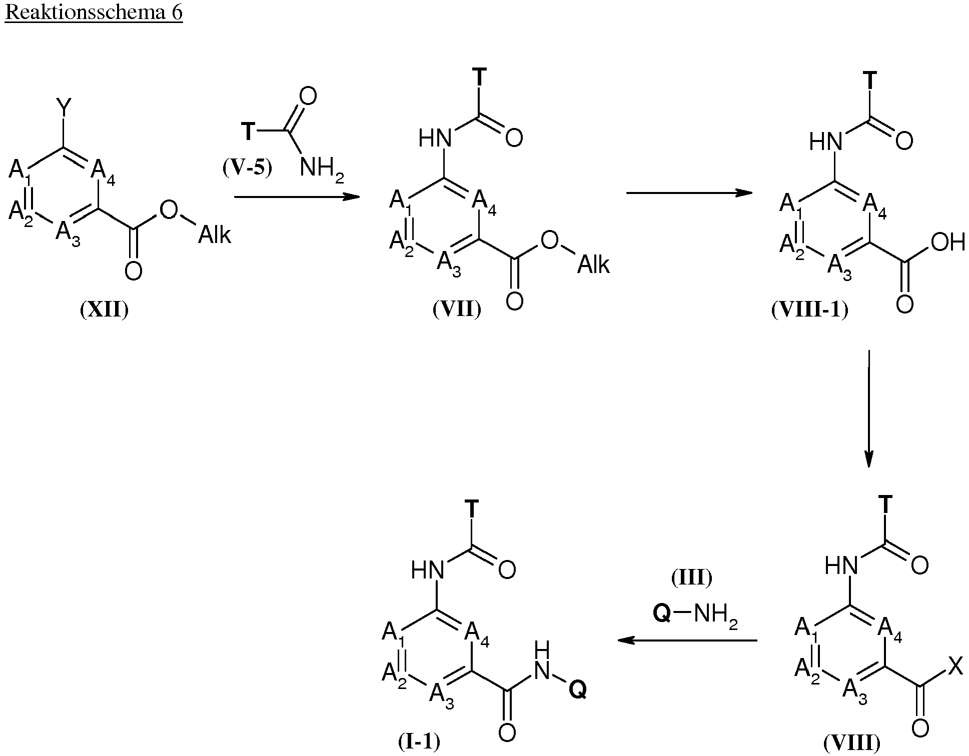

Die Reste A₁-A₄, Q und T haben die oben beschriebenen Bedeutungen. Y steht für Brom, Iod bzw. Triflat. X steht für eine beliebige Abgangsgruppe und Alk für einen Alkylrest, wie z.B. Methyl oder Ethyl.

Verbindungen des Typs **(I-1)** können ausgehend von Verbindungen der allgemeinen Struktur **(VII)** nach dem im Darstellungsverfahren B beschriebenen Verfahren dargestellt werden. Verbindungen der allgemeinen Struktur **(VII)** können unter anderem durch eine metall-vermittelte Kupplung von Verbindungen der allgemeinen Struktur **(XII)** mit Carbonsäureamiden allgemeinen Struktur **(V**-**5)** dargestellt werden. Die Reaktion kann analog zu bekannten Literaturvorschriften durchgeführt werden [Chemistry - A European Journal 2008, 14(12), 3527-3529; Organic Letters 2007, 9(23), 4749-4751; Journal of the American Chemical Society 2002, 124(21), 6043-6048; Bioorganic & Medicinal Chemistry 2008, 16(6), 3091-3107].

Aktivierte Carbonsäurederivate der allgemeinen Formel **(II)** sind aus zyklischen Aminocarbonsäuren der allgemeinen Formel **(II-1)** nach bereits im Darstellungsverfahren A beschriebenen Methoden zur Darstellung von **(V)** zugänglich. Substanzen der allgemeinen Formel **(II-1)** sind allgemein bekannte Verbindungen der organischen Chemie, die nach etablierten Syntheseverfahren erhalten werden können. Mögliche Synthesewege der zyklischen Aminocarbonsäuren der allgemeinen Formel **(II-1)** sind im Reaktionsschema 7 abgebildet.

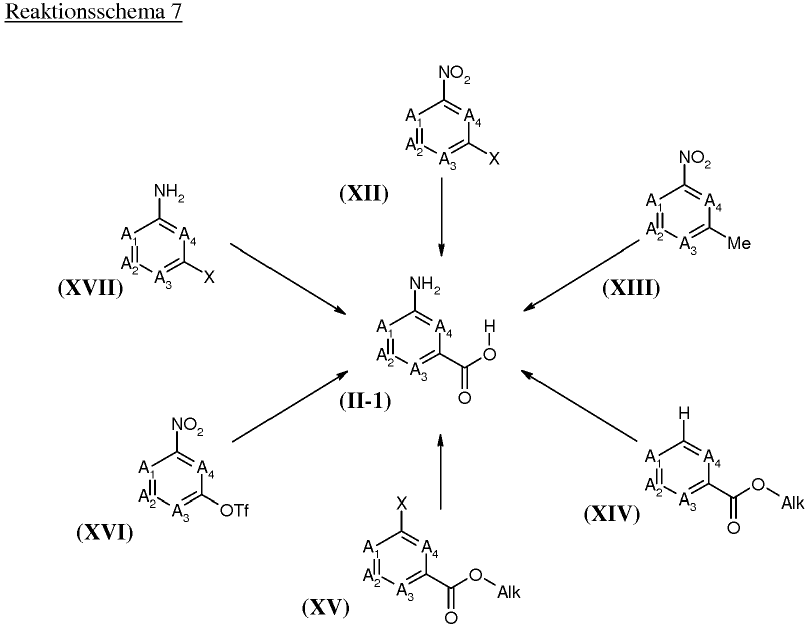

Als Edukte zur Darstellung von Aminocarbonsäuren der allgemeinen Struktur **(II-1)** können z.B. halogenierte (hetero)aromatische Nitro- bzw. Aminoverbindungen dienen, wie sie durch die Formeln **(XII)** und **(XVII)** repräsentiert werden. Dabei wird die Abgangsgruppe X durch eine Cyanogruppe ersetzt und diese anschließend sauer oder basisch hydrolysiert. Der Halogen-Cyano-Austausch kann z.B. durch eine nucleophile Substitution am Aromaten mit einer Cyanidspezies wie z.B. Natriumcyanid [US 4766219] oder auch durch eine Kupfer-vermittelte Reaktion [Journal of Antibiotics 1994, 47(12), 1456-65] erfolgen.

Im Fall der Nitroverbindungen **(XVII)** kann anschließend noch eine Reduktion der Nitrofunktion in eine Aminofunktion erfolgen. Geeignete Verfahren für solche Reduktionen sind Hydrierungen und Metall-vermittelte Reaktionen wie z.B. Zinn(II)chlorid, Eisenpulver, Zinkpulver und diesen ähnliche Verbindungen.

Hydrierungen können in einem geeigneten Lösungsmittel in Anwesenheit eines Katalysators unter Wasserstoffatmosphäre (Normaldruck oder Hochdruck). Als Katalysatoren können Palladiumkatalysatoren wie z.B. Palladium auf Kohle, Nickelkatalysatoren wie Raney-Nickel, Kobaltkatalysatoren, Rutheniumkatalysatoren, Rhodiumkatalysatoren, Platinkatalysatoren und diesen ähnliche Verbindungen verwendet werden. Geeignete Lösungsmittel sind Wasser, Alkohole wie Methanol und Ethanol, aromatische Kohlenwasserstoffe wie Benzol und Toluol, offenkettige oder zyklische Ether wie Diethylether, Dioxan und Tetrahydrofuran sowie Ester wie Essigsäureethylester. Die Reduktionen können in einem Druckbereich von 1 bar bis 100 bar durchgeführt werden, wobei die Temperatur zwischen -20°C und dem Siedepunkt des eingesetzten Lösungsmittels variieren kann. Je nach Reaktionsbedingungen liegen die Reaktionszeiten zwischen wenigen Minuten und 96 Stunden.

Die Metall-vermittelten Reduktionen wie z.B. mit Zinn(II)chlorid können nach einem in Organic Syntheses Coll. Vol. (III), 453 beschriebenen Verfahren durchgeführt werden.

Des Weiteren können (hetero)aromatische Aminocarbonsäuren der allgemeinen Struktur **(II-1)** auch aus den entsprechenden Methylvorläufern des Typs **(XIII)** durch Oxidation dargestellt werden. Für solche Oxidationen geeignete Oxidationsmittel sind z.B. Kaliumpermanganat, Natriumdichromat, Chromtrioxid und diesen ähnliche Verbindungen. [Tetrahedron Letters 1995, 36(25), 4369-72; Bioorganic & Medicinal Chemistry Letters 2007, 17(4), 1043-1046] Ebenso können für solche Oxidationen auch enzymatische Verfahren verwendet werden. [PCT Int. Appl., 9502061] Die anschließend notwendige Reduktion der Nitrofunktion kann analog zu den oben beschriebenen Verfahren durchgeführt werden.

Eine weitere Methode zur Darstellung von (hetero)aromatischen Aminocarbonsäuren der allgemeinen Struktur **(II-1)** ist die Nitrierung von Carbonsäurevorläufern repräsentiert durch die Formel **(XIV)** bzw. **(XV)** und die anschließende Reduktion der Nitrofunktion. Die Nitrierungen können nach literaturbekannten Verfahren durchgeführt werden [Justus Liebigs Annalen der Chemie 1958, 611, 194-205; Organikum, Wiley-VCH, 22. Auflage, 358ff]. Die anschließend notwendige Reduktion der Nitrofunktion kann analog zu den oben beschriebenen Verfahren durchgeführt werden.

Des Weiteren können (hetero)aromatische Aminocarbonsäuren der allgemeinen Struktur **(II-1)** aus den entsprechenden (Hetero)aryl-Triflaten des Typs **(XVI)** mit Hilfe eines Palladium-katalysierten Verfahrens hergestellt werden [Synthesis 2006, (4), 594-596].

Mögliche Synthesen der heterozyklischen Carbonsäurederivate der allgemeinen Formel **(V)** sind im Reaktionsschema 8 abgebildet.

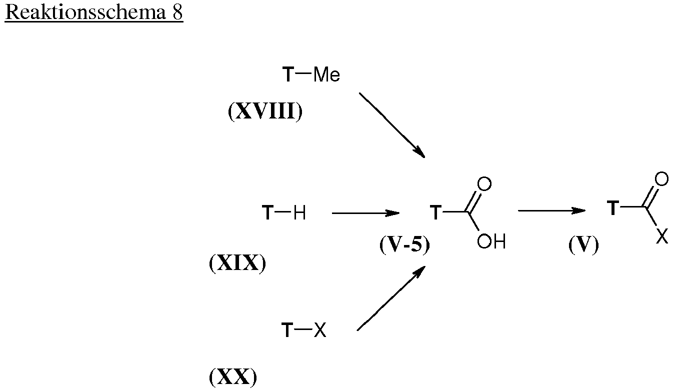

Heterozyklischen Carbonsäuren der allgemeinen Struktur (**V**-**5**) können unter anderem aus Methylderivaten der allgemeinen Formel **(XVIII)** durch Oxidation der Methylfunktion dargestellt werden. Dabei können die bereits bei der Oxidation von Methylgruppen der Verbindungen der allgemeinen Struktur **(XIII)** genannten Verfahren angewendet werden.

Heterozyklischen Carbonsäuren der allgemeinen Struktur (**V**-**5**) können aus Vorläufern der allgemeinen Struktur **(XIX)** durch Deprotonierung mit einer geeigneten Base und durch Abfangen des entsprechenden Carbanions mit Kohlendioxid dargestellt werden [Journal of Medicinal Chemistry 2008, 51(4), 937-947; Bioorganic & Medicinal Chemistry Letters 2007, 17(22), 6274-6279]. Als Base eignen sich z.B. Lithiumdiisopropylamid, n-Butyllithium, sec-Butyllithium und diesen ähnliche Verbindungen.

Für das oben beschriebene Verfahren zur Darstellung von heterozyklischen Carbonsäuren der allgemeinen Struktur (**V**-**5**) eignen sich ebenfalls die entsprechenden halogenierten Heterozyklen **(XX).** Dabei wird das Carbanion allerdings nicht durch Deprotonierung generiert, sondern durch eine Metallierungsreaktion [Angewandte Chemie, International Edition 2008, 47(2), 311-315]. Für diese Metallierungsreaktionen eignen sich vorzugsweise n-Butyllithium, tert-Butyllithium und iso-Propylmagnesiumchlorid.

Heterozyklischen Carbonsäuren der allgemeinen Struktur (**V**-**5**) können ebenfalls aus halogenierten Vorläufern der allgemeinen Struktur **(XX)** mit Hilfe von literaturbekannten Palladium-katalysierten Reaktionen in die entsprechenden heterozyklischen Carbonsäureester überführt [Russian Journal of Applied Chemistry 2007, 80(4), 571-575].

Heterozyklischen Carbonsäuren der allgemeinen Struktur (**V**-**5**) können des Weiteren aus halogenierten Verbindungen der allgemeinen Struktur **(XX)** durch eine Substitutionsreaktion der Halogene mit Cyaniden und anschließender Hydrolyse der Nitrilfunktion mit starken Säure oder Basen dargestellt werden [WO 2005079801].

Heterocyclische aktivierte Carbonsäurederivate, wie z.B. Carbonsäurehalogenide, wie sie durch die allgemeine Struktur **V** repräsentiert werden, können durch Umsetzung einer zyklischen Carbonsäure repräsentiert durch die Formel (**V**-**5**) mit Halogenierungsreagenzien wie Thionylchlorid, Thionylbromid, Phosphorylchlorid, Oxalylchlorid, Phosphortrichlorid, etc. hergestellt werden [Organikum, Wiley-VCH, 22. Auflage, 496ff].

Aktivierte Carbonsäurederivate der allgemeinen Struktur (II) können nach allgemein bekannten Literaturverfahren aus Carbonsäuren der Formel **(II-1)** dargestellt werden [Organikum, Wiley-VCH, 22. Auflage, 496ff; Chem. Ber. 1970, 788; J. Am. Chem. Soc 1967, 5012]. Die Verbindungen der Formel **(II-1**) sind kommerziell erhältlich oder können nach bekannten Literaturverfahren hergestellt werden [Synthesis 2006, (4), 594-596; Tetrahedron Letters 1995, 36(25), 4369-72; Bioorganic & Medicinal Chemistry Letters 2007, 17(4), 1043-1046; PCT Int. Appl., 9502061, Journal of Organic Chemistry 1954, 19, 357-64; WO 2001083459].

Verbindungen der allgemeinen Struktur **III** sind käuflich und/oder können nach allgemein in der Fachliteratur bekannten Verfahren hergestellt werden [Houben-Weyl (1992), Vol. E, 16d, 646ff; Tetrahedron Letters, 33(24), 3487-90; 1992].

Verbindungen der allgemeinen Struktur (V) sind im Allgemeinen käuflich und/oder können nach bekannten Literaturverfahren dargestellt werden [Journal of Medicinal Chemistry 2008, 51(4), 937-947; Bioorganic & Medicinal Chemistry Letters 2007, 17(22), 6274-6279; Russian Journal of Applied Chemistry 2007, 80(4), 571-575; WO 2005079801; Journal of Organic Chemistry 2008, 73(9), 3523-3529; Bioorganic & Medicinal Chemistry Letters 2005, 15(22), 4898-4906; US2006069270]

Die Pyrazolcarbonsäuren der Formel (V-6) sind neu und ebenfalls Gegenstand der Erfindung.

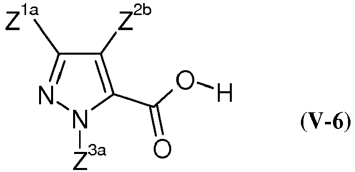

Wobei unabhängig voneinander
- Z^{1a}: für 1,2,2,2-Tetrafluorethyl, 1-Chlor-1,2,2,2-tetrafluorethyl, Pentafluorethyl, Heptafluor-n-propyl, Heptafluor-isopropyl, Nonafluor-n-butyl, Difluormethoxy, Difluormethyl,
- Z^{2b}: für Fluor, Chlor, Brom, Iod, Methyl, Ethyl, Trifluormethyl, 2-Propenyl, ,
- Z^{3a}: für Ethyl, Isopropyl, tert-Butyl, 1-Fluor-1-methylethyl,
stehen.

Die Verbindungen der allgemeinen Struktur **(VI)** können nach literaturbekannten Verfahren aus den Verbindungen der alllgemeinen Struktur **(II)** dargestellt werden [Journal of the American Chemical Society 2001, 123(34), 8177-8188; Inorganica Chimica Acta 2006, 359(6), 1912-1922].

Verbindungen der allgemeinen Strukturen **(X)** bis **XX** sind käuflich und/oder aus der einschlägigen Fachliteratur bekannt.

Oxidationsmittel für die Oxidation alkoholischer Gruppen sind bekannt (vgl. z. B. Oxidationsreagenzien in Organic Synthesis by Oxidation with Metal Compounds, Mijs, de Jonge, Plenum Verlag, New York, 1986; Manganese Compounds as Oxidizing Agens in Organic Chemistry, Arndt, Open Court Publishing Company, La Salle, IL, 1981; The Oxidation of Organic Compounds by Permanganate Ion and Hexavalent Chromium, Lee, Open Court Publishing Company, La Salle, IL, 1980). Eine Oxidation kann beispielsweise in Gegenwart von Permanganaten (z.B. Kaliumpermanganat), Metalloxiden (z.B. Mangandioxid, Chromoxide die beispielsweise in Dipyridin-chrom(VI)-oxid als Collins Reagenz (vgl. J. C. Collins et al., Tetrahedron Lett. 30, 3363-3366, 1968) verwendet werden) durchgeführt werden. Ebenfalls in Gegenwart von Pyridiniumchlorochromat (z.B. Corey's Reagenz) (vgl. auch R. O. Hutchins et al., Tetrahedron Lett. 48, 4167-4170, 1977; D. Landini et al. Synthesis 134-136, 1979) oder Ruthenium-tetroxid (vgl. S.-I. Murahashi, N. Komiya Ruthenium-catalyzed Oxidation of Alkenes, Alcohols, Amines, Amides, β-Lactams, Phenols and Hydrocarbons, in: Modern Oxidation Methods, Baeckvall, Jan-Erling (Eds.), Wiley-VCH-Verlag GmbH & Co. KGaA, 2004). Ebenfalls geeignet sind Ultraschall-induzierte Oxidationsreaktionen, sowie die Verwendung von Kaliumpermanganat (vgl. J. Yamawaki et al., Chem. Lett. 3, 379-380, 1983).

Zur Deblockierung/Abspaltung der Schutzgruppe SG können alle bekannten geeigneten sauren oder basischen Reaktionshilfsmittel nach den in der Literatur beschriebenen Verfahrensweises verwendet werden. Bei Verwendung von Schutzgruppen für Aminogruppen des Carbamat-Typs werden bevorzugt saure Reaktionshilfsmittel verwendet. Bei Verwendung der tert-Butylcarbamat-Schutzgruppe (BOC-Gruppe) werden beispielsweise Mischungen von Mineralsäuren wie Salzsäure, Bromwasserstoffsäure, Salpetersäure, Schwefelsäure, Phosphorsäure oder organischen Säuren wie Benzoesäure, Ameisensäure, Essigsäure, Trifluoressigsäure, Methansulfonsäure, Benzolsulfonsäure oder Toluolsulfonsäure und einem geeigneten Verdünnungsmittel wie Wasser und/oder einem organischen Lösungsmittel wie Tetrahydrofuran, Dioxan, Dichlormethan, Chloroform, Essigester, Ethanol oder Methanol verwendet. Bevorzugt sind Mischungen von Salzsäure oder Essigsäure mit Wasser und/oder einem organischen Lösungsmittel wie Essigester.

Es ist bekannt, dass manche Reaktionen und Herstellungsverfahren besonders gut in Gegenwart von Verdünnungs- bzw. Lösungsmittel und basischer oder saurer Reaktionshilfsmitteln durchführbar sind. Mischungungen der Verdünnungs- bzw. Lösungsmittel sind ebenfalls einsetzbar. Die Verdünnungs- bzw. Lösungsmittel werden vorteilhafterweise in einer solchen Menge eingesetzt, dass das Reaktionsgemisch während des ganzen Verfahrens gut rührbar ist.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, n-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, *N,N*-Dimethyl-formamid, *N,N*-Dipropyl-formamid, *N,N*-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, *N,N*'-1,4-Diformyl-piperazin) und Ketone (z.B. Aceton, Acetophenon, Methylethylketon, Methylbutylketon).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Natriums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetrabutyl-guanidin (CyTBG), Cyclohexyltetramethylguanidin (CyTMG), *N,N,N,N-*Tetramethyl-1,8-naphthalindiamin, Pentamethylpiperidin) und Amine, insbesondere tertiäre Amine, (z.B. Triethylamin, Trimethylamin, Tribenzylamin, Trüsopropylamin, Tributylamin, Tricyclohexylamin, Triamylamin, Trihexylamin, *N,N*-Dimethylanilin, *N,N*-Dimethyl-toluidin, *N,N-*Dimethyl-p-aminopyridin, N-Methyl-pyrrolidin, N-Methyl-piperidin, N-Methyl-imidazol, N-Methyl-pyrazol, N-Methyl-morpholin, N-Methyl-hexamethylendiamin, Pyridin, 4-Pyrrolidinopyridin, 4-Dimethylamino-pyridin, Chinolin, α-Picolin, β-Picolin, Isochinolin, Pyrimidin, Acridin, *N,N*,N',N'-Tetramethylendiamin, *N,N*,N',N'-Tetraethylendiamin, Chinoxalin, N-Propyl-diisopropylamin, N-Ethyl-diisopropylamin, *N,N*'-Dimethyl-cyclohexylamin, 2,6-Lutidin, 2,4-Lutidin oder Triethyldiamin).

Als saure Reaktionshilfsmittel zur Durchführung der erfindungsgemäßen Verfahren können alle Mineralsäuren (z.B. Halogenwasserstoff-säuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)-chlorid, Bortrifluorid oder sein Etherat, Titan(V)chlorid, Zinn(V)-chlorid, und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure eingesetzt werden.

Sofern in den Reaktionsschemata Schutzgruppen vorgesehen sind, können alle allgemein bekannten Schutzgruppen verwendet werden. Insbesondere solche, die von Greene T. W., Wuts P. G. W. in Protective Groups in Organic Synthesis; John Wiley & Sons, Inc. 1999, "Protection for the hydroxyl group including 1,2- and 1,3-diols" beschrieben sind.

Weiterhin eignen sich auch Schutzgruppen
vom Typ eines substituierten Methylethers (z.B. Methoxymethylether (MOM), Methylthiomethylether (MTM), (Phenyl-dimethylsilyl)methoxymethylether (SNOM-OR), Benzyloxymethylether (BOM-OR) para-Methoxybenzyloxymethylether (PMBM-OR), para-Nitrobenzyloxymethyl-ether, ortho-Nitrobenzyloxymethylether (NBOM-OR), (4-Methoxyphenoxy)-methylether (p-AOM-OR), Guaiacolmethylether (GUM-OR), tert-Butoxymethylether, 4-Pentyloxy-methylether (POM-OR), Silyloxymethylether, 2-Methoxy-ethoxy-methylether (MEM-OR), 2,2,2-Trichlorethoxymethylether, Bis(2-chlorethoxy)-methylether, 2-(Trimethyl-silyl)ethoxymethylether (SEM-OR), Methoxymethylether (MM-OR));
vom Typ eines substituierten Ethylethers (z.B. 1-Ethoxyethylether (EE-OR), 1-(2-Chlorethoxy)ethylether (CEE-OR), 1-[2-(Trimethylsilyl)ethoxy]ethylether (SEE-OR), 1-Methyl-1-methoxyethylether (MIP-OR), 1-Methyl-1-benzyloxyethylether (MBE-OR), 1-Methyl-1-benzyloxy-2-fluor-ethylether (MIP-OR), 1-Methyl-1-phenoxyethylether, 2,2,-Trichlorethylether, 1,1-Dianisyl-2,2,2-trichlorethylether (DATE-OR), 1,1,1,3,3,3-Hexafluor-2-phenylisopropylether (HIP-OR), 2-Trimethylsilylethylether, 2-(Benzylthio)ethylether, 2-(Phenylselenyl)ethylether), eines Ethers (z.B. Tetrahydropyranylether (THP-OR), 3-Brom-tetrahydropyranylether (3-BrTHP-OR), Tetrahydrothiopyranylether, 1-Methoxy-cyclohexylether, 2- und 4-Picolylether, 3-Methyl-2-picolyl-N-oxido-ether, 2-Quinolinylmethylether (Qm-OR), 1-Pyrenylmethylether, Dipenylmethylether (DPM-OR), para, para'-Dinitrobenzhydrylether (DNB-OR), 5-Dibenzosuberylether, Triphenylmethylether (Tr-OR), alpha-Naphthyldiphenylmethylether, para-Methoxy-phenyldiphenylmethylether (MMTrOR), Di(para-methoxy-phenyl)phenylmethylether (DMTr-OR), Tri(para-methoxy-phenyl)phenylmethylether (TMTr-OR), 4-(4'-Brom-phenacyloxy) phenyldiphenylmethylether, 4,4',4"-Tris(4,5-dichlorphthalimido-phenyl)methylether (CPTr-OR), 4,4',4"-Tris(benzoyloxyphenyl)-methylether (TBTr-OR), 4,4'-Dimethoxy-3"-[N-(imidazolylmethyl)]-tritylether (IDTr-OR), 4,4'-Dimethoxy-3"-[N-(imidazolyl-ethyl)carbamoyl]tritylether (IETr-OR), 1,1-Bis(4-methoxy-phenyl)-1'-pyrenyl-methylether (Bmpm-OR), 9-Anthrylether, 9-(9-Phenyl)xanthenylether (Pixyl-OR), 9-(9-Phenyl-10-oxo)anthryl (Tritylon-Ether), 4-Methoxy-tetrahydropyranylether (MTHP-OR), 4-Methoxy-tetrahydrothiopyranylether, 4-Methoxy-tetrahydrothiopyranyl-S,S-dioxid, 1-[(2-Chlor-4-methyl)phenyl]-4-methoxypiperidin-4-yl-ether (CTMP-OR), 1-(2-Fluorphenyl)-4-methoxy-piperidin-4-yl-ether (Fpmp-OR), 1,4-Dioxan-2-yl-ether, Tetrahydrofuranylether, Tetrahydrothiofuranylether, 2,3,3a,4,5,6,7,7a-Octahydro-7,8,8-trimethyl-4,7-methanbenzofuran-2-yl-ether (MBF-OR), tert-Butylether, Allylether, Propargylether, para-Chlor-phenylether, para-Methoxy-phenylether, para-Nitro-phenylether, para-2,4-Dinitro-phenylether (DNP-OR), 2,3,5,6-Tetrafluor-4-(trifluormethyl)phenylether, Benzylether (Bn-OR));
vom Typ eines sustituierten Benzylethers (z.B. para-Methoxy-benzylether (MPM-OR), 3,4-Dimethoxy-benzylether (DMPM-OR), ortho-Nitro-benzylether, para-Nitro-benzylether, para-Halobenzylether, 2,6-Dichlor-benzylether, para-Aminoacyl-benzylether (PAB-OR), para-Azidobenzylether (Azb-OR), 4-Azido-3-chlor-benzylether, 2-Trifluormethyl-benzylether, para-(Methylsulfinyl)benzylether (Msib-OR));
vom Typ eines Silylethers (z.B. Trimethylsilylether (TMS-OR), Triethylsilylether (TES-OR), Triisopropylsilylether (TIPS-OR), Dimethylisopropylsilylether (IPDMS-OR), Diethylisopropylsilylether (DEIPS-OR), Dimethylhexylsilylether (TDS-OR), tert-Butyldimethylsilylether (TBDMS-OR), tert-Butyldiphenylsilylether(TBDPS-OR), Tribenzylsilylether, Tri-para-xylylsilylether, Triphenylsilylether (TPS-OR), Diphenylmethylsilylether (DPMS-OR), Di-tert-butylmethylsilylether (DTBMS-OR), Tris(trimethylsilyl)silylether (Sisylether), Di-tert-butylmethylsilylether (DTBMS-OR), Tris(trimethylsilyl)silylether (Sisylether), (2-Hydroxystyryl)-dimethylsilylether (HSDMS-OR), (2-Hydroxystyryl)diisopropylsilylether (HSDIS-OR), tert-Butylmethoxyphenyl-silylether (TBMPS-OR), tert-Butoxydiphenylsilylether (DPTBOS-OR));
vom Typ eines Esters (z.B. Formiatester, Benzoylformiatester, Acetatester (Ac-OR), Chloracetatester, Dichloracetatester, Trichloracetatester, Trifluoracetatester, (TFA-OR), Methoxyacetatester, Triphenylmethoxyacetatester, Phenoxyacetatester, para-Chlorphenoxyacetatester, Phenylacetatester, Diphenylacetatester (DPA-OR), Nicotinatester, 3-Phenylpropionatester, 4-Pentoatester, 4-Oxo-pentoatester (Levulinate) (Lev-OR) 4,4-(Ethylendithio)-pentanoatester (LevS-OR), 5-[3-Bis(4-methoxyphenyl)hydroxy-methoxyphenoxy]-levulinatester, Pivaloatester (Pv-OR), 1-Adamantanoatester, Crotonatester, 4-Methoxy-crotonatester, Benzoatester (Bz-OR), para-Phenyl-benzoatester, 2,4,6-Trimethyl-benzoatester (Mesitoate), 4-(Methylthiomethoxy)-butyratester (MTMB-OR), 2-(Methylthiomethoxymethyl)-benzoatester (MTMT-OR),
vom Typ eines Esters (z.B. Methylcarbonat, Methoxymethylcarbonat, 9-Fluorenylmethylcarbonat (Fmoc-OR), Ethylcarbonat, 2,2,2-Trichlorethylcarbonat (Troc-OR), 1,1-Dimethyl-2,2,2-trichlorethylcarbonat (TCBOC-OR), 2-(Trimethylsilyl)ethylcarbonat (TMSEC-OR), 2-(Phenylsulfonyl)-ethylcarbonat (Psec-OR), 2-(Triphenylphosphonio)-ethylcarbonat (Peoc-OR), tert-Butylcarbonat (Boc-OR), Isobutylcarbonat, Vinylcarbonat, Allylcarbonat (Alloc-OR), para-Nitro-phenylcarbonat, Benzylcarbonat (Z-OR), para-Methoxy-benzylcarbonat, 3,4-Dimethoxy-benzylcarbonat, ortho-Nitro-benzylcarbonat, para-Nitro-benzylcarbonat, 2-Dansylethylcarbonat (Dnseoc-OR), 2-(4-Nitrophenyl)ethylcarbonat (Npeoc-OR), 2-(2,4-Dinitrophenyl)ethylcarbonat (Dnpeoc)), und
vom Typ eines Sulfats (z.B. Allylsulfonat (Als-OR), Methansulfonat (Ms-OR), Benzylsulfonat, Tosylat (Ts-OR), 2-[(4-Nitrophenyl)ethyl]sulfonat (Npes-OR)).

Als Katalysatoren zur Durchführung einer katalytischen Hydrierung im erfindungsgemäßen Verfahren sind alle üblichen Hydrierkatalysatoren, wie beispielsweise Platin-Katalysatoren (z.B. Platin-Platte, Platin-Schwamm, Platin-Schwarz, kolloidales Platin, Platinoxid, Platindraht), Palladium-Katalysatoren (z.B. Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid , Nickel-Katalysatoren (z.B. reduziertes Nickel, Nickeloxid, Raney-Nickel), Ruthenium-Katalysatoren, Cobalt-Katalysatoren (z.B. reduziertes Cobalt, Raney-Cobalt), Kupfer-Katalysatoren (z.B. reduziertes Kupfer, Raney-Kupfer, Ullmann-Kupfer) geeignet. Bevorzugt werden Edelmetalllkatalysatoren (z.B. Platin- und Palladium- oder Ruthenium-Katalysatoren) verwendet, die gegebenenfalls auf einem geeigneten Träger (z.B. Kohlenstoff oder Silizium) aufgebraucht sind, Rhodium-Katalysatoren (z.B. Tris(triphenylphosphin)rhodium(I)-chlorid in Gegenwart von Triphenylphosphin). Ferner können "chiralen Hydrierkatalysatoren" (z. B. solche die chirale Diphosphinliganden enhalten wie (2S,3S)-(-)-2,3-Bis(diphenylphosphino)-butan [(S,S)-Chiraphos] oder (R)-(+)-2,2'- bzw. (S)-(-)-2,2'-Bis(diphenylphosphino)-1,1'-binaphthalin [R(+)-BINAP bzw. S(-)-BINAP]) verwendet werden, wodurch der Anteil eines Isomers im Isomerengemisch erhöht wird bzw. das Entstehen eines anderen Isomers nahezu vollständig unterdrückt wird.

Die Herstellung von Salzen der erfindungsgemäßen Verbindungen erfolgt nach Standardverfahren. Repräsentative Säureadditionssalze sind beispielsweise solche die durch Reaktion mit anorganischen Säuren, wie beispielsweise Schwefelsäure, Salzsäure, Bromwasserstoffsäure, Phosphorsäure oder organischen Carbonsäuren wie Essigsäure, Trifluoressigsäure, Zitronensäure, Bernsteinsäure, Buttersäure, Milchsäure, Ameisensäure, Fumarsäure, Maleinsäure, Malonsäure, Camphersäure, Oxalsäure, Phthalsäure, Propionsäure, Glycolsäure, Glutarsäure, Stearinsäure, Salicylsäure, Sorbinsäure, Weinsäure, Zimtsäure, Valeriansäure, Pikrinsäure, Benzoesäure oder organischen Sulfonsäuren wie Methansulfonsäure und 4-Toluolsulfonsäure gebildet werden.

Repräsentativ sind auch Salze von erfindungsgemäßen Verbindungen, die aus organischen Basen, wie beispielsweise Pyridin oder Triethylamine gebildet werden oder solche, die aus anorganischen Basen, wie beispielsweise Hydride, Hydroxide oder Karbonate des Natriums, Lithiums, Calciums, Magnesiums oder Bariums, gebildet werden, wenn die Verbindungen der allgemeinen Formel (I) ein zu dieser Salzbildung geeignetes Strukturelement aufweist.

Synthesemethoden zur Darstellung heterocyclischer N-Oxide und tert-Aminen sind bekannt. Sie können mit Peroxysäuren (z.B. Peressigsäure und meta-Chlor-perbenzoesäure (MCPBA), Wasserstoffperoxid), Alkylhydroperoxide (z.B. tert-Butylhydroperoxid), Natriumperborat und Dioxiranen (z.B. Dimethyldioxiran) erhalten werden. Diese Methoden sind beispielsweise von T. L. Gilchrist, in Comprehensive Organic Synthesis, Vol. 7, S. 748-750, 1992, S. V. Ley, (Ed.), Pergamon Press; M. Tisler, B. Stanovnik, in Comprehensive Heterocyclic Chemistry, Vol. 3, S. 18-20, 1984, A. J. Boulton, A. McKillop, (Eds.), Pergamon Press; M. R. Grimmett, B. R. T. Keene in Advances in Heterocyclic Chemistry, Vol. 43, S. 149-163, 1988, A. R. Katritzky, (Ed.), Academic Press; M. Tisler, B. Stanovnik, in Advances in Heterocyclic Chemistry, Vol. 9, S. 285-291, 1968, A. R. Katritzky, A. J. Boulton (Eds.), Academic Press; G. W. H. Cheeseman, E. S. G. Werstiuk in Advances in Heterocyclic Chemistry, Vol. 22, S. 390-392, 1978, A. R. Katritzky, A. J. Boulton, (Eds.), Academic Press beschrieben.

### Experimenteller Teil

### Darstellungsverfahren A

### Erfindungsgemäßes Beispiel (Ik-1) N-[4-Chlor-3-(cyclopropylcarbamoyl)phenyl]-1-methyl-3-(pentafluorethyl)-4-(trifluormethyl)-1H-pyrazol-5-carbonsäureamid

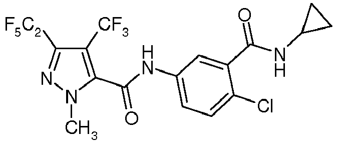

560 mg (1,79 mmol) 1-Methyl-3-pentafluorethyl-4-trifluormethyl-pyrazol-5-carbonsäure werden in 10 mL Dichlormethan suspendiert. Die Suspension wird auf 0 °C gekühlt und anschließend nacheinander mit 0,02 mL *N,N*-Dimethylformamid und 188 µL (2,15 mmol) Oxalylchlorid versetzt. Das Reaktionsgemisch wird erst 0,5 h bei 0 °C und dann 3 Stunden bei Raumtemperatur gerührt. Das Lösungsmittel wird unter vermindertem Druck am Rotationsverdampfer entfernt. Das enstandene 1-Methyl-3-(pentafluorethyl)-4-(trifluormethyl)-1*H*-pyrazol-5-carbonsäurechlorid wird ohne weitere Aufarbeitung für den nachfolgenden Syntheseschritt eingesetzt.

95,3 mg (0,45 mmol) 5-Amino-2-chlor-*N*-cyclopropylbenzamid, 3,7 mg (0,03 mmol) *N,N-*Dimethylpyridin-4-amin werden in 2,5 mL Ethylacetat gelöst. Die Lösung wird mit einem Eisbad auf 0 °C gekühlt und mit 158 µL (0,91 mmol) *N*-Ethyl-diisopropylamin versetzt. 100 mg (0,30 mmol) 1-Methyl-3-(pentafluorethyl)-4-(trifluormethyl)-1*H*-pyrazol-5-carbonsäurechlorid werden in 2,5 mL Ethylacetat suspendiert und dann zu der gekühlten Reaktionslösung hinzugegeben. Das Reaktionsgemisch wird vier Stunden auf 50 °C erhitzt und anschließend 16 Stunden bei Raumtemperatur gerührt. Die Reaktionslösung wird mit 10,0 mL Ethylacetat verdünnt. Die organische Phase wird dreimal mit 1 M Salzsäure, zweimal mit 1 M Natronlauge und einmal mit gesättigter Natriumchloridlösung gewaschen. Die organische Phase wird über Natriumsulfat getrocknet, filtriert und Lösungsmittel am Rotationsverdampfer unter vermindertem Druck entfernt. Man erhält 151 mg *N*-[4-Chlor-3-(cyclopropylcarbamoyl)phenyl]-1-methyl-3-(pentafluorethyl)-4-(trifluormethyl)-1*H*-pyrazol-5-carbonsäureamid (97%) als weißen Feststoff.

¹H-NMR (400 MHz, d₃-Acetonitril): δ = 9,35 (br. s, 1H), 7,71 (d, 1H), 7,65 (dd, 1H), 7,45 (d, 1H), 6,94 (br. s, 1H), 3,98 (s, 3H), 2,82 (m, 1H), 0,76 (m, 2H), 0,58 (m, 2H) ppm.

HPLC-MS^{a)} : logP = 3,34; Masse (m/z) = 505 [M+H]⁺.

### Beispiel (Ib-27) N-[4-Chlor-3-(cyclopropylcarbamoyl)phenyl]-4-(difluormethyl)-2-(pentafluorethyl)pyrimidin-5-carboxamid

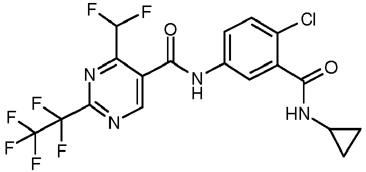

In 10 mL Dichlormethan werden 467 mg 4-(Difluormethyl)-2-(pentafluorethyl)pyrimidin-5-carbonsäure mit zwei Tropfen *N,N*-Dimethylformamid versetzt und auf 0 °C gekühlt. Nach tropfenweiser Zugabe von 167 µL Oxalsaeuredichlorid wird das Reaktionsgemisch drei Stunden bei Raumtemperatur gerührt und anschließend eingeengt. Man erhält das Rohprodukt 4-(Difluormethyl)-2-(pentafluorethyl)pyrimidin-5-carbonylchlorid.

In 2 mL Ethylacetat werden 63 mg (0,3 mmol) 5-Amino-2-chlor-*N*-cyclopropyl-benzamid, 3,1 mg (0,025 mmol) *N,N*-Dimethylpyridin-4-amin gelöst. Die Lösung wird mit einem Eisbad auf 0 °C gekühlt und mit 96,9 mg (750 µmol) *N*-Ethyl-diisopropylamin versetzt. 77,6 mg (0.25 mmol) 4-(Difluormethyl)-2-(pentafluorethyl)pyrimidin-5-carbonylchlorid (wird als Rohprodukt eingesetzt) werden in 2 mL absolutem Ethylacetat gelöst bzw. suspendiert und dann zu der gekühlten Reaktionslösung hinzugegeben. Das Reaktionsgemisch wird vier Stunden auf 50 °C erhitzt und anschließend 16 Stunden bei Raumtemperatur weitergerührt. Die Reaktionslösung wird mit 8 mL Ethylacetat verdünnt. Die organische Phase wird zweimal mit 1 M Salzsäure, einmal mit 1 M Natronlauge und einmal mit gesättigter Natriumchloridlösung gewaschen. Die organische Phase wird über Natriumsulfat getrocknet, filtriert und Lösungsmittel am Rotationsverdampfer unter vermindertem Druck entfernt. Man erhält 112 mg *N*-[4-Chlor-3-(cyclopropylcarbamoyl)phenyl]-4-(difluormethyl)-2-(pentafluorethyl)pyrimidin-5-carboxamid (92%) als weißen Feststoff.

¹H-NMR (400 MHz, d₆-DMSO): δ = 10,91 (br. s, 1H), 9,56 (s, 1H), 8,33 (m, 1H), 7,70 (s, 1H), 7,67 (dd, 1H), 7,33 (t, 1H), 2,84 (m, 1H), 0,70 (m, 2H), 0,53 (m, 2H) ppm.

HPLC-MS^{a)}: logP = 2,92 Masse (m/z) = 485 [M+H]⁺.

### Erfindungsgemäßes Beispiel (Ik-124) N-{4-Chlor-3-[(2,2,2-trifluorethyl)carbamoyl]phenyl}-3-(pentafluorethyl)-4-(trifluormethyl)-1H-pyrazol-5-carboxamid

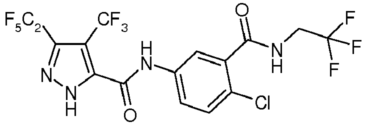

250 mg (0,84 mmol) 3-(Pentafluorethyl)-4-(trifluormethyl)-1*H*-pyrazol-5-carbonsäure werden in 5,0 mL Dichlormethan suspendiert und nacheinander mit einem Tropfen *N,N*-Dimethylformamid und 219 µL (2,52 mmol) Oxalylchlorid versetzt. Das Reaktionsgemisch wird 30 Minuten bei Raumtemperatur und 30 Minuten unter Rückfluß gerührt. Das Lösungsmittel wird unter vermindertem Druck am Rotationsverdampfer entfernt. Das entstandene Produkt 3-(Pentafluorethyl)-4-(trifluormethyl)-1*H*-pyrazol-5-carbonylchlorid wird ohne weitere Aufarbeitung für den nachfolgenden Syntheseschritt eingesetzt.

Eine Suspension von 212 mg (0,84 mmol) 5-Amino-2-chlor-*N*-(2,2,2-trifluorethyl)benzamid und 168 mg (1,26 mmol) Silber(I)cyanid in 5,0 mL Dichlormethan wird mit einer Lösung 265 mg (0,84 mmol) 3-(Pentafluorethyl)-4-(trifluormethyl)-1*H*-pyrazol-5-carbonylchlorid in 3,0 mL Dichlormethan versetzt und 16 h bei Raumtemperatur gerührt. Die Suspension wird anschließend über Silicagel filtriert und das Produkt mit einem Gemisch aus Dichlormethan und Methanol (1:1) eluiert. Die Lösungsmittel werden unter vermindertem Druck am Rotationsverdampfer entfernt und das erhaltene Rohprodukt in Ethylacetat aufgenommen. Die organische Phase wird nacheinander zweimal mit 1 M Salzsäure und einmal mit ges. Natriumchloridlösung gewaschen. Anschließend wird die organische Phase über Magnesiumsulfat getrocknet, filtriert und am Rotationsverdampfer unter vermindertem Druck eingeengt. Das Rohprodukt wird dann mit Gemisch aus Dichlormethan, Methyl-*tert*-butylether und Cyclohexan (1:1:1) trituriert. Das aus der Mutterlauge erhaltene Rohprodukt wird anschließend mittels präparativer HPLC aufgreinigt. Auf diese Weise wurden 49 mg *N*-{4-Chlor-3-[(2,2,2-trifluorethyl)carbamoyl]phenyl}-3-(pentafluorethyl)-4-(trifluormethyl)-1*H*-pyrazol-5-carboxamid (11%) als farbloser Feststoff erhalten.

¹H-NMR (400 MHz, d₃-Acetonitril): δ = 9,09 (br. s, 1H), 7,78 (d, 1H), 7,68 (dd, 1H), 7,48 (d, 1H), 7,23 (br. s, 1H), 4,03-4,12 (m, 2H) ppm.

HPLC-MS^{a)}: logP = 3,26 Masse (m/z) = 533 [M+H]⁺.

### Erfindungsgemäßes Beispiel (Ik-125) N-{4-Chlor-3-[(2,2,2-trifluorethyl)carbamoyl]phenyl}-1-methyl-3,4-bis(trifluormethyl)-1H-pyrazol-5-carboxamid

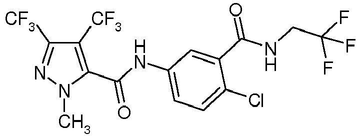

50 mg (0,19 mmol) 1-Methyl-3,4-bis(trifluormethyl)-1*H*-pyrazol-5-carbonsäure werden in 5,0 mL Dichlormethan suspendiert und nacheinander mit einem Tropfen *N,N*-Dimethylformamid und 50 µL (2,52 mmol) Oxalylchlorid versetzt. Das Reaktionsgemisch wird 30 Minuten bei Raumtemperatur und 30 Minuten unter Rückfluß gerührt. Das Lösungsmittel wird unter vermindertem Druck am Rotationsverdampfer entfernt. Das entstandene Produkt 1-Methyl-3,4-bis(trifluormethyl)-1*H*-pyrazol-5-carbonylchlorid wird ohne weitere Aufarbeitung für den nachfolgenden Syntheseschritt eingesetzt.

Eine Suspension von 53 mg (0,21 mmol) 5-Amino-2-chlor-*N*-(2,2,2-trifluorethyl)benzamid und 38 mg (0,29 mmol) Silber(I)cyanid in 5,0 mL Dichlormethan wird mit einer Lösung 53 mg (0,19 mmol) 3-(Pentafluorethyl)-4-(trifluormethyl)-1*H*-pyrazol-5-carbonylchlorid in 3,0 mL Dichlormethan versetzt und 16 h bei Raumtemperatur gerührt. Die Suspension wird anschließend über Silicagel filtriert und das Produkt mit einem Gemisch aus Cyclohexan und Ethylacetat (1:1) eluiert. Die organische Phase wird nacheinander dreimal mit 1 M Salzsäure, zweimal mit 1 M Natronlauge und einmal mit ges. Natriumchloridlösung gewaschen. Anschließend wird die organische Phase über Magnesiumsulfat getrocknet, filtriert und am Rotationsverdampfer unter vermindertem Druck eingeengt. Das Rohprodukt wird dann mit Cyclohexan trituriert, das Cyclohexan dekantiert und das Produkt am Vakuum getrocknet Auf diese Weise wurden 63 mg *N-*{4-Chlor-3-[(2,2,2-trifluorethyl)carbamoyl]phenyl}-1-methyl-3,4-bis(trifluormethyl)-1*H*-pyrazol-5-carboxamid (66%) als beiger Feststoff erhalten.

¹H-NMR (400 MHz, d₃-Acetonitril): δ = 9,10 (br. s, 1H), 7,70 (d, 1H), 7,66 (dd, 1H), 7,49 (d, 1H), 7,23 (br. s, 1H), 4,02-4,14 (m, 2H), 3,98 (s, 3H) ppm.

HPLC-MS^{a)}: logP = 3,25 Masse (m/z) = 497 [M+H]⁺.

### Erfindungsgemäßes Beispiel (Ik-233) N-[4-Chlor-3-(cyclopropylcarbamoyl)phenyl]-3-[cyclopropyl(fluor)methyl]-1-methyl-4-(trifluormethyl)-1H-pyrazol-5-carboxamid

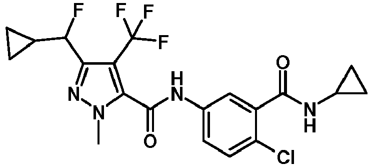

Zu einer Lösung aus 410 mg (1,55 mmol) 3-[Cyclopropyl(hydroxy)methyl]-1-methyl-4-(trifluormethyl)-1*H*-pyrazol-5-carbonsäure und 327 mg (1,55 mmol) 5-Amino-2-chlor-*N-*cyclopropylbenzamid in Tetrahydrofuran (10 ml) gibt man 515 mg (1,86 mmol) 4-(4,6-Dimethoxy-1,3,5-triazin-2-yl)-4-methylmorpholiniumchlorid und rührt sechs Stunden bei Raumtemperatur. Das Reaktionsgemisch wird eingeengt, mit Wasser versetzt, mit Dichlormethan extrahiert und die vereinigten organischen Phasen über Natriumsulfat getrocknet. Nach Einengen im Vakuum wird der Feststoff mit Dichlormethan gewaschen und man erhält 308 mg (N-[4-Chlor-3-(cyclopropylcarbamoyl)phenyl]-3-[cyclopropyl(hydroxy)methyl]-1-methyl-4-(trifluormethyl)-1*H-*pyrazol-5-carboxamid (44%).
¹H-NMR (400 MHz, d₆-DMSO): δ = 11,10 (s, 1H), 8,30 (d, 1H), 7,67 (m, 2H), 7,45 (d, 1H), 5,12 (br. s, 1H), 4,02 (br. d, 1H), 3,84 (s, 3H), 2,83 (m, 1H), 1,36 (m, 1H), 0,77 (m, 2H), 0,51 (m, 3H), 0,44 (m, 2H), 0,20 (m, 1H) ppm.

HPLC-MS^{a)}: logP = 2.00; Masse (m/z) 439 [M-H₂O+H]⁺.

Ausgehend von *N*-[4-Chlor-3-(cyclopropylcarbamoyl)phenyl]-3-[cyclopropyl(hydroxy)methyl]-1-methyl-4-(trifluormethyl)-1*H*-pyrazol-5-carboxamid wird analog zur Herstellung von Ethyl-3-(2-fluorpropan-2-yl)-1-methyl-4-(trifluormethyl)-1*H*-pyrazol-5-carboxylat *N*-[4-Chlor-3-(cyclopropylcarbamoyl)phenyl]-3-[cyclopropyl(fluor)methyl]-1-methyl-4-(trifluormethyl)-1H-pyrazol-5-carboxamid hergestellt.

¹H-NMR (400 MHz, d₆-DMSO): δ = 11,10 (s, 1H), 8,34 (br. d, 1H), 7,66-7,68 (m, 2H), 7,47 (d, 1H), 4,87 (dd, 1H), 2,80-2,86 (m, 1H), 1,60-1,65 (m, 1H), 0,67-0,76 (m, 5H), 0,51-0,55 (m, 2H), 0,34-0,39 (m, 1H) ppm.

HPLC-MS^{a)}: logP = 2,76; Masse (m/z) 439 [M-F+H]⁺.

### Darstellungsverfahren B

### Erfindungsgemäßes Beispiel (Ik-2) 2-Chlor-5-({[1-methyl-3-(pentafluorethyl)-4-(trifluormethyl)-1H-pyrazol-5-yl]carbonyl}amino)benzoesäure

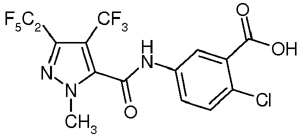

6,74 g (36,3 mmol) Methyl-5-amino-2-chlorbenzoat, 0,22 g (1,8 mmol) 4*-N,N-*Dimethylaminopyridin und 9,49 mL (54,4 mmol) *N*-Ethyl-diisopropylamin werden in 50,0 mL Ethylacetat gelöst und auf 0 °C gekühlt. Zu dieser Reaktionsmischung wird eine Lösung aus 6,0 g (18,1 mmol) 1-Methyl-3-(pentafluorethyl)-4-(trifluormethyl)-1*H*-pyrazol-5-carbonsäurechlorid in 100 mL Ethylacetat innerhalb einer Stunde hinzugefügt. Nach Beendigung der Zugabe wird die Reaktionsmischung 16 Stunden bei Raumtemperatur gerührt. Die Reaktionsmischung wird mit 250 mL Ethylacetat verdünnt und die organische Phase anschließend dreimal mit je 100 mL 1 M Salzsäure, dreimal mit 1 M Natronlauge und einmal mit gesättigter Natriumchloridlösung gewaschen. Man erhält 8,0 g eines Gemisches von Methyl-2-chlor-5-({[1-methyl-3-(pentafluorethyl)-4-(trifluormethyl)-1*H*-pyrazol-5-yl]carbonyl}amino)benzoat und Methyl-5-(bis{[1-methyl-3-(pentafluorethyl)-4-(trifluormethyl)-1*H*-pyrazol-5-yl]carbonyl}amino)-2-chlorbenzoat im Verhältnis 6:4.

### Methyl-2-chlor-5-({[1-methyl-3-(pentafluorethyl)-4-(trifluormethyl)-1H-pyrazol-5-yl]carbonyl}amino)benzoat:

HPLC-MS^{a)}: logP = 4,05; Masse (m/z) = 480 [M+H]⁺.

### Methyl-5-(bis{[1-methyl-3-(pentafluorethyl)-4-(trifluormethyl)-1H-pyrazol-5-yl]carbonyl}amino)-2-chlorbenzoat:

HPLC-MS^{a)}: logP = 5,85; Masse (m/z) = 790 [M-H+H₂O]⁻.

8.0 g des Produktgemisches aus der Darstellung von Methyl-2-chlor-5-({[1-methyl-3-(pentafluorethyl)-4-(trifluormethyl)-1*H*-pyrazol-5-yl]carbonyl}amino)benzoat werden in 150 mL Methanol suspendiert und dann mit 15,6 mL 2 M Natronlauge versetzt. Das Reaktionsgemisch wird 16 Stunden bei Raumtemperatur gerührt und dann langsam in eine Mischung aus 150 mL 1 M Salzsäure und 250 mL Eis getropft. Nach Beendigung der Zugabe wird noch eine Stunde nachgerührt. Der sich bildende weiße Niederschlag wird abfiltriert und mit kaltem Wasser gewaschen. Nach der Trocknung des Feststoffs im Ölpumpenvakuum, wird dieser in 50 mL warmen Ethylacetat suspendiert und anschließend durch die langsame Zugabe von 500 mL Cyclohexan wieder ausgefällt. Der Niederschlag wird abfiltriert und getrocknet. Man erhält 4,55 g 2-Chlor-5-({[1-methyl-3-(pentafluorethyl)-4-(trifluormethyl)-1*H*-pyrazol-5-yl]carbonyl}amino)benzoesäure (54%) als weißen Feststoff.

¹H-NMR (400 MHz, d₃-Acetonitril): δ = 9,17 (br. s, 1H), 8,11 (d, 1H), 7,73 (dd, 1H), 7,52 (d, 1H), 3,98 (s, 3H) ppm.

HPLC-MS^{a)}: logP = 3,22; Masse (m/z) = 466 [M+H]⁺.

### Erfindungsgemäßes Beispiel (Ik-3) N-[4-Chlor-3-(prop-2-in-1-ylcarbamoyl)phenyl]-1-methyl-3-(pentafluorethyl)-4-(trifluormethyl)-1H-pyrazol-5-carboxamid

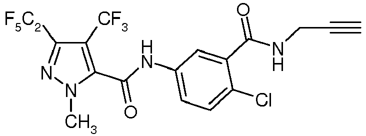

70 mg (0,15 mmol) 2-Chlor-5-({[1-methyl-3-(pentafluorethyl)-4-(trifluormethyl)-1*H*-pyrazol-5-yl]carbonyl}amino)benzoesäure werden in 2,0 mL Dichlormethan suspendiert und anschließend nacheinander mit 20,3 mg (0,15 mmol) 1-Hydroxybenztriazol, 28,8 mg (0,20 mmol) N-(3-Dimethylaminopropyl)-*N*'-ethyl-carbodiimid-hydrochlorid und 52,3 µL (0,30 mmol) N-Ethyl-diisopropylamin versetzt. Das Reaktionsgemisch wird 20 Minuten bei Raumtemperatur gerührt und anschließend mit 13,4 µL (0,20 mmol) Propargylamin versetzt. Das Reaktionsgemisch wird über Nacht bei Raumtemperatur nachgerührt und dann am Rotationsverdampfer unter vermindertem Druck eingeengt. Das Rohprodukt wird anschließend mittels präparativer HPLC (C18, Saphir 110, 5 µm, 20x125mm; Gradient: 0-1,5 min 94% Wasser, 5% Acetonitril, 1% Ameisensäure, 1,5-6,0 min linearer Gradient auf 4% Wasser, 95% Acetonitril, 1% Ameisensäure, 6,0-14,0 min 4% Wasser, 95% Acetonitril, 1% Ameisensäure) aufgereinigt. Man erhält 57 mg *N*-[4-Chlor-3-(prop-2-in-1-ylcarbamoyl)phenyl]-1-methyl-3-(pentafluorethyl)-4-(trifluormethyl)-1*H*-pyrazol-5-carboxamid (75%) als weißen Feststoff.

HPLC-MS^{b)}: logP = 3,11; Masse (m/z) = 503 [M+H]⁺.

### Erfindungsgemäßes Beispiel (Ik-127) N-{3-Brom-[2-(1R,2S)-2-fluor-cycloprop-1-yl carbamoyl]pyrid-6-yl}-1-methyl-3-(pentafluorethyl)-4-(trifluormethyl)-1H-pyrazol-5-carboxamid

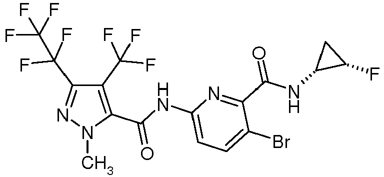

Zu einer Lösung aus 369 mg (1,59 mmol) Methyl-6-amino-3-brompyridin-2-carboxylat (vgl. WO 2008/084717) in 13,4 mL Dichlormethan werden 528 mg (1,59 mmol) 1-Methyl-3-(pentafluorethyl)-4-(trifluormethyl)-1*H*-pyrazol-5-carbonylchlorid und eine Suspension aus 214 mg Silber(I)-cyanid in 13,4 mL Acetonitril gegeben. Anschließend wird das Reaktionsgemisch 24 Stunden bei Raumtemperatur gerührt. Danach wird die Reaktionsmischung filtriert und das Lösungsmittelgemisch im Vakuum abgezogen. Der verbleibende Rückstand wird mittels Säulenchromatographie an Kieselgel mit dem Laufmittelgemisch Cyclohexan:Aceton (Gradient) gereinigt. Man erhält 302 mg Methyl-3-brom-6-({[1-methyl-3-(pentafluorethyl)-4-(trifluormethyl)-1*H*-pyrazol-5-yl]carbonyl}amino)pyridin-2-carboxylat (34%).

HPLC-MS^{a)}: logP = 4,06; Masse (m/z) = 527 [M+H]⁺.

425 mg (0,80 mmol) Methyl-3-brom-6-({[1-methyl-3-(pentafluorethyl)-4-(trifluormethyl)-1*H-*pyrazol-5-yl]carbonyl}amino)pyridin-2-carboxylat werden in 13,6 mL Methanol suspendiert und dann mit 0,60 mL 2 M Natronlauge versetzt. Das Reaktionsgemisch wird 4 Stunden bei 50 °C gerührt. Danach wird der Reaktionsansatz in Ethylacetat aufgenommen und einmal gegen 1 M Salzsäure geschüttelt. Die organische Phase wird abgetrennt, getrocknet und im Vakuum eingeengt. Man erhält 360 mg 3-Brom-6-({[1-methyl-3-(pentafluorethyl)-4-(trifluormethyl)-1*H*-pyrazol-5-yl]carbonyl}-amino)pyridin-2-carbonsäure (65%; Reinheit: 75%ig), die ohne weitere Reinigung für Folgereaktionen verwendet werden kann.

HPLC-MS^{a)}: logP = 2,78; Masse (m/z) = 513 [M+H]⁺.

400 mg (783 µmol) 3-Brom-6-({[1-methyl-3-(pentafluorethyl)-4-(trifluormethyl)-1*H*-pyrazol-5-yl]carbonyl}-amino)pyridin-2-carbonsäure werden in 36 mL Dichlormethan suspendiert und anschließend nacheinander mit 193 mg (783 µmol) (1*R*,2*S*)-2-Fluorcyclopropanaminium-4-methylbenzolsulfonat, 387 mg (1,02 mmol) 2-(7-Aza-1*H*-benzotriazole-1-yl)-1,1,3,3-tetramethyluronium hexafluorophosphat (HATU) und 388 µL (2,35 mmol) N-Ethyl-diisopropylamin versetzt. Das Reaktionsgemisch wird 30 Stunden bei Raumtemperatur gerührt. Zur Aufarbeitung wird der Reaktionsansatz nacheinander mit 1 M Salzsäure und Natriumhydrogencarbonat-Lösung gewaschen. Die organische Phase wird abgetrennt, über Natriumsulfat getrocknet und im Vakuum eingeengt. Der verbleibende Rückstand wird mittels Säulenchromatographie an Kieselgel mit dem Laufmittelgemisch Cyclohexan:Aceton (Gradient) gereinigt. Man erhält 228 mg *N*-{3-Brom-[2-(1*R*,2*S*)-2-fluor-cycloprop-1-yl-carbamoyl]pyrid-6-yl}-1-methyl-3-(pentafluorethyl)-4-(trifluormethyl)-1*H*-pyrazol-5-carboxamid (51%).

¹H-NMR (400 MHz, d₆-DMSO): δ = 8,40 (d, 1H), 7,85 (d, 1H), 4,78 (m, 1H), 4,11 (s, 3H), 3,18 (br. s, 1H), 1,15-1,21 (m, 2H) ppm.

¹³C-NMR (600 MHz, d₆-DMSO; 328K): δ = 147,5; 143,9; 140,0; 121,5; 118,8; 108,8; 82,1; 69,8; 53,4; 38,9; 25,0; 11,4 ppm.

HPLC-MS^{a)}: logP = 3,29; Masse (m/z) = 568, 570 [M+1]⁺.

### Erfindungsgemäßes Beispiel (Ik-128) 5-Brom-N-[(1R,2S)-2-fluorcyclopropyl-2-({[1-methyl-3-(pentafluorethyl)-4-(trifluormethyl)-1H-pyrazol-5-yl]carbonyl}amino)isonicotinamid

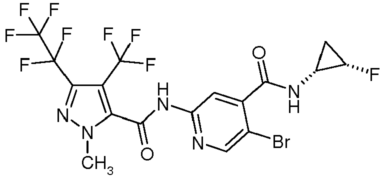

Die Reaktion mit Methyl-2-amino-5-bromisonicotinat (vgl. WO 2006/020830; Bromierung von Methyl-2-aminoisonicotinat) erfolgt analog der Reaktionsvorschrift des Beispiels **Ik-127** unter Verwendung von:
738 mg (3,19 mmol) Methyl-2-amino-5-bromisonicotinat in
26,8 mL Dichlormethan,
1056 mg (3,19 mmol) 1-Methyl-3-(pentafluorethyl)-4-(trifluormethyl)-1*H*-pyrazol-5-carbonylchlorid,
428 mg Silber(I)-cyanid in
26,8 mL Acetonitril.

Der verbleibende Rückstand wird mittels Säulenchromatographie an Kieselgel mit dem Laufmittelgemisch Cyclohexan:Aceton (Gradient) gereinigt. Man erhält 250 mg Methyl-5-brom-2-({[1-methyl-3-(pentafluorethyl)-4-(trifluormethyl)-1*H*-pyrazol-5-yl]carbonyl}amino)isonicotinat (15%).

HPLC-MS^{a)}: logP = 4,04; Masse (m/z) = 527 [M+H]⁺.

Die nachfolgende Esterspaltung erfolgt analog der Reaktionsvorschrift des Beispiels **(Ik-127)** unter Verwendung von:
335 mg (0,82 mmol) Methyl-5-brom-2-({[1-methyl-3-(pentafluorethyl)-4-(trifluormethyl)-1*H-*pyrazol-5-yl]carbonyl}amino)isonicotinat,
15,2 mL Methanol,
1,24 mL 2 M Natronlauge.

Man erhält 380 mg 5-Brom-2-({[1-methyl-3-(pentafluorethyl)-4-(trifluormethyl)-1*H*-pyrazol-5-yl]carbonyl}amino)isonicotinsäure (85%), die ohne weitere Reinigung für Folgereaktionen verwendet werden kann.

HPLC-MS^{a)}: logP = 2,85; Masse (m/z) = 513 [M+H]⁺.

Die Reaktion der 5-Brom-2-({[1-methyl-3-(pentafluorethyl)-4-(trifluormethyl)-1*H*-pyrazol-5-yl]carbonyl}amino)isonicotinsäure erfolgt analog der Reaktionsvorschrift des Beispiels **(Ik-127)** unter Verwendung von:
100 mg (196 µmol) 5-Brom-2-({[1-methyl-3-(pentafluorethyl)-4-(trifluormethyl)-1*H*-pyrazol-5-yl]carbonyl}amino)isonicotinsäure, 9,0 mL Dichlormethan, 48,3 mg (196 µmol) (1*R*,2*S*)-2-Fluorcyclopropanaminium-4-methylbenzolsulfonat, 96,6 mg (254 µmol) HATU, 0,097 mL (587 µmol) Hünig's Base.

Der verbleibende Rückstand wird zunächst mittels Säulenchromatographie an Kieselgel mit dem Laufmittelgemisch Cyclohexan:Aceton (Gradient) gereinigt. Man erhält 89,4 mg 5-Brom-*N-*[(1*R*,2*S*)-2-fluorcyclopropyl]-2-({[1-methyl-3-(pentafluorethyl)-4-(trifluormethyl)-1*H*-pyrazol-5-yl]carbonyl}amino)isonicotinamid (80%).

¹H-NMR (600 MHz, d₃-Acetonitril): δ = 9,65 (br. s, 1H), 8,52 (d, 1H), 8,22 (d, 1H), 4,75 (m, 1H), 3,97 (s, 3H), 2,88 (br. s, 1H), 1,01-1,22 (m, 2H) ppm.

¹³C-NMR (600 MHz, d₃-Acetonitril): δ = 157,5; 151,8; 150,7; 148,3; 137,1; 114,2; 113,4; 111,2; 70,8; 39,6; 26,4; 12,2 ppm.

HPLC-MS^{a)}: logP = 3,19; Masse (m/z) = 568, 570 [M+1]⁺.

### Erfindungsgemäßes Beispiel (Ik-129) 5-Chlor-2-({[1-methyl-3-(pentafluorethyl)-4-(trifluormethyl)-1H-pyrazol-5-yl]carbonyl}amino)isonicotinsäure

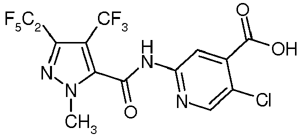

1,50 g (9,85 mmol) Methyl-2-aminoisonicotinat (kommerziell erhältlich) werden in 15 mL Dimethylformamid verrührt und bei einer Temperatur von -18 °C prortionsweise mit 1,83 g (13,8 mol) *N*-Chlorsuccinimid versetzt. Danach wir noch eine Stunde bei -18 °C weitergerührt. Zur Aufarbeitung wird der gesamte Reaktionsansatz in Ethylacetat aufgenommen und gegen eine wässrige Natriumthiosulfat-Lösung geschüttelt. Anschließend wird die organische Phase mit Wasser gewaschen, über Natriumsulfat getrocknet und im Vakuum eingeengt. Der verbleibende Rückstand wird mittels Säulenchromatographie an Kieselgel mit dem Laufmittelgemisch Cyclohexan:Aceton (Gradient) gereinigt. Man erhält 383mg Methyl-2-amino-5-chlorisonicotinat (16%).

¹H-NMR (400 MHz, d₆-DMSO): δ = 8,00 (s, 1H), 6,78 (s, 1H), 6,25 (br. s, 2H), 3,85 (s, 3H) ppm.

HPLC-MS^{a)}: logP = 1,30; Masse (m/z) = 187 [M+H]⁺.

Darüber hinaus wurden auch 500 mg Methyl-2-amino-3,5-dichlorisonicotinat (23%) als Nebenprodukt isoliert.

¹H-NMR (400 MHz, d₆-DMSO): δ = 8,04 (s, 1H), 6,62 (br. s, 2H), 3,92 (s, 3H) ppm.

HPLC-MS^{a)}: logP = 1,75; Masse (m/z) = 221 [M+1]⁺.

Die Reaktion mit Methyl-2-amino-5-chlorisonicotinat erfolgt analog der Reaktionsvorschrift des Beispiels (**Ik**-**127**) unter Verwendung von:
601 mg (3,21 mmol) Methyl-2-amino-5-chlorisonicotinat in
28,0 mL Dichlormethan,
1064 mg (3,21 mmol) 1-Methyl-3-(pentafluorethyl)-4-(trifluormethyl)-1*H*-pyrazol-5-carbonyl-chlorid,
431 mg Silber(I)-cyanid in 28,0 mL Acetonitril.

Der verbleibende Rückstand wird mittels Säulenchromatographie an Kieselgel mit dem Laufmittelgemisch Cyclohexan:Aceton (Gradient) gereinigt. Man erhält 563 mg Methyl-5-chlor-2-({[1-methyl-3-(pentafluorethyl)-4-(trifluormethyl)-1*H*-pyrazol-5-yl]carbonyl}amino)isonicotinat (36%).

Die nachfolgende Esterspaltung erfolgt analog der Reaktionsvorschrift des Beispiels **(Ik-127)** unter Verwendung von:
563 mg (1,17 mmol) Methyl-5-chlor-2-({[1-methyl-3-(pentafluorethyl)-4-(trifluormethyl)-1*H-*pyrazol-5-yl]carbonyl}amino)isonicotinat,
15,3 mL Methanol,
1,57 mL 2 M Natronlauge.

Man erhält 542 mg 5-Chlor-2-({[1-methyl-3-(pentafluorethyl)-4-(trifluormethyl)-1*H*-pyrazol-5-yl]carbonyl}amino)isonicotinsäure (97%; Reinheit: 98%ig), die ohne weitere Reinigung für Folgereaktionen verwendet werden kann.

HPLC-MS^{a)}: logP = 2,82; Masse (m/z) = 467 [M+1]⁺.

### Erfindungsgemäßes Beispiel (Ik-130) N-[2-(2,2-Difluor-cycloprop-1-yl-carbamoyl)pyrid-4-yl]-1-methyl-3-(pentafluorethyl)-4-(trifluormethyl)-1H-pyrazol-5-carboxamid

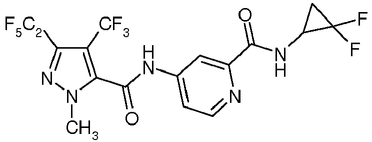

Die Reaktion mit Methyl-4-aminopyridin-2-carboxylat (vgl. WO 2001/074788) erfolgt analog der Reaktionsvorschrift des Beispiels **(Ik-127)** unter Verwendung von:
486 mg (3,19 mmol) Methyl-4-aminopyridin-2-carboxylat in
26,8 mL Dichlormethan,
1056 mg (3,19 mmol) 1-Methyl-3-(pentafluorethyl)-4-(trifluormethyl)-1*H*-pyrazol-5-carbonylchlorid,
428 mg Silber(I)-cyanid in
26,8 mL Acetonitril.

Der verbleibende Rückstand wird mittels Säulenchromatographie an Kieselgel mit dem Laufmittelgemisch Cyclohexan:Aceton (Gradient) gereinigt. Man erhält 984 mg Methyl-4-({[1-methyl-3-(pentafluorethyl)-4-(trifluormethyl)-1*H*-pyrazol-5-yl]carbonyl}amino)pyridin-2-carboxylat (69%).

¹H-NMR (400 MHz, d₆-DMSO): δ = 8,67 (d, 1H), 8,31 (s, 1H), 7,80 (d, 1H), 4,03 (s, 3H), 3,90 (s, 3H) ppm.

HPLC-MS ^{a)}: logP = 3,01; Masse (m/z) = 447 [M+H]⁺.

Die nachfolgende Esterspaltung erfolgt analog der Reaktionsvorschrift des Beispiels (**Ik**-**127**) unter Verwendung von:
900 mg (2,01 mmol) Methyl-4-({[1-methyl-3-(pentafluorethyl)-4-(trifluormethyl)-1*H*-pyrazol-5-yl]carbonyl}amino)pyridin-2-carboxylat,
25,0 mL Methanol,
1,51 mL 2 M Natronlauge.

Man erhält 807 mg 4-({[1-Methyl-3-(pentafluorethyl)-4-(trifluormethyl)-1*H*-pyrazol-5-yl]carbonyl}-amino)pyridin-2-carbonsäure (58%; Reinheit: 63%), die für Folgereaktionen verwendet werden kann.

HPLC-MS ^{a)}: logP = 2,06; Masse (m/z) = 433 [M+H]⁺.

Die Reaktion der 4-({[1-Methyl-3-(pentafluorethyl)-4-(trifluormethyl)-1*H*-pyrazol-5-yl]carbonyl}-amino)pyridin-2-carbonsäure erfolgt analog der Reaktionsvorschrift des Beispiels **(Ik-127)** unter Verwendung von:
150 mg (347 µmol) 4-({[1-methyl-3-(pentafluorethyl)-4-(trifluormethyl)-1*H*-pyrazol-5-yl]carbonyl}-amino)pyridin-2-carbonsäure,
13.5 mL Dichlormethan,
85,8 mg (1.50 mmol) 2,2-Difluor-cyclopropylamin,
171,4 mg (451 µmol) HATU,
172 µL (587 µmol) Hünig's Base.

Der verbleibende Rückstand wird zunächst mittels Säulenchromatographie an Kieselgel mit dem Laufmittelgemisch Cyclohexan:Aceton (Gradient) und anschließend mittels präparativer HPLC mit dem Laufmittelgemisch Aceton:Wasser (neutral) gereinigt. Man erhält 68 mg *N*-[2-(2,2-Difluorcycloprop-1-yl-carbamoyl)pyrid-4-yl]-1-methyl-3-(pentafluorethyl)-4-(trifluormethyl)-1*H*-pyrazol-5-carboxamid (38%).

¹H-NMR (600 MHz, d₃-Acetonitril): δ = 9,55 (br. s, 1H), 8,31 (br. s, 1H), 8,56 (d, 1H), 8,27 (d, 1H), 7,81 (dd, 1H), 3,99 (s, 3H), 3,47 (br. s, 1H), 1,64-1,93 (m, 2H) ppm.

¹³C-NMR (600 MHz, d₃-Acetonitril): δ = 166,0; 157,8; 151,6; 150,9; 146,6; 140,4; 137,2; 121,8; 119,6; 117,2; 112,8; 112,7; 111,2; 109,6; 39,8; 31,3; 18,1 ppm.

HPLC-MS ^{a)}: logP = 3,59; Masse (m/z) = 508 [M+H]⁺.

### Erfindungsgemäßes Beispiel (Ik-131) 2-Chlor-5-({[-methyl-3-(pentafluorethyl)-4-(trifluormethyl)-1H-pyrazol-5 -yl]carbonyl}amino)nicotinsäure

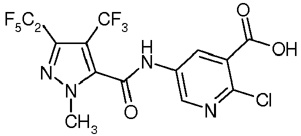

Die Reaktion mit Methyl-5-amino-2-chlornicotinat (vgl. WO 2006/050506) erfolgt analog der Reaktionsvorschrift des Beispiels **(Ik-127)** unter Verwendung von:
601 mg (3,21 mmol) Methyl-5-amino-2-chlornicotinat in
28,0 mL Dichlormethan,
1,06 g (3,19 mmol) 1-Methyl-3-(pentafluorethyl)-4-(trifluormethyl)-1*H*-pyrazol-5-carbonylchlorid, 431 mg Silber(I)-cyanid in
28,0 mL Acetonitril.

Der verbleibende Rückstand wird mittels Säulenchromatographie an Kieselgel mit dem Laufmittelgemisch Cyclohexan:Aceton (Gradient) gereinigt. Man erhält 980 mg Methyl-2-chlor-5-({[1-methyl-3-(pentafluorethyl)-4-(trifluormethyl)-1*H*-pyrazol-5-yl]carbonyl}amino)nicotinat (62°C0).

¹H-/¹³C-Korrelation (HMQC) NMR (600 MHz, d₃-Acetonitril): δ = 8,75(s, 1H), 8,57 (s, 1H), 4,04 (s, 3H), 3,91 (s, 3H) ppm.

¹³C-NMR (600 MHz, d₃-Acetonitril): δ = 164,2; 156,3; 143,5; 143,3; 140,2; 135,5; 134,0; 131,0; 126,6; 120,8; 118,3; 110,1; 109,6; 53,4; 39,3 ppm.

HPLC-MS ^{a)}: logP = 3,70; Masse (m/z) = 481 [M+H]⁺.

Die nachfolgende Esterspaltung erfolgt analog der Reaktionsvorschrift des Beispiels **(Ik-127)** unter Verwendung von:
940 mg (1,95 mmol) Methyl-2-chlor-5-({[1-methyl-3-(pentafluorethyl)-4-(trifluormethyl)-1*H-*pyrazol-5-yl]carbonyl}amino)nicotinat,
32,8 mL Methanol,
2,93 mL 2 M Natronlauge.

Man erhält 912 mg 2-Chlor-5-({[1-methyl-3-(pentafluorethyl)-4-(trifluormethyl)-1*H*-pyrazol-5-yl]carbonyl}-amino)pyridin-3-carbonsäure (96%; Reinheit: 96%ig), die ohne weitere Reinigung für Folgereaktionen verwendet werden kann.

HPLC-MS ^{a)}: logP = 2,27; Masse (m/z) = 467 [M+H]⁺.

### Darstellungsverfahren C

### Erfindungsgemäßes Beispiel (Ik-4) N-[4-Chlor-3-(cyclopropylcarbamoyl)phenyl]-1-methyl-3-(pentafluorethyl)-4-Iod-1H-pyrazol-5-carboxamid

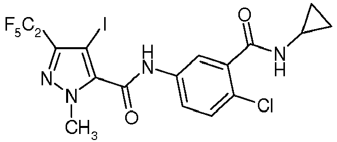

Zu einer Lösung von 1,28 g (2,34 mmol) 4-Iod-1-methyl-3-pentafluorethyl-1*H*-pyrazolcarbonsäure und 0,54 g (2,81 mmol) 1-Ethyl-3-[3-dimethylaminopropyl]carbodiimid Hydrochlorid in 20 mL Dioxan werden 0,49 g (2,34 mmol) 4-Chlor-3-(cyclopropylcarbamoyl)anilin gelöst in 0,5 mL Dioxan getropft und das Reaktionsgemisch drei Tage bei Raumtemperatur gerührt. Das Dioxan wird zum Großteil bei vermindertem Druck am Rotationsverdampfer abdestilliert und der Rückstand mit 20 mL Wasser versetzt. Die wässrige Phase wird dreimal mit Ethylacetat extrahiert und die organische Phase anschließend dreimal mit gesättigter Natriumchloridlösung gewaschen. Nach Trocknen über Natriumsulfat wird das Lösungsmittel am Rotationsverdampfer bei vermindertem Druck abdestilliert und der Rückstand mittels Flashchromatographie an Kieselgel gereinigt (Eluent: Cyclohexan/Ethylacetat). Man erhält 0,70 g *N*-[4-Chlor-3-(cyclopropylcarbamoyl)phenyl]-1-methyl-3-(pentafluorethyl)-4-Iod-1*H*-pyrazol-5-carboxamid (53%) als Öl.

¹H-NMR (400 MHz, d₃-Acetonitril): δ = 8,84 (br. s, 1H), 7,68 (d, 1H), 7,66 (dd, 1H), 7,45 (d, 1H), 6,80 (br. s, 1H), 4,04 (s, 3H), 2,85 (m, 1H), 0,91 (m, 2H), 0,77 (m, 2H) ppm.

HPLC-MS ^{a)}: logP = 3,16; Masse (m/z) = 563 [M+H]⁺.

### Erfindungsgemäßes Beispiel (Ik-5) N-[4-Chlor-3-(cyclopropylcarbamoyl)phenyl]-1-methyl-3-(pentafluorethyl)-4-ethenyl-1H-pyrazol-5-carboxamid

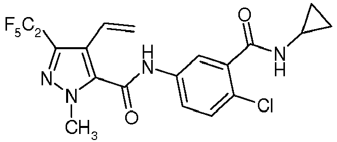

Zu einer Lösung von 180 mg (0,32 mmol) *N*-[4-Chlor-3-(cyclopropylcarbamoyl)phenyl]-1-methyl-3-(pentafluorethyl)-4-Iod-1*H*-pyrazol-5-carbonsäureamid aus Beispiel **Ik-4** in 3 mL Dimethoxyethan werden nacheinander 18,5 mg (0,016 mmol) Tetrakis(triphenylphoshin)palladium, 0,044 g (0,320 mmol) Kaliumcarbonat in 1 mL Wasser sowie 0,077 g (320 µmol) 2,4,6-Trivinylcyclotriboroxan-Pyridin-Komplex gegeben und das Reaktionsgemisch 20 Stunden unter Rückfluß erhitzt. Das Lösungsmittel wird am Rotationsverdampfer bei vermindertem Druck vollständig abdestilliert und der Rückstand mittels Flashchromatographie an Kieselgel gereinigt (Eluent: Cyclohexan/Ethylacetat; Gradient: 2 Stunden, von 0% auf 100% Ethylacetat). Man erhält 0,107 g *N*-[4-Chlor-3-(cyclopropylcarbamoyl)phenyl]-1-methyl-3-(pentafluorethyl)-4-ethenyl-1*H-*pyrazol-5-carboxamid (70%) als Öl.

¹H-NMR (400 MHz, d₃-Acetonitril): δ = 8,78 (br. s, 1H), 7,68 (d, 1H), 7,62 (dd, 1H), 7,42 (d, 1H), 6,79 6,71 (m, 2H), 5,46 (m, 1H), 5,43 (m, 1H) 3,99 (s, 3H), 2,85 (m, 1H), 0,92 (m, 2H), 0,77 (m, 2H) ppm.

HPLC-MS ^{a)}: logP = 3,11; Masse (m/z) = 463 [M+H]⁺.

### Erfindungsgemäßes Beispiel (Ik-126) N-{4-Chlor-3-[(2,2,2-trifluorethyl)carbamoyl]phenyl}-3-(difluormethoxy)-1-methyl-4-(trifluormethyl)-1H-pyrazol-5-carboxamid

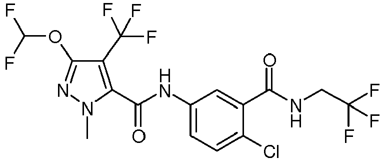

36,2 mg (0,19 mmol) Kupfer(I)iodid und 8,8 mg (0,15 mmol) Kaliumfluorid werden in einem ausgeheiztem Vial unter Argon vorgelegt und 4 mL *N,N*-Dimethylformamid und 70,0 mg (0,12 mmol) *N*-{4-Chlor-3-[(2,2,2-trifluorethyl)carbamoyl]phenyl}-3-(difluormethoxy)-4-iod-1-methyl-1*H*-pyrazol-5-carboxamid und 36,0 mg (254 µmol) Trifluormethyltrimethylsilan werden zugegeben. Das Reaktionsgemisch wird im Ultraschall entgast, mit Argon gespült und das geschlossene Vial wird für zwei Stunden auf 80 °C erhitzt. Die Reaktionslösung wird auf Wasser gegossen und mit Ethylacetat extrahiert. Die organische Phase wird über Natriumsulfat getrocknet, filtriert und am Rotationsverdampfer eingeengt. Nach chromatographischer Aufreinigung an Kieselgel werden 8,00 mg *N*-{4-Chlor-3-[(2,2,2-trifluorethyl)carbamoyl]phenyl}-3-(difluormethoxy)-1-methyl-4-(trifluormethyl)-1*H*-pyrazol-5-carboxamid (13%) erhalten.

¹H-NMR (400 MHz, d₆-DMSO): δ = 11,18 (s, 1H), 9,02 (m, 1H), 7,71 (m, 2H), 7,53 (d, 1H), 7,38 (t, 1H), 4,03 (m, 2H), 3,84 (s, 3H) ppm.

HPLC-MS ^{a)}: logP = 3,01; Masse (m/z) 495 [M+H]⁺.

### Darstellungsverfahren D

### Beispiel (Ib-2) N-[4-Chlor-3-(cyclopropylcarbamoyl)phenyl]-2-(2,2,2-trifluorethoxy)-4-trifluormethyl)pyrimidin-5-carboxamid

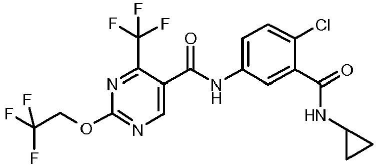

2-Chlor-4-trifluormethyl-5-pyrimidincarbonsäure und 5-Amino-2-chlor-*N*-cyclopropylbenzamid werden nach der im Herstellungsverfahren A beschriebenen Methode gekuppelt. Man erhält 2-Chlor-*N*-[4-chlor-3-(cyclopropylcarbamoyl)phenyl]-4-(trifluormethyl)pyrimidin-5-carboxamid.

¹H-NMR (400 MHz, d₆-DMSO): δ = 9,35 (s, 1H), 8,32 (d, 1H), 7,67 (m, 1H), 7,64 (s, 1H), 7,47 (dd, 1H), 2,83 (m, 1H), 0,69 (m, 2H), 0,55 (m, 2H) ppm.

HPLC-MS ^{a)}: logP = 2,36; Masse (m/z) = 419 [M+H]⁺.

In 5 mL Acetonitril werden 105 mg (250 µmol) 2-Chlor-*N*-[4-chlor-3-(cyclopropylcarbamoyl)phenyl]-4-(trifluormethyl)pyrimidin-5-carboxamid und 67,5 mg (675 µmol) Trifluorethanol gelöst. Bei -5 °C werden 64,5 mg (575 µmol) portionsweise hinzugegeben. Das Reaktionsgemisch wird über Nacht bei Raumtemperatur gerührt und dann mit 5 mL 1 M Salzsäure versetzt. Die wässrige Phase wird zweimal mit 5 mL Ethylacetat extrahiert, dann über Natriumsulfat getrocknet, filtriert und das Lösungsmittel am Rotationsverdampfer unter vermindertem Druck entfernt. Der Rückstand wird mittels Flashchromatographie an Kieselgel gereinigt (Eluent: Cyclohexan/Ethylacetat 2/1). Man erhält 60 mg *N*-[4-Chlor-3-(cyclopropylcarbamoyl)phenyl]-2-(2,2,2-trifluorethoxy)-4-trifluormethyl)pyrimidin-5-carboxamid (50%) als weißen Feststoff.

¹H-NMR (400 MHz, d₆-DMSO): δ = 10,78 (s, 1H), 9,22 (s, 1H), 8,31 (d, 1H), 7,66 (m, 2H), 7,45 (dd, 1H), 2,83 (m, 1H), 0,70 (m, 2H), 0,53 (m, 2H) ppm.

HPLC-MS ^{a)}: logP = 2,76; Masse (m/z) = 483 [M+H]⁺.

Auf gleiche Weise wurden erhalten:

### Beispiel (Ii-7) N-[4-Chlor-3-(cyclopropylcarbamoyl)Phenyl]-5-(2,2,2-trifluorethoxy)-3-(trifluormethyl)pyridin-2-carboxamid

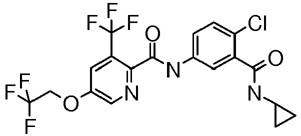

5-Chlor-3-trifluormethyl-pyridin-2-carbonsäure und 5-Amino-2-chlor-*N*-cyclopropylbenzamid werden nach der im Herstellungsverfahren A beschriebenen Methode gekuppelt. Man erhält 5-Chlor-*N*-[4-chlor-3-(cyclopropylcarbamoyl)phenyl]-3-(trifluormethyl)pyridin-2-carboxamid.

¹H-NMR (400 MHz, d₆-DMSO): δ = 10,80 (s, 1H), 8,99 (s, 1H), 8,53 (s, 1H), 8,31 (d, 1H), 7,72 (m, 2H), 7,44 (dd, 1H), 2,83 (m, 1H), 0,69 (m, 2H), 0,53 (m, 2H) ppm.

HPLC-MS ^{a)}: logP = 2,55; Masse (m/z) = 418 [M+H]⁺.

Analog zur Herstellung von **(Ib-2)** wird aus 5-Chlor-*N*-[4-chlor-3-(cyclopropylcarbamoyl)phenyl]-3-(trifluormethyl)pyridin-2-carboxamid und 2,2,2-Trifluorethanol unter Zugabe von 0,2 Äquivalenten 18-Krone-6 *N*-[4-Chlor-3-(cyclopropylcarbamoyl)phenyl]-5-(2,2,2-trifluorethoxy)-3-(trifluormethyl)pyridin-2-carboxamid dargestellt.

¹H-NMR (400 MHz, d₃-Acetonitril): δ = 9,78 (s, 1H), 8,57 (d, 1H), 7,87 (d, 1H), 7,81 (s, 1H), 7,77 (dd, 1H), 7,42 (dd, 1H), 6,90 (bs, 1H), 4,77 (q, 2H), 2,83 (m, 2H), 0,75 (m, 1H), 0,58 (m, 2H) ppm.

HPLC-MS ^{a)}: logP = 2,90; Masse (m/z) = 482 [M+H]⁺.

### Beispiel (Ij-1) N-[4-Chlor-3-(cyclopropylcarbamoyl)phenyl]-6-(2,2,2-trifluorethoxy)-4-(trifluormethyl)pyridin-3-carboxamid

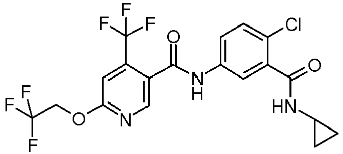

2-Chlor-4-trifluormethyl-pyridin-5-carbonsäure und 5-Amino-2-chlor-*N*-cyclopropylbenzamid werden nach der im Herstellungsverfahren A beschriebenen Methode gekuppelt. Man erhält 2-Chlor-*N*-[4-chlor-3-(cyclopropylcarbamoyl)phenyl]-4-(trifluormethyl)pyridin-5-carboxamid.

¹H-NMR (400 MHz, d₆-DMSO): δ = 10,80 (s, 1H), 8,85 (s, 1H), 8,30 (d, 1H), 8,05 (s, 1H), 7,67 (m, 2H), 7,45 (m, 1H), 2,83 (m, 1H), 0,69 (m, 2H), 0,53 (m, 2H) ppm.

HPLC-MS ^{a)}: logP = 2,35; Masse (m/z) = 418 [M+H]⁺.

Analog zur Herstellung von **(Ib-2)** wird aus 2-Chlor-*N*-[4-chlor-3-(cyclopropylcarbamoyl)phenyl]-4-(trifluormethyl)pyridin-5-carboxamid und 2,2,2-Trifluorethanol *N*-[4-Chlor-3-(cyclopropylcarbamoyl)phenyl]-5-(2,2,2-trifluorethoxy)-3-(trifluormethyl)pyridin-2-carboxamid dargestellt.

¹H-NMR (400 MHz, d₆-DMSO): δ = 8,62 (s, 1H), 8,30 (d, 1H), 7,68 (m, 2H), 7,45 (m, 2H), 5,13 (q, 2H), 2,83 (m, 1H), 0,69 (m, 2H), 0,53 (m, 2H) ppm.

HPLC-MS ^{a)}: logP = 3,02; Masse (m/z) = 482 [M+H]⁺.

### Darstellungsverfahren F

### Beispiel (Ia-1) N-[4-Chlor-3-(cyclopropylcarbamoyl)phenyl]-2-methyl-6-(pentafluorethyl)-5-(trifluormethyl)pyrimidin-4-carboxamid

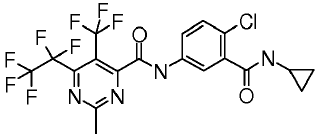

In einem ausgeheizten Kolben werden 975 mg (3,02 mmol) 2-Methyl-6-(pentafluorethyl)-5-(trifluormethyl)pyrimidin-4-carboxamid, 833 mg (6.03 mmol) Kaliumcarbonat, 287 mg (1,51 mmol) Kupferiodid und Molsieb unter Argon vorgelegt. Anschließend werden 795 mg (3,02 mmol) Ethyl-5-bromo-2-chlorobenzoat, 429 mg (3,02 mmol) *N,N'*-Dimethylcyclohexan-1,2-diamin und 5 mL Toluol zugetropft. Das Reaktionsgemisch wird in der Mikrowelle 45 min bei 100 °C gerührt und dann mit Ethylacetat verdünnt, über Celite filtriert und am Rotationsverdampfer eingeengt. Der Rückstand wird mittels Flashchromatographie an Kieselgel gereinigt (Eluent: Cyclohexan / Ethylacetat 1/5). Man erhält 0,16 g Ethyl-2-chlor-5-({[2-methyl-6-(pentafluorethyl)-5-(trifluormethyl)pyrimidin-4-yl]carbonyl}amino)benzoat (10%) als beigen Feststoff.

¹H-NMR (400 MHz, d₆-DMSO): δ = 8,05 (d, 1H), 7,81-7,78 (m, 1H), 7,59-7,52 (m, 1H), 4,36 (q, 2H), 2,88 (s, 3H), 1,33 (t, 3H) ppm.

HPLC-MS ^{a)}: logP = 4,55; Masse (m/z) = 506 [M+H]⁺.

Zu einer Lösung von 142 mg (0,28 mmol) Ethyl-2-chlor-5-({[2-methyl-6-(pentafluorethyl)-5-(trifluormethyl)pyrimidin-4-yl]carbonyl}amino)benzoat in einer 1:1 Tetrahydrofuran-Wasser Gemisch werden 32 mg (1,22 mmol) Lithiumhydroxid hinzugegeben. Das Reaktionsgemisch wird über Nacht bei Raumtemperatur gerührt, angesäuert und eingedampft. Der Rückstand wird in Ethylacetat aufgenommen, mit 1 M Salzsäure gewaschen, getrocknet und eingeengt. Man erhält 94 mg 2-Chlor-5-({[2-methyl-6-(pentafluorethyl)-5-(trifluormethyl)pyrimidin-4-yl]carbonyl}amino)-benzoesäure (70%).

¹H-NMR (400 MHz, d₃-Acetonitril): δ = 9,36 (br. s, 1H), 8,20 (m, 1H), 7,81-7,77 (m, 1H), 7,54-7,51 (m, 1H), 2,87 (s, 3H) ppm.

HPLC-MS ^{a)}: logP = 3,30 Masse (m/z) = 478 [M+H]⁺.

In 10 mL Dichlormethan werden 94 mg (0,19 mmol) 2-Chlor-5-({[2-methyl-6-(pentafluorethyl)-5-(trifluormethyl)pyrimidin-4-yl]carbonyl}amino)benzoesäure mit zwei Tropfen *N,N-*Dimethylformamid versetzt und auf 0 °C gekühlt. Nach Zugabe von 0,05 mL Oxalsaeuredichlorid wird das Reaktionsgemisch drei Stunden bei Raumtemperatur gerührt und anschließend eingeengt.

^{a)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 micron; Eluent A: Acetonitril (0,1% Ameisensäure); Eluent B: Wasser (0,09% Ameisensäure); linearer Gradient von 10% Acetonitril bis 95% Acetonitril in 4,25 min, dann 95% Acetonitril für weitere 1,25 min; Ofentemperatur 55 °C; Fluß: 2,0 mL/min. Die Massendetektion erfolgt über ein Agilend MSD-System.

^{b)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18). HP1100; 50*4,6 Zorbax Eclipse Plus C18 1,8 micron; Eluent A: Acetonitril (0,1% Ameisensäure); Eluent B: Wasser (0,08% Ameisensäure); linearer Gradient von 5% Acetonitril bis 95% Acetonitril in 1,70 min, dann 95% Acetonitril für weitere 1,00 min; Ofentemperatur 55°C; Fluß: 2,0 mL/min. Die Massendetektion erfolgt über den Massendetektor Micromass ZQ2000 der Firma Waters.

Mit Hilfe der oben beschriebenen Darstellungsverfahren A bis E wurden die in den Tabellen 1 -6 aufgeführten Verbindungen dargestellt.

### Herstellung der Ausgangsverbindungen

### Ethyl-4-(difluormethyl)-2-(pentafluorethyl)pyrimidin-5-carboxylat

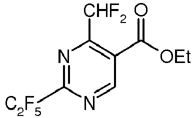

Eine Mischung von 1,62 g (10 mmol) 2,2,3,3,3-Pentafluorpropanimidamid und 2,22 g (10 mmol) Ethyl-2-(ethoxymethylen)-4,4-difluor-3-oxobutanoat (Herstellung s. WO 2005/123690) in 10 mL Ethanol wird nach Zugabe von 0,68 g (10 mmol) Natriumethylat 4 Tage unter Rückfluss gerührt. Anschließend wird im Vakuum eingeengt und der Rückstand in 10 mL Wasser aufgenommen und zweimal mit 10 mL Ethylacetat extrahiert. Die organischen Phasen werden successiv mit 5 mL Wasser und 5 mL gesättigter Natriumchloridlösung gewaschen, über Natriumsulfat getrocknet, filtriert und im Vakuum eingeengt. Nach chromatographischer Reinigung mit einer Mischung von Cyclohexan und Ethylacetat erhält man 1,26 g Ethyl-4-(difluormethyl)-2-(pentafluorethyl)pyrimidin-5-carboxylat (40%) als weißen Feststoff.

¹H-NMR (400 MHz, d₆-DMSO): δ = 9,58 (s, 1H), 7,49 (t, 1H), 4,45 (q, 2H), 1,38 (t, 3H) ppm.

HPLC-MS ^{a)}: logP = 3,42; Masse (m/z) = 321 [M+H]⁺.

### Auf gleiche Weise wurden erhalten:

### Ethyl-2-(pentafluorethyl)-4-(trifluormethyl)pyrimidin-5-carboxylat aus Ethyl-2-(ethoxymethylen)-4,4,4-trifluor-3-oxobutanoat und 2,2,3,3,3-Pentafluorpropanimidamid

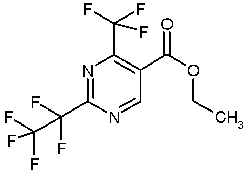

¹H-NMR (400 MHz, d₆-DMSO): δ = 9,66 (s, 1H), 4,45 (q, 2H), 1,36 (t, 3H) ppm.

HPLC-MS ^{a)}: logP = 3,86; Masse (m/z) = 339 [M+H]⁺.

### Ethyl-2-(heptafluorpropyl)-4-(trifluormethyl)pyrimidin-5-carboxylat aus Ethyl-2-(ethoxymethylen)-4,4,4-trifluor-3-oxobutanoat und 2,2,3,3,4,4,4-Heptafluorbutanimidamid

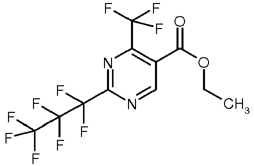

¹H-NMR (400 MHz, d₆-DMSO): δ = 9,68 (s, 1H), 4,46 (q, 2H), 1,36 (t, 3H) ppm.

HPLC-MS ^{a)}: logP = 4,32; Masse (m/z) = 389 [M+H]⁺.

### Ethyl-4,6-dimethyl-2-(pentafluorethyl)pyrimidin-5-carboxylat aus Ethyl-(2E)-2-acetyl-3-ethoxybut-2-enoat (Herstellung s. Journal of Medicinal Chemistry 2006, 49, 6351) und 2,2,3,3,3-Pentafluorpropanimidamid

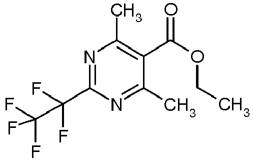

¹H-NMR (400 MHz, d₆-DMSO): δ = 4,46 (q, 2H), 3,10 (s, 6H), 1,36 (t, 3H) ppm.

HPLC-MS ^{a)}: logP = 3,68; Masse (m/z) = 299 [M+H]⁺.

### Ethyl-2,4-bis(pentafluorethyl)pyrimidin-5-carboxylat aus Ethyl-2-(ethoxymethylen)-4,4,5,5,5-pentafluor-3-oxopentanoat (Herstellung analog WO 2005/123690) und 2,2,3,3,3-Pentafluorpropanimidamid.

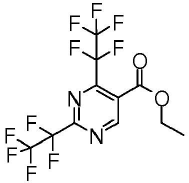

¹H-NMR (400 MHz, d₆-DMSO): δ = 9,68 (s, 1H), 4,46 (q, 2H), 1,35 (t, 3H) ppm.

HPLC-MS: logP = 4,41; Masse (m/z) = 389 [M+H]⁺.

### Ethyl-4-(pyridin-2-yl)-2-(trifluormethyl)pyrimidin-5-carboxylat aus Ethyl-3-ethoxy-2-(pyridin-2-ylcarbonyl)acrylat (Herstellung analog WO 2005/123690) und 2,2,2-Trifluorpropanimidamid

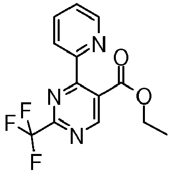

¹H-NMR (400 MHz, d₆-DMSO): δ = 9,28 (s, 1H), 8,69-8,71 (m, 1H), 8,27-8,30 (m, 1H), 8,09 (dt, 1H), 7,61-7,64 (m, 1H), 4,31 (q, 2H), 1,18 (t, 3H) ppm.

HPLC-MS: logP = 3,09; Masse (m/z) = 298 [M+H]⁺.

### Ethyl-4-methyl-2-(pentafluorethyl)pyrimidin-5-carboxylat kann analog zu dem Vorschrift

*Bioorg. Med. Chem. Letters* **2005***, 15,* 4898 synthetisiert werden.

### 4-(Difluormethyl)-2-(pentafluorethyl)pyrimidin-5-carbonsäure

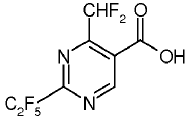

In 4 mL Ethanol werden 1,15 g (3,59 mmol) Ethyl-4-(difluormethyl)-2-(pentafluorethyl)pyrimidin-5-carboxylat gelöst. Es werden 5,39 mL (10,8 mmol) 2 M Natronlauge zugegeben und das Reaktionsgemisch wird vier Stunden bei Raumtemperatur gerührt. Es wird durch Zugabe von 2 M Salzsäure auf pH 2-3 gestellt. Der entstandene Feststoff wird abgesaugt, mit wenig Wasser gewaschen und mit Cyclohexan verrieben. Man erhält 870 mg 4-(Difluormethyl)-2-(pentafluorethyl)pyrimidin-5-carbonsäure (83%) als weißen Feststoff.

¹H-NMR (400 MHz, d₆-DMSO): δ = 9,55 (s, 1H), 7,58 (t, 1H) ppm.

HPLC-MS ^{a)}: logP = 1,80; Masse (m/z) = 293 [M+H]⁺.

Auf gleiche Weise wurden erhalten:

### 2-(Pentafluorethyl)-4-(trifluormethyl)pyrimidin-5-carbonsäure aus Ethyl-2-(pentafluorethyl)-4-(trifluormethyl)pyrimidin-5-carboxylat

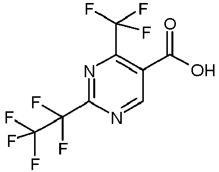

¹H-NMR (400 MHz, d₆-DMSO): δ = 9,40 (s, 1H) ppm.

HPLC-MS ^{a)}: logP = 1,80; Masse (m/z) = 311 [M+H]⁺.

### 2-(Heptafluorpropyl)-4-(trifluormethyl)pyrimidin-5-carbonsäure aus Ethyl-2-(heptafluorpropyl)-4-(trifluormethyl)pyrimidin-5-carboxylat

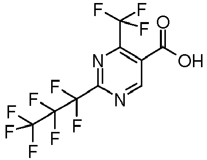

¹H-NMR (400 MHz, d₆-DMSO): δ = 9,50 (s, 1H) ppm.

HPLC-MS ^{a)}: logP = 2,23 Masse (m/z) = 361 [M+H]⁺.

### 4-Methyl-2-(trifluormethyl)pyrimidin-5-carbonsäure aus Ethyl-4-methyl-2-(trifluormethyl)pyrimidin-5-carboxylat

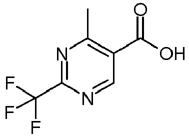

¹H-NMR (400 MHz, d₆-DMSO): δ = 9,19 (s, 1H) ppm.

HPLC-MS ^{a)}: logP = 1,26; Masse (m/z) = 207 [M+H]⁺.

### 4-Methyl-2-(pentafluorethyl)pyrimidin-5-carbonsäure aus Ethyl-4-methyl-2-(pentafluorethyl)pyrimidin-5-carboxylat

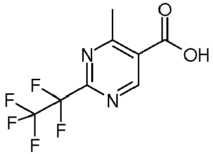

¹H-NMR (400 MHz, d₆-DMSO): δ = 9,25 (s, 1H) ppm.

HPLC-MS ^{a)}: logP = 1,97 Masse (m/z) = 257 [M+H]⁺.

### 4,6-Dimethyl-2-(pentafluorethyl)pyrimidin-5-carbonsäure aus Ethyl-4,6-dimethyl-2-(pentafluorethyl)pyrimidin-5-carboxylat

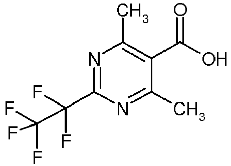

¹H-NMR (400 MHz, d₆-DMSO): δ = 2,58 (s, 6H) ppm.

HPLC-MS ^{a)}: logP = 1,63 Masse (m/z) = 271 [M+H]⁺.

### 4-Chlor-3-(trifluormethyl)pyridin-2-carbonsäure wurde analog zur Literaturstelle European Journal of Organic Chemistry 2004, 18, 3793 aus 4-Chlor-3-(trifluormethyl)pyridin hergestellt

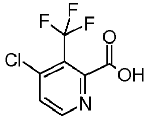

¹H-NMR (400 MHz, d₆-DMSO): δ = 9,13 (d, 1H), 9,07 (d, 1H) ppm.

HPLC-MS: logP = 1,16 Masse (m/z) = 226 [M+H]⁺.

### 2,4-Bis(pentafluorethyl)pyrimidin-5-carbonsäure aus Ethyl-2,4-bis(pentafluorethyl)pyrimidin-5-carboxylat

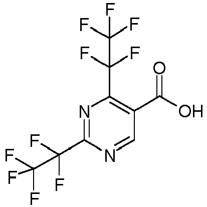

¹H-NMR (400 MHz, d₆-DMSO): δ = 9,56 (s, 1H) ppm.

HPLC-MS: logP = 2,24; Masse (m/z) = 361 [M+H]⁺.

### 4-(Pyridin-2-yl)-2-(trifluormethyl)pyrimidin-5-carbonsäure

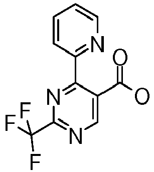

¹H-NMR (400 MHz, d₆-DMSO): δ = 9,27 (s, 1H), 8,71-8,69 (m, 1H), 8,23-8,21 (m, 1H), 8,09-8,04 (m, 1H), 7,62-7,59 (m, 1H) ppm.

HPLC-MS: logP = 1,50; Masse (m/z) = 270 [M+H]⁺.

### 5-Cyano-1-methyl-3-pentafluorethyl-4-trifluormethyl-1H-pyrazol

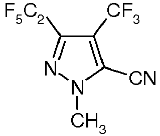

42,0 g (147 mmol) 5-Fluor-1-methyl-3-pentafluorethyl-4-trifluormethyl-pyrazol [Darstellung s. Izvestiya Akademii Nauk SSSR, Seriya Khimicheskaya 1990, (11), 2583-9] und 11,5 g (235 mmol) Natriumcyanid werden in 150 mL Acetonitril suspendiert und anschließend unter Schutzgasatmosphäre unter Rückflußtemperatur erhitzt. Nach dem Abkühlen wird das Reaktionsgemisch auf ein Gemisch aus 300 mL destilliertem Wasser und 300 mL Diethylether gegossen. Die wässrige Phase wird dreimal mit Diethylether extrahiert. Die vereinigten organischen Phasen werden zweimal mit Wasser und einmal mit gesättigter wässriger Natriumchlorid-Lösung gewaschen. Die organische Phase wird über Natriumsulfat getrocknet und anschließend filtriert. Das Lösungsmittel wird am Rotationsverdampfer unter vermindertem Druck entfernt und der so erhaltene Rückstand im Vakuum fraktioniert destilliert. Man erhält 37,0 g 5-Cyano-1-methyl-3-pentafluorethyl-4-trifluormethyl-pyrazol (82%) als farblose Flüssigkeit (Sdp. 74° C / 10 mbar).

¹H-NMR (400 MHz, d₃-Acetonitril): δ = 4,11 (s, 3H) ppm.

GC-MS: Retentionszeit 2,67 min; Masse (m/z) = 224 [M]⁺.

### 1-Methyl-3-pentafluorethyl-4-trifluormethyl-1H-pyrazol-5-carbonsäure

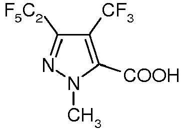

11,0 g (37,5 mmol) 5-Cyano-1-methyl-3-pentafluorethyl-4-trifluormethyl-1*H*-pyrazol, 22 mL 50%-ige Natronlauge und 7,0 mL destilliertes Wasser werden im Ölbad erhitzt bis der Feststoff geschmolzen ist. Das Reaktionsgemisch wird anschließend über Nacht gerührt (Ölbadtemperatur 100 °C). Nach Abkühlen wird das Reaktionsgemisch auf ein Gemisch aus 150 mL konzentrierter Salzsäure und 150 mL Eis gegossen. Es werden 30 Minuten nachgerührt und der Feststoff abfiltriert. Der Feststoff wird mit wenig Wasser gewaschen und dann im Ölpumpenvakuum getrocknet. Man erhält 11,2 g (95%) 1-Methyl-3-pentafluorethyl-4-trifluormethyl-1*H*-pyrazol-5-carbonsäure als weißen Feststoff.

¹H-NMR (400 MHz, d₃-Acetonitril): δ = 4,08 (s, 3H) ppm.

HPLC-MS ^{a)}: logP = 1,86; Masse (m/z) = 313 [M+H]⁺.

### 3-Chlor-2-[5-fluor-3-(pentafluorethyl)-4-(trifluormethyl)-1H-pyrazol-1-yl]pyridin

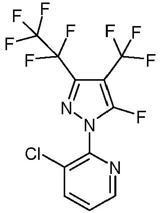

1,0 g (7,0 mmol) 3-Chlor-2-hydrazinopyridin werden in 2,9 mL (21 mmol) Triethylamin und 40,0 mL Acetonitril suspendiert und bei -65 °C zu 2,1 g (7 mmol) (1,1,1,3,4,4,5,5,5-Nonafluor-2-(trifluormethyl)pent-2-en hinzugefügt. Das Reaktionsgemisch wird langsam auf Raumtemperatur erwärmt und dann 60 Stunden bei Raumtemperatur gerührt. Der Feststoff wird abfiltriert und mit Diethylether nachgewaschen. Das Lösungsmittel wird unter vermindertem Druck am Rotationsverdampfer entfernt. Das Produkt wird dreimal mit Cyclohexan aus dem Rückstand extrahiert. Die organischen Phasen werden vereinigt und das Lösungsmittel unter vermindertem Druck am Rotationsverdampfer entfernt. Man erhält 1,95 g 3-Chlor-2-[5-fluor-3-(pentafluorethyl)-4-(trifluormethyl)-1*H*-pyrazol-1-yl]pyridin (73%) als orangenes Öl.

¹H-NMR (400 MHz, d₃-Acetonitril): δ = 8,58-8,62 (m, 1H), 8,12-8,18 (m, 1H), 7,62-7,68 (m, 1H) ppm.

GC-MS: Retentionszeit 5,88 min; Masse (m/z) = 383 [M]⁺.

### 3-Chlor-2-[5-cyano-3-(pentafluorethyl)-4-(trifluormethyl)-1H-pyrazol-1-yl]pyridin

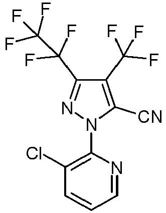

1,95 g (5,1 mmol) 3-Chlor-2-[5-fluor-3-(pentafluorethyl)-4-(trifluormethyl)-1*H*-pyrazol-1-yl]pyridin und 0,30 g (6,1 mmol) Natriumcyanid (werden in 20 mL Acetonitril suspendiert und das Reaktionsgemisch anschließend 16 Stunden unter Rückfluß gerührt. Nach dem Abkühlen wird das Reaktionsgemisch auf ein Wasser-Diethylether Gemisch gegossen. Die wässrige Phase wird dreimal mit Diethylether extrahiert. Die vereinigten organischen Phasen werden zweimal mit Wasser und einmal mit gesättigter Natriumchloridlösung gewaschen und dann über Magnesiumsulfat getrocknet und filtriert. Das Lösungsmittel wird unter vermindertem Druck am Rotationsverdampfer entfernt. Das Rohprodukt wird mittels Flashchromatographie an Kieselgel gereinigt. Man erhält 1,7 g 3-Chlor-2-[5-cyano-3-(pentafluorethyl)-4-(trifluormethyl)-1*H*-pyrazol-1-yl]pyridin (84%) als gelbes Öl.

¹H-NMR (400 MHz, d₃-Acetonitril): δ = 8,61 (dd, 1H), 8,20 (dd, 1H), 7,70 (dd, 1H) ppm.

GC-MS: Retentionszeit 6,43 min; Masse (m/z) = 390 [M]⁺.

### 3-(Pentafluorethyl)-4-(trifluormethyl)-1H-pyrazol-5-carbonsäure

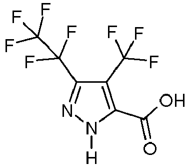

4,9 g (12,5 mmol) 3-Chlor-2-[5-cyano-3-(pentafluorethyl)-4-(trifluormethyl)-1*H*-pyrazol-1-yl]pyridin wird 24 mL Methanol gelöst und mit 32,6 mL (494 mmol) 50%-iger Natronlauge (494 mmol) versetzt. Das Reaktionsgemisch wird 7 Tage bei Raumtemperatur gerührt. Das Reaktionsgemisch wird mit 10 mL Wasser verdünnt und dann auf die Hälfte des Volumens eingeengt. Das Gemisch wird anschließend auf konzentrierte Salzsäure mit Eis getropft. Der ausfallende farblose Feststoff wird abfiltriert un dam Vakuum getrocknet. Man erhält 2,95 g 3-(Pentafluorethyl)-4-(trifluormethyl)-1*H*-pyrazol-5-carbonsäure (79%) als farblosen Feststoff.

HPLC-MS ^{a)}: logP = 2,00; Masse (m/z) = 299 [M+H]⁺.

### Methyl-3-(pentafluorethyl)-4-(trifluormethyl)-1H-pyrazol-5-carboxylat

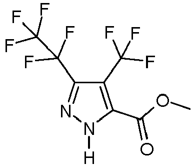

0,8 g (2,6 mmol) 3-(Pentafluorethyl)-4-(trifluormethyl)-1*H*-pyrazol-5-carbonsäure werden in 15,0 mL Methanol gelöst und dann langsam tropfenweise mit 0,58 mL (7,9 mmol) Thionylchlorid versetzt. Die Reaktionslösung wird anschließend 16 Stunden unter Rückfluß erhitzt. Nach dem Abkühlen wird das Lösungsmittel unter vermindertem Druck am Rotationsverdampfer entfernt und der Rückstand in Ethylacetat aufgenommen. Die organische Phase wird vorsichtig mit gesättigter Natriumhydrogencarbonat-Lösung versetzt. Die organische Phase wird mit gesättigter Natriumchloridlösung gewaschen, über Magnesiumsulfat getrocknet und filtriert. Das Lösungsmittel wird unter vermindertem Druck am Rotationsverdampfer entfernt. Man erhält 0,75 g Methyl-3-(pentafluorethyl)-4-(trifluormethyl)-1*H*-pyrazol-5-carboxylat (96%) als farbloses Öl.

¹H-NMR (400 MHz, d₃-Acetonitril): δ = 3,92 (s, 3H) ppm.

HPLC-MS ^{a)}: logP = 3,02; Masse (m/z) = 313 [M+H]⁺.

### 1-Ethyl-3-(pentafluorethyl)-4-(trifluormethyl)-1H-pyrazol-5-carbonsäure

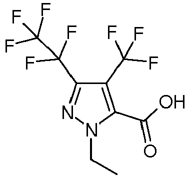

0,23 g (0,72 mmol) Methyl-3-(pentafluorethyl)-4-(trifluormethyl)-1*H*-pyrazol-5-carboxylat und 0,3 g (2,2 mmol) Kaliumcarbonat werden in 7,0 mL Aceton suspendiert und mit 0,12 mL Iodethan (1,4 mmol) versetzt. Das Reaktionsgemisch wird über Nacht bei Raumtemperatur gerührt. Zu der Suspension werden 1,1 mL (2,2 mmol) 2 N Natronlauge gegeben. Die Lösung wird anschließend über Nacht bei Raumtemperatur gerührt. Das Reaktionsgemisch wird mit Wasser verdünnt und mit 1 M Salzsäure auf pH 2-3 eingestellt. Die wässrige Reaktionslösung wird dreimal mit Ethylacetat extrahiert. Die vereinigten organischen Phasen werden über Magnesiumsulfat getrocknet, filtriert und unter vermindertem Druck am Rotationsverdampfer eingeengt. Man erhält 0,22 g 1-Ethyl-3-(pentafluorethyl)-4-(trifluormethyl)-1*H*-pyrazol-5-carbonsäure (91%) als farblosen Feststoff.

¹H-NMR (400 MHz, d₃-Acetonitril): δ = 4,44 (q, 2H), 1,44 (t, 3H) ppm.

HPLC-MS ^{a)}: logP = 2,18; Masse (m/z) = 327 [M+H]⁺.

### 1-Isopropyl-4-(pentafluorethyl)-3-(trifluormethyl)-1H-pyrazol-5-carbonsäure

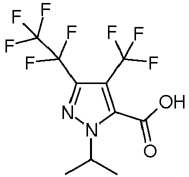

Die Verbindung wird analog zur Herstellung von 1-Ethyl-3-(pentafluorethyl)-4-(trifluormethyl)-1*H*-pyrazol-5-carbonsäure aus Methyl-3-(pentafluorethyl)-4-(trifluormethyl)-1*H*-pyrazol-5-carboxylat und 2-Iodpropan dargestellt.

¹H-NMR (400 MHz, d₃-Acetonitril): δ = 5,03 (sep, 1H), 1,47 (d, 6H) ppm.

HPLC-MS ^{a)}: logP = 2,55; Masse (m/z) = 341 [M+H]⁺.

### 1-(Methoxymethyl)-4-(pentafluorethyl)-3-(trifluormethyl)-1H-pyrazol-5-carbonsäure

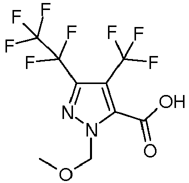

Die Verbindung wird analog zur Herstellung von 1-Ethyl-3-(pentafluorethyl)-4-(trifluormethyl)-1*H*-pyrazol-5-carbonsäure aus Methyl-3-(pentafluorethyl)-4-(trifluormethyl)-1*H*-pyrazol-5-carboxylat und Methoxymethylchlorid dargestellt.

¹H-NMR (400 MHz, d₃-Acetonitril): δ = 5,03 (sep, 1H), 1,47 (d, 6H) ppm.

HPLC-MS ^{a)}: logP = 1,90; Masse (m/z) = 343 [M+H]⁺.

### Ethyl-3,4-bis(trifluormethyl)-1H-pyrazol-5-carboxylat

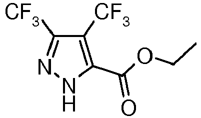

7,57 g (63,0 mmol) Diazoethylacetat werden unter Schutzgas in 200 mL Diethylether vorgelegt und auf -70°C temperiert. Es werden anschließend 20,4 g (126 mmol) Hexafluorbutin in die gekühlte Lösung eingeleitet. Das Reaktionsgemisch wird langsam auf Raumtemperatur erwärmt und 16 Stunden gerührt. Anschließend wird das Lösungsmittel am Rotationsverdampfer entfernt. Man erhält 17,0 g Ethyl-3,4-bis(trifluormethyl)-1*H*-pyrazol-5-carboxylat (98%) als gelbes Öl.

¹H-NMR (400 MHz, d₃-Acetonitril): δ = 4,42 (q, 2H), 1,38 (t, 3H) ppm.

GC-MS: Retentionszeit 3,48 min; Masse (m/z) = 276 [M]⁺.

### 1-Methyl-3,4-bis(trifluormethyl)-1H-pyrazol-5-carbonsäure

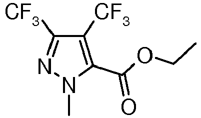

### 1-Methyl-3,4-bis(trifluormethyl)-1H-pyrazol-5-carbonsäure wird analog zur Herstellung von 1-Ethyl-3-(pentafluorethyl)-4-(trifluormethyl)-1H-pyrazol-5-carbonsäure aus Ethyl-3,4-bis(trifluormethyl)-1H-pyrazol-5-carboxylat und Iodmethan dargestellt.

¹H-NMR (400 MHz, d₃-Acetonitril): δ = 4,12 (s, 3H) ppm.

HPLC-MS ^{a)}: logP = 1,47; Masse (m/z) = 263 [M+H]⁺.

### 1,4-Dimethyl-3-(pentafluorethyl)-1H-pyrazol-5-amin

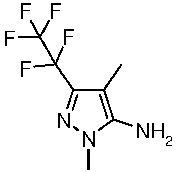

Eine Lösung von 39 g (388 mmol) N,N-DÜspropylamin in 500 mL Tetrahydrofuran wird bei -5 °C mit 232 mL (371 mmol) einer 1,6 M Lösung von *n*-Butyllithium in Hexan versetzt. Die Lösung wird 30 Minuten bei 0 °C gerührt und dann auf -78 °C gekühlt. Dann werden 18,5 g (337 mmol) n-Propionitril dazugetropft. Nach Beendigung der Zugabe wird die Lösung 15 Minuten gerührt. Anschließend werden 30 g (169 mmol) Methyl-pentafluorpropanoat langsam hinzugefügt. Nach Beendigung der Zugabe wird das Reaktionsgemisch noch 45 min bei -78 °C gerührt. Anschließend wird das Gemisch auf Raumtermperatur erwärmt und 1 Stunde bei Raumtermperatur gerührt. Das Reaktionsgemisch wird auf 0 °C heruntergekühlt und mit 700 mL Wasser versetzt. Der pH-Wert der Lösung wird mit konz. Salzsäure auf 1 eingestellt. Die wässrige Phase wird mit Ethylacetat extrahiert und die vereinigten org. Phasen über Magnesiumsulfat getrocknet und unter vermindertem Druck am Rotationsverdampfer eingeengt. Der Rückstand wird in 200 mL Ethanol gelöst und mit 10,5 g (224 mmol) Methylhydrazin und 16 mL konz. Salzsäure versetzt. Die Mischung wird fünf Stunden unter Rückfluß erwärmt. Das Ethanol wird unter vermindertem Druck am Rotationsverdampfer entfernt und der pH-Wert der wässrigen Phase auf 14 eingestellt. Die wässrige Phase wird mehrfach mit Dichlormethan extrahiert. Die vereinigten org. Phasen werden über Magnesiumsulfat getrocknet und unter vermindertem Druck am Rotationsverdampfer eingeengt. Man erhält 13,0 g 1,4-Dimethyl-3-(pentafluorethyl)-1*H*-pyrazol-5-amin (34%).

¹H-NMR (400 MHz, CDCl₃): δ = 3,83 (s, 3H), 3,32 (br. s, 2H), 1,98 (s, 3H) ppm.

### 1,4-Dimethyl-3-(pentafluorethyl)-1H-pyrazol-5-carbonsäure

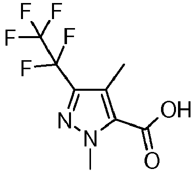

8,66 g (85,1 mmol) *tert*-Butylnitrit und 6,0 g (67,4 mmol) Kupfer(I)cyanid werden in 360 mL Acetonitril suspendiert und auf 65 °C erwärmt. Dann wird eine Lösung von 13,0 g (57,2 mmol) 1,4-Dimethyl-3-(pentafluorethyl)-1*H*-pyrazol-5-amin in 20 mL Acetonitril langsam hinzugefügt. Die Reaktion wird 24 Stunden bei 65 °C weitergerührt. Anschließend wird das Reaktionsgemisch über Celite filtriert. Das Filtrat wird unter vermindertem Druck am Rotationsverdampfer eingeengt. Der Rückstand wird in Wasser aufgenommen und mit konz. Salzsäure angesäuert. Die wässrige Phase wird mehrfach mit Dichlormethan extrahiert. Die vereinigten org. Phasen werden unter vermindertem Druck am Rotationsverdampfer eingeengt und der Rückstand säulenchromatographisch an Kieselgel (Hexan: Ethylacetat = 5:1) aufgereinigt. Das so erhaltene Produkt (2,3 g, 9,7 mmol) wird mit 3,44 g (63 mmol) Kaliumhydroxid in 20 mL Wasser unter Rückfluß erhitzt. Nach 3h wird die Reaktionslösung auf 0 °C gekühlt und mit verdünnter Salzsäure auf pH 6 eingestellt. Der entstandene Feststoff wird filtriert und getrocknet. Man erhält 0,3 g 1,4-Dimethyl-3-(pentafluorethyl)-1*H*-pyrazol-5-carbonsäure (2%) als weißen Feststoff.

¹H-NMR (400 MHz, CDCl₃): δ = 4,22 (s, 3H), 2,44 (s, 3H) ppm.

### 1-Methyl-3-pentafluorethyl-1H-pyrazol

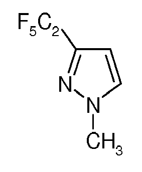

Zu einer Lösung von 30,9 g (142 mmol) (1*E*)-1-Ethoxy-4,4,5,5,5-pentafluorpent-1-en-3-on (Herstellung: Synthesis, 2000, 5, 738-742.) in 56 mL Methanol werden 7,18 g (156 mmol) Methylhydrazin getropft und das Reaktionsgemisch 18 Stunden unter Rückfluß erhitzt. Das Methanol wird zum Großteil bei Normaldruck abdestilliert und der Rückstand auf Eis gegeben. Die wässrige Phase wird dreimal mit Dichlormethan extrahiert und die organische Phase anschließend dreimal mit gesättigter Natriumchloridlösung gewaschen. Nach Trocknen über Natriumsulfat wird das Lösungsmittel am Rotationsverdampfer bei vermindertem Druck abdestilliert. Man erhält 15,8 g 1-Methyl-3-pentafluorethyl-1*H*-pyrazol (52%) als Öl.

¹H-NMR (400 MHz, d₃-Acetonitril): δ = 7,61 (m, 1H), 6,57 (m, 1H), 3,89 (s, 3H) ppm.

HPLC-MS ^{a)}: logP = 2,29; Masse (m/z) = 201 [M+H]⁺.

Auf gleiche Weise wurden erhalten:

### 1-Methyl-3-(1-chlor,1,2,2,2-tetrafluorethyl-1H-pyrazol aus (1E)-4-Chlor-1-ethoxy-4,5,5,5-tetrafluorpent-1-en-3 -on

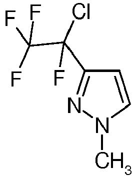

¹H-NMR (400 MHz, d₃-Acetonitril) δ = 7,58 (m, 1H), 6,54 (m, 1H), 3,89 (s, 3H) ppm.

HPLC-MS ^{a)}: logP = 2,46; Masse (m/z) = 217 [M+H]⁺.

### 1-Methyl-3-heptafluorpropyl-1H-pyrazol aus (1E)-1-Ethoxy-4,4,5,5,6,6,6-heptafluorhex-1-en-3-on

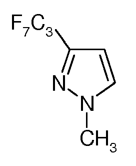

¹H-NMR (400 MHz, d₃-Acetonitril): δ = 7,91 (m, 1H), 6,65 (m, 1H), 3,94 (s, 3H) ppm.

HPLC-MS ^{a)}: logP = 2,84; Masse (m/z) = 251 [M+H]⁺.

### 1-Methyl-3-nonafluorbutyl-1H-pyrazol aus (1E)-1-Ethoxy-4,4,5,5,6,6,7,7,7-nonafluorhept-1-en-3-on

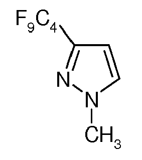

¹H-NMR (400 MHz, d₃-Acetonitril): δ = 7,61 (m, 1H), 6,57 (m, 1H), 3,97 (s, 3H) ppm.

HPLC-MS ^{a}': logP = 3,38; Masse (m/z) = 301 [M+H]⁺.

### 3-{[Difluor(trifluormethoxy)methoxy](difluor)methyl}-1-methyl-1H-pyrazol aus(3E)-1-[Difluor(trifluormethoxy)methoxy]-4-ethoxy-1,1-difluorbut-3-en-2-on

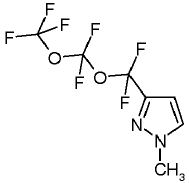

¹H-NMR (400 MHz, d₃-Acetonitril): δ = 7,58 (m, 1H), 6,54 (m, 1H), 3,90 (s, 3H) ppm.

HPLC-MS ^{a)}: logP = 3,79; Masse (m/z) = 333 [M+H]⁺.

### 4-Brom-1-Methyl-3-heptafluorpropyl-1H-pyrazol

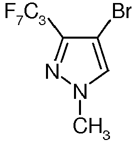

Zu einer Lösung von 4,65 g (18,6 mmol) 1-Methyl-3-heptafluorpropyl-1*H*-pyrazol in 18 mL Wasser werden bei 40 °C 3,27 g (20,5 mmol) Brom getropft und das Reaktionsgemisch zunächst eine Stunde bei 60 °C und dann 18 Stunden bei Raumtemperatur nachgerührt. Die wässrige Phase wird dreimal mit Dichlormethan extrahiert und die organische Phase über Natriumsulfat getrocknet. Das Dichlormethan wird am Rotationsverdampfer bei vermindertem Druck abdestilliert. Man erhält 5,75 g 1-Methyl-3-heptafluorpropyl-4-bromo-1*H*-pyrazol (78%) als Öl.

¹H-NMR (400 MHz, d₃-Acetonitril): δ = 7,73 (m, 1H), 3,90 (s, 3H) ppm.

HPLC-MS ^{a)}: logP = 3,53; Masse (m/z) = 330 [M+H]⁺.

Auf gleiche Weise wurden erhalten:

### 4-Brom-1-Methyl-3-pentafluorethyl-1H-pyrazol aus 1-Methyl-3-pentafluorethyl-1H-pyrazol

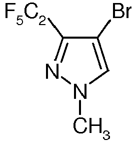

¹H-NMR (400 MHz, d₃-Acetonitril): δ = 7,77 (m, 1H), 3,90 (s, 3H) ppm.

HPLC-MS ^{a)}: logP = 2,99; Masse (m/z) = 280 [M+H]⁺.

### 4-Brom-1-Methyl-3-(1-chlor-1,2,2,2-tetrafluorethyl-1H-pyrazol aus 1-Methyl-3-(1-chlor,1,2,2,2-tetrafluorethyl-1H-pyrazol

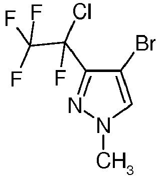

¹H-NMR (400 MHz, d₃-Acetonitril) δ = 7,75 (m, 1H), 3,89 (s, 3H) ppm.

HPLC-MS ^{a)}: logP = 3,17; Masse (m/z) = 296 [M+H]⁺.

### 1-Methyl-3-pentafluorethyl-1H-pyrazol-5-carbonsäure

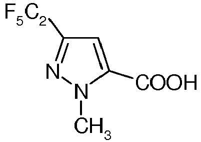

Unter Argonatmosphäre werden 5,00 g (25,0 mmol) 1-Methyl-3-pentafluorethyl-1*H*-pyrazol in Diethylether vorgelegt und die Lösung auf -78 °C gekühlt. Man tropft 11,1 mL (27,7 mmol) 2 M Lithiumdüsopropylamidlösung in Tetrahydrofuran/Heptan zu und gibt bei -30 °C unter starkem Rühren 450 g zerstoßenes Trockeneis zu. Nach Beendigung der Gasentwicklung versetzt man das Reaktionsgemisch mit 235 mL Wasser und stellt mit 1 M Natronlauge pH 11 ein. Die alkalische Lösung wird dreimal mit Ethylacetat extrahiert und daraufhin mit 1N Salzsäure auf pH 2 gestellt. Die wässrige Phase wird dreimal mit Ethylacetat extrahiert und die organische Phase über Natriumsulfat getrocknet. Nach Abdestillieren des Lösungsmittels am Rotationsverdampfer unter vermindertem Druck erhält man 1,20 g 1-Methyl-3-pentafluorethyl-1*H*-pyrazol-5-carbonsäure (18%) als Feststoff.

¹H-NMR (400 MHz, d₃-Acetonitril): δ = 7,14 (m, 1H), 4,16 (s, 3H) ppm.

HPLC-MS ^{a)}: logP = 2,08; Masse (m/z) = 245 [M+H]⁺.

Auf gleiche Weise wurden erhalten:

### 4-Brom-1-Methyl-3-pentafluoroethyl-1H-pyrazol-5-carbonsäure aus 4-Brom-1-Methyl-3-pentafluorethyl-1H-pyrazol

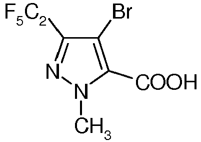

¹H-NMR (400 MHz, d₃-Acetonitril) δ = 4,15 (s, 3H) ppm.

HPLC-MS ^{a)}: logP = 4,69; Masse (m/z) = 324 [M+H]⁺.

### 4-Brom-1-Methyl-3-heptafluorpropyl-1H-pyrazol-5-carbonsäure aus 4-Brom-1-Methyl-3-hepta-fluorpropyl-1H-pyrazol

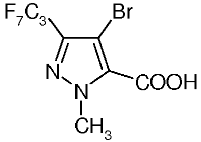

¹H-NMR (400 MHz, d₃-Acetonitril): δ = 4,15 (s, 3H) ppm.

HPLC-MS ^{a)}: logP = 2,26; Masse (m/z) = 374 [M+H]⁺.

### 4-Brom-1-Methyl-3-(1-chloro-1,2,2,2-tetrafluoroethyl)-1H-pyrazol-5-carbonsäure aus 4-Brom-1-Methyl-3-(1-chlor-1,2,2,2-tetrafluorethyl)-1H-pyrazol

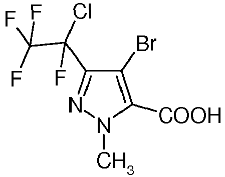

¹H-NMR (400 MHz, d₃-Acetonitril): δ = 4,14 (s, 3H) ppm.

HPLC-MS ^{a)}: logP = 2,43; Masse (m/z) = 340 [M+H]⁺.

### 1-Methyl-3-nonafluorbutyl-1H-pyrazol-5-carbonsäure aus 1-Methyl-3-nonafluorbutyl-1H-pyrazol

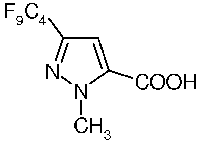

¹H-NMR (400 MHz, d₃-Acetonitril): δ = 7,14 (m, 1H), 4,17 (s, 3H) ppm.

HPLC-MS ^{a)}: logP = 3,01; Masse (m/z) = 345 [M+H]⁺.

### 3-{[Difluor(trifluormethoxy)methoxy](difluor)methyl}-1-methyl-1H-pyrazol-5-carbonsäure aus 3-{[Difluor(trifluormethoxy)methoxy] (difluor)methyl}-1-methyl-1H-pyrazol

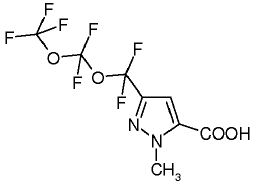

¹H-NMR (400 MHz, d₃-Acetonitril): δ = 7,11 (m, 1H), 4,16 (s, 3H) ppm.

HPLC-MS ^{a)}: logP = 3,38; Masse (m/z) = 377 [M+H]⁺.

### 4-Brom-1-Methyl-3-nonafluorbutyl-1H-pyrazol-5-carbonsäure

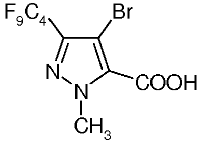

Zu einer Lösung von 0,50 g (1,45 mmol) 1-Methyl-3-nonafluorbutyl-1*H*-pyrazol-5-carbonsäure in 3,5 mL Wasser werden bei 40 °C 255 mg (1,60 mmol) Brom getropft und das Reaktionsgemisch zunächst eine Stunde bei 60 °C und dann drei Tage bei Raumtemperatur nachgerührt. Die wässrige Phase wird dreimal mit Dichlormethan extrahiert und die organische Phase über Natriumsulfat getrocknet. Das Dichlormethan wird am Rotationsverdampfer bei vermindertem Druck abdestilliert. Man erhält 0,54 g 4-Brom-1-methyl-3-nonafluorbutyl-1*H*-pyrazol-5-carbonsäure (80%) als Öl.

¹H-NMR (400 MHz, d₃-Acetonitril): δ = 4,16 (s, 3H) ppm.

HPLC-MS ^{a)}: logP = 3,17; Masse (m/z) = 424 [M+H]⁺.

Auf gleiche Weise wurden erhalten:

### 4-Brom-3-{[difluoro(trifluoromethoxy)methoxy](difluoro)methyl}-1-methyl-1H-pyrazol-5-carbonsäure aus 3-{[Difluor(trifluormethoxy)methoxy](difluor)methyl}-1-methyl-1H-pyrazol-5-carbonsäure

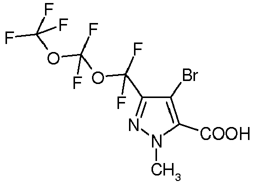

¹H-NMR (400 MHz, d₃-Acetonitril): δ = 4,14 (s, 3H) ppm.

HPLC-MS ^{a)}: logP = 3,56; Masse (m/z) = 456 [M+H]⁺.

### 1-Methyl-3-pentafluorethyl-4-iodo-1H-pyrazol-5-carbonsäure

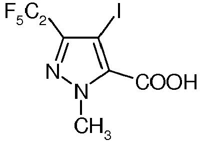

Zu einer Lösung von 1,20 g (4,91 mmol) 1-Methyl-3-pentafluorethyl-1*H*-pyrazol-5-carbonsäure in 4,3 mL Acetonitril werden 1,34 g (2,46 mmol) Ammoniumcer(IV)nitrat und anschließend 0,75 g (2,95 mmol) Iod gegeben und das Reaktionsgemisch 18 Stunden unter Rückfluß erhitzt. Nach Zusatz von 20 mL Dichlormethan wäscht man zunächst mit Wasser, mit Natriumdisulfitlösung und schließlich mit gesättigter Natriumchloridlösung. Die organische Phase wird über Natriumsulfat getrocknet und das Lösungsmittel wird am Rotationsverdampfer bei vermindertem Druck abdestilliert. Man erhält 1,28 g 4-Iod-1-Methyl-3-pentafluorethyl-1*H*-pyrazol-5-carbonsäure (47%) als Öl.

¹H-NMR (400 MHz, d₃-Acetonitril): δ = 4,16 (s, 3H) ppm.

HPLC-MS: logP = 2,33; Masse (m/z) = 371 [M+H]⁺.

### 4-Fluor-2-methyl-6-(pentafluorethyl)-5-(trifluormethyl)pyrimidin kann analog zu den Vorschriften aus dem Patent JP 07196622 und Russ. Chem.. Bull. 1997, 46, 1920 synthetisiert werden.

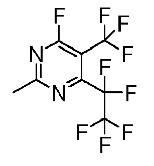

### 2-Methyl-6-(pentafluorethyl)-5-(trifluormethyl)pyrimidin-4-carbonitril

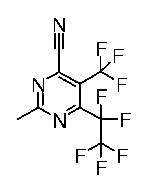

In 40 mL Acetonitril werden 5,0 g (16,8 mmol) 4-Fluor-2-methyl-6-(pentafluorethyl)-5-(trifluormethyl)pyrimidin gelöst. Es werden 986 mg (20,1 mmol) Natriumcyanid zugegeben und das Reaktionsgemisch wird 18 Stunden bei 50 °C gerührt. Es werden nochmals 493 mg (10,7 mmol) Natriumcyanid zugegeben und das Reaktionsgemisch drei Stunden unter Rückfluss gerührt. Nach Zugabe von 100 mL Wasser und 100 mL Ethylacetat wird die organische Phase abgetrennt. Die wässrige Phase wird dreimal mit Ethylacetat extrahiert. Die vereinigten organischen Phasen werden zweimal mit Wasser und mit einer gesättigten Natriumchloridlösung gewaschen, anschließend über Natriumsulfat getrocknet, filtriert und das Lösungsmittel am Rotationsverdampfer entfernt. Man erhält 4,71 g 2-Methyl-6-(pentafluorethyl)-5-(trifluormethyl)pyrimidin-4-carbonitril (92%) als dunkles Öl.

¹H-NMR (400 MHz, d₃-Acetonitril): δ = 2,87 (s, 3H) ppm.

GC-MS: Masse (m/z) = 305 [M]⁺.

### 2-Methyl-6-(pentafluorethyl)-5-(trifluormethyl)pyrimidin-4-carboxamid

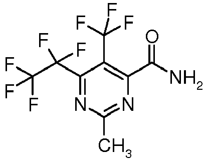

In 80 mL konzentrierter Schwefelsäure werden 4,70 g (15,4 mmol) 2-Methyl-6-(pentafluorethyl)-5-(trifluormethyl)pyrimidin-4-carbonitril gelöst. Das Reaktionsgemisch wird 12 Stunden bei 100 °C gerührt und anschließend auf 300 mL Eiswasser gegeben. Die wässrige Phase wird viermal mit Ethylacetat extrahiert. Die vereinigten organischen Phasen werden über Natriumsulfat getrocknet und das Lösungsmittel am Rotationsverdampfer unter vermindertem Druck eingeengt. Die Hälfte des Rohprodukts wurde mittels Flashchromatographie an Kieselgel gereinigt (Eluent: Cyclohexan/Ethylacetat = 1/1). Man erhält 0,63 g 2-Methyl-6-(pentafluorethyl)-5-(trifluormethyl)pyrimidin-4-carboxamid (13%) als zähflüssiges Öl.

¹H-NMR (400 MHz, d₆-DMSO): δ = 8,16 (s, 1H), 8,13 (s, 1H), 2,83 (s, 3H) ppm.

HPLC-MS: logP = 2,33; Masse (m/z) = 324 [M+H]⁺.

### Methyl-3-(difluormethoxy)-1-methyl-1H-pyrazol-5-carboxylat

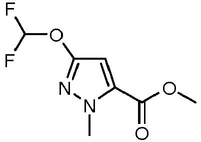

Eine Lösung von 2,00 g (12,8 mmol) Methyl-3-hydroxy-1-methyl-1*H*-pyrazol-5-carboxylat (Herstellung: Chem. Ber. 1974, 107, 1318-1328) in 28 mL *N,N*-Dimethylformamid wird mit 5,09 g (32,0 mmol, 96%) Chlordifluoressigsäurenatriumsalz und 2,66 g (19,2 mmol) Kaliumcarbonat versetzt und das Reaktionsgemisch wird über Nacht auf 80 °C erwärmt. Das Reaktionsgemisch auf 300 mL Wasser gegeben und mehrmals mit Ethylacetat extrahiert. Die organische Phase wird über Magnesiumsulfat getrocknet, filtriert und im Vakuum am Rotationsverdampfer eingeengt. Nach chromatographischer Aufreinigung werden 1,07 g Methyl-3-(difluormethoxy)-1-methyl-1*H-*pyrazol-5-carboxylat (40%) erhalten.

¹H-NMR (400 MHz, d₆-DMSO): δ = 7,19 (t, 1H), 6,55 (s, 1H), 4,00 (s, 3H), 3,55 (s, 3H) ppm.

HPLC-MS: logP = 2,04; Masse (m/z) = 207 [M+H]⁺.

Die Herstellung des Ethylesters wird in WO2007/071900A1 beschrieben.

### 3-(Difluormethoxy)-1-methyl-1H-pyrazol-5-carbonsäure

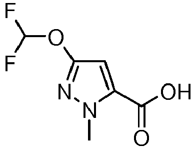

200 mg (0,97 mmol) Methyl-3-(difluormethoxy)-1-methyl-1*H*-pyrazol-5-carboxylat werden in 6,5 mL Methanol gelöst und 1,94 mL 1 M Natronlauge wird zugegeben. Das Reaktionsgemisch wird über Nacht bei Raumtemperatur gerührt, dann mit 1 M Salzsäure versetzt und mit Ethylacetat extrahiert. Die organische Phase wird über Natriumsulfat getrocknet, filtriert und im Vakuum eingeengt. Es werden 180 mg 3-Difluormehoxy-1-methyl-1*H*-pyrazol-5-carbonsäure (97%) erhalten.

¹H-NMR (400 MHz, d₆-DMSO): δ = 7,26 (t, 1H), 6,49 (s, 1H), 3,99 (s, 3H) ppm.

HPLC-MS: logP = 1,15; Masse (m/z) = 193 [M+H]⁺.

### Methyl-4-chlor-3-(difluormethoxy)-1-methyl-1H-pyrazol-5-carboxylat

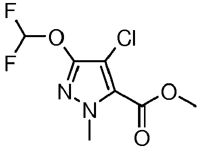

Eine Lösung von 400 mg (1,94 mmol) Methyl-3-(difluormethoxy)-1-methyl-1*H*-pyrazol-5-carboxylat in 10 mL *N,N*-Dimethylformamid wird bei 0 °C mit 389 mg (2,91 mmol) N-Chlorsuccinimid versetzt und dann für 8 Stunden bei 80 °C gerührt. Die Reaktionslösung wird auf Wasser gegossen, zweimal mit Ethylacetat extahiert. Die vereinigten organischen Phasen werden über Natriumsulfat getrocknet und im Vakuum eingeengt. Es werden 466 mg Methyl-4-chlor-3-(difluormethoxy)-1-methyl-1*H*-pyrazol-5-carboxylat (99%) erhalten.

¹H-NMR (400 MHz, d₆-DMSO): δ = 7,25 (t, 1H), 4,01 (s, 3H), 3,91 (s, 3H) ppm.

HPLC-MS: logP = 2,57. Masse (m/z) 241 [M+H]⁺.

### Methyl-4-brom-3-(difluormethoxy)-1-methyl-1H-pyrazol-5-carboxylat

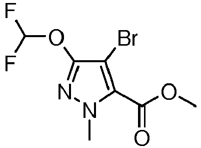

Eine Lösung von 700 mg (3,39 mmol) Methyl-3-(difluormethoxy)-1-methyl-1*H*-pyrazol-5-carboxylat in 20 mL Chloroform wird tropfenweise mit einer Lösung von 570 mg (3,56 mmol) Brom in 10 mL Chloroform versetzt und dann für drei Tage bei Raumtemperatur verrührt. Das Reaktionsgemisch wird auf 80 mL Wasser mit etwas Natriumbisulfit gegeben und geschüttelt. Die organische Phase wird abgetrennt, über Magnesiumsulfat getrocknet, filtriert und im Vakuum eingeengt: 792 mg Methyl-4-brom-3-(difluormethoxy)-1-methyl-1*H*-pyrazol-5-carboxylat (82%) fallen als weiße Kristalle an.

¹H-NMR (400 MHz, d₆-DMSO): δ = 7,24 (t, 1H), 4,01 (s, 3H), 3,90 (s, 3H) ppm.

HPLC-MS: logP = 2,62; Masse (m/z) = 285; 287 [M+H]⁺.

### Methyl-3-(difluormethoxy)-4-iod-1-methyl-1H-pyrazol-5-carboxylat

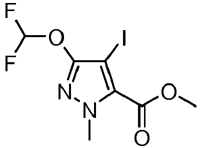

Eine Lösung von 500 mg (2,42 mmol) Methyl-3-(difluormethoxy)-1-methyl-1*H*-pyrazol-5-carboxylat in 20 mL Acetonitril wird mit 665 mg (1,21 mmol) Ammoniumcer(IV)nitrat und 369 mg (1,45 mmol) Iod versetzt und für drei Stunden unter Rückfluß erhitzt und über Nacht bei Raumtemperatur nachgerührt. Das Reaktionsgemisch wird mit Wasser und Ethylacetat verdünnt, die organische Phase wird mit gesättigter Natriumthiosulfat-Lösung gewaschen, über Natriumsulfat getrocknet, filtriert und im Vakuum eingeengt. Es werden 750 mg Methyl-3-(difluormethoxy)-4-iod-1-methyl-1*H*-pyrazol-5-carboxylat (93%) erhalten.

¹H-NMR (400 MHz, d₆-DMSO): δ = 7,22 (t, 1H), 4,02 (s, 3H), 3,88 (s, 3 H) ppm.

HPLC-MS: logP = 2,69; Masse (m/z) = 333 [M+H]⁺.

Analog der oben genannten Verseifung wurden die folgenden Säuren hergestellt:

### 4-Chlor-3-(difluormethoxy)-1-methyl-1H-pyrazol-5-carbonsäure

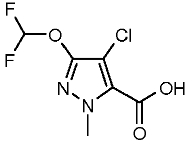

¹H-NMR (400 MHz, d₆-DMSO): δ = 7,23 (t, 1H), 3,99 (s, 3H) ppm.

HPLC-MS: logP = 1,50; Masse (m/z) = 227 [M+H]⁺.

### 4-Brom-3-(difluormethoxy)-1-methyl-1H-pyrazol-5-carbonsäure

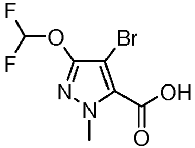

¹H-NMR (400 MHz, d₆-DMSO): δ = 7,31 (t, 1H), 4,01 (s, 3H) ppm;

HPLC-MS: logP = 1,52; Masse (m/z) = 271 [M+H]⁺.

### 4-Iod-3-(difluormethoxy)-1-methyl-1H-pyrazol-5-carbonsäure

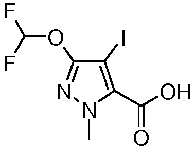

¹H-NMR (400 MHz, d₆-DMSO): δ = 7,20 (t, 1H), 4,01 (s, 3H) ppm.

HPLC-MS: logP = 1,64; Masse (m/z) = 319 [M+H]⁺.

### Methyl-1-methyl-3-(2,2,2-trifluorethoxy)-1H-pyrazol-5-carboxylat

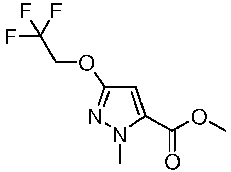

Eine Lösung von 200 mg (1,28 mmol) 3-Hydroxy-1-methyl-5-pyrazolcarbonsäure-methylester (Herstellung siehe oben) in 10 mL *N,N*-Dimethylformamid wird mit 254 mg (2,56 mmol) 2,2,2-Trifluorethyl-4-methylbenzolsulfonat und 138 mg (2,56 mmol) Kaliumcarbonat versetzt und das Reaktionsgemisch wird für fünf Stunden auf 100 °C erwärmt und über zwei Tage bei Raumtemperatur nachgerührt. Das Reaktionsgemisch auf Wasser gegeben und mehrmals mit Ethylacetat extrahiert. Die organische Phase wird über Natriumsulfat getrocknet, filtriert und im Vakuum am Rotationsverdampfer eingeengt. Es werden 500 mg Methyl-1-methyl-3-(2,2,2-trifluorethoxy)-1*H*-pyrazol-5-carboxylat (50%ig, 82%) erhalten.

¹H-NMR (400 MHz, d₆-DMSO): δ = 6,41 (s, 1H), 4,77 (m, 2H), 3,94 (s, 3H), 3,83 (s, 3H) ppm.

HPLC-MS: logP = 2,61; Masse (m/z) = 239 [M+H]⁺.

### Methyl-4-fluor-1-methyl-3-(2,2,2-trifluorethoxy)-1H-pyrazol-5-carboxylat

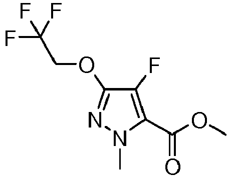

Eine Lösung von 300 mg (1,26 mmol) Methyl-1-methyl-3-(2,2,2-trifluorethoxy)-1*H*-pyrazol-5-carboxylat und 404 mg (1,52 mmol) 1-(Chlormethyl)-4-fluor-1,4-diazoniabicyclo[2.2.2]octanditetrafluoroborat (Selectfluor) in 37,5 mL Acetonitril wird für fünf Stunden unter Rückfluß erhitzt. Nach dem Erkalten wird die Lösung mit 1 M Salzsäure versetzt und zweimal mit Ethylacetat extrahiert. Die organische Phase wird mit ges. Natriumchloridlösung gewaschen, über Na₂SO₄ getrocknet, filtriert und im Vakuum eingeengt. Chromatographische Reinigung an Kieselgel gibt 190 mg Methyl-4-fluor-1-methyl-3-(2,2,2-trifluorethoxy)-1*H*-pyrazol-5-carboxylat (26%ig, 15%).

HPLC-MS: logP = 2,79; Masse (m/z) = 257 [M+H]⁺.

Die Chlorierung, Bromierung und Iodierung verläuft analog den oben gezeigten Umsetzungen:

### Methyl-4-chlor-1-methyl-3-(2,2,2-trifluorethoxy)-1H-pyrazol-5-carboxylat

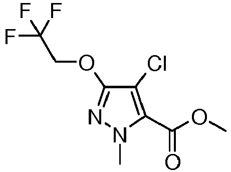

¹H-NMR (400 MHz, d₆-DMSO): δ = 4,83 (m, 2H), 3,95 (s, 3H), 3,89 (s, 3H) ppm.

HPLC-MS: logP = 3,08; Masse (m/z) = 273 [M+H]⁺.

### Methyl-4-brom-1-methyl-3-(2,2,2-trifluorethoxy)-1H-pyrazol-5-carboxylat

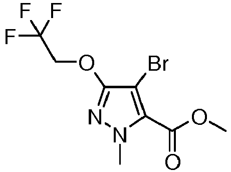

¹H-NMR (400 MHz, d₆-DMSO): 8 = 4,81 (m, 2H), 3,97 (s, 3H), 3,88 (s, 3H) ppm.

HPLC-MS: logP = 3,14; Masse (m/z) = 317, 319 [M+H]⁺.

### Methyl-4-iod-1-methyl-3-(2,2,2-trifluorethoxy)-1H-pyrazol-5-carboxylat

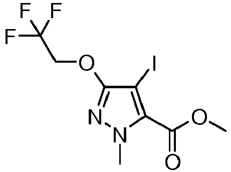

¹H-NMR (400 MHz, d₆-DMSO): δ = 4,81 (m, 2H), 3,98 (s, 3H), 3,87 (s, 3H) ppm.

HPLC-MS: logP = 3,17; Masse (m/z) = 365 [M+H]⁺.

Analog der oben genannten Verseifung wurden die folgenden Säuren hergestellt:

### 1-Methyl-3-(2,2,2-trifluorethoxy)-1H-pyrazol-5-carbonsäure

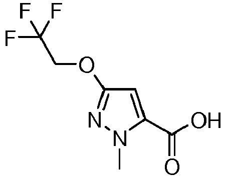

¹H-NMR (400 MHz, d₆-DMSO): δ = 4,74 (m, 2H), 3,94 (s, 3H) ppm.

HPLC-MS: logP = 1,65; Masse (m/z) = 225 [M+H]⁺.

### 4-Fluor-1-methyl-3-(2,2,2-trifluorethoxy)-1H-pyrazol-5-carbonsäure

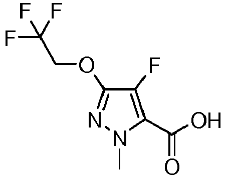

¹H-NMR (400 MHz, d₆-DMSO): δ = 4,83 (m, 2H), 3,89 (s, 3H) ppm.

HPLC-MS: logP = 1,83; Masse (m/z) = 243 [M+H]⁺.

### 4-Chlor-1-methyl-3-(2,2,2-trifluorethoxy)-1H-pyrazol-5-carbonsäure

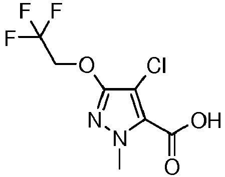

¹H-NMR (400 MHz, d₆-DMSO): δ = 4,85 (m, 2H), 3,94 (s, 3H) ppm.

HPLC-MS: logP = 2,00; Masse (m/z) = 259 [M+H]⁺.

### 4-Brom-1-methyl-3-(2,2,2-trifluorethoxy)-1H-pyrazol-5-carbonsäure

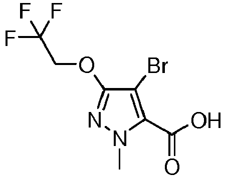

¹H-NMR (400 MHz, d₆-DMSO): δ = 4,84 (m, 2H), 3,96 (s, 3H) ppm.

HPLC-MS: logP = 2,03; Masse (m/z) = 303 [M+H]⁺.

### 4-Iod-1-methyl-3-(2,2,2-trifluorethoxy)-1H-pyrazol-5-carbonsäure

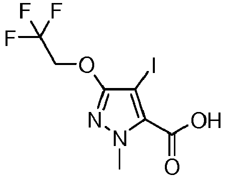

¹H-NMR (400 MHz, d₆-DMSO): δ = 4,75 (m, 2H), 3,87 (s, 3H) ppm.

HPLC-MS: logP = 2,11; Masse (m/z) = 351 [M+H]⁺.

### 4-Brom-3-cyclopropyl-1-methyl-1H-pyrazol-5-carbonsäure

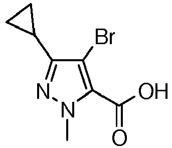

Eine auf 0 °C gekühlte Lösung von 100 mg (0,60 mmol) 3-Cyclopropyl-1-methyl-1H-pyrazol-5-carbonsäure in 5 mL Essigsäure wird mit 173 mg (1,08 mmol) Brom versetzt und über Nacht bei Raumtemperatur nachgerührt. Essigsäure und Brom werden am Rotationsverdampfer abgezogen und der feste Rückstand wird mit Cyclohexan und wenig Diethylether gewaschen. Es werden 69,0 mg 4-Brom-3-cyclopropyl-1-methyl-1H-pyrazol-5-carbonsäure (47%) als gelblicher Feststoff erhalten.

¹H-NMR (400 MHz, d₆-DMSO): δ = 3,96 (s, 3H), 1,84 (m, 1H), 0,89 (m, 2H), 0,77 (m, 2H) ppm.

HPLC-MS: logP = 1,76; Masse (m/z) = 245 [M+H]⁺.

### Ethyl-3-cyclopropyl-4-iod-1-methyl-1H-pyrazol-5-carboxylat

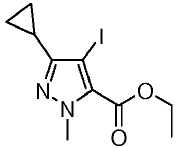

Eine Lösung von 500 mg (2,57 mmol) Ethyl-3-cyclopropyl-1-methyl-1H-pyrazol-5-carboxylat (aus Ethyl-3-cyclopropyl-1H-pyrazol-5-carboxylat nach Bioorg. Med. Chem. Lett. 1996, 6, 1819-1824 hergestellt) in 5 mL Acetonitril wird mit 706 mg (1,28 mmol) Ammoniumcer(IV)nitrat und 392 mg (1,53 mmol) Iod versetzt und für drei Stunden unter Rückfluß erhitzt und über Nacht bei Raumtemperatur nachgerührt. Das Reaktionsgemisch wird mit Wasser und Ethylacetat verdünnt, die organische Phase wird mit gesättigter Natriumthiosulfat-Lösung gewaschen, über Natriumsulfat getrocknet, filtriert und im Vakuum eingeengt. Nach chromatographischer Aufreinigung werden 506 mg Ethyl-3-cyclopropyl-4-iod-1-methyl-1*H*-pyrazol-5-carboxylat (66%) erhalten.

¹H-NMR (400 MHz, d₆-DMSO): δ = 4,34 (m, 2H), 3,98 (s, 3H), 1,82 (m, 1H), 1,36 (m, 3H), 0,89 (m, 2H), 0,75 (m, 2H) ppm.

HPLC-MS: logP = 3,56; Masse (m/z) = 321 [M+H]⁺.

### Ethyl-3-cyclopropyl-1-methyl-4-(trifluormethyl)-1H-pyrazol-5-carboxylat

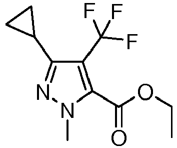

268 mg (1,40 mmol) Kupfer(I)iodid und 65,3 mg (1,12 mmol) Kaliumfluorid werden in einem ausgeheiztem Vial unter Argon vorgelegt und 3 mL *N*,*N*-Dimethylformamid und 300 mg (0,93 mmol) Ethyl-3-cyclopropyl-4-iod-1-methyl-1*H*-pyrazol-5-carboxylat und 0,28 mL (1,87 mmol) Trimethyl(trifluormethyl)silan werden zugegeben. Das Reaktionsgemisch wird im Ultraschall entgast, mit Argon gespült und das geschlossene Vial wird für drei Stunden auf 80 °C erhitzt. Die erkaltete Reaktionslösung wird mit wenig Wasser versetzt, mit Ethylacetat verdünnt, über Kieselgel filtriert (Ethylacetat) und am Rotationsverdampfer eingeengt. Nach chromatographischer Aufreinigung an Kieselgel werden 212 mg Ethyl-3-cyclopropyl-1-methyl-4-(trifluormethyl)-1*H-*pyrazol-5-carboxylat (86%) erhalten.

¹H-NMR (400 MHz, d₆-DMSO): δ = 4,36 (m, 2H), 3,94 (s, 3H), 1,97 (m, 1H), 1,31 (m, 3H), 0,90 (m, 2H), 0,83 (m, 2H) ppm.

HPLC-MS: logP = 3,66; Masse (m/z) = 263 [M+H]⁺.

### 3-Cyclopropyl-1-methyl-4-(trifluormethyl)-1H-pyrazol-5-carbonsäure

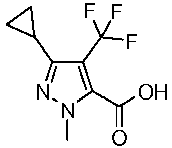

Diese Verbindung wird analog zur Herstellung von 3-(Difluormethoxy)-1-methyl-1*H*-pyrazol-5-carbonsäure aus Ethyl-3-cyclopropyl-1-methyl-4-(trifluormethyl)-1*H*-pyrazol-5-carboxylat hergestellt:
¹H-NMR (400 MHz, d₆-DMSO): δ = 3,92 (s, 3H), 1,94 (m, 1H), 0,89 (m, 2H), 0,82 (m, 2H) ppm;
HPLC-MS: logP = 1,74; Masse (m/z) = 235 [M+H]⁺.

### Methyl-1-methyl-3-[4-(trifluormethyl)phenyl]-1H-pyrazol-5-carboxylat

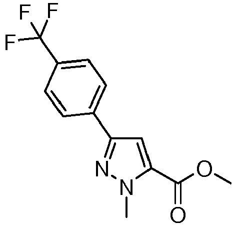

1,50 g (5,36 mmol) Methyl-1-methyl-3-(4,4,5,5-tetramethyl-1,3,2-dioxaborolan-2-yl)-1*H*-pyrazol-5-carboxylat (Herstellung siehe WO2007/034278A2), 1,21 g (5,36 mmol) 1-Brom-4-(trifluormethyl)benzol und Bis(triphenylphosphine)palladium(II)chlorid werden unter Argon vorgelegt und mit 6,69 mL einer 2 M Lösung von Natriumcarbonat in Wasser und 20 mL Dioxan versetzt. Das Reaktionsgemisch wird für drei Stunden auf 100 °C erhitzt und nach dem Abkühlen über Celite und Natriumsulfat filtriert. Der Filterkuchen wird mit Ethylacetat nachgewaschen und das Filtrat wird am Rotationsverdampfer eingeengt. Nach chromatographischer Aufreinigung an Kieselgel werden 529 mg Methyl-1-methyl-3-[4-(trifluormethyl)phenyl]-1*H*-pyrazol-5-carboxylat (35%) erhalten (Herstellung siehe auch DD1984/210265).

¹H-NMR (400 MHz, d₆-DMSO): δ = 8,05 (d, 2H), 7,75 (d, 2H), 7,44 (s, 1H), 4,16 (s, 3H), 3,88 (s, 3H) ppm.

HPLC-MS: logP = 3,82; Masse (m/z) = 285 [M+H]⁺.

### Methyl-4-iod-1-methyl-3-[4-(trifluormethyl)phenyl]-1H-pyrazol-5-carboxylat

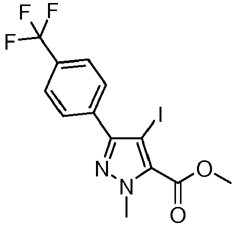

Diese Verbindung wird analog zur Herstellung von Ethyl-3-cyclopropyl-4-iod-1-methyl-1*H-*pyrazol-5-carboxylat aus Methyl-1-methyl-3-[4-(trifluormethyl)phenyl]-1*H*-pyrazol-5-carboxylat und Iod hergestellt.

¹H-NMR (400 MHz, d₆-DMSO): δ = 7,97 (d, 2H), 7,82 (d, 2H), 4,16 (s, 3H), 3,92 (s, 3H) ppm.

HPLC-MS: logP = 4,23; Masse (m/z) = 411 [M+H]⁺.

### Methyl-1-methyl-4-(trifluormethyl)-3-[4-(trifluormethyl)phenyl]-1H-prazol-5-carboxylat

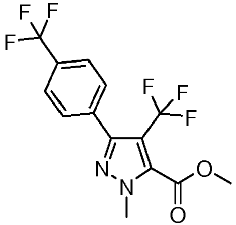

Diese Verbindung wird analog zur Herstellung von Ethyl-3-cyclopropyl-1-methyl-4-(trifluormethyl)-1*H*-pyrazol-5-carboxylat aus Methyl-4-iod-1-methyl-3-[4-(trifluormethyl)phenyl]-1*H*-pyrazol-5-carboxylat hergestellt.

¹H-NMR (400 MHz, d₆-DMSO): δ = 7,86 (d, 2H), 7,72 (d, 2H), 4,12 (s, 3H), 3,96 (s, 3H) ppm.

HPLC-MS: logP = 4,14; Masse (m/z) = 353 [M+H]⁺.

### 1-Methyl-4-(trifluormethyl)-3-[4-(trifluormethyl)-phenyl]-1H-pyrazol-5-carbonsäure

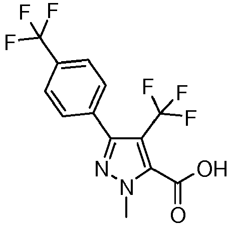

Diese Verbindung wird analog zur Herstellung von 3-(Difluormethoxy)-1-methyl-1*H*-pyrazol-5-carbonsäure aus Methyl-1-methyl-4-(trifluormethyl)-3-[4-(trifluormethyl)phenyl]-1*H*-pyrazol-5-carboxylat hergestellt.

¹H-NMR (400 MHz, d₆-DMSO): δ = 7,85 (d, 2H), 7,71 (d, 2H), 4,09 (s, 3H) ppm.

HPLC-MS: logP = 1,24; Masse (m/z) = 339 [M+H]⁺.

### 5-(Benzylsulfanyl)-1-methyl-3-(pentafluorethyl)-4-(trifluormethyl)-1H-pyrazol

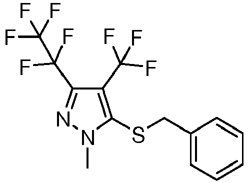

Eine Lösung aus 3,00 g (10,5 mmol) 5-Fluor-1-methyl-3-(pentafluorethyl)-4-(trifluormethyl)-1*H-*pyrazol und 1,48 mL (12,6 mmol) Benzylmercaptan werden bei 0 °C in 24 mL Acetonitril vorgelegt. Dann werden 4,78 mL (34,3 mmol) Triethylamin zugetropft und das Reaktionsgemisch für zwei Stunden zwischen 0 °C und 10 °C gerührt. Nach Einengen im Vakuum wird das erhaltene Öl chromatographisch an Kieselgel gereinigt und es werden 3,19 g 5-(Benzylsulfanyl)-1-methyl-3-(pentafluorethyl)-4-(trifluormethyl)-1*H*-pyrazol (78%) erhalten.

¹H-NMR (400 MHz, d₆-DMSO): δ = 7,27 (m, 3H), 7,08 (m, 2H), 4,13 (s, 2H), 3,68 (s, 3H) ppm.

HPLC-MS: logP = 5,00; Masse (m/z) = 391 [M+H]⁺.

### 1-Methyl-3-(pentafluorethyl)-4-(trifluormethyl)-1H-pyrazol-5-sulfonylchlorid

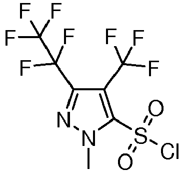

Eine Lösung von 1,00 g (2,49 mmol) 5-(Benzylsulfanyl)-1-methyl-3-(pentafluorethyl)-4-(trifluormethyl)-1*H*-pyrazol in 17 mL Dichlormethan wird mit 20 mL Salzsäure (16%) und 30 mL Natriumhypochloridlösung (13%) versetzt und für 18 Stunden bei Raumtemperatur gerührt. Nach Zugaben von 10 mL Chlorlauge und weiteren sieben Stunden bei Raumtemperatur wird mehrfach mit Dichlormethan extrahiert, die organische Phase über Magnesiumsulfat getrocknet, filtriert und im Vakuum eingeengt. Das Rohprodukt wird ohne weitere Aufreinigung zu den entsprechenden Sulfonamiden umgesetzt.

¹H-NMR (400 MHz, d₆-DMSO): δ = 4,10 (s, 3H) ppm.

### Ethyl-3-(hydroxymethyl)-1-methyl-4-(trifluormethyl)-1H-pyrazol-5-carboxylat

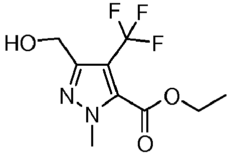

Eine Lösung von 6,00 g (20,4 mmol) Diethyl-1-methyl-4-(trifluormethyl)-1*H*-pyrazol-3,5-dicarboxylat in 80 mL Tetrahydrofuran wird unter Argon bei -78 °C mit 38,9 mL (42,8 mmol) einer 1,1 M Lösung von Diisobutylaluminiumhydrid in Cyclohexan versetzt und über Nacht bei dieser Temperatur gerührt. Die Reaktionsmischung wird mit Wasser versetzt und zweimal mit Ethylacetat extrahiert. Die organische Phase wird über Natriumsulfat getrocknet, über Kieselgel filtriert und im Vakuum eingeengt. Chormatographische Reinigung an Kieselgel ergibt 4,10 g an Ethyl-3-(hydroxymethyl)-1-methyl-4-(trifluormethyl)-1*H*-pyrazol-5-carboxylat (80%).

¹H-NMR (400 MHz, d₆-DMSO): δ = 5,03 (br. s, 1H), 4,49 (s, 2H), 4,37 (q, 2H), 4,02 (s, 3H), 1,32 (t, 3H) ppm.

HPLC-MS: logP = 1,74; Masse (m/z) = 253 [M+H]⁺.

### Ethyl-3-formyl-1-methyl-4-(trifluormethyl)-1H-pyrazol-5-carboxylat

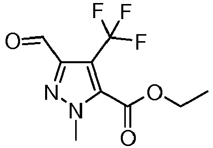

Eine Lösung von 1,50 g (5,95 mmol) Ethyl-3-(hydroxymethyl)-1-methyl-4-(trifluormethyl)-1*H-*pyrazol-5-carboxylat in 100 mL Dichlormethan wird mit 4,14 g (47,6 mmol) Mangan(IV)oxid versetzt und für vier Stunden unter Rückfluß erhitzt. Das erkaltete Reaktionsgemisch wird über Celite filtriert und am Rotationsverdampfer eingeengt. Es werden 1,36 g an Ethyl-3-formyl-1-methyl-4-(trifluormethyl)-1*H*-pyrazol-5-carboxylat (91%) erhalten.

¹H-NMR (400 MHz, d₃-Acetonitril): δ = 9,99 (s, 1H), 4,44 (q, 2H), 4,11 (s, 3H), 1,37 (t, 3H) ppm.

HPLC-MS: logP = 2,40; Masse (m/z) = 251 [M+H]⁺.

### Ethyl-3-(difluormethyl)-1-methyl-4-(trifluormethyl)-1H-pyrazol-5-carboxylat

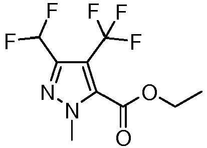

Eine Lösung von 1,00 g (4,00 mmol) Ethyl-3-formyl-1-methyl-4-(trifluormethyl)-1*H*-pyrazol-5-carboxylat in 40 mL Dichlormethan wird unter Argon auf -10 °C gekühlt und 1,11 mL (8,39 mmol) Diethylaminoschwefeltrifluorid wird über 10 Minuten zugetropft. Das Reaktionsgemisch wird für eine Stunde bei -10 °C gerührt, erst mit festem Natriumcarbonat und dann mit ges. Natriumcarbonatlösung versetzt. Die wässrige Phase wird zweimal mit Dichlormethan extrahiert, die organische Phase wird über Natriumsulfat getrocknet und eingeengt. Chormatographische Reinigung an Kieselgel ergibt 585 mg an Ethyl-3-(difluormethyl)-1-methyl-4-(trifluormethyl)-1*H-*pyrazol-5-carboxylat (54%).

¹H-NMR (400 MHz, d₆-DMSO): δ = 7,11 (t, 1H), 4,41 (q, 2H), 4,11 (s, 3H), 1,33 (t, 3H) ppm.

HPLC-MS: logP = 3,05; Masse (m/z) = 273 [M+H]⁺.

### 3-(Difluormethyl)-1-methyl-4-(trifluormethyl)-1H-pyrazol-5-carbonsäure

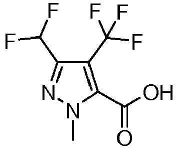

Eine Lösung von 600 mg (2,20 mmol) Ethyl-3-(difluormethyl)-1-methyl-4-(trifluormethyl)-1*H-*pyrazol-5-carboxylat in 10 mL Methanol wird mit 331 mg (3,31 mmol) 40%iger Natronlauge versetzt und für drei Stunden bei Raumtemperatur gerührt. Die Reaktionsmischung wird eingeengt, mit Wasser versetzt und mit Diethylether extrahiert. Die wässrige Phase wird mit verdünnter Salzsäure angesäuert und zweimal mit Ethylacetat extrahiert. Die organische Phase wird über Natriumsulfat getrocknet und nach Filtration eingeengt. Es werden 452 mg an 3-(Difluormethyl)-1-methyl-4-(trifluormethyl)-1*H*-pyrazol-5-carbonsäure (84%) erhalten.

¹H-NMR (400 MHz, d₆-DMSO): δ = 7,07 (t, 1H), 4,09 (s, 3H) ppm.

HPLC-MS: logP = 1,10; Masse (m/z) = 245 [M+H]⁺.

### Ethyl-3-[cyclopropyl(hydroxy)methyl]-1-methyl-4-(trifluormethyl)-1H-pyrazol-5-carboxylat

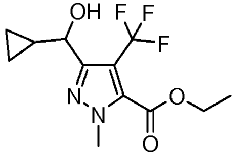

Eine Lösung von 1,82 g (7,28 mmol) Ethyl-3-formyl-1-methyl-4-(trifluormethyl)-1*H*-pyrazol-5-carboxylat in 80 mL Tetrahydrofuran wird unter Argon bei -78 °C mit 17,5 mL (8,73 mmol) 0,5 M Cyclopropylmagenesiumbromid-Lösung in Tetrahydrofuran versetzt, für 30 Minuten bei dieser Temperatur und dann über Nacht bei Raumtemperatur gerührt. Die Reaktionslösung wird mit ges. Ammoniumchloridlösung versetzt, mit Ethylacetat extrahiert, die organische Phase über Natriumsulfat getrocknet und am Rotationsverdampfer eingeengt. Chormatographische Reinigung an Kieselgel ergibt 0,67 g an Ethyl-3-[cyclopropyl(hydroxy)methyl]-1-methyl-4-(trifluormethyl)-1*H*-pyrazol-5-carboxylat (31%).

¹H-NMR (400 MHz, d₆-DMSO): δ = 5,08 (d, 1H), 4,37 (q, 2H), 4,06 (m, 1H), 4,01 (s, 3H), 1,31 (m, 4H), 0,52 (m, 1H), 0,43 (m, 2H), 0,18 (m, 1H) ppm.

HPLC-MS: logP = 2,38; Masse (m/z) = 275 [M-H₂O+H]⁺.

### 3-[Cyclopropyl(hydroxy)methyl]-1-methyl-4-(trifluormethyl)-1H-pyrazol-5-carbonsäure

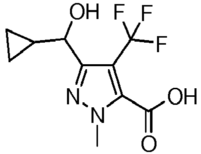

Diese Verbindung wird analog zur Herstellung von 3-(Difluormethyl)-1-methyl-4-(trifluormethyl)-1*H*-pyrazol-5-carbonsäure aus Ethyl-3-[cyclopropyl(hydroxy)methyl]-1-methyl-4-(trifluormethyl)-1*H*-pyrazol-5-carboxylat hergestellt.

¹H-NMR (400 MHz, d₆-DMSO): δ = 3,88 (m, 1H), 3,83 (s, 3H), 1,16 (m, 1H), 1,01 (t, 3H), 0,33 (m, 1H), 0,25 (m, 2H), 0,02 (m, 1H) ppm.

HPLC-MS: logP = 0,72; Masse (m/z) = 247 [M-H₂O+H]⁺.

### Ethyl-3-(2-hydroxypropan-2-yl)-1-methyl-4-(trifluormethyl)-1H-pyrazol-5-carboxylat

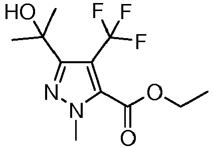

Zu einer Lösung von 2,00 g (6,80 mmol) Ethyl-3-formyl-1-methyl-4-(trifluormethyl)-1*H*-pyrazol-5-carboxylat in 30 mL Tetrahydrofuran werden bei -78 °C 4,76 mL (14,3 mmol) einer 3 M Methylmagnesiumchlorid in Tetrahydrofuran über 10 Minuten zugetropft. Das Reaktionsgemisch wird für vier Stunden bei -78 °C gerührt und dann auf ges. Ammoniumchloridlösung gegeben und dreimal mit Ethylacetat extrahiert. Die organische Phase wird über Natriumsulfat getrocknet und am Rotationsverdampfer eingeengt. Chormatographische Reinigung an Kieselgel ergibt 976 mg an Ethyl-3-(2-hydroxypropan-2-yl)-1-methyl-4-(trifluormethyl)-1*H*-pyrazol-5-carboxylat (51%).

¹H-NMR (400 MHz, d₆-DMSO): δ = 4,88 (br. s, 1H), 4,38 (q, 2H), 3,88 (s, 3H), 1,48 (s, 6H), 1,31 (t, 3H) ppm.

HPLC-MS: logP = 2,33; Masse (m/z) = 263 [M-H₂O+H]⁺.

### Ethyl-3-(2-fluorpropan-2-yl)-1-methyl-4-(trifluormethyl)-1H-pyrazol-5-carboxylat

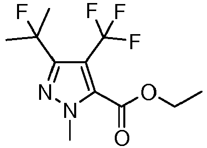

Zu einer Lösung aus 1,00 g (3,57 mmol) Ethyl-3-(2-hydroxypropan-2-yl)-1-methyl-4-(trifluormethyl)-1*H*-pyrazol-5-carboxylat in 20 mL Dichlormethan tropft man bei -10 °C 0,52 ml (3,9 mmol) Diethylaminoschwefeltrifluorid. Es wird eine Stunde bei -10 °C nachgerührt, Natriumcarbonat zugegeben, und 10 Minuten bei Raumtemperatur gerührt. Nach Zugabe von ges. Natriumcarbonatlösung wird mit Dichlormethan extrahiert, die vereinigten organischen Phasen über Natriumsulfat getrocknet und im Vakuum am Rotationsverdampfer eingeengt. Man reinigt per Flashchromatographie an Kieselgel (Cyclohexan/Ethylacetat) zu 700 mg Ethyl-3-(2-fluorpropan-2-yl)-1-methyl-4-(trifluormethyl)-1*H*-pyrazol-5-carboxylat (67%).

¹H-NMR (400 MHz, d₆-DMSO): δ = 4,40 (q, 2H), 3,95 (s, 3H), 1,70 (d, 6H), 1,32 (t, 3H) ppm.

HPLC-MS: logP = 3,55; Masse (m/z) = 283 [M+H]⁺.

### 3-(2-Fluorpropan-2-yl)-1-methyl-4-(trifluormethyl)-1H-pyrazol-5-carbonsäure

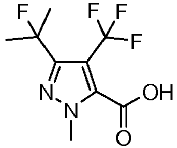

Eine Lösung von 700 mg (2,48 mmol) Ethyl-3-(2-fluorpropan-2-yl)-1-methyl-4-(trifluormethyl)-1*H*-pyrazol-5-carboxylat in 10 mL Methanol wird mit 208 mg (3,72 mmol) Kaliumhydroxid und 1 mL Wasser versetzt und für eine Stunde auf 50 °C erhitzt. Die Reaktionsmischung wird eingeengt, mit Wasser versetzt und mit Diethylether extrahiert. Die wässrige Phase wird mit verdünnter Salzsäure angesäuert, zweimal mit Ethylacetat extrahiert, die organische Phase wird über Natriumsulfat getrocknet und nach Filtration eingeengt. Es werden 498 mg 3-(2-Fluorpropan-2-yl)-1-methyl-4-(trifluormethyl)-1*H*-pyrazol-5-carbonsäure (79%) erhalten.

¹H-NMR (400 MHz, d₆-DMSO): δ = 3,92 (s, 3H), 1,69 (d, 6H) ppm.

HPLC-MS: logP = 1,43; Masse (m/z) = 255 [M+H]⁺.

### Ethyl-2-(pentafluorethyl)-4-(trifluormethyl)-1,3-thiazol-5-carboxylat

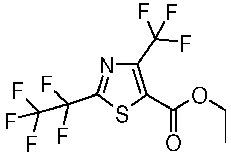

Ein Gemisch aus 2,00 g (12,3 mmol) 2,2,3,3,3-Pentafluorpropanamid und 2,48 g (6,13 mmol) Lawesson-Reagenz werden mit 20 mL Tetrahydrofuran versetzt und für 3,5 Stunden unter Rückfluß erhitzt. Nach einengen im Vakuum wird das entstandene 2,2,3,3,3-Pentafluorpropanthioamid destillativ gereinigt (561 mg).

557 mg (3,10 mmol) 2,2,3,3,3-Pentafluorpropanthioamid und 746 mg (3,41 mmol) Ethyl-2-chlor-4,4,4-trifluor-3-oxobutanoat (kommerziell erhältlich oder nach Journal of Fluorine Chemistry 2004, 125, 1287-1290 herstellbar) werden in 15 mL Acetonitril gelöst und 0,86 mL (6,19 mmol) Triethylamin zugetropft. Das Reaktionsgemisch wird für 5 Stunden am Rückfluß erhitzt und dann über zwei Tage bei Raumtemperatur belassen. Chromatographische Reinigung an Kieselgel ergibt 568 mg Ethyl-2-(pentafluorethyl)-4-(trifluormethyl)-1,3-thiazol-5-carboxylat (13%).

¹H-NMR (400 MHz, d₆-DMSO): δ = 4,42 (q, 2H), 1,33 (t, 3H) ppm.

HPLC-MS: logP = 4,36.

### 2-(Pentafluorethyl)-4-(trifluormethyl)-1,3-thiazol-5-carbonsäure

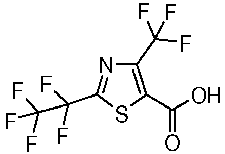

Eine Lösung von 540 mg (1,57 mmol) Ethyl-2-(pentafluorethyl)-4-(trifluormethyl)-1,3-thiazol-5-carboxylat in 3 mL Methanol und 1 mL Wasser wird mit 94,4 mg (2,36 mmol) Natronlauge versetzt und für zwei Stunden auf 60 °C erhitzt. Die Reaktionsmischung mit konz. Salzsäure angesäuert, viermal mit Dichlormethan extrahiert, die organische Phase wird mit ges. Natriumchloridlösung gewaschen, über Magnesiumsulfat getrocknet und nach Filtration eingeengt. Es werden 417 mg (min. 50%ig, max. 82%) an 2-(Pentafluorethyl)-4-(trifluormethyl)-1,3-thiazol-5-carbonsäure erhalten.

HPLC-MS: logP = 2,06; Masse (m/z) = 316 [M+H]⁺.

### 2,2,2-Trifluor-1-[1-methyl-4-(trifluormethyl)-1H-imidazol-2-yl]ethanamin

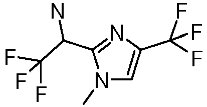

2,0 g (8,12 mmol) 2,2,2-Trifluor-1-[1-methyl-4-(trifluormethyl)-1*H*-imidazol-2-yl]ethanon [Herstellung in Analogie zu Synthesis 2008, 6, 948-956] wird unter Schutzgasatmosphäre in 40 mL Toluol gelöst und dann mit 8,93 mL (8,93 mmol) einer 1 M Lösung von Lithium(bistrimethylsilyl)amid in Toluol versetzt. Die Lösung wird anschließend 1h bei Raumtemperatur gerührt. Dann werden 8,12 mL (16,2 mmol) einer 2 M Lösung von Borandimethylsulfid-Komplex in Tetrahydrofuran hinzugefügt und weitere 16 Stunden bei Raumtemperatur gerührt. Anschließend werden 6,0 mL 2 M Natronlauge hinzugefügt. Die organische Phase wird über Magnesiumsulfat getrocknet und dann unter vermindertem Druck am Rotationsverdampfer eingeengt. Das Produkt wird an säulenchromatographisch an Kieselgel aufgereinigt (Dichlormethan: Methanol = 9:1). Man erhält 0,85 g 2,2,2-Trifluor-1-[1-methyl-4-(trifluormethyl)-1*H*-imidazol-2-yl]ethanamin (42%) als gelbes Öl.

¹H-NMR (400 MHz, d₃-Acetonitril): δ = 7,60 (s, 1H), 4,80 (m, 1H), 3,84 (s, 3H) ppm.

¹³C-NMR (400 MHz, d₃-Acetonitril): δ = 145,7, 130,5, 126,2, 123,9, 123,0, 51,0, 34,1 ppm.

**Tabelle 1**

| | | | | | | | | | | | | | | |
|---|---|---|---|---|---|---|---|---|---|---|---|---|---|---|
| 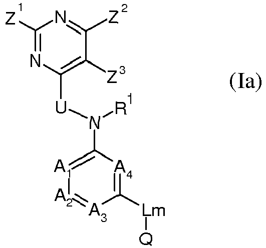 | | | | | | | | | | | | | | |

| **Bsp**.-**Nr**. | **Verfahren** | **Z¹** | **Z²** | **Z³** | **R¹** | **A₄** | **A₃** | **A₂** | **A₁** | **Lm** | **U** | **Q** | **logP** | **Masse** [**m/z**]**¹** |
|---|---|---|---|---|---|---|---|---|---|---|---|---|---|---|
| Ia-1 | F | CH₃ | CF₂CF₃ | CF₃ | H | C-H | C-Cl | C-H | C-H | CONH | CO | Cyclopropyl | 3.33^{a)} | 517.0^{a)} |

**Tabelle 2**

| | | | | | | | | | | | | | | |
|---|---|---|---|---|---|---|---|---|---|---|---|---|---|---|
| 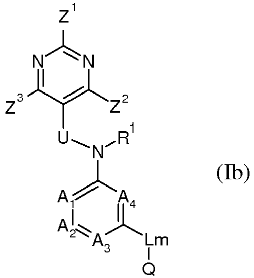 | | | | | | | | | | | | | | |

| **Bsp**.-**Nr**. | **Verfahren** | **Z¹** | **Z²** | **Z³** | **R¹** | **A₄** | **A₃** | **A₂** | **A₁** | **Lm** | **U** | **Q** | **logP** | **Masse** [**m**/**z**]**¹** |
|---|---|---|---|---|---|---|---|---|---|---|---|---|---|---|
| Ib-2 | D | CF₃CH ₂O | CF₃ | - | H | C-H | C-Cl | C-H | C-H | CONH | CO | Cyclopropyl | 2,76^{a)} | 483,0^{a)} |
| Ib-3 | A | CF₃ | CF₃ | - | H | C-H | C-Cl | C-H | C-H | CONH | CO | Cyclopropyl | 2,78^{a)} | 453,1 ^{a)} |
| Ib-4 | | CF₃ | CF₃ | - | H | C-H | C-Cl | C-H | C-H | CONH | CO | 2,2,2-Trifluorethyl | 3,14^{a)} | 495,0^{a)} |
| Ib-5 | A | CF₃ | CF₃ | - | H | C-H | C-Cl | C-H | C-H | CONH | CO | 3-Chlor-prop-2-enyl | 3,19^{a)} | 487,0^{a)} |
| Ib-6 | A | CF₃ | CF₃ | - | H | C-H | C-Cl | C-H | C-H | CONH | CO | Benzyl | 3,39^{a)} | 503,1^{a)} |
| Ib-7 | A | C₂F₅ | CF₃ | - | H | C-H | C-Cl | C-H | C-H | CONH | CO | Cyclopropyl | 3,20^{a)} | 503,1^{a)} |
| Ib-8 | A | C₂F₅ | Me | Me | H | C-H | C-Cl | C-H | C-H | CONH | CO | Cyclopropyl | 2,82^{a)} | 463,1 ^{a)} |
| Ib-9 | A | C₂F₅ | CF₃ | - | H | C-H | C-Cl | C-H | C-H | CONH | CO | 2,2,2-Trifluorethyl | 3,53^{a)} | 545,0^{a)} |
| Ib-10 | A | C₂F₅ | CF₃ | - | H | C-H | C-Cl | C-H | C-H | CONH | CO | 3-Chlor-prop-2-enyl | 3,59^{a)} | 537,0^{a)} |
| Ib-11 | A | C₂F₅ | CF₃ | - | H | C-H | C-Cl | C-H | C-H | CONH | CO | Benzyl | 3,78^{a)} | 553,1 ^{a)} |
| Ib-12 | A | C₂F₅ | Me | Me | H | C-H | C-Cl | C-H | C-H | CONH | CO | 2,2,2-Trifluorethyl | 3,22^{a)} | 505,1^{a)} |
| Ib-13 | A | C₂F₅ | Me | Me | H | C-H | C-Cl | C-H | C-H | CONH | CO | 3-Chlor-prop-2-enyl | 3,26^{a)} | 497,1^{a)} |
| Ib-14 | A | C₂F₅ | Me | Me | H | C-H | C-Cl | C-H | C-H | CONH | CO | Benzyl | 3,49^{a)} | 513,1^{a)} |
| Ib-15 | A | C₃F₇ | CF₃ | - | H | C-H | C-Cl | C-H | C-H | CONH | CO | Cyclopropyl | 3,56^{a)} | 553,1 ^{a)} |
| Ib-16 | A | C₃F₇ | CF₃ | - | H | C-H | C-Cl | C-H | C-H | CONH | CO | 2,2,2-Trifluorethyl | 3,88 ^{a)} | 595,0 ^{a)} |
| Ib-17 | A | C₃F₇ | CF₃ | - | H | C-H | C-Cl | C-H | C-H | CONH | CO | 3-Chlor-prop-2-enyl | 3,92 ^{a)} | 587,0 ^{a)} |
| Ib-18 | A | C₃F₇ | CF₃ | - | H | C-H | C-Cl | C-H | C-H | CONH | CO | Benzyl | 4,13 ^{a)} | 603,1 ^{a)} |
| Ib-19 | A | CF₃ | Me | - | H | C-H | C-Cl | C-H | C-H | CONH | CO | Cyclopropyl | 2,24v | 399,0 ^{a)} |
| Ib-20 | A | CF₃ | Me | - | H | C-H | C-Cl | C-H | C-H | CONH | CO | 2,2,2-Trifluorethyl | 2,65 ^{a)} | 441,0 ^{a)} |
| Ib-21 | A | CF₃ | Me | - | H | C-H | C-Cl | C-H | C-H | CONH | CO | 3-Chlor-prop-2-enyl | 2,69 ^{a)} | 433,0 ^{a)} |
| Ib-22 | A | CF₃ | Me | - | H | C-H | C-Cl | C-H | C-H | CONH | CO | Benzyl | 2,94 ^{a)} | 449,0 ^{a)} |
| Ib-23 | A | C₂F₅ | Me | - | H | C-H | C-Cl | C-H | C-H | CONH | CO | Cyclopropyl | 2,76 ^{a)} | 449,0 ^{a)} |
| Ib-24 | A | C₂F₅ | Me | - | H | C-H | C-Cl | C-H | C-H | CONH | CO | 2,2,2-Trifluorethyl | 3,16 ^{a)} | 491,0 ^{a)} |
| Ib-25 | A | C₂F₅ | Me | - | H | C-H | C-Cl | C-H | C-H | CONH | CO | 3-Chlor-prop-2-enyl | 3,2 ^{a)} | 483,0 ^{a)} |
| Ib-26 | A | C₂F₅ | Me | - | H | C-H | C-Cl | C-H | C-H | CONH | CO | Benzyl | 3,43 ^{a)} | 499,0 ^{a)} |
| Ib-27 | A | C₂F₅ | CHF₂ | - | H | C-H | C-Cl | C-H | C-H | CONH | CO | Cyclopropyl | 2,92 ^{a)} | 485,1 ^{a)} |
| Ib-28 | A | C₂F₅ | CHF₂ | - | H | C-H | C-Cl | C-H | C-H | CONH | CO | 2,2,2-Trifluorethyl | 3,27 ^{a)} | 527,0 ^{a)} |
| Ib-29 | A | C₂F₅ | CHF₂ | - | H | C-H | C-Cl | C-H | C-H | CONH | CO | Benzyl | 3,51 ^{a)} | 535,1 ^{a)} |
| Ib-30 | A | C₂F₅ | Cycloprop yl | - | H | C-H | C-Cl | C-H | C-H | CONH | CO | Cyclopropyl | 3,28 ^{a)} | 475,0 ^{a)} |
| Ib-31 | A | C₂F₅ | Cycloprop yl | - | H | C-H | C-Cl | C-H | C-H | CONH | CO | 2,2,2-Trifluorethyl | 3,65 ^{a)} | 517,0 ^{a)} |
| Ib-32 | A | CF₂CF₃ | CHF₂ | H | H | C-H | C-Cl | C-H | C-H | CONH | CO | 3-Chlor-prop-2-enyl | 3.29 ^{a)} | 519.0 ^{a)} |
| Ib-33 | A | CF₂CF₃ | CF₂CF₃ | H | H | C-H | C-Cl | C-H | C-H | CONH | CO | Cyclopropyl | 3.64^{a)} | 553.0 ^{a)} |
| Ib-34 | A | CF₂CF₃ | CF₂CF₃ | H | H | C-H | C-Cl | C-H | C-H | CONH | CO | 2,2,2-Trifluorethyl | 3.94^{a)} | 595.0 ^{a)} |
| Ib-35 | A | CF₂CF₃ | CF₂CF₃ | H | H | C-H | C-Cl | C-H | C-H | CONH | CO | Benzyl | 4.20^{a)} | 603.0 |
| Ib-36 | A | CF₂CF₃ | CF₃ | H | H | C-H | C-Cl | C-Br | C-H | CONH | CO | Cyclopropyl | 3.62^{a)} | 582.9 ^{a)} |
| Ib-37 | A | CF₂CF₃ | CF₃ | H | H | C-H | C-Cl | C-H | C-H | CONH | CO | 1-Cyclopropylethyl | 3.76^{a)} | 531.1 ^{a)} |
| Ib-38 | A | CF₂CF₃ | CF₃ | H | CH3 | C-H | C-Cl | C-H | C-H | CONH | CO | Cyclopropyl | 3.2^{a)} | 517.1 ^{a)} |

Außerordentlich bevorzugt sind auch Verbindungen der allgemeinen Formeln (Ib), die sich aus einer beliebigen Kombination der in der Tabelle 2 aufgeführten Reste Z¹, Z², Z3, R¹, A₄, A₃, A₂, A₁, Lₘ, U und Q ergeben.

**Tabelle 3**

| | | | | | | | | | | | | | |
|---|---|---|---|---|---|---|---|---|---|---|---|---|---|
| 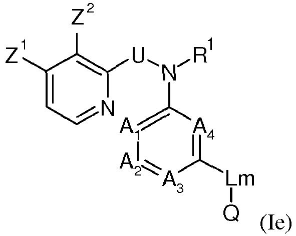 | | | | | | | | | | | | | |

| **Bsp**.-**Nr**. | **Verfah-ren** | **Z¹** | **Z²** | **R¹** | **A₄** | **A₃** | **A₂** | **A₁** | **Lm** | **U** | **Q** | **logP** | **Masse** [**m**/**z**]**¹** |
|---|---|---|---|---|---|---|---|---|---|---|---|---|---|
| Ie-1 | A | CF₃ | Cl | H | C-H | C-Cl | C-H | C-H | CONH | CO | Cyclopropyl | 2,48 ^{a)} | 418,0 ^{a)} |
| Ie-2 | A | CF₃ | Cl | H | C-H | C-Cl | C-H | C-H | CONH | CO | 1-Methylethyl | 2,72 ^{a)} | 420,0 ^{a)} |
| Ie-3 | A | CF₃ | Cl | H | C-H | C-Cl | C-H | C-H | CONH | CO | 2,2,2-Trifluorethyl | 2,88 ^{a)} | 460,0 ^{a)} |
| Ie-4 | A | CF₃ | Cl | H | C-H | C-Cl | C-H | C-H | CONH | CO | 3-Chlor-prop-2-enyl | 2,92 ^{a)} | 452,0 ^{a)} |
| Ie-5 | A | CF₃ | Cl | H | C-H | C-Cl | C-H | C-H | CONH | CO | Benzyl | 3,16 ^{a)} | 468,1 ^{a)} |

Außerordentlich bevorzugt sind auch Verbindungen der allgemeinen Formeln (Ie), die sich aus einer beliebigen Kombination der in der Tabelle 3 aufgeführten Reste Z¹, Z², Z3, R¹, A₄, A₃, A₂, A₁, Lₘ, U und Q ergeben.

**Tabelle 4**

| | | | | | | | | | | | | | |
|---|---|---|---|---|---|---|---|---|---|---|---|---|---|
| 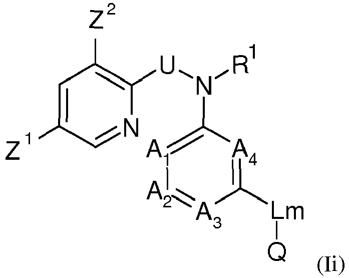 | | | | | | | | | | | | | |

| **Bsp**.-**Nr.** | **Verfahren** | **Z¹** | **Z²** | **R¹** | **A₄** | **A₃** | **A₂** | **A₁** | **Lₘ** | **U** | **Q** | **logP** | **Masse [m/z]¹** |
|---|---|---|---|---|---|---|---|---|---|---|---|---|---|
| Ii-1 | A | CF₃ | Cl | H | C-H | C-Cl | C-H | C-H | CONH | CO | Cyclopropyl | 2,46 ^{a)} | 418,0 ^{a)} |
| Ii-2 | A | CF₃ | Cl | H | C-H | C-Cl | C-H | C-H | CONH | CO | 1-Methylethyl | 2,73 ^{a)} | 420,0 ^{a)} |
| Ii-3 | A | CF₃ | Cl | H | C-H | C-Cl | C-H | C-H | CONH | CO | 2,2,2-Trifluorethyl | 2,86 ^{a)} | 460,0 ^{a)} |
| Ii-4 | A | CF₃ | Cl | H | C-H | C-Cl | C-H | C-H | CONH | CO | 3-Chlor-prop-2-enyl | 2,93 ^{a)} | 451,9 ^{a)} |
| Ii-5 | A | CF₃ | Cl | H | C-H | C-Cl | C-H | C-H | CONH | CO | Benzyl | 3,17 ^{a)} | 468,0 ^{a)} |

Außerordentlich bevorzugt sind auch Verbindungen der allgemeinen Formeln (Ii), die sich aus einer beliebigen Kombination der in der Tabelle 4 aufgeführten Reste Z¹, Z², Z3, R¹, A₄, A₃, A₂, A₁, Lₘ, U und Q ergeben.

**Tabelle 5**

| | | | | | | | | | | | |
|---|---|---|---|---|---|---|---|---|---|---|---|
| 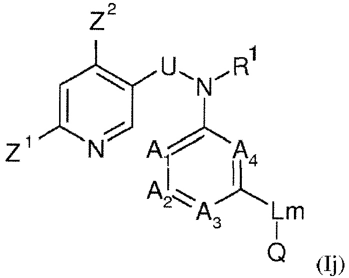 | | | | | | | | | | | |

| **Bsp**.-**Nr.** | **Verfahren** | **Z¹** | **Z²** | **R¹** | **A₄** | **A₃** | **A₂** | **A₁** | **Lₘ** | **U** | **Q** |
|---|---|---|---|---|---|---|---|---|---|---|---|
| Ij-1 | D | CF₃CH₂O | CF₃ | H | C-H | C-Cl | C-H | C-H | CONH | CO | Cyclopropyl |
| Ij-2 | D | CF₃CH(Me)O | CF₃ | H | C-H | C-Cl | C-H | C-H | CONH | CO | Cyclopropyl |
| Ij-3 | D | C₂F₅CH₂O | CF₃ | H | C-H | C-Cl | C-H | C-H | CONH | CO | Cyclopropyl |
| Ij-4 | A | (CF₃)₂CHO | CF₃ | H | C-H | C-Cl | C-H | C-H | CONH | CO | Cyclopropyl |

Außerordentlich bevorzugt sind auch Verbindungen der allgemeinen Formeln (Ij), die sich aus einer beliebigen Kombination der in der Tabelle 5 aufgeführten Reste Z¹, Z², Z3, R¹, A₄, A₃, A₂, A₁, Lₘ, U und Q ergeben.

**Tabelle 6**

| | | | | | | | | | | | | | | |
|---|---|---|---|---|---|---|---|---|---|---|---|---|---|---|
| 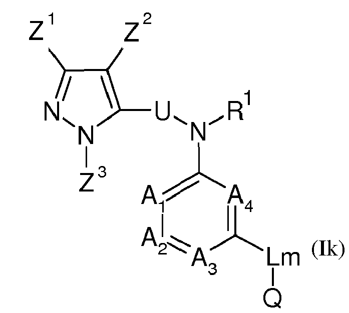 | | | | | | | | | | | | | | |

| **Bsp**.-**Nr.** | **Verfahren** | **Z¹** | **Z²** | **Z³** | **R¹** | **A₄** | **A₃** | **A₂** | **A₁** | **Lₘ** | **U** | **Q** | **logP** | **Masse** [**m**/**z**]**¹** |
|---|---|---|---|---|---|---|---|---|---|---|---|---|---|---|
| Ik-1 | A | C₂F₅ | CF₃ | CH₃ | H | C-H | C-Cl | C-H | C-H | CONH | CO | Cyclopropyl | 3,34 ^{a)} | 505,0 ^{a)} |
| Ik-2 | B | C₂F₅ | CF₃ | CH₃ | H | C-H | C-Cl | C-H | C-H | CONH | CO | H | 3,22 ^{a)} | 466,0 ^{a)} |
| Ik-3 | B | C₂F₅ | CF₃ | CH₃ | H | C-H | C-Cl | C-H | C-H | CONH | CO | Prop-2-inyl | 3,11 ^{b)} | 502,9 ^{b)} |
| Ik-4 | C | C₂F₅ | I | CH3 | H | C-H | C-Cl | C-Cl | C-H | CONH | CO | Cyclopropyl | 3,15 ^{a)} | 562,9 ^{a)} |
| Ik-5 | C | C₂F₅ | Vinyl | CH₃ | H | C-H | C-Cl | C-H | C-H | CONH | CO | Cyclopropyl | 3.11 | 463,1 ^{a)} |
| Ik-6 | B | C₂F₅ | CF₃ | CH₃ | H | C-H | C-Cl | C-H | C-H | CONH | CO | 2,2-Difluorcyclopropyl | 3,33^{b)} | 541,1 ^{b)} |
| Ik-7 | A | C₂F₅ | CF₃ | CH₃ | H | C-H | C-Cl | C-Br | C-H | CONH | CO | Cyclopropyl | 3,81 ^{a)} | 582,9 ^{a)} |
| Ik-8 | B | C₂F₅ | CF₃ | CH₃ | H | C-H | C-Cl | C-H | C-H | CONH | CO | *trans*-2-Fluorcyclopropyl | 3,24 ^{b)} | 523,1 ^{b)} |
| Ik-9 | B | C₂F₅ | CF₃ | CH₃ | H | C-H | C-Cl | C-H | C-H | CONH | CO | *cis*-2-Fluorcyclopropyl | 3,28 ^{b)} | 523,1 ^{b)} |
| Ik-10 | A | C₂F₅ | CF₃ | C₂H₅ | H | C-H | C-Cl | C-OC H₃ | C-H | CONH | CO | Benzyl | 4,18 ^{a)} | 563,1 ^{a)} |
| Ik-11 | A | C₂F₅ | Br | CH₃ | H | C-H | C-Cl | C-H | C-H | CONH | CO | 1-Trifluormethylethyl | 2,00^{a)} | 572,9 ^{a)} |
| Ik-12 | A | C₂F₅ | Br | CH₃ | H | C-H | C-Cl | C-H | C-H | CONH | CO | 1-Cyclopropylethyl | 3,84 ^{a)} | 543,0 ^{a)} |
| Ik-13 | A | C₂F₅ | CF₃ | CH₃ | H | C-H | C-H | C-H | C-H | CONH | CO | Cyclopropyl | 3,15 ^{a)} | 471,1 ^{a)} |
| Ik-14 | A | C₂F₅ | CF₃ | CH₃ | H | C-H | C-H | C-H | C-H | CONH | CO | Benzyl | 3,77 ^{a)} | 521,1 ^{a)} |
| Ik-15 | A | C₂F₅ | CF₃ | CH₃ | H | C-H | C-H | CC ON HcP r | C-H | CONH | CO | Cyclopropyl | 2,74^{a)} | 554,2 ^{a)} |
| Ik-16 | A | CF₃CClF | Br | CH₃ | H | C-H | C-Cl | C-H | C-H | CONH | CO | Cyclopropyl | 3,35 ^{a)} | 530,9 ^{a)} |
| Ik-17 | A | C₂F₅ | H | CH₃ | H | C-H | C-Cl | C-H | C-H | CONH | CO | Cyclopropyl | 2,94^{a)} | 437,0 ^{a)} |
| Ik-18 | A | CF₃ | Cl | CH₃ | H | C-H | C-Cl | C-H | C-H | CONH | CO | Cyclopropyl | 3,77 ^{a)} | 421,1 ^{a)} |
| Ik-19 | A | CF₃ | Cl | CH₃ | H | C-H | C-Cl | C-H | C-H | CONH | CO | 3-Chlor-prop-2-enyl | 3,22 ^{a)} | 455,0 ^{a)} |
| Ik-20 | A | CF₃ | Cl | CH₃ | H | C-H | C-Cl | C-H | C-H | CONH | CO | Benzyl | 3,46 ^{a)} | 471,1^{a)} |
| Ik-21 | A | CF₃ | Br | CH₃ | H | C-H | C-Cl | C-H | C-H | CONH | CO | Cyclopropyl | 3,75 ^{a)} | 467,0 ^{a)} |
| Ik-22 | A | CF₃ | Br | CH₃ | H | C-H | C-Cl | C-H | C-H | CONH | CO | 3-Chlor-prop-2-enyl | 3,20 ^{a)} | 501,0 ^{a)} |
| Ik-23 | A | CF₃ | Br | CH₃ | H | C-H | C-Cl | C-H | C-H | CONH | CO | Benzyl | 3,44 ^{a)} | 517,0 ^{a)} |
| Ik-24 | A | C₂F₅ | CF₃ | CH₃ | H | C-H | C-Cl | C-H | C-H | CONH | CO | Methylsulfonyl | 3.13 ^{a)} | 543,0 ^{a)} |
| Ik-25 | A | C₂F₅ | CF₃ | CH₃ | H | C-H | COCHF 2 | C-H | C-H | CONH | CO | Cyclopropyl | 3,49 ^{a)} | 537,1 ^{a)} |
| Ik-26 | A | C₂F₅ | Br | CH₃ | H | C-H | C-Cl | C-H | C-H | CONH | CO | Cyclopropyl | 3,22 ^{a)} | 515,0 ^{a)} |
| Ik-27 | A | C₂F₅ | Br | CH₃ | H | C-H | C-Cl | C-H | C-H | CONH | CO | 1-Methylethyl | 3,49 ^{a)} | 517,0 ^{a)} |
| Ik-28 | A | C₂F₅ | Br | CH₃ | H | C-H | C-Cl | C-H | C-H | CONH | CO | 3-Chlor-prop-2-enyl | 3,64 ^{a)} | 550,9 ^{a)} |
| Ik-29 | A | C₂F₅ | CF₃ | CH₃ | H | C-H | C-Cl | C-H | C-H | CONH | CO | 1,2,4-Triazol-3-ylmethyl | 2,31 ^{b)} | 546,0 ^{b)} |
| Ik-30 | B | C₂F₅ | CF₃ | CH₃ | H | C-H | C-Cl | C-H | C-H | CONH | CO | Propyl | 3,23 ^{b)} | 507,0 ^{b)} |
| Ik-31 | B | C₂F₅ | CF₃ | CH₃ | H | C-H | C-Cl | C-H | C-H | CONCH₃ | CO | Cyclopropyl | 3,51 ^{b)} | 519,0 ^{b)} |
| Ik-32 | B | C₂F₅ | CF₃ | CH₃ | H | C-H | C-Cl | C-H | C-H | CONH | CO | Oxetan-3-yl | 2,76 ^{b)} | 521,0 ^{b)} |
| Ik-33 | B | C₂F₅ | CF₃ | CH₃ | H | C-H | C-Cl | C-H | C-H | CONH | CO | 2-Hydroxypropyl | 2,65 ^{b)} | 523,0 ^{b)} |
| Ik-34 | B | C₂F₅ | CF₃ | CH₃ | H | C-H | C-Cl | C-H | C-H | CONH | CO | Methoxycarbonylmet hyl | 3,04 ^{b)} | 537,0 ^{b)} |
| Ik-35 | B | C₂F₅ | CF₃ | CH₃ | H | C-H | C-Cl | C-H | C-H | CONCH₃ | CO | 1-Methylethyl | 3,73 ^{b)} | 521,0 ^{b)} |
| Ik-36 | B | C₂F₅ | CF₃ | CH₃ | H | C-H | C-Cl | C-H | C-Cl | CONH | CO | Cyclobutyl | 3,53 ^{b)} | 519,0 ^{b)} |
| Ik-37 | B | C₂F₅ | CF₃ | CH₃ | H | C-H | C-Cl | C-H | C-H | CONH | CO | 2,6-Difluorphenylmethyl | 3,73 ^{b)} | 591,0 ^{b)} |
| Ik-38 | B | C₂F₅ | CF₃ | CH₃ | H | C-H | C-Cl | C-H | C-H | CONH | CO | 1-Phenylethyl | 3,89 ^{b)} | 569,0 ^{b)} |
| Ik-39 | B | C₂F₅ | CF₃ | CH₃ | H | C-H | C-Cl | C-H | C-H | CONH | CO | Cyclopentyl | 3,78 ^{b)} | 533.0 ^{b)} |
| Ik-40 | B | C₂F₅ | CF₃ | CH₃ | H | C-H | C-Cl | C-H | C-H | CONH | CO | 4-Chlorphenylethyl | 4,23 ^{b)} | 603,0 ^{b)} |
| Ik-42 | B | C₂F₅ | CF₃ | CH₃ | H | C-H | C-Cl | C-H | C-H | CONH | CO | 2,2-Dimethyl-3-fluorpropyl | 3,68 ^{b)} | 553,0 ^{b)} |
| Ik-43 | B | C₂F₅ | CF₃ | CH₃ | H | C-H | C-Cl | C-H | C-H | CONH | CO | Isoxazol-3-ylmethyl | 3,02 ^{b)} | 546,0 ^{b)} |
| Ik-44 | B | C₂F₅ | CF₃ | CH₃ | H | C-H | C-Cl | C-H | C-H | CONH | CO | 3,3-Dichlor-1,1-dimethylprop-2-enyl | 4,28 ^{b)} | 602,9 ^{b)} |
| Ik-45 | B | C₂F₅ | CF₃ | CH₃ | H | C-H | C-Cl | C-H | C-H | CONH | CO | 3-Methyloxetan-3-ylmethyl | 2,98 ^{b)} | 548,9 ^{b)} |
| Ik-46 | B | C₂F₅ | CF₃ | CH₃ | H | C-H | C-Cl | C-H | C-H | CONH | CO | 2,2-Dimethylcyclopropyl -methyl | 4,11 ^{b)} | 547,0 ^{b)} |
| Ik-47 | B | C₂F₅ | CF₃ | CH₃ | H | C-H | C-Cl | C-H | C-H | CONH | CO | 2-Phenylcyclopropyl | 4,00 ^{b)} | 581.0 ^{b)} |
| Ik-48 | B | C₂F₅ | CF₃ | CH₃ | H | C-H | C-Cl | C-H | C-H | CONH | CO | Cyanomethyl | 2,93 ^{b)} | 503,9 ^{b)} |
| Ik-49 | B | C₂F₅ | CF₃ | CH₃ | H | C-H | C-Cl | C-H | C-H | CONH | CO | 3-Fluorphenylmethyl | 3,78 ^{b)} | 573,0 ^{b)} |
| Ik-50 | B | C₂F₅ | CF₃ | CH₃ | H | C-H | C-Cl | C-H | C-H | CONH | CO | 2-Methylprop-2-enyl | 3,53 ^{b)} | 519,0 ^{b)} |
| Ik-52 | B | C₂F₅ | CF₃ | CH₃ | H | C-H | C-Cl | C-H | C-H | CONH | CO | 2-Fluorphenylmethyl | 3,73 ^{b)} | 573,0 ^{b)} |
| Ik-53 | B | C₂F₅ | CF₃ | CH₃ | H | C-H | C-Cl | C-H | C-H | CONH | CO | 2-Ethoxyethyl | 3,28 ^{b)} | 537,0 ^{b)} |
| Ik-54 | B | C₂F₅ | CF₃ | CH₃ | H | C-H | C-Cl | C-H | C-H | CONH | CO | 2,5-Difluorphenylmethyl | 3,84 ^{b)} | 591,0 ^{b)} |
| Ik-55 | B | C₂F₅ | CF₃ | CH₃ | H | C-H | C-Cl | C-H | C-H | CONH | CO | 2-Trifluormethylphenyl ethyl | 4,28 ^{b)} | 591,0 ^{b)} |
| Ik-56 | B | C₂F₅ | CF₃ | CH₃ | H | C-H | C-Cl | C-H | C-H | CONH | CO | 2-Hydroxypropyl | 2,65 ^{b)} | 637,0 ^{b)} |
| Ik-57 | B | C₂F₅ | CF₃ | CH₃ | H | C-H | C-Cl | C-H | C-H | CONH | CO | 2-Methylbutyl | 4,00 ^{b)} | 523,0 ^{b)} |
| Ik-58 | B | C₂F₅ | CF₃ | CH₃ | H | C-H | C-Cl | C-H | C-H | CONH | CO | trans-4-Hydroxycyclohexyl | 2,69 ^{b)} | 535,0 ^{b)} |
| Ik-59 | B | C₂F₅ | CF₃ | CH₃ | H | C-H | C-Cl | C-H | C-H | CONCH₃ | CO | Propyl | 3,78 ^{b)} | 663,0 ^{b)} |
| Ik-60 | B | C₂F₅ | CF₃ | CH₃ | H | C-H | C-Cl | C-H | C-H | CONH | CO | Pyrimidin-2-ylmethyl | 2,84 ^{b)} | 521,0 ^{b)} |
| Ik-61 | B | C₂F₅ | CF₃ | CH₃ | H | C-H | C-Cl | C-H | C-H | CONH | CO | Dicyclopropylmethyl | 4,00 ^{b)} | 557,0 ^{b)} |
| Ik-62 | B | C₂F₅ | CF₃ | CH₃ | H | C-H | C-Cl | C-H | C-H | CONH | CO | 1-Cyclopropylethyl | 3,73 ^{b)} | 559,0 ^{b)} |
| Ik-63 | B | C₂F₅ | CF₃ | CH₃ | H | C-H | C-Cl | C-H | C-H | CONH | CO | 1-Trifluormethylethyl | 3,68 ^{b)} | 533,0 ^{b)} |
| Ik-64 | B | C₂F₅ | CF₃ | CH₃ | H | C-H | C-Cl | C-H | C-H | CONH | CO | 2,2-Difluorpropyl | 3,37 ^{b)} | 561,0 ^{b)} |
| Ik-65 | B | C₂F₅ | CF₃ | CH₃ | H | C-H | C-Cl | C-H | C-H | CONH | CO | 4-Trifluormethylcycloh exyl | 4,11 ^{b)} | 543,0 ^{b)} |
| Ik-66 | B | C₂F₅ | CF₃ | CH₃ | H | C-H | C-Cl | C-H | C-H | CONH | CO | Ethyl | 3,15 ^{b)} | 615,1 ^{b)} |
| Ik-67 | B | C₂F₅ | CF₃ | CH₃ | H | C-H | C-Cl | C-H | C-H | CONH | CO | 1,1-Dimethylethyl | 3,84 ^{b)} | 492,9 ^{b)} |
| Ik-68 | B | C₂F₅ | CF₃ | CH₃ | H | C-H | C-Cl | C-H | C-H | CONH | CO | 2-Cyanoethyl | 2,89 ^{b)} | 521,0 ^{b)} |
| Ik-69 | A | C₂F₅ | CF₃ | CH₃ | H | C-F | C-H | C-H | C.H | CONH | CO | 1-Methylethyl | 3,45 ^{a)} | 517,9 ^{a)} |
| Ik-70 | A | CF₃ | H | CH₃ | H | C-H | C-Cl | C-H | C-H | CONH | CO | 1-Methylethyl | 2,74 ^{a)} | 491,1 ^{a)} |
| Ik-71 | A | CF₃ | H | CH₃ | H | C-H | C-Cl | C-H | C-H | CONH | CO | Cyclopropyl | 2,48 ^{a)} | 389,1 ^{a)} |
| Ik-72 | A | C₃F₇ | Br | CH₃ | H | C-H | C-Cl | C-H | C-H | CONH | CO | 1-Methylethyl | 3,87 ^{a)} | 387,1 ^{a)} |
| Ik-73 | A | C₃F₇ | Br | CH₃ | H | C-H | C-Cl | C-H | C-H | CONH | CO | Cyclopropyl | 3,58 ^{a)} | 567,0 ^{a)} |
| Ik-74 | A | C₂F₅ | CF₃ | CH₃ | H | C-F | C-F | C-H | C-H | CONH | CO | 1-Methylethyl | 3,56 ^{a)} | 564,9 ^{a)} |
| Ik-75 | A | C₂F₅ | CF₃ | CH₃ | H | C-H | C-F | C-H | C-H | CONH | CO | 1-Methylethyl | 3,39 ^{a)} | 509,1 ^{a)} |
| Ik-76 | A | C₂F₅ | CF₃ | CH₃ | H | C-H | C-CH₃ | C-H | C-H | CONH | CO | 1-Methylethyl | 3,40 ^{a)} | 491,1 ^{a)} |
| Ik-77 | A | C₂F₅ | CF₃ | CH₃ | H | C-Cl | C-H | C-H | C-H | CONH | CO | 1-Methylethyl | 3,47 ^{a)} | 507,0 ^{a)} |
| Ik-78 | A | C₂F₅ | CF₃ | CH₃ | H | C-H | C-Cl | C-H | C-H | CONH | CO | 1-Pyridin-2-ylethyl | 3,18 ^{b)} | 570,1 ^{b)} |
| Ik-79 | A | C₂F₅ | CF₃ | CH₃ | H | C-F | C-H | C-H | C.H | CONH | CO | Pyridin-2-ylmethyl | 2,80 ^{a)} | 556,1 ^{a)} |
| Ik-80 | A | C₂F₅ | CF₃ | CH₃ | H | C-H | C-Cl | C-H | C-H | CONH | CO | 5-Fluorpyridin-2-ylmethyl | 4,75 ^{a)} | 574,1 ^{a)} |
| Ik-81 | A | C₂F₅ | CF₃ | CH₃ | H | C-H | C-Cl | C-H | C-H | CONH | CO | 1,1-Dimethylbut-2-inyl | 4,03 ^{a)} | 545,1 ^{a)} |
| Ik-82 | A | C₂F₅ | CF₃ | CH₃ | H | C-H | C-Cl | C-H | C-H | CONH | CO | 2-(Methylsulfanyl)ethyl | 3,57 ^{a)} | 539,0 ^{a)} |
| Ik-83 | A | C₂F₅ | CF₃ | CH₃ | H | C-H | C-Cl | C-H | C-H | CONH | CO | 3-Chlor-prop-2-enyl | 3,72 ^{a)} | 539,0 ^{a)} |
| Ik-84 | A | C₂F₅ | CF₃ | CH₃ | H | C-H | C-Cl | C-H | C-H | CONH | CO | Benzyl | 3,90 ^{a)} | 555.1 ^{a)} |
| Ik-85 | A | C₂F₅ | CF₃ | CH₃ | H | C-H | C-Cl | C-H | C-H | CONH | CO | CH3 | 3,04 ^{a)} | 479,0 ^{a)} |
| Ik-86 | A | C₂F₅ | CF₃ | CH₃ | H | C-H | C-Cl | C-H | C-H | CONH | CO | 2,2-Difluorethyl | 3,41 ^{a)} | 529,0 ^{a)} |
| Ik-87 | A | C₂F₅ | CF₃ | CH₃ | H | C-H | C-Cl | C-H | C-H | CONH | CO | 2,2,2-Trifluorethyl | 3,67 ^{a)} | 547,0 ^{a)} |
| Ik-88 | A | C₂F₅ | CF₃ | CH₃ | H | C-H | C-Cl | C-H | C-H | CONH | CO | 2-Fluorethyl | 3,21 ^{a)} | 511,0 ^{a)} |
| Ik-89 | A | C₂F₅ | CF₃ | CH₃ | H | C-H | C-Cl | C-H | C-H | CONH | CO | 1-CH3-2-(ethylsulfanyl)ethyl | 4,09 ^{a)} | 567,0 ^{a)} |
| Ik-90 | A | C₂F₅ | CF₃ | CH₃ | H | C-H | C-Cl | C-H | C-H | CONH | CO | 1-Methylethyl | 3,60 ^{a)} | 507,1 ^{a)} |
| Ik-91 | A | C₂F₅ | CF₃ | CH₃ | H | C-H | C-Cl | C-H | C-H | CONH | CO | 1-Methylpropyl | 3,87 ^{a)} | 521,1 ^{a)} |
| Ik-92 | A | C₂F₅ | CF₃ | CH₃ | H | C-H | C-Cl | C-H | C-H | CONH | CO | 2-Methylpropyl, | 3,90 ^{a)} | 521,1 ^{a)} |
| Ik-93 | A | C₂F₅ | CF₃ | CH₃ | H | C-H | C-Cl | C-H | C-H | CONH | CO | Prop-2-enyl | 3,44 ^{a)} | 505,1 ^{a)} |
| Ik-94 | A | CF₃ | H | CH₃ | H | C-H | C-Cl | C-H | C-H | CONH | CO | Prop-2-enyl | 2,62 ^{a)} | 387,1 ^{a)} |
| Ik-95 | A | C₂F₅ | CF₃ | CH₃ | H | C-H | C-Cl | C-F | C-H | CONH | CO | Cyclopropyl | 3,43 ^{a)} | 489,0 ^{a)} |
| Ik-96 | A | C₂F₅ | CF₃ | CH₃ | H | C-H | C-Cl | C-Cl | C-H | CONH | CO | Cyclopropyl | 3,71 ^{a)} | 503,0 ^{a)2)} |
| Ik-97 | A | C₂F₅ | CF₃ | CH₃ | H | C-H | C-F | C-F | C-H | CONH | CO | Benzyl | 4,19 ^{a)} | 557,1 ^{a)} |
| Ik-98 | A | C₂F₅ | I | CH₃ | H | C-H | C-Cl | C-H | C-H | CONH | CO | 3-Chlor-prop-2-enyl | 3,54 ^{a)} | 596,9 ^{a)} |
| Ik-99 | A | C₂F₅ | I | CH₃ | H | C-H | C-Cl | C-H | C-H | CONH | CO | 2,2,2-Trifluorethyl | 3,52 ^{a)} | 604,9 ^{a)} |
| Ik-100 | A | CHFCF₃ | CF₃ | CF(CH₃ )₂ | H | C-H | C-Cl | C-H | C-H | CONH | CO | Cyclopropyl | 3,39 ^{a)} | 531.1 ^{a)2)} |
| Ik-101 | A | C₂F₅ | CF₃ | CH₃ | H | C-H | C-Cl | C-H | C-Cl | CONH | CO | 3-Chlor-prop-2-enyl | 3,80 ^{a)} | 539,0 ^{a)} |
| Ik-102 | A | C₂F₅ | CF₃ | CH₃ | H | C-H | C-Cl | C-H | C-Cl | CONH | CO | Cyclopropyl | 3,38 ^{a)} | 505,0 ^{a)} |
| Ik-103 | A | C₂F₅ | CF₃ | CH₃ | H | C-H | C-Cl | C-H | C-H | C(O)O | CO | Ethyl | 4.42 ^{a)} | 494,1 ^{a)} |
| Ik-104 | A | C₂F₅ | CF₃ | CH₃ | H | C-H | C-OCHF₂ | C-H | C-H | C(O)O | CO | H | 3.23 ^{a)} | 496,1 ^{a)2)} |
| Ik-105 | A | C₂F₅ | CF₃ | CH₃ | H | C-H | Bindung zu Q | C-H | C-F | C(O)O | CO | -CH₂-[Bindung zu A₃] | 3.27 ^{a)} | 462.0^{a)} |
| Ik-106 | A | C₂F₅ | CF₃ | CH₃ | H | C-H | Bindung zu Q | C-H | C-H | C(O)O | CO | -CH₂-[Bindung zur A₃] | 3.20 ^{a)} | 444,0 ^{a)} |
| Ik-107 | E | C₂F₅ | CF₃ | CH₃ | H | C-H | C-Cl | C-H | C-H | C(O)OCH₂C (O) | CO | Methoxy | 4.03 ^{a)} | 555,0 ^{a)} |
| Ik-108 | E | C₂F₅ | CF₃ | CH₃ | H | C-H | C-Cl | C-H | C-H | C(O)OCH₂C (O) | CO | *N,N*-Dimethylamino | 3.57 ^{a)} | 551,1 ^{a)} |
| Ik-109 | E | C₂F₅ | CF₃ | CH₃ | H | C-H | C-Cl | C-H | C-H | C(O)OCH₂C (O) | CO | *N,N*-Diethylamino | 4.11 ^{a)} | 579,1 ^{a)} |
| Ik-110 | E | C₂F₅ | CF₃ | CH₃ | H | C-H | C-Cl | C-H | C-H | C(O)OCH₂C (O)NHC(O) | CO | NH₂ | 3.10 ^{a)} | 566,1 ^{a)} |
| Ik-112 | E | C₂F₅ | CF₃ | CH₃ | H | C-H | C-Cl | C-H | C-H | C(O)OCH₂C (O) | CO | *N*-Allylamino | 3.66 ^{a)} | 581.2 ^{a)} |
| Ik-113 | E | C₂F₅ | CF₃ | CH₃ | H | C-H | C-Cl | C-H | C-H | C(O)OCH₂C (O)NH | CO | Methoxycarbonyl | 3.60 ^{a)} | 567,2 ^{a)} |
| Ik-114 | E | C₂F₅ | CF₃ | CH₃ | H | C-H | C-Cl | C-H | C-H | C(O)OCH₂C (O)NMe | CO | Methoxy | 3.87 ^{a)} | 551,2 ^{a)2)} |
| Ik-115 | E | C₂F₅ | CF₃ | CH₃ | H | C-H | C-Cl | C-H | C-H | C(O)OCH₂C (O)NH | CO | Hydroxymethyl | 2.93 ^{a)} | 551,2 ^{a)} |
| Ik-116 | E | C₂F₅ | CF₃ | CH₃ | H | C-H | C-Cl | C-H | C-H | C(O)OCH₂C (O) | CO | *N*-Ethylamino | 3.52 ^{a)} | 646,9 ^{a)} |
| Ik-117 | A | CF₃OC₂F₄OC F₂ | Br | CH₃ | H | C-H | C-Cl | C-H | C-H | CONH | CO | Cyclopropyl | 4.31 ^{a)} | 690,9 ^{a)} |
| Ik-118 | A | CF₃OC₂F₄OC F₂ | Br | CH₃ | H | C-H | C-Cl | C-H | C-H | CONH | CO | 2,2,2-Trilfuorethyl | 4.61 ^{a)} | 682,9 ^{a)} |
| Ik-119 | A | CF₃OC₂F₄OC F₂ | Br | CH₃ | H | C-H | C-Cl | C-H | C-H | CONH | CO | 3-Chlor-prop-2-enyl | 4.66 ^{a)} | 615,0 ^{a)} |
| Ik-120 | A | C₄F₉ | Br | CH₃ | H | C-H | C-Cl | C-H | C-H | CONH | CO | Cyclopropyl | 3.99^{a)} | 654,9^{a)2)} |
| Ik-121 | A | C₄F₉ | Br | CH₃ | H | C-H | C-Cl | C-H | C-H | CONH | CO | 2,2,2-Trilfuorethyl | 4.29 ^{a)} | 648,9^{a)2)} |
| Ik-122 | A | C₄F₉ | Br | CH₃ | H | C-H | C-Cl | C-H | C-H | CONH | CO | 3-Chlor-prop-2-enyl | 4.35 ^{a)} | 531,1 ^{a)} |
| Ik-123 | C | C₂F₅ | 4-Fluorphenyl | CH₃ | H | C-H | C-Cl | C-H | C-H | CONH | CO | Cyclopropyl | 3.60 ^{a)} | 444,0^{a)} |
| Ik-124 | A | CF₂CF₃ | CF₃ | H | H | C-H | C-Cl | C-H | C-H | CONH | CO | 2.2.2-Trifluorethyl | 3.26 ^{a)} | 533.0 ^{a)} |
| Ik-125 | A | CF₃ | CF₃ | CH₃ | H | C-H | C-Cl | C-H | C-H | CONH | CO | 2.2.2-Trifluorethyl | 3.25 ^{a)} | 497.0 ^{a)} |
| Ik-126 | C | OCHF₂ | CF₃ | CH₃ | H | C-H | C-Cl | C-H | C-H | CONH | CO | 2,2,2-Trifluorethyl | 3.01 ^{a)} | 495.0 ^{a)} |
| Ik-127 | B | CF₂CF₃ | CF₃ | CH₃ | H | N | C-Br | C-H | C-H | CONH | CO | (1S,2R)-2-Fluorcyclopropyl | 3,29 ^{a)} | 568,0 ^{a)} |
| Ik-128 | B | CF₂CF₃ | CF₃ | CH₃ | H | C-H | C-Br | C-H | N | CONH | CO | (1S,2R)-2-Fluorcyclopropyl | 3,19 ^{a)} | 568,0 ^{a)} |
| Ik-129 | B | CF₂CF₃ | CF₃ | CH₃ | H | C-H | C-Cl | C-H | N | COO | CO | H | 2.82 ^{a)} | 467.0 ^{a)} |
| Ik-130 | B | CF₂CF₃ | CF₃ | CH₃ | H | C-H | N | C-H | C-H | CONH | CO | 2,2-Difluorcyclopropyl | 3.59 ^{a)} | 508.0 ^{a)} |
| Ik-131 | B | CF₂CF₃ | CF₃ | CH₃ | H | C-H | C-Cl | N | C-H | CONH | CO | H | 2.77 ^{a)} | 467.0 ^{a)} |
| Ik-132 | A | CF₂CF₃ | CF₃ | CH₃ | CH₃ | C-H | C-Cl | C-H | C-H | CONH | CO | Cyclopropyl | 3.30 ^{a)} | 519.1 ^{a)} |
| Ik-133 | A | CF₂CF₃ | CF₃ | CH₃ | CH₃ | C-H | C-Cl | C-H | C-H | CONH | CO | 2.2.2-Trifluorethyl | 3.66 ^{a)} | 561.0 ^{a)} |
| Ik-134 | A | CF₂CF₃ | CF₃ | CH₃ | CH₃ | C-H | C-Cl | C-H | C-H | CONH | CO | Benzyl | 3.94 ^{a)} | 569.1 ^{a)} |
| Ik-135 | A | CF₂CF₃ | CF₃ | CH₃ | CH₂O CH₃ | C-H | C-Cl | C-H | C-H | CONH | CO | Cyclopropyl | 3.51 ^{a)} | 549.1 ^{a)} |
| Ik-136 | A | CF₂CF₃ | CF₃ | CH₃ | Ethyl | C-H | C-Cl | C-H | C-H | CONH | CO | Cyclopropyl | 3.55 ^{a)} | 533.1 ^{a)} |
| Ik-137 | A | CF₂CF₃ | CF₃ | CH₃ | 1-Methy lethyl | C-H | C-Cl | C-H | C-H | CONH | CO | Cyclopropyl | 3.74 ^{a)} | 547.1 ^{a)} |
| Ik-138 | A | CF₂CF₃ | CF₃ | CH₃ | Ethyl | C-H | C-Cl | C-H | C-H | CONH | CO | 2,2,2-Trifluorethyl | 3.81 ^{a)} | 575.1 ^{a)} |
| Ik-139 | A | CF₂CF₃ | CF₃ | CH₃ | 1-Methy lethyl | C-H | C-Cl | C-H | C-H | CONH | CO | 2,2,2-Trifluorethyl | 4.02 ^{a)} | 589.1^{a)} |
| Ik-140 | A | CF₂CF₃ | CF₃ | CH₃ | H | C-H | C-F | C-F | C-H | CONH | CO | Cyclopropyl | 3.60 ^{a)} | 507.1 al |
| Ik-141 | A | CF₂CF₃ | CF₃ | CH₃ | H | C-H | C-F | C-F | C-H | CONH | CO | 2,2,2-Trifluorethyl | 3.89^{a)} | 549.1^{a)} |
| Ik-142 | A | CF₂CF₃ | CF₃ | CH₃ | H | C-H | C-Cl | C-H | C-F | CONH | CO | Benzyl | 4.00^{a)} | 573.0^{a)} |
| Ik-143 | A | CF₂CF₃ | CF₃ | CH₃ | H | C-H | C-Cl | C-H | C-F | CONH | CO | Cyclopropyl | 3.39^{a)} | 523.0^{a)} |
| Ik-144 | A | CF₂CF₃ | CF₃ | CH₃ | H | C-H | C-Cl | C-H | C-F | CONH | CO | 2,2,2-Trifluorethyl | 3.73^{a)} | 565.0^{a)} |
| Ik-145 | A | CF₂CF₃ | CF₃ | CH₃ | H | C-H | C-F | C-H | C-F | CONH | CO | Cyclopropyl | 3.32^{a)} | 507.1^{a)} |
| Ik-146 | A | CF₂CF₃ | CF₃ | CH₃ | H | C-H | C-F | C-H | C-F | CONH | CO | 2,2,2-Trifluorethyl | 3.64^{a)} | 549.0^{a)} |
| Ik-147 | A | CF₂CF₃ | CF₃ | CH₃ | H | C-H | C-Cl | C-H | C-Cl | CONH | CO | Cyclopropyl | 3.61^{a)} | 539.0^{a)} |
| Ik-148 | A | CF₂CF₃ | CF₃ | CH₃ | H | C-H | C-F | C-H | C-Cl | CONH | CO | Cyclopropyl | 3.53^{a)} | 523.2^{a)} |
| Ik-149 | A | CF₂CF₃ | CF₃ | CH₃ | H | C-Cl | C-Cl | C-H | C-H | CONH | CO | Benzyl | 4.33^{a)} | 589.0^{a)} |
| Ik-150 | A | CF₂CF₃ | CF₃ | CH₃ | H | C-H | C-Cl | C-H | C-Cl | CONH | CO | Benzyl | 4.23^{a)} | 589.2^{a)} |
| Ik-151 | A | CF₂CF₃ | CF₃ | CH₃ | H | C-H | C-F | C-H | C-Cl | CONH | CO | 2,2,2-Trifluorethyl | 3.83^{a)} | 565.2^{a)} |
| Ik-152 | B | CF₃ | Cl | CH₃ | H | C-H | C-Cl | C-H | C-H | CONH | CO | CH(CH₃)CF₃ | 3.44^{a)} | 477.0^{a)} |
| Ik-153 | B | CF₃ | Cl | CH₃ | H | C-H | C-Cl | C-H | C-H | CONH | CO | 2-Fluorcyclopropyl | 2.86^{a)} | 439.0^{a)} |
| Ik-154 | A | CF₃ | Cl | CH₃ | H | C-H | C-Cl | C-H | C-H | CONH | CO | 2,2,2-Trifluorethyl | 3.20^{a)} | 463.0^{a)} |
| Ik-155 | A | CF₂CF₃ | CF₃ | CH₃ | H | C-H | C-F | C-H | C-H | CONH | CO | Cyclopropyl | 3.31^{a)} | 489.1^{a)} |
| Ik-156 | A | CF₂CF₃ | CF₃ | CH₃ | H | C-H | C-F | C-H | C-H | CONH | CO | 2,2,2-Trifluorethyl | 3.66^{a)} | 531.1^{a)} |
| Ik-157 | A | CF₂CF₃ | CF₃ | CH₃ | H | C-H | C-F | C-H | C-H | CONH | CO | Benzyl | 3.95^{a)} | 537.1^{a)} |
| Ik-158 | B | CF₃ | Br | CH₃ | H | C-H | C-Cl | C-H | C-H | CONH | CO | 1-Trifluormethylethyl | 3.40^{a)} | 522.9^{a)} |
| Ik-159 | B | CF₃ | Br | CH₃ | H | C-H | C-Cl | C-H | C-H | CONH | CO | 2-Fluorcyclopropyl | 2.82^{a)} | 485.0^{a)} |
| Ik-160 | B | CF₃ | Br | CH₃ | H | C-H | C-Cl | C-H | C-H | CONH | CO | 2,2,2-Trifluorethyl | 3.17^{a)} | 508.9^{a)} |
| Ik-161 | B | CF₂CF₃ | CF₃ | CH₃ | H | C-H | C-F | C-H | C-H | CONH | CO | CH(CH₃)CF₃ | 3.89^{a)} | 545.1^{a)} |
| Ik-162 | B | CF₂CF₃ | CF₃ | CH₃ | H | C-H | C-H | C-F | C-H | CONH | CO | Benzyl | 4.05^{a)} | 537.2^{a)} |
| Ik-163 | B | CF₂CF₃ | CF₃ | CH₃ | H | C-H | C-Cl | C-H | C-H | CONH | CO | H | 2.83^{a)} | 465.1^{a)} |
| Ik-164 | A | CF₂CF₃ | CF₃ | CH₃ | H | C-H | C-Cl | C-Cl | C-H | CONH | CO | 1-Trifluormethylethyl | 5.44 ^{a)} | 595.0^{a)} |
| Ik-165 | A | CF₂CF₃ | CF₃ | CH₃ | H | C-H | C-Cl | C-Cl | C-Cl | CONH | CO | Cyclopropyl | 3.96 ^{a)} | 573.0^{a)} |
| Ik-166 | A | CF₂CF₃ | CF₃ | CH₃ | H | C-H | C-Cl | C-H | C-CH₃ | CONH | CO | Cyclopropyl | 3.43 ^{a)} | 519.2^{a)} |
| Ik-167 | B | CF₂CF₃ | CF₃ | C(CH₃)₃ | H | C-H | C-Cl | C-H | C-H | CONH | CO | Cyclopropyl | 4.17^{a)} | 547.1^{a)} |
| Ik-168 | A | CF₂CF₃ | CF₃ | CH₃ | H | C-H | C-F | C-H | C-H | CONH | CO | 2-Fluorcyclopropyl | 3.35 ^{a)} | 507.0^{a)} |
| Ik-169 | A | CF₂CF₃ | CF₃ | CH₃ | H | C-F | C-F | C-F | C-H | CONH | CO | Cyclopropyl | 3.53 ^{a)} | 525.1^{a)} |
| Ik-170 | A | CF₂CF₃ | CF₃ | CH₃ | H | C-Cl | C-H | C-Cl | C-H | CONH | CO | Cyclopropyl | 3.78^{a)} | 538.9^{a)} |
| Ik-171 | A | CF₂CF₃ | CF₃ | CH₃ | H | C-H | C-Cl | C-H | C-Cl | CONH | CO | 2.2.2-Trifluorethyl | 3.97^{a)} | 581.0 ^{a)} |
| Ik-172 | A | CF₂CF₃ | CF₃ | CH₃ | H | C-H | C-Br | C-H | C-H | CONH | CO | Benzyl | 4.03 ^{a)} | 599.0 ^{a)} |
| Ik-173 | A | CF₂CF₃ | CF₃ | CH₃ | H | C-H | C-Br | C-H | C-H | CONH | CO | 2.2.2-Trifluorethyl | 3.77^{a)} | 592.9^{a)} |
| Ik-174 | A | CF₂CF₃ | CF₃ | CH₃ | H | C-H | C-Br | C-H | C-H | CONH | CO | 1-Trifluormethylethyl | 3.95 ^{a)} | 604.9 ^{a)} |
| Ik-175 | A | CF₂CF₃ | CF₃ | CH₃ | H | C-H | C-Br | C-H | C-H | CONH | CO | Cyclopropyl | 3.39 ^{a)} | 549.0^{a)} |
| Ik-176 | B | CF₂CF₃ | CF₃ | Phenyl | H | C-H | C-Cl | C-H | C-H | CONH | CO | Cyclopropyl | 4.00^{a)} | 567.0^{a)} |
| Ik-177 | B | CF₂CF₃ | CF₃ | CH₃ | H | C-H | C-Cl | C-Br | C-H | CONH | CO | Benzyl | 4.42^{a)} | 635.0 ^{a)} |
| Ik-178 | B | CF₂CF₃ | CF₃ | CH₃ | H | C-H | C-Cl | C-Br | C-H | CONH | CO | 2.2.2-Trifluorethyl | 4.14 ^{a)} | 626.9^{a)} |
| Ik-179 | A | CF₂CF₃ | CF₃ | H | H | C-H | C-Cl | C-H | C-H | CONH | CO | Cyclopropyl | 2.95^{a)} | 491.0 ^{a)} |
| Ik-180 | B | CF₂CF₃ | CF₃ | CH₃ | H | C-H | C-NO₂ | C-H | C-H | CONH | CO | Cyclopropyl | 3.27^{a)} | 516.0^{a)} |
| Ik-181 | B | CF₂CF₃ | CF₃ | CH₃ | H | C-H | C-NO₂ | C-H | C-H | CONH | CO | 2.2.2-Trifluorethyl | 3.58^{a)} | 557.9^{a)} |
| Ik-182 | A | CF₃ | I | CH₃ | H | C-H | C-Cl | C-H | C-H | CONH | CO | Cyclopropyl | 2.70^{a)} | 513.0 ^{a)} |
| Ik-183 | B | CF₂CF₃ | CF₃ | CH₃ | H | C-H | C-I | C-H | C-H | CONH | CO | Cyclopropyl | 3.45 ^{a)} | 597.0^{a)} |
| Ik-184 | B | CF₂CF₃ | CF₃ | CH₃ | H | C-H | C-I | C-H | C-H | CONH | CO | Benzyl | 4.07^{a)} | 647.0^{a)} |
| Ik-185 | B | CF₂CF₃ | CF₃ | CH₃ | H | C-H | C-OCH₃ | C-H | C-H | CONH | CO | Cyclopropyl | 3.32^{a)} | 501.1 ^{a)} |
| Ik-186 | B | CF₂CF₃ | CF₃ | CH₃ | H | C-H | C-Cl | C-H | C-H | CONH | CO | 3.3-Dichlorprop-2-en-1-yl | 4.09 ^{a)} | 572.9^{a)} |
| Ik-187 | B | CF₂CF₃ | CF₃ | CH₃ | H | C-H | C-Cl | C-Cya n | C-H | CONH | CO | Cyclopropyl | 3.40^{a)} | 530.0^{a)} |
| Ik-188 | B | CF₂CF₃ | CF₃ | CH₃ | H | C-H | C-Cl | C-F | C-H | CONH | CO | Cyclopropyl | 3.59^{a)} | 523.1^{a)} |
| Ik-189 | B | CF₂CF₃ | CF₃ | CH₃ | H | C-H | C-Cl | C-F | C-H | CONH | CO | Benzyl | 4.14^{a)} | 573.1^{a)} |
| Ik-190 | B | CF₂CF₃ | CF₃ | CH₃ | H | C-H | C-Cl | C-F | C-H | CONH | CO | 2.2.2-Trifluorethyl | 3.91 ^{a)} | 565.0^{a)} |
| Ik-191 | B | CF₂CF₃ | CF₃ | CH₃ | H | C-H | C-SCH₃ | C-F | C-H | CONH | CO | Cyclopropyl | 3.57^{a)} | 535.1 ^{a)} |
| Ik-192 | B | CF₂CF₃ | CF₃ | CH₃ | H | C-H | C-SCH₃ | C-F | C-H | CONH | CO | Benzyl | 4.15 ^{a)} | 585.1 ^{a)} |
| Ik-193 | B | CF₂CF₃ | CF₃ | CH₃ | H | C-H | C-SCH₃ | C-F | C-H | CONH | CO | 2.2.2-Trifluorethyl | 3.90^{a)} | 577.0 ^{a)} |
| Ik-194 | B | CF₂CF₃ | CF₃ | CH₃ | H | C-H | C-SO₂CH₃ | C-F | C-H | CONH | CO | Cyclopropyl | 2.92^{a)} | 567.1^{a)} |
| Ik-195 | A | CF₃ | CF₃ | CH₃ | H | C-H | C-Cl | C-H | C-H | CONH | CO | Cyclopropyl | 2.89^{a)} | 455.1 ^{a)} |
| Ik-196 | A | CF₃ | CF₃ | CH₃ | H | C-H | C-Cl | C-H | C-H | CONH | CO | 3-Chlor-prop-2-enyl | 3.28^{a)} | 489.1^{a)} |
| Ik-197 | A | CF₃ | CF₃ | H | H | C-H | C-Cl | C-H | C-H | CONH | CO | Cyclopropyl | 2.55^{a)} | 441.1 ^{a)} |
| Ik-198 | B | CF₂CF₃ | CF₃ | CH₃ | H | C-H | C-SCH₃ | C-Br | C-H | CONH | CO | Cyclopropyl | 3.87^{a)} | 596.9^{a)} |
| Ik-199 | B | CF₂CF₃ | CF₃ | CH₃ | H | C-H | C-SCH₃ | C-Br | C-H | CONH | CO | Benzyl | 4.43 ^{a)} | 647.0^{a)} |
| Ik-200 | B | CF₂CF₃ | CF₃ | CH₃ | H | C-H | C-SCH₃ | C-Br | C-H | CONH | CO | 2,2,2-Trifluorethyl | 4.22 ^{a)} | 639.0 ^{a)} |
| Ik-201 | B | CF₂CF₃ | CF₃ | CH₃ | H | C-H | C-SCH₃ | C-Cl | C-H | CONH | CO | Cyclopropyl | 3.80^{a)} | 551.0^{a)} |
| Ik-202 | B | CF₂CF₃ | CF₃ | CH₃ | H | C-H | C-SCH₃ | C-Cl | C-H | CONH | CO | Benzyl | 4.39 ^{a)} | 601.0 ^{a)} |
| Ik-203 | B | CF₂CF₃ | CF₃ | CH₃ | H | C-H | C-SCH₃ | C-Cl | C-H | CONH | CO | 2.2.2-Trifluorethyl | 4.15 ^{a)} | 593.0 ^{a)} |
| Ik-204 | B | CF₂CF₃ | CF₃ | CH₃ | H | C-H | C-I | C-Cl | C-H | CONH | CO | Cyclopropyl | 3.84^{a)} | 630.9 ^{a)} |
| Ik-205 | B | CF₂CF₃ | CF₃ | CH₃ | H | C-H | C-I | C-Cl | C-H | CONH | CO | Benzyl | 4.45 ^{a)} | 681.0^{a)} |
| Ik-206 | B | CF₂CF₃ | CF₃ | CH₃ | H | C-H | C-I | C-Cl | C-H | CONH | CO | 2.2.2-Trifluorethyl | 4.18^{a)} | 672.9^{a)} |
| Ik-207 | B | CF₂CF₃ | CF₃ | CH₃ | H | C-H | C-Br | C-Br | C-H | CONH | CO | Cyclopropyl | 3.84^{a)} | 628.8^{a)} |
| Ik-208 | B | CF₂CF₃ | CF₃ | CH₃ | H | C-H | C-Br | C-Br | C-H | CONH | CO | Benzyl | 4.42^{a)} | 678.9^{a)} |
| Ik-209 | B | CF₂CF₃ | CF₃ | CH₃ | H | C-H | C-Br | C-Br | C-H | CONH | CO | 2,2,2-Trifluorethyl | 4.18^{a)} | 670.9^{a)} |
| Alk-2 10 | B | CF₂CF₃ | CF₃ | CH₃ | H | C-H | C-I | C-Br | C-H | CONH | CO | Cyclopropyl | 3.92^{a)} | 676.9^{a)} |
| Alk-211 | B | CF₂CF₃ | CF₃ | CH₃ | H | C-H | C-I | C-Br | C-H | CONH | CO | Benzyl | 4.49 ^{a)} | 726.9^{a)} |
| Ik-212 | B | CF₂CF₃ | CF₃ | CH₃ | H | C-H | C-I | C-Br | C-H | CONH | CO | 2,2,2-Trifluorethyl | 4.15 ^{a)} | 718.8 ^{a)} |
| Ik-213 | B | CF₂CF₃ | CF₃ | CH₃ | H | C-H | C-Br | C-Cl | C-H | CONH | CO | Cyclopropyl | 3.81^{a)} | 584.9^{a)} |
| Ik-214 | B | CF₂CF₃ | CF₃ | CH₃ | H | C-H | C-Br | C-Cl | C-H | CONH | CO | Benzyl | 4.42^{a)} | 635.0 ^{a)} |
| Ik-215 | B | CF₂CF₃ | CF₃ | CH₃ | H | C-H | C-Br | C-Cl | C-H | CONH | CO | 2,2,2-Trifluorethyl | 4.14 ^{a)} | 627.0^{a)} |
| Ik-216 | A | CF₂CF₃ | CF₃ | Ethyl | H | C-H | C-Cl | C-H | C-H | CONH | CO | 2,2,2-Trifluorethyl | 3.93 ^{a)} | 561.1 ^{a)} |
| Ik-217 | A | CF₂CF₃ | CF₃ | Ethyl | H | C-H | C-Cl | C-H | C-H | CONH | CO | Cyclopropyl | 3.63 ^{a)} | 519.1 ^{a)} |
| Ik-218 | A | CF₂CF₃ | CF₃ | 1-Methylethyl | H | C-H | C-Cl | C-H | C-H | CONH | CO | Cyclopropyl | 4.02^{a)} | 533.1 ^{a)} |
| Ik-219 | A | CF₂CF₃ | CF₃ | CH₂OC H₃ | H | C-H | C-Cl | C-H | C-H | CONH | CO | Cyclopropyl | 3.50^{a)} | 535.1 ^{a)} |
| Ik-220 | A | CF₂CF₃ | CF₃ | CH₂OC H₃ | H | C-H | C-Cl | C-H | C-H | CONH | CO | 2,2,2-Trifluorethyl | 3.82^{a)} | 577.1 ^{a)} |
| Ik-221 | A | CF₂CF₃ | CF₃ | CH₃ | H | C-H | C-H | C-Br | C-H | CONH | CO | Cyclopropyl | 3.74 ^{a)} | 551.0^{a)} |
| Ik-222 | A | CF₂CF₃ | CF₃ | CH₃ | H | C-H | C-H | C-Br | C-H | CONH | CO | Benzyl | 4.35 ^{a)} | 601.1 ^{a)} |
| Ik-223 | A | CF₂CF₃ | CF₃ | CH₃ | H | C-H | C-H | C-Br | C-H | CONH | CO | 2,2,2-Trifluorethyl | 4.09 ^{a)} | 593.0 ^{a)} |
| Ik-224 | B | CF₂CF₃ | CF₃ | CH₃ | H | C-H | C-I | C-F | C-H | CONH | CO | 3-Chlor-prop-2-enyl | 3.99 ^{a)} | 649.0 ^{a)} |
| Ik-225 | B | CF₂CF₃ | CF₃ | CH₃ | H | C-H | C-I | C-F | C-H | CONH | CO | Cyclopropyl | 3.63 ^{a)} | 615.0 ^{a)} |
| Ik-226 | B | CF₂CF₃ | CF₃ | CH₃ | H | C-H | C-I | C-F | C-H | CONH | CO | 2,2,2-Trifluorethyl | 3.95 ^{a)} | 657.0^{a)} |
| Ik-227 | B | CF₂CF₃ | CF₃ | CH₃ | H | C-H | C-I | C-F | C-H | CONH | CO | Benzyl | 4.20^{a)} | 665.0 ^{a)} |
| Ik-228 | B | CF₂CF₃ | CF₃ | CH₃ | H | C-H | C-Br | C-F | C-H | CONH | CO | 2,2,2-Trifluorethyl | 3.88^{a)} | 611.0 ^{a)} |
| Ik-229 | B | CF₂CF₃ | CF₃ | CH₃ | H | C-H | C-Br | C-F | C-H | CONH | CO | Benzyl | 4.14 ^{a)} | 619.0 ^{a)} |
| Ik-230 | A | CF(CH₃)₂ | CF₃ | CH₃ | H | C-H | C-Cl | C-H | C-H | CONH | CO | Cyclopropyl | 2.70 [M-F]^{a)} | 427.0^{a)} |
| Ik-231 | A | CHF2 | CF₃ | CH₃ | H | C-H | C-Cl | C-H | C-H | CONH | CO | Cyclopropyl | 2.46^{a)} | 437.0^{a)} |
| Ik-232 | A | CHF2 | CF₃ | CH₃ | H | C-H | C-Cl | C-H | C-H | CONH | CO | 3-Chlor-prop-2-enyl | 2.92^{a)} | 471.0^{a)} |
| Ik-234 | A | OCHF₂ | H | CH₃ | H | C-H | C-Cl | C-H | C-H | CONH | CO | Cyclopropyl | 2.17^{a)} | 385.1^{a)} |
| Ik-235 | A | Cyclopropyl | Br | CH₃ | H | C-H | C-Cl | C-H | C-H | CONH | CO | Cyclopropyl | 2.60^{a)} | 437.0^{a)} |
| Ik-236 | A | OCHF₂ | Br | CH₃ | H | C-H | C-Cl | C-H | C-H | CONH | CO | 2,2,2- Trifluorethyl | 2.88^{a)} | 505.0^{a)} |
| Ik-237 | A | OCHF₂ | Br | CH₃ | H | C-H | C-Cl | C-H | C-H | CONH | CO | Cyclopropyl | 2.51^{a)} | 462.9^{a)} |
| Ik-238 | A | OCHF₂ | Cl | CH₃ | H | C-H | C-Cl | C-H | C-H | CONH | CO | Cyclopropyl | 2.52^{a)} | 419.0 ^{a)} |
| Ik-239 | A | OCHF₂ | Cl | CH₃ | H | C-H | C-Cl | C-H | C-H | CONH | CO | 2,2,2-Trifluorethyl | 2.89 ^{a)} | 460.9 al |
| Ik-240 | A | OCHF₂ | I | CH₃ | H | C-H | C-Cl | C-H | C-H | CONH | CO | Cyclopropyl | 2.54^{a)} | 511.0 ^{a)} |
| Ik-241 | A | OCHF₂ | I | CH₃ | H | C-H | C-Cl | C-H | C-H | CONH | CO | 2,2,2-Trifluorethyl | 2.92 ^{a)} | 553.0^{a)} |
| Ik-242 | A | CF₃CH₂O | Cl | CH₃ | H | C-H | C-Cl | C-H | C-H | CONH | CO | Cyclopropyl | 2.89^{a)} | 451.0 ^{a)} |
| Ik-243 | A | CF₃CH₂O | Cl | CH₃ | H | C-H | C-Cl | C-H | C-H | CONH | CO | 2,2,2-Trifluorethyl | 3.27^{a)} | 492.9^{a)} |
| Ik-244 | A | CF₃CH₂O | I | CH₃ | H | C-H | C-Cl | C-H | C-H | CONH | CO | 2,2,2-Trifluorethyl | 3.24 ^{a)} | 584.9^{a)} |
| Ik-245 | A | CF₃CH₂O | I | CH₃ | H | C-H | C-Cl | C-H | C-H | CONH | CO | Cyclopropyl | 2.85^{a)} | 542.9^{a)} |
| Ik-246 | A | CF₃CH₂O | H | CH₃ | H | C-H | C-Cl | C-H | C-H | CONH | CO | 2,2,2-Trifluorethyl | 2.92^{a)} | 459.0 ^{a)} |
| Ik-247 | A | CF₃CH₂O | H | CH₃ | H | C-H | C-Cl | C-H | C-H | CONH | CO | Cyclopropyl | 2.55^{a)} | 417.1^{a)} |
| Ik-248 | A | 4-Fluorphenyl | Br | CH₃ | H | C-H | C-Cl | C-H | C-H | CONH | CO | Cyclopropyl | 3.04 ^{a)} | 492.9^{a)} |
| Ik-249 | A | 4-(Trifluormeth yl)phenyl | Br | CH₃ | H | C-H | C-Cl | C-H | C-H | CONH | CO | Cyclopropyl | 3.63 ^{a)} | 543.1 ^{a)} |
| Ik-250 | A | 2,4-Dichlorphenyl | Br | CH₃ | H | C-H | C-Cl | C-H | C-H | CONH | CO | Cyclopropyl | 3.59^{a)} | 543.0 ^{a)} |
| Ik-251 | A | 4-(Trifluormeth oxy)phenyl | Br | CH₃ | H | C-H | C-Cl | C-H | C-H | CONH | CO | Cyclopropyl | 3.76 ^{a)} | 557.0 ^{a)} |
| Ik-252 | A | 2-Chlorphenyl | Br | CH₃ | H | C-H | C-Cl | C-H | C-H | CONH | CO | 2,2,2-Trifluorethyl | 3.41 ^{a)} | 549.0 ^{a)} |
| Ik-253 | A | 4-(Trifluormeth yl)phenyl | Br | CH₃ | H | C-H | C-Cl | C-H | C-H | CONH | CO | 2,2,2-Trifluorethyl | 3.99 ^{a)} | 585.0 ^{a)} |
| Ik-254 | A | 2,4-Dichlorphenyl | Br | CH₃ | H | C-H | C-Cl | C-H | C-H | CONH | CO | 2,2,2-Trifluorethyl | 4.04 ^{a)} | 585.0 ^{a)} |
| Ik-255 | A | 4-(Trifluormeth oxy)phenyl | Br | CH₃ | H | C-H | C-Cl | C-H | C-H | CONH | CO | 2,2,2-Trifluorethyl | 4.15 ^{a)} | 601.0 ^{a)} |
| Ik-256 | A | CF₃CH₂O | Br | CH₃ | H | C-H | C-Cl | C-H | C-H | CONH | CO | Cyclopropyl | 2.87 ^{a)} | 497.0 ^{a)} |
| Ik-257 | A | CF₃CH₂O | Br | CH₃ | H | C-H | C-Cl | C-H | C-H | CONH | CO | 2,2,2-Trifluorethyl | 3.26 ^{a)} | 538.9 ^{a)} |
| Ik-258 | A | 3,5-Bis(trifluorme thyl)phenyl | CF₃ | CH₃ | H | C-H | C-Cl | C-H | C-H | CONH | CO | 2,2,2-Trifluorethyl | 4.46 ^{a)} | 641.0 ^{a)} |
| Ik-259 | A | 3,5-Bis(trifluorme thyl)phenyl | CF₃ | CH₃ | H | C-H | C-Cl | C-H | C-H | CONH | CO | Cyclopropyl | 4.19 ^{a)} | 599.1 ^{a)} |
| Ik-260 | A | CF₃CH₂O | F | CH₃ | H | C-H | C-Cl | C-H | C-H | CONH | CO | Cyclopropyl | 2.75 ^{a)} | 435.1 ^{a)} |
| Ik-261 | A | Cyclopropyl | CF₃ | CH₃ | H | C-H | C-Cl | C-H | C-H | CONH | CO | Cyclopropyl | 2.78 ^{a)} | 427.1 ^{a)} |
| Ik-262 | C | CF₂CF₃ | Prop-2-enyl | CH₃ | H | C-H | C-Cl | C-H | C-H | CONH | CO | Cyclopropyl | 3.37 ^{a)} | 475.2 ^{a)} |
| Ik-263 | C | CF₂CF₃ | Ethyl | CH₃ | H | C-H | C-H | C-H | C-H | CONH | CO | Cyclopropyl | 3.05 ^{a)} | 431.1 ^{a)} |
| Ik-264 | C | CF₃OC₂F4OC F₂ | I | CH₃ | H | C-H | C-Cl | C-H | C-H | CONH | CO | Cyclopropyl | 3.66 ^{a)} | 694.8 ^{a)} |
| Ik-265 | C | C4F9 | I | CH₃ | H | C-H | C-Cl | C-H | C-H | CONH | CO | Cyclopropyl | 3.90 ^{a)} | 663.0 ^{a)} |
| Ik-266 | A | C4F9 | H | CH₃ | H | C-H | C-Cl | C-H | C-H | CONH | CO | Cyclopropyl | 3.74 ^{a)} | 536.8 ^{a)} |
| Ik-267 | C | C4F9 | I | CH₃ | H | C-H | C-Cl | C-H | C-H | CONH | CO | 1-Cyclopropylethyl | 4.38 ^{a)} | 691.0 ^{a)} |
| Ik-268 | C | C4F9 | I | CH₃ | H | C-H | C-Cl | C-H | C-H | CONH | CO | Cyclobutyl | 4.16 ^{a)} | 6%6.9 ^{a)} |
| Ik-269 | C | C4F9 | I | CH₃ | H | C-H | C-Cl | C-H | C-H | CONH | CO | 3-Chlor-prop-2-enyl | 4.27 ^{a)} | 696.9 ^{a)} |
| Ik-270 | B | CF₂CF₃ | CF₃ | CH₃ | H | C-H | C-Cl | C-H | C-H | CONH | CO | 1-(Pyridin-3-yl)ethyl | 2,27 ^{b)} | 570.1 ^{b)} |
| Ik-271 | B | CF₂CF₃ | CH₃ | CH₃ | H | C-H | C-Cl | C-H | C-H | CONH | CO | Benzyl | 3,42 ^{b)} | 501.0 ^{b)} |
| Ik-272 | B | CF₂CF₃ | Ethyl | CH₃ | H | C-H | C-Cl | C-H | C-H | CONH | CO | Benzyl | 3,68 ^{b)} | 515.0^{b)} |
| Ik-273 | B | CF₂CF₃ | CF₃ | CH₃ | H | C-H | C-Cl | C-H | C-H | CONH | CO | [4-(Trifluormethyl)-1.3-thiazol-2-yl]methyl | 3,75 ^{b)} | 629.9^{b)} |
| Ik-274 | B | CF₂CF₃ | CF₃ | CH₃ | H | C-H | C-Cl | C-H | C-H | CONH | CO | 2-Cyanpropan-2-yl | 3,37 ^{b)} | 531.9^{b)} |
| Ik-275 | B | CF₂CF₃ | CF₃ | CH₃ | H | C-H | C-Cl | C-H | C-H | CONH | CO | 2-Cyan-1-methoxypropan-2-yl | 3,37 ^{b)} | 562.0^{b)} |
| Ik-276 | B | CF₂CF₃ | CF₃ | CH₃ | H | C-H | C-Cl | C-H | C-H | CONH | CO | 2.2.2-Trifluor-1-[1-methyl-4-(trifluormethyl)-1H-imidazol-2-yl]ethyl | 4,00 ^{b)} | 695.1^{b)} |
| Ik-277 | B | CF₂CF₃ | CF₃ | CH₃ | H | C-H | C-Cl | C-H | C-H | CONH | CO | 2,2,2-Trifluor-1-(4-methyl-1,3-thiazol-2-yl)ethyl | 4,00 ^{b)} | 644.1 ^{b)} |
| Ik-278 | B | CF₂CF₃ | CF₃ | CH₃ | H | C-H | C-Cl | C-H | C-H | CONH | CO | 1.1-Dioxido-2.3-dihydrothiophen-3-yl | 2,80 ^{b)} | 581.0^{b)} |
| Ik-279 | B | CF₂CF₃ | CF₃ | CH₃ | H | C-H | C-Cl | C-H | C-H | CONH | CO | (1R,2S)-2-Methylcyclopropyl | 3,37 ^{b)} | 519.0^{b)} |
| Ik-280 | B | CF₂CF₃ | CF₃ | CH₃ | H | C-H | C-Cl | C-H | C-H | CONH | CO | (1R,2R)-2-Methylcyclopropyl | 3,46 ^{b)} | 519.0^{b)} |
| Ik-281 | B | CF₂CF₃ | CF₃ | CH₃ | H | C-H | C-Cl | C-H | C-H | CONH | CO | 2-(2,2,2-Trifluorethoxy)ethyl | 3,53 ^{b)} | 591.1 ^{b)} |
| Ik-282 | B | CF₂CF₃ | CF₃ | CH₃ | H | C-H | C-Cl | C-H | C-H | CONH | CO | 2-Chlorbenzyl | 4,05 ^{b)} | 589.1 ^{b)} |
| Ik-283 | B | CF₂CF₃ | CF₃ | CH₃ | H | C-H | C-Cl | C-H | C-H | CONH | CO | 4-Chlorbenzyl | 4,05 ^{b)} | 589.1 ^{b)} |
| Ik-284 | B | CF₂CF₃ | CF₃ | CH₃ | H | C-H | C-Cl | C-H | C-H | CONH | CO | 1-(2-Fluorphenyl)ethyl | 4,00 ^{b)} | 587.1 ^{b)} |
| Ik-285 | B | CF₂CF₃ | CF₃ | CH₃ | H | C-H | C-Cl | C-H | C-H | CONH | CO | Tetrahydro-2H-pyran-4-yl | 3,06 ^{b)} | 549.1 ^{b)} |
| Ik-286 | B | CF₂CF₃ | CF₃ | CH₃ | H | C-H | C-Cl | C-H | C-H | CONH | CO | 2-Ethylcyclopropyl | 3,84 ^{b)} | 533.1 ^{b)} |
| Ik-287 | B | CF₂CF₃ | CF₃ | CH₃ | H | C-H | C-Cl | C-H | C-H | CONH | CO | Cyclopent-3-en-1-yl | 3,63 ^{b)} | 531.1 ^{b)} |
| Ik-288 | B | CF₂CF₃ | CF₃ | CH₃ | H | C-H | C-Cl | C-H | C-H | CONH | CO | 1-(1-Chlorcyclopropyl)eth yl | 4,05 ^{b)} | 567^{b)} |
| Ik-289 | B | CF₂CF₃ | CF₃ | CH₃ | H | C-H | C-Cl | C-H | C-H | CONH | CO | 2-Methyl-1-(methylsulfanyl)prop an-2-yl | 4,05 ^{b)} | 567.1 ^{b)} |
| Ik-290 | B | CF₂CF₃ | CF₃ | CH₃ | H | C-H | C-Cl | C-H | C-H | CONH | CO | 1-Fluorpropan-2-yl | 3,28 ^{b)} | 525.1 ^{b)} |
| Ik-291 | B | CF₂CF₃ | CF₃ | CH₃ | H | C-H | C-Cl | C-H | C-H | CONH | CO | 3,3,3-Trifluorpropyl | 3,53 ^{b)} | 561.0^{b)} |
| Ik-292 | B | CF₂CF₃ | CF₃ | CH₃ | H | C-H | C-Cl | C-H | C-H | CONH | CO | 1-Cyanethyl | 3,02 ^{b)} | 517.9^{b)} |
| Ik-293 | B | CF₂CF₃ | CF₃ | CH₃ | H | C-H | C-Cl | C-H | C-H | CONH | CO | But-3-in-2-yl | 3,28 ^{b)} | 516.9^{b)} |
| Ik-294 | B | CF₂CF₃ | CF₃ | CH₃ | H | C-H | C-Cl | C-H | C-H | CONH | CO | (2S)-1,1,1-Trifluorpropan-2-yl | 3,63 ^{b)} | 561.1 ^{b)} |
| Ik-295 | B | CF₂CF₃ | CF₃ | CH₃ | H | C-H | C-Cl | C-H | C-H | CONH | CO | (2Z)-3-Chlorbut-2-en-1-yl | 3,73 ^{b)} | 553.1^{b)} |
| Ik-296 | B | CF₂CF₃ | CF₃ | CH₃ | H | C-H | C-Cl | C-H | C-H | CONH | CO | 1-(Trifluormethyl)cyclo propyl | 3,58 ^{b)} | 573.1 ^{b)} |
| Ik-297 | B | CF₂CF₃ | CF₃ | CH₃ | H | C-H | C-Cl | C-H | C-H | CONH | CO | 1,1,1-Trifltiorbtitan-2-yl | 3,89 ^{b)} | 575.1 ^{b)} |
| Ik-298 | B | CF₂CF₃ | CF₃ | CH₃ | H | C-H | C-Cl | C-H | C-H | CONH | CO | 1,1,1-Trifltiorpentan-2-yl | 4,11 ^{b)} | 589.1 ^{b)} |
| Ik-299 | B | CF₂CF₃ | CF₃ | CH₃ | H | C-H | C-Cl | C-H | C-H | CONH | CO | 4,4,4-Trifluorbutan-2-yl | 3,63 ^{b)} | 575.1 ^{b)} |
| Ik-300 | B | CF₂CF₃ | CF₃ | CH₃ | H | C-H | C-Cl | C-H | C-H | CONH | CO | 4,4,4-Trifluor-2-methylbutan-2-yl | 4,00 ^{b)} | 589.1 ^{b)} |
| Ik-301 | B | CF₂CF₃ | CF₃ | CH₃ | H | C-H | C-Cl | C-H | C-H | CONH | CO | 2-Methyl-1-(methylsulfonyl)prop an-2-yl | 3,02^{b)} | 599.1^{b)} |
| Ik-302 | B | CF₂CF₃ | CF₃ | CH₃ | H | C-H | C-Cl | C-H | C-H | CONH | CO | 2,2,3,3,3-Pentafluorpropyl | 3,78^{b)} | 597.1^{b)} |
| Ik-303 | B | CF₂CF₃ | CF₃ | CH₃ | H | C-H | C-Cl | C-H | C-H | CONH | CO | 1-(3-Fluorphenyl)ethyl | 3,89^{b)} | 587.1^{b)} |
| Ik-304 | B | CF₂CF₃ | CF₃ | CH₃ | H | C-H | C-Cl | C-H | C-H | CONH | CO | 3-Methylbenzyl | 3,94^{b)} | 569.1^{b)} |
| Ik-305 | B | CF₂CF₃ | CF₃ | CH₃ | H | C-H | C-Cl | C-H | C-H | CONH | CO | 2-Methylbenzyl | 3,89^{b)} | 569.1^{b)} |
| Ik-306 | B | CF₂CF₃ | CF₃ | CH₃ | H | C-H | C-Cl | C-H | C-H | CONH | CO | 4-Methylbenzyl | 3,94^{b)} | 569.1^{b)} |
| Ik-307 | B | CF₂CF₃ | CF₃ | CH₃ | H | C-H | C-Cl | C-H | C-H | CONH | CO | (2R)-1,1,1-Trifluorpropan-2-yl | 3,78^{b)} | 561.0^{b)} |
| Ik-308 | B | CF₂CF₃ | CF₃ | CH₃ | H | C-H | C-Cl | C-H | C-H | CONH | CO | 2,4-Difluorbenzyl | 3,94^{b)} | 591.0^{b)} |
| Ik-309 | B | CF₂CF₃ | CF₃ | CH₃ | H | C-H | C-Cl | C-H | C-H | CONH | CO | 3,4-Difluorbenzyl | 3,94^{b)} | 591.1^{b)} |
| Ik-310 | B | CF₂CF₃ | CF₃ | CH₃ | H | C-H | C-Cl | C-H | C-H | CONH | CO | (2S)-1,1,1-Trifluor-3-methylbutan-2-yl | 3,89^{b)} | 589.1^{b)} |
| Ik-311 | B | CF₂CF₃ | CF₃ | CH₃ | H | C-H | C-Cl | C-H | C-H | CONH | CO | (1S,2R)-2-Fluorcyclopropyl | 3,28^{b)} | 523.1^{b)} |
| Ik-312 | B | CF₂CF₃ | CF₃ | CH₃ | H | C-H | C-Cl | C-H | C-H | CONH | CO | 4-Fluorbenzyl | 3,73^{b)} | 573.0^{b)} |
| Ik-313 | B | CF₂CF₃ | CF₃ | CH₃ | H | C-H | C-Cl | C-H | C-H | CONH | CO | Pyridin-4-ylmethyl | 1,93^{b)} | 555.9^{b)} |
| Ik-314 | B | CF₂CF₃ | CF₃ | CH₃ | H | C-H | C-Cl | C-H | N | CONH | CO | (1S,2R)-2-Fluorcyclopropyl | 3,14^{a)} | 524,1^{a)} |
| Ik-315 | B | CF₂CF₃ | CF₃ | CH₃ | H | C-H | C-Br | C-H | N | CONH | CO | Prop-2-enyl | 3,42^{a)} | 552,0^{a)} |
| Ik-316 | B | CF₂CF₃ | CF₃ | CH₃ | H | C-H | C-Cl | C-H | N | CONH | CO | Prop-2-enyl | 3,38^{a)} | 506,1^{a)} |
| Ik-317 | B | CF₂CF₃ | CF₃ | CH₃ | H | C-H | C-Cl | C-H | N | CONH | CO | 2,2,2-Trifluorethyl | 3,61^{a)} | 548,1^{a)} |
| Ik-318 | B | CF₂CF₃ | CF₃ | CH₃ | H | C-H | C-Cl | C-H | N | CONH | CO | Cyclopropyl | 3,24^{a)} | 506,1^{a)} |
| Ik-319 | B | CF₂CF₃ | CF₃ | CH₃ | H | C-H | C-Cl | N | C-H | CONH | CO | (1S,2R)-2-Fluorcyclopropyl | 2.98^{a)} | 524,1^{a)} |
| Ik-320 | B | CF₂CF₃ | CF₃ | CH₃ | H | C-H | C-Cl | N | C-H | CONH | CO | 2,2,2-Trifluorethyl | 3,37^{a)} | 548,0^{a)} |
| Ik-321 | B | CF₂CF₃ | CF₃ | CH₃ | H | C-H | C-Cl | N | C-H | CONH | CO | Prop-2-enyl | 3,15^{a)} | 506,0^{a)} |
| Ik-322 | B | CF₂CF₃ | CF₃ | CH₃ | H | C-H | C-Cl | N | C-H | CONH | CO | Cyclopropyl | 3,03^{a)} | 506,1^{a)} |
| Ik-323 | B | CF₂CF₃ | CF₃ | CH₃ | H | C-H | C-Cl | N | C-H | CONH | CO | 2,2-Difluorcyclopropyl | 3,22^{a)} | 542,0^{a)} |
| Ik-324 | B | CF₂CF₃ | CF₃ | CH₃ | H | C-H | C-Cl | N | C-H | CONH | CO | Propyl | 3,33^{a)} | 508,0^{a)} |
| Ik-325 | B | CF₂CF₃ | CF₃ | CH₃ | H | C-H | C-Cl | N | C-H | CONH | CO | 2-Methylcyclopropyl | 3,35^{a)} | 520.1^{a)} |
| Ik-326 | B | CF₂CF₃ | CF₃ | CH₃ | H | C-H | C-Cl | N | C-H | CONH | CO | 1-Methylethyl | 3,28^{a)} | 508,1^{a)} |
| Ik-327 | B | CF₂CF₃ | CF₃ | CH₃ | H | C-H | C-Cl | N | C-H | CONH | CO | (1S,2R)-2-Fluorcyclopropyl | 2.98^{a)} | 524,1^{a)} |
| Ik-328 | B | CF₂CF₃ | CF₃ | CH₃ | H | C-H | C-Cl | N | C-H | CONH | CO | 3-Chlor-prop-2-enyl | 3,43^{a)} | 540,0^{a)} |
| Ik-329 | B | CF₂CF₃ | CF₃ | CH₃ | H | C-H | C-Cl | N | C-H | CONH | CO | 1-Trifluormethylethyl | 3,62^{a)} | 562.1^{a)} |
| Ik-330 | B | CF₂CF₃ | CF₃ | CH₃ | H | N | C-H | C-H | C-H | CONH | CO | Cyclopropyl | n.d.^{a)} | 472.1^{a)} |
| Ik-331 | B | CF₂CF₃ | CF₃ | CH₃ | H | N | C-H | C-H | C-H | CONH | CO | 2-Fluorcyclopropyl | 3,46^{a)} | 490,1^{a)} |
| Ik-332 | B | CF₂CF₃ | CF₃ | CH₃ | H | N | C-Br | C-H | C-H | CONH | CO | 2-Fluorcyclopropyl | 3,65^{a)} | 568,0^{a)} |
| Ik-333 | B | CF₂CF₃ | CF₃ | CH₃ | H | N | C-Br | C-H | C-H | CONH | CO | 2,2,2-Trifluorethyl | 3,97^{a)} | 592,0^{a)} |
| Ik-334 | B | CF₂CF₃ | CF₃ | CH₃ | H | N | C-Br | C-H | C-H | CONH | CO | 1-Trifluormethylethyl | 4,23^{a)} | 606,6^{a)} |
| Ik-335 | B | CF₂CF₃ | CF₃ | CH₃ | H | N | C-Br | C-H | C-H | CONH | CO | 2-Methyl-1-(methylsulfanyl)prop an-2-yl | 4,50^{a)} | 613,9^{a)} |
| Ik-336 | B | CF₂CF₃ | CF₃ | CH₃ | H | N | C-Br | C-H | C-H | CONH | CO | Propyl | 3,82^{a)} | 552,0^{a)} |
| Ik-337 | B | CF₂CF₃ | CF₃ | CH₃ | H | N | C-Br | C-H | C-H | CONH | CO | 1-Cyclopropylethyl | 4,14^{a)} | 578,0^{a)} |
| Ik-338 | B | CF₂CF₃ | CF₃ | CH₃ | H | N | C-Br | C-H | C-H | CONH | CO | Prop-2-enyl | 3,73^{a)} | 549,9^{a)} |
| Ik-339 | B | CF₂CF₃ | CF₃ | CH₃ | H | C-H | C-I | C-F | C-H | CON(CH₂C H₃) | CO | Cyclopropyl | 4.37^{a)} | 643.1^{a)} |
| Ik-340 | B | CF₂CF₃ | CF₃ | CH₃ | H | C-H | C-Cl | C-H | C-H | CON(CH₃) | CO | 2,2,2-Trifluorethyl | 3,84 ^{b)} | 561.0^{a)} |
| Ik-341 | B | CF₂CF₃ | CF₃ | CH₃ | H | C-H | C-Cl | C-H | C-H | CON(CH₃) | CO | 2,2-Difluorethyl | 3,53 ^{b)} | 543.0^{b)} |
| Ik-342 | B | CF₂CF₃ | CF₃ | CH₃ | H | C-H | C-Cl | C-H | C-H | CON(Cyclo propyl) | CO | Pyridin-2-ylmethyl | 3,47 ^{b)} | 596.1^{b)} |
| Ik-343 | B | CF₂CF₃ | CF₃ | CH₃ | H | C-H | C-Cl | C-H | C-H | | CO | 3,3-Difluorazetidin-1-yl | 3,58^{b)} | 541.0^{b)} |
| Ik-344 | B | CF₂CF₃ | CF₃ | CH₃ | H | C-H | C-Cl | C-H | C-H | CONH(CH₂ CH₃) | CO | Cyclopropyl | 3,84^{b)} | 533.1^{b)} |
| Ik-345 | B | CF₂CF₃ | CF₃ | CH₃ | H | C-H | C-Cl | C-H | C-H | CO | CO | 4-(Trifluormethyl)piper idin-1-yl | 3,94^{b)} | 601.1^{b)} |
| Ik-346 | B | CF₂CF₃ | CF₃ | CH₃ | H | C-H | C-Cl | C-H | C-H | CON(Cyclo propyl) | CO | Cyclopropyl(tetrahyd ro-2H-pyran-4-yl)amino | 3,77^{b)} | 589.1^{b)} |
| Ik-347 | A | CF₂CF₃ | CF₃ | CH₃ | H | C-H | C-Cl | C-H | C-H | CONH | SO₂ | Cyclopropyl | 3.38^{a)} | 541.0^{a)} |
| Ik-348 | A | CF₂CF₃ | CF₃ | CH₃ | H | C-H | C-Cl | C-H | C-H | CONH | SO₂ | 1-Cyclopropylethyl | 4.08^{a)} | 569.1^{a)} |

Außerordentlich bevorzugt sind auch Verbindungen der allgemeinen Formeln (Ik), die sich aus einer beliebigen Kombination der in der Tabelle 6 aufgeführten Reste Z¹, Z²-, Z3, R¹, A₄, A₃, A₂, A₁, Lₘ, U und Q ergeben.

**Tabelle 7**

| 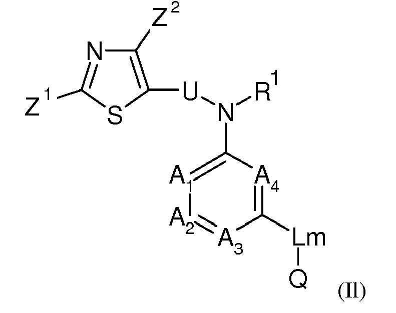 | | | | | | | | | | | | | |
|---|---|---|---|---|---|---|---|---|---|---|---|---|---|
| **Bsp.-Nr.** | **Verfahren** | **Z¹** | **Z²** | **R¹** | **A₄** | **A₃** | **A₂** | **A₁** | **Lₘ** | **U** | **Q** | **logP** | **Masse [m/z]¹** |
| Il-1 | A | CF₂CF₃ | CF₃ | H | C-H | C-Cl | C-H | C-H | CONH | CO | Cyclopropyl | 3.37^{a)} | 508.0^{a)} |

**Tabelle 8**

| | | | | | | | | | | | | | |
|---|---|---|---|---|---|---|---|---|---|---|---|---|---|
| 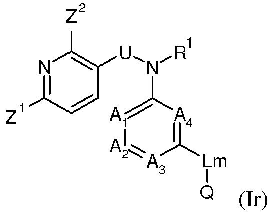 | | | | | | | | | | | | | |

| **Bsp.-Nr.** | **Verfahren** | **Z¹** | **Z²** | **R1** | **A4** | **A3** | **A2** | **A1** | **L** | **U** | **Q** | **logP** | **Masse [m/z]¹** |
|---|---|---|---|---|---|---|---|---|---|---|---|---|---|
| Ir-1 | A | CF₃ | Cl | H | C-H | C-Cl | C-H | C-H | CONH | CO | Cyclopropyl | 2,48^{a)} | 418,0^{a)} |
| Ir-2 | A | CF₃ | Me | H | C-H | C-Cl | C-H | C-H | CONH | CO | Cyclopropyl | 2,27^{a)} | 398,0^{a)} |
| Ir-3 | A | CF₃ | Cl | H | C-H | C-Cl | C-H | C-H | CONH | CO | 1-Methyl-ethyl | 2,68^{a)} | 420,0^{a)} |
| Ir-4 | A | CF₃ | Me | H | C-H | C-Cl | C-H | C-H | CONH | CO | 1-Methyl-ethyl | 2,53^{a)} | 400,1^{a)} |
| Ir-5 | A | CF₃ | Me | H | C-H | C-Cl | C-H | C-H | CONH | CO | Methyl | 1,97^{a)} | 372,0^{a)} |
| Ir-6 | A | CF₃ | Cl | H | C-H | C-Cl | C-H | C-H | CONH | CO | Methyl | 2,16^{a)} | 392,0^{a)} |
| Ir-7 | A | CF₃ | Cl | H | C-H | C-Cl | C-H | C-Me | CONH | CO | Prop-2-enyl | 2,68^{a)} | 432,0^{a)} |
| Ir-8 | A | CF₃ | Me | H | C-H | C-Cl | C-H | C-Me | CONH | CO | Prop-2-enyl | 2,45^{a)} | 412,1 ^{a)} |
| Ir-9 | A | CF₃ | Cl | H | C-H | C-Cl | C-H | C-H | CONH | CO | 2,2,2-Trifluorethyl | 2,82^{a)} | 460,0^{a)} |
| Ir-10 | A | CF₃ | Cl | H | C-H | C-Cl | C-H | C-H | CONH | CO | 3-Chlor-prop-2-enyl | 2,86^{a)} | 453,0^{a)} |
| Ir-11 | A | CF₃ | CH₃ | H | C-H | C-Cl | C-H | C-H | CONH | CO | 2,2,2- Trifluorethyl | 2.67^{a)} | 440.0 ^{a)} |

Außerordentlich bevorzugt sind auch Verbindungen der allgemeinen Formeln (Ir), die sich aus einer beliebigen Kombination der in der Tabelle 8 aufgeführten Reste Z¹, Z², Z3, R¹, A₄, A₃, A₂, A₁, Lₘ, U und Q ergeben.
¹ Bei der angegebenen Masse handelt es sich um den Peak des Isotopenmusters des [M+H]⁺ Ions mit der höchsten Intensität; falls das [M-H]⁻ Ion detektiert wurde, ist die Massenangabe mit ² gekennzeichnet.
² Bei der angegebenen Masse handelt es sich um den Peak des Isotopenmusters des [M-H]⁻ Ions mit der höchsten Intensität.
^{a)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 micron; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilend MSD-System.
^{b)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18). HP1100; 50*4,6 Zorbax Eclipse Plus C18 1,8 micron; Eluent A: Acetonitril (0,1 % Ameisensäure); Eluent B: Wasser (0,08 % Ameisensäure); linearer Gradient von 5 % Acetonitril bis 95 % Acetonitril in 1,70 min, dann 95 % Acetonitril für weitere 1,00 min; Ofentemperatur 55°C; Fluß:2,0 mL/min. Die Massendetektion erfolgt über den Massendetektor Micromass ZQ2000 der Firma Waters.

**Tabelle 9**

| **Bsp.-Nr.** | **NMR**-**Daten** |
|---|---|
| Ia-1 | ¹H-NMR (400 MHz, d₃-Acetonitril): δ = 9.31 (bs, 1H), 7.73-7-68 (m, 2H), 7.45 (d, 1H), 6.90 (bs, 1H), 2.86 (s, 3H), 2.85-2.81 (m, 1H), 0.81-0.74 (m, 2H), 0.60-0.56 (m, 2H) ppm. |
| Ib-1 | ¹H-NMR (400 MHz, d₆-DMSO): δ = 10.85 (s, 1H), 9.35 (s, 1H), 8.32 (d, 1H), 7.66-7.64 (m, 2H), 7.48--7.46 (m, 1H), 2.86-2.80 (m, 1H), 0.72-0.67 (m, 2H), 0.55-0.51 (m, 2H) ppm. |
| Ib-2 | ¹H-NMR (400 MHz, d₆-DMSO): δ = 10.78 (s, 1H), 9.22 (s, 1H), 8.31 (d, 1H), 7.67-7.65 (m, 2H), 7.47-7.45 (m, 1H), 5.21-5.14 (q, 2H), 2.85-2.81 (m, 1H), 0.72-0.67 (m, 2H), 0.55-0.51 (m, 2H) ppm. |
| Ib-3 | ¹H-NMR (400 MHz, d₆-DMSO): δ = 10.91 (s, 1H), 9.66 (s, 1H), 8.33 (d, 1H), 7.66-7.64 (m, 2H), 7.50-7.48 (m, 1H), 2.86-2.81 (m, 1H), 0.72-0.67 (m, 2H), 0.56-0.52 (m, 2H) ppm. |
| Ib-4 | ¹H-NMR (400 MHz, d₆-DMSO): δ = 10.97 (s, 1H), 9.67 (s, 1H), 9.05-9.02 (m, 1H), 7.73-7.71 (m, 2H), 7.55-7.53 (m, 1H), 4.11-4.04 (m, 2H) ppm. |
| Ib-5 | ¹H-NMR (400 MHz, d₆-DMSO): δ = 10.94 (s, 1H), 9.66 (s, 1H), 8.61-8.55 (m, 1H), 7.72-7.65 (m, 2H), 7.51 (d, 1H), 6.43-6.37 (m, 1H), 6.06-5.96 (m, 1H), 4.06-4.01 (m, 1H), 3.92-3.89 (m, 1H) ppm. |
| Ib-9 | ¹H-NMR (400 MHz, d₆-DMSO): δ = 10.97 (s, 1H), 9.70 (s, 1H), 9.04 (t, 1H), 7.73-7.71 (m, 2H), 7.56-7.53 (m, 1H), 4.11-4.02 (m, 2H) ppm. |
| Ib-14 | ¹H-NMR (400 MHz, d₆-DMSO): δ = 10.80 (s, 1H), 8.81 (s, 1H), 7.77-7.72 (m, 2H), 7.50 (d, 1H), 7.38-7.31 (m, 3H), 7.26-7.24 (m, 1H), 4.46 (d, 2H), 2.57 (s, 6H) ppm. |
| Ib-16 | ¹H-NMR (400 MHz, d₃-Acetonitril): δ = 9.49 (bs, 1H), 9.40 (s, 1H), 7.82 (d, 1H), 7.73-7.70 (m, 1H), 7.50 (d, 1H), 7.38-7.36 (m, 1H), 4.13-4.03 (m, 2H) ppm. |
| Ib-19 | ¹H-NMR (400 MHz, d₆-DMSO): δ = 10.73 (s, 1H), 9.12 (s, 1H), 8.30 (d, 1H), 7.73-7.70 (m, 2H), 7.46 (d, 1H), 2.86-2.81 (m, 1H), 2.69 (s, 3H), 0.72-0.68 (m, 2H), 0.56-0.52 (m, 2H) ppm. |
| Ib-24 | ¹H-NMR (400 MHz, d₆-DMSO): δ = 10.80 (s, 1H), 9.15 (s, 1H), 9.00 (t, 1H), 7.80-7.76 (m, 2H), 7.52 (d, 1H), 4.11-4.02 (m, 2H), 2.70 (s, 3H) ppm. |
| Ib-27 | ¹H-NMR (400 MHz, d₆-DMSO): δ = 10.91 (s, 1H), 9.56 (s, 1H), 8.33 (m, 1H), 7.70 -7.67 (m, 2H), 7.48 (d, 1H), 7.33 (t, 1H), 2.87-2.80 (m, 1H), 0.72-0.68 (m, 2H), 0.56-0.52 (m, 2H) ppm. |
| Ib-30 | ¹H-NMR (400 MHz, d₆-DMSO): δ = 10.85 (s, 1H), 9.04 (s, 1H), 8.30 (d, 1H), 7.76 -7.70 (m, 2H), 7.6 (d, 1H), 2.86-2.80 (m, 1H), 2.53-2.45 (m, 1H), 1.39-1.21 (m, 2H), 1.20-1.16 (m, 2H), 0.72-0.68 (m, 2H), 0.56-0.52 (m, 2H) ppm. |
| Ib-33 | ¹H-NMR (400 MHz, d₃-Acetonitril): δ = 9.39 (s, 1H), 9.06 (bs, 1H), 7.78 (d, 1H), 7.68-7.66 (m, 2H), 7.50 (d, 1H), 7.35 (bs, 1H), 4.13-4.04 (m, 2H) ppm. |
| Ib-34 | ¹H-NMR (400 MHz, d₃-Acetonitril): δ = 9.39 (s, 1H), 9.06 (bs, 1H), 7.78 (d, 1H), 7.68-7.66 (m, 2H), 7.50 (d, 1H), 7.35 (bs, 1H), 4.13-4.04 (m, 2H) ppm. |
| Ib-35 | ¹H-NMR (400 MHz, d₃-Acetonitril): δ = 9.38 (s, 1H), 9.06 (bs, 1H), 7.75 (d, 1H), 7.67-7.64 (m, 1H), 7.47 (d, 1H), 7.40-7.28 (m, 6H), 4.53 (d, 2H) ppm. |
| Ib-36 | ¹H-NMR (400 MHz, d₆-DMSO): δ = 11.06 (s, 1H), 9.70 (bs, 1H), 8.44 (d, 1H), 8.10 (d, 1H), 7.54 (d, 1H), 2.85-2.78 (m, 1H), 0.73-0.68 (m, 2H), 0.55-0.51 (m, 2H) ppm. |
| Ib-37 | ¹H-NMR (400 MHz, d₃-Acetonitril): δ = 9.39 (s, 1H), 9.16 (bs, 1H), 7.70 (d, 1H), 7.65 (dd, 1H), 7.46 (d, 1H), 6.84 (d, 1H), 3.55-3.49 (m, 1H), 1.26 (d, 3H), 0.96-0.92 (m, 1H), 0.52-0.43 (m, 2H), 0.38-0.27(m, 2H) ppm. |
| Ie-1 | ¹H-NMR (400 MHz, d₆-DMSO): δ = 10.83 (s, 1H), 8.87 (d, 1H), 8.31 (d, 1H), 8.02 (d, 1H), 7.75-7.71 (m, 2H), 7.46 (d, 1H), 2.86-2.81 (m, 1H), 0.72-0.67 (m, 2H), 0.56-0.52 (m, 2H) ppm. |
| Ii-1 | ¹H-NMR (400 MHz, d₆-DMSO): δ = 10.83 (s, 1H), 8.87 (d, 1H), 8.32 (d, 1H), 8.02 (d, 1H), 7.75-7.71 (m, 2H), 7.46 (d, 1H), 2.86-2.81 (m, 1H), 0.72-0.67 (m, 2H), 0.56-0.52 (m, 2H) ppm. |
| Ij-1 | ¹H-NMR (400 MHz, d₆-DMSO): δ = 10.69 (s, 1H), 8.62 (s, 1H), 8.30 (d, 1H), 7.69-7.66 (m, 2H), 7.47-7.42 (m, 2H), 5.17-5.10 (q, 2H), 2.85-2.80 (m, 1H), 0.72-0.67 (m, 2H), 0.55-0.51 (m, 2H) ppm. |
| Ij-2 | ¹H-NMR (400 MHz, d₆-DMSO): δ = 10.68 (s, 1H), 8.62 (s, 1H), 8.30 (d, 1H), 7.69-7.66 (m, 2H), 7.44-7.41 (m, 2H), 6.02-5.95 (m, 1H), 2.85-2.80 (m, 1H), 1.52 (d, 3H), 0.72-0.67 (m, 2H), 0.55-0.51 (m, 2H) ppm. |
| Ij-4 | ¹H-NMR (400 MHz, d₃-Acetonitril): δ = 9.01 (bs, 1H), 8.58 (s, 1H), 7.70 (d, 1H), 7.67-7.64 (m, 1H), 7.48 (s, 1H), 7.42 (d, 1H), 6.91-6.82 (m, 2H), 2.84-2.80 (m, 1H), 0.78-0.73 (m, 2H), 0.59-0.55 (m, 2H) ppm. |
| Ik-1 | ¹H-NMR (400 MHz, d₃-Acetonitril): δ = 9.45 (bs, 1H), 7.71 (d, 1H), 7.65 (dd, 1H), 7.45 (d, 1H), 6.94 (bs, 1H), 3.98 (s, 3H), 2.79-2.85 (m, 1H), 0.72-0.79 (m, 2H), 0.57-0.60 (m, 2H) ppm. |
| Ik-2 | ¹H-NMR (400 MHz, d₃-Acetonitril): δ = 9.16 (bs, 1H), 8.11 (d, 1H), 7.74 (dd, 1H), 7.52 (d, 1H), 3.98 (s, 3H) ppm. |
| Ik-3 | ¹H-NMR (d₆-DMSO) 11.24 (s, 1H), 8.49 (1H, t, J= 2 Hz, NH), 7.71 (1H, d, J= 2.5 Hz), 7.68 (1H, dd: J= 9, 2 Hz), 7.50 (1H, d, J= 9 Hz), 4.05 (2H, dd, J= 5.5, 2.5 Hz), 4.00 (3H, s), 3.87 (1H,s) ppm. |
| Ik-6 | ¹H-NMR (d₃-d₃-Acetonitril) 9.40 (s, 1H, br), 7.77 (d, 1H), 7.67 (dd, 1H), 7.48 (d, 1H), 7.25 (s, 1H, br), 3.98 (s, 3H), 3.45 (m, 1H), 1.90 (m, 1H), 1.55 (m, 1H) ppm. |
| Ik-7 | ¹H-NMR (400 MHz, d₃-Acetonitril): δ = 9.21 (bs, 1H), 8.08 (d, 1H), 7.57 (d, 1H), 6.93 (bs, 1H), 3.98 (s, 3H), 2.79-2.84 (m, 1H), 0.73-0.79 (m, 2H), 0.58-0.61 (m, 2H) ppm. |
| Ik-9 | ¹H-NMR (d₆-DMSO) 8.49 (1H, d, J= 4 Hz, NH), 7.70 (1H, dd, J= 8, 2.5 Hz), 7.68 (1H, s), 7.50 (1H, d, J= 8 Hz), 4.7 (1H, ddd, J= 62, 4,4 Hz), 4.00 (3H, s), 2.85 (1H, m), 1.15 (1H, m), 1.05 (1H, m) ppm. |
| Ik-18 | ¹H-NMR (400 MHz, d₃-Acetonitril): δ = 8.80 (bs, 1H), 7.73 (d, 1H), 7.67 (dd, 1H), 7.43 (d, 1H), 6.79 (bs, 1H), 4.07 (s, 3H), 2.81-2.87 (m, 1H), 0.74-0.79 (m, 2H), 0.57-0.62 (m, 2H) ppm. |
| Ik-25 | ¹H-NMR (400 MHz, d₃-Acetonitril): δ = 9.23 (bs, 1H), 7.92 (d, 1H), 7.76 (dd, 1H), 7.25 (d, 1H), 7.08 (bs, 1H), 6.78 (t, 1H), 3.98 (s, 3H), 2.81-2.86 (m, 1H), 0.74-0.79 (m, 2H), 0.55-0.60 (m, 2H) ppm. |
| Ik-43 | ¹H-NMR (d₆-DMSO) 11.43 (s, 1H), 9.12 (t, 1H), 8.86 (s, 1H), 7.75-7.70 (m, 2H), 7.56 (d, 1H), 4.53 (d, 2H), 4.02 (s, 3H) ppm. |
| Ik-47 | ¹H-NMR (d₆-DMSO) 11.42 (s, 1H), 11.30 (s, 1H, Isomer), 8.78 (d, 1H, NH), 8.44 (d, 1H, Isomer), 7.72 (d, 1H), 7.70 (dd, 1H), 7.52 (d, 1H), 7.40 (d, 1H), 7.30-7.10 (m, 4H), 4.02 (s, 3H), 3.00 (m, 1H), 2.05 (m, 1H), 1.30-1.20 (m, 4H) ppm. |
| Ik-51 | ¹H-NMR (d₆-DMSO) 11.23 (s, 1H), 8.40 (1H, s, NH), 7.70 (1H, s), 7.65 (1H, dd, J= 8, 2.5 Hz), 7.50 (1H, d, J= 8 Hz), 4.7 (1H, ddd, J= 62, 4,4 Hz), 4.00 (3H, s), 2.50 (1H, m), 1.40 (1H, m), 1.00 (1H, m) ppm. |
| Ik-52 | ¹H-NMR (d₆-DMSO) 11.20 (s, 1H), 8.82 (t, 1H, NH), 7.70 (m, 2H), 7.52 (d, 1H), 7.45 (dd, 1H), 7.35-7.30 (m, 1H), 7.15 (m, 1H), 4.49 (d, 2H), 4.00 (s, 3H) ppm. |
| Ik-59 | ¹H-NMR (d₆-DMSO) 11.23 (s, 1H), 7.65-7.60 (m, 2H), 7.50 (d, 1H), 4.01 (6H, m), 1.55 (m, 2H), 0.95 (t, 3H), 0.70 (t, 2H) ppm. |
| Ik-60 | ¹H-NMR (d₆-DMSO) 11.44 (s, 1H), 9.00 (t, 1H), 8.79 (d, 2H), 7.87 (d, 1H), 7.75 (dd, 1H), 7.55 (d, 1H), 7.41 (t, 1H), 4.62 (d, 2H), 4.03 (s, 3H) ppm. |
| Ik-62 | ¹H-NMR (d₆-DMSO) 11.42 (s, 1H), 8.44 (d, 1H, NH), 7.70 (dd, 1H), 7.68 (s, 1H), 7.53 (d, 1H), 4.00 (s, 3H), 3.50 (m, 1H), 1.20 (d, 3H), 0.90 (m, 1H), 0.50-0.25 (m, 3H) ppm. |
| Ik-63 | ¹H-NMR (d₆-DMSO) 11.25 (s, 1H), 8.95 (d, 1H, NH), 7.72 (dd, 1H), 7.68 (m, 1H), 7.53 (d, 1H), 4.75 (m, 1H), 4.00 (s, 3H), 1.33 (d, 3H) ppm. |
| Ik-64 | ¹H-NMR (d₆-DMSO) 11.25 (s, 1H), 8.72 (t, 1H, NH), 7.70 (m, 2H), 7.50 (d, 1H), 4.00 (s, 3H), 3.72-3.65 (ddd, 2H), 1.65 (t, 3H, J(H,F)=18Hz) ppm. |
| Ik-83 | ¹H-NMR (400 MHz, d₃-Acetonitril): δ = 9.18 (bs, 1H), 7.75 (s, 1H), 7.65 (dd, 1H), 7.46 (d, 1H), 6.94 (bs, 1H), 6.25-6.36 (m, 1H), 5.95-6.09 (m, 1H), 3.98 (s, 3H) ppm. (mixture of E/Z isomers) |
| Ik-84 | ¹H-NMR (400 MHz, d₃-Acetonitril): δ = 9.29 (bs, 1H), 7.78 (d, 1H), 7.67 (dd, 1H), 7.48 (d, 1H), 7.21-7.52 (m, 6H), 4.54 (d, 2H), 3.97 (s, 3H) ppm. |
| Ik-87 | ¹H-NMR (400 MHz, d₃-Acetonitril): δ = 9.23 (bs, 1H), 7.78 (d, 1H), 7.67 (dd, 1H), 7.49 (d, 1H), 7.24 (bs, 1H), 4.02-4.12 (m, 1H), 3.98 (s, 3H) ppm. |
| Ik-90 | ¹H-NMR (400 MHz, d₃-Acetonitril): δ = 9.46 (bs, 1H), 7.71 (d, 1H), 7.65 (dd, 1H), 7.45 (d, 1H), 6.73 (bs, 1H), 4.09-4.16 (m, 1H), 3.98 (s, 3H), 1.21 (d, 6H) ppm. |
| Ik-93 | ¹H-NMR (400 MHz, d₃-Acetonitril): δ = 9.19 (bs, 1H), 7.80 (d, 1H), 7.74 (dd, 1H), 7.37 (d, 1H), 6.92 (bs, 1H), 5.90-5.99 (m, 1H), 5.23 (dd, 1H), 5.13 (dd, 1H), 3.98 (s, 3H) ppm. |
| Ik-100 | ¹H-NMR (400 MHz, d₃-Acetonitril): δ = 9.28 (bs, 1H), 7.57-7.62 (m, 2H), 7.43 (d, 1H), 6.92 (bs, H), 6.18 (dq, 1H), 2.78-2.83 (m, 1H), 1.98 (d, 1H), 0.73-0.79 (m, 2H), 0.57-0.60 (m, 2H) ppm. |
| Ik-107 | ¹H-NMR (400 MHz, d₃-Acetonitril): δ = 9.19 (bs, 1H), 8.22 (d, 1H), 7.76 (dd, 1H), 7.55 (d, 1H), 4.86 (s, 2H), 3.98 (s, 3H), 3.76 (s, 3H) ppm. |
| Ik-117 | (d₆-DMSO) 0.45 (m, 2H), 0.72 (m, 2H), 2.82 (m, 1H) 4.21 (s, 3H), 7.46 (m, 1H), 7.50 (s, 1H), 7.75 (m, 1H), 8.30 (br. s, 1H). |
| Ik-118 | (d₃-Acetonitril) 3.38 (m, 1H) 4.07 (s, 3H), 7.44 (m, 1H), 7.51 (s, 1H), 7.76 (m, 1H), 8.87 (br. s, 1H). |
| Ik-123 | (d₃-Acetonitril) 0.45 (m, 2H), 0.72 (m, 2H), 2.79 (m, 1H) 4.11 (s, 3H), 7.15 - 7.25 (m, 3H), 7.3 -7.4 (m, 2H), 7.4 -7.5 (m, 2H), 7,91 (br. s, 1H). |
| Ik-132 | ¹H-NMR (400 MHz, d₃-Acetonitrile; Mischung aus *cis* und *trans* konfigurierten Amiden): δ = 6.55-7.63 (m, 4H), 3.98 & 3.84 (2 s, zusammen 3H), 3.45 & 3.21 (2 s, zusammen 3H), 2.77-2.90 (m ,1H), 0.72-0.81 (m 2 H), 0.51-0.63 (m, 2H) ppm. |
| Ik-137 | ¹H-NMR (400 MHz, d₃-Acetonitrile: Mischung aus *cis* und *trans* konfigurierten Amiden): δ = 7.58 & 7.39 (2 d, zusammen 1H), 7.16-7.31 (m, 2H), 6.77 & 6.99 (2 bs, zusammen 1H), 4.90-4.98 (m, 1H), 3.86 & 3.98 (2 s, zusammen 3H), 2.76-2.82 (m, 1H), 1.17-1.23 (m, 6H), 0.73-0.80 (m ,2H), 0.53-0.58 (m, 2H) ppm. |
| Ik-138 | ¹H-NMR (400 MHz, d₃-Acetonitrile): δ = 7.38-7.50 (m, 1H), 7.28 (d, 1H), 7.19 (dd, 1H), 7.10 (bs, 1H), 3.84-4.12 (m, 7H), 1.24 (t, 3H) ppm. |
| Ik-142 | ¹H-NMR (400 MHz, d₃-Acetonitril): δ = 9.16 (bs, 1H), 8.26 (d, 1H), 7.27-7.42 (m, 6H), 4.54 (d, 2H), 3.98 (s, 3H) ppm. |
| Ik-146 | ¹H-NMR (400 MHz, d₃-Acetonitril): δ = 9.08 (bs, 1H), 8.52 (dd, 1H), 7.43 (bs, 1H), 7.22 (t, 1H), 4.08-4.16 (m, 2H), 4.00 (s, 3H) ppm. |
| Ik-147 | ¹H-NMR (400 MHz, d₃-Acetonitril): δ = 9.00 (bs, 1H), 8.11 (s, 1H), 7.64 (s, 1H), 6.98 (bs, 1H), 4.01 (s, 3H), 2.80-2.85 (m. 1H), 0.74-0.79 (m, 2H), 0.58-0.62 (m, 2H) ppm. |
| Ik-149 | ¹H-NMR (400 MHz, d₃-Acetonitril): δ = 9.30 (bs, 1H), 7.90-7.93 (m, 1H), 7.65-7.66 (m, 1H), 7.57-7.61 (m, 1H), 7.23-7.35 (m, 5H), 4.54 (d, 2H), 3.98 (s, 3H) ppm. |
| Ik-151 | ¹H-NMR (400 MHz, d₃-Acetonitril): δ = 8.99 (bs, 1H), 8.44 (d, 1H), 7.49 (d, 1H), 7.45 (bs, 1H), 4.07-4.16 (m, 2H), 4.03 (s, 3H) ppm. |
| Ik-154 | ¹H-NMR (400 MHz, d₃-Acetonitril): δ = 8.83 (bs, 1H), 7.81 (d, 1H), 7.71 (dd, 1H), 7.48 (d, 1H), 7.21 (bs, 1H), 4.02-4.12 (m, 2H), 4.07 (s, 3H) ppm. |
| Ik-155 | ¹H-NMR (400 MHz, d₃-Acetonitril): δ = 9.11 (bs, 1H), 7.97-8.00 (m, 1H), 7.70-7.76 (m, 1H), 7.20 (dd, 1H), 6.92 (bs, 1H), 3.98 (s, 3H), 2.85-2.90 (m, 1H), 0.75-0.79 (m, 2H), 0.59-0.63 (m, 2H) ppm. |
| Ik-156 | ¹H-NMR (400 MHz, d₃-Acetonitril): δ = 9.13 (bs, 1H), 8.04-8.07 (m, 1H), 7.76-7.82 (m, 1H), 7.35 (bs, 1H), 7.26 (dd, 1H), 4.05-4.16 (m, 1H), 3.98 (s, 3H) ppm. |
| Ik-158 | ¹H-NMR (400 MHz, d₃-Acetonitril): δ = 8.84 (bs, 1H), 7.77 (d, 1H), 7.70 (dd, 1H), 7.47 (d, 1H), 7.10 (bs, 1H), 4.79-4.88 (m, 1H), 4.07 (s, 3H), 1.39 (d, 3H) ppm. |
| Ik-161 | ¹H-NMR (400 MHz, d₃-Acetonitril): δ = 9.18 (bs, 1H), 7.99 (dd, 1H), 7.75-7.80 (m, 1H), 7.25 (dd, 1H), 7.10 (bs, 1H), 4.85-4.93 (m, 1H), 3.98 (s, 3H), 1.41 (d, 3H) ppm. |
| Ik-162 | ¹H-NMR (400 MHz, d₃-Acetonitril): δ = 9.26 (bs, 1H), 7.95 (s, 1H), 7.65-7.70 (m, 1H), 7.24-7.48 (m, 7H), 4.54 (d, 2H), 3.98 (s, 3H) ppm. |
| Ik-167 | ¹H-NMR (400 MHz, d₃-Acetonitril): δ = 9.40 (bs, 1H), 7.64 (d, 1H), 7.61 (dd, 1H), 7.44 (d, 1H), 6.93 (bs, 1H), 2.79-2.84 (m, 1H), 1.66 (s, 9H), 0.73-0.78 (m, 2H), 0.56-0.60 (m, 2H) ppm. |
| Ik-169 | ¹H-NMR (400 MHz, d₃-Acetonitril): δ = 9.17 (bs, 1H), 8.12-819 (m, 1H), 7.61 (dd, 1H), 7.16 (bs, 1H), 3.98 (s, 3H), 2.81-2.87 (m, 1H), 0.73-0.80 (m, 2H), 0.53-0.59 (m, 2H) ppm. |
| Ik-170 | ¹H-NMR (400 MHz, d₃-Acetonitril): δ = 8.93 (bs, 1H), 8.15 (d, 1H), 7.34 (d, 1H), 6.79 (bs, 1H), 4.01 (s, 3H), 2.78-2.85 (m, 1H), 0.73-0.79 (m, 2H), 0.54-0.59 (m, 2H) ppm. |
| Ik-172 | ¹H-NMR (400 MHz, d₃-Acetonitril): δ = 9.13 (bs, 1H), 7.70-7.71 (m, 1H), 7.63 (d, 1H), 7.54-7.58 (m, 1H), 7.26-7.42 (m, 5H), 7.13 (bs, 1H), 4.54 (d, 2H), 3.97 (s, 3H) ppm. |
| Ik-173 | ¹H-NMR (400 MHz, d₃-Acetonitril): δ = 9.16 (bs, 1H), 7.72 (d, 1H), 7.66 (d, 1H), 7.55-7.60 (m, 1H), 7.20 (bs, 1H), 4.02-4.11 (m 2H), 3.97 (s, 3H) ppm. |
| Ik-174 | ¹H-NMR (400 MHz, d₃-Acetonitril): δ = 9.14 (bs, 1H), 7.68-7.77 (m, 1H), 7.65 (d, 1H), 7.58 (dd, 1H), 7.07 (bs, 1H), 4.78-4.88 (m 2H), 3.97 (s, 3H) ppm. |
| Ik-175 | ¹H-NMR (400 MHz, d₃-Acetonitril): δ = 9.25 (bs, 1H), 7.53-7.65 (m, 3H), 6.77 (bs, 1H), 3.98 (s, 3H), 2.79-2.86 (m, 1H), 0.73-0.79 (m, 2H), 0.60-0.62 (m, 2H) ppm. |
| Ik-178 | ¹H-NMR (400 MHz, d₆-DMSO): δ = 11.40 (s, 1H), 9.15 (t, 1H), 8.17 (d, 3H), 7.63 (d, 1H), 4.01-4.12 (m, 5H) ppm. |
| Ik-179 | ¹H-NMR (400 MHz, d₃-Acetonitrile): δ = 9.15 (bs, 1H), 7.70-7.72 (m, 1H), 7.65 (dd, 1H), 7.42 (d, 1H), 6.79 (bs, 1H), 2.81-2.89 (m,1H), 0.72-0.81 (m 2 H), 0.51-0.64 (m, 2H) ppm. |
| Ik-181 | ¹H-NMR (400 MHz, d₃-Acetonitrile): δ = 9.51 (bs, 1H), 8.14 (d, 1H), 7.86 (dd, 1H), 7.82 (d, 1H), 7.32 (bs, 1H), 4.04-4.14 (m,1H), 3.99 (s, 3H) ppm. |
| Ik-183 | ¹H-NMR (400 MHz, d₃-Acetonitrile): δ = 9.11 (bs, 1H), 7.86 (d, 1H), 7.58 (d, 1H), 7.38 (dd, 1H), 6.68 (bs, 1H), 3.97 (s, 3H), 2.79-2.85 (m ,1H), 0.73-0.79 (m, 2H), 0.62-0.65 (m, 2H) ppm. |
| Ik-187 | ¹H-NMR (400 MHz, d₃-Acetonitrile): δ = 9.33 (bs, 1H), 8.09 (d, 1H), 7.85 (d, 1H), 6.88 (bs, 1H), 3.97 (s, 3H), 2.82-2.87 (m, 1H), 0.75-0.81 (m, 2H), 0.58-0.63 (m, 2H) ppm. |
| Ik-189 | ¹H-NMR (400 MHz, d₃-Acetonitrile): δ = 7.78 (d, 1H), 7.49 (d, 1H), 7.24-7.42 (m, 6H), 4.54 (d, 2H), 3.97 (s, 3H) ppm. |
| Ik-192 | ¹H-NMR (400 MHz, d₃-Acetonitrile): δ = 9.26 (bs, 1H), 7.63 (dd, 1H), 7.22-7.46 (m, 7H), 4.55 (d, 2H), 3.97 (s, 3H), 2.38 (s, 3H) ppm. |
| Ik-194 | ¹H-NMR (400 MHz, d₃-Acetonitrile): δ = 9.44 (bs, 1H), 7.74 (dd, 1H), 7.34 (s, 1H), 6.76 (bs, 1H), 3.97 (s, 3H), 3.24 (s, 3H), 2.72-2.78 (m, 1H), 0.70-0.76 (m, 2H), 0.58-0.62 (m, 2H) ppm. |
| Ik-197 | ¹H-NMR (400 MHz, d₃-Acetonitrile): δ = 9.08 (bs, 1H), 7.71 (d, 1H), 7.67 (dd, 1H), 7.42 (d, 1H), 6.78 (bs, 1H), 3.95 (s, 3H), 2.82-2.88 (m, 1H), 0.73-0.80 (m, 2H), 0.54-0.61 (m, 2H) ppm. |
| Ik-199 | ¹H-NMR (400 MHz, d₃-Acetonitrile): δ = 9.38 (bs, 1H), 8.08 (d, 1H), 7.57 (d, 1H), 7.43 (dd, 2H), 7,38 (dt, 2H), 7.27-7.31 (m, 2H), 4.54 (d, 2H), 3.97 (s, 3H), 2.36 (s, 3H) ppm. |
| Ik-203 | ¹H-NMR (400 MHz, d₃-Acetonitrile): δ = 9.35 (bs, 1H), 7.92 (d, 1H), 7.54 (d, 1H), 7.33 (bs, 1H), 4.07-4.11 (m, 1H), 3.98 (s, 3H), 2.38 (s, 3H) ppm. |
| Ik-204 | ¹H-NMR (400 MHz, d₃-Acetonitrile): δ = 9.25 (bs, 1H), 7.88 (d, 1H), 7.40 (d, 1H), 6.71 (bs, 1H), 3.97 (s, 3H), 2.79-2.86 (m, 1H), 0.73-0.81 (m, 2H), 0.58-0.64 (m, 2H) ppm. |
| Ik-208 | ¹H-NMR (400 MHz, d₃-Acetonitrile): δ = 9.25 (bs, 1H), 8.07 (d, 1H), 7.58 (d, 1H), 7.26-7.41 (m, 5H), 7.14 (bs, 1H), 4.54 (d, 2H), 3.97 (s, 3H) ppm. |
| Ik-213 | ¹H-NMR (400 MHz, d₃-Acetonitrile): δ = 9.24 (bs, 1H), 7.90 (d, 1H), 7.48 (d, 1H), 6.75 (bs, 1H), 3.97 (s, 3H), 2.76-2.89 (m, 1H), 0.73-0.80 (m, 2H), 0.58-0.62 (m, 2H) ppm. |
| Ik-216 | ¹H-NMR (400 MHz, d₃-Acetonitril): δ = 9.18 (bs, 1H), 7.76 (d, 1H), 7.66 (dd, 1H), 7.49 (d, 1H), 7.22 (bs, 1H), 4.30 (q, 2H), 4.03-4.13 (m, 2H), 1.43-1.48 (m, 3H) ppm. |
| Ik-218 | ¹H-NMR (400 MHz, d₃-Acetonitril): δ = 9.19 (bs, 1H), 7.68 (d, 1H), 7.62 (dd, 1H), 7.44 (d, H), 6.78 (bs, 1H), 4.66-1.75 (m, 1H), 2.81-2.87 (m, 1H), 1.49 (d, 6H), 0.72-0.79 (m, 2H), 0.58-0.62 (m, 2H) ppm. |
| Ik-220 | ¹H-NMR (400 MHz, d₃-Acetonitril): δ = 9.20 (bs, 1H), 7.75 (d, 1H), 7.65 (dd, 1H), 7.49 (d, H), 7.23 (bs, 1H), 5.55 (s, 2H), 4.02-4.11 (m, 2H), 3.37 (s, 3H) ppm. |
| Ik-222 | ¹H-NMR (400 MHz, d₃-Acetonitril): δ = 9.33 (bs, 1H), 8.05 (t, 1H), 7.97 (t, 1H), 7.80 (t, H), 7.62 (bs, 1H), 7.24-7.48 (m, 5H), 4.54 (d, 2H), 3.98 (s, 3H) ppm. |
| Ik-229 | ¹H-NMR (400 MHz, d₃-Acetonitril): δ = 9.54 (bs, 1H), 7.74 (dd, 1H), 7.27-7.44 (m, 7H), 4.54 (d, 2H), 3.97 (s, 3H) ppm. |
| Ik-230 | ^{1H-NMR} (400 MHz, d₆-DMSO): δ = 11.09 (br. s, 1H), 8.32 (br. d, 1H), 7.65-7.68 (m, 2H), 7.45-7.47 (m, 1H), 3.83 (s, 3H), 2.82-2.83 (m, 1H), 1.74 (d, 6H), 0.68-0.71 (m, 2H), 0.51-0.55 (m, 2H) ppm. |
| Ik-231 | ^{1H-NMR} (400 MHz, d₆-DMSO): δ = 11.38 (s, 1H), 8.57 (d, 1H), 7.69-7.74 (m, 2H), 7.55 (d, 1H), 7.24 (t, 1H), 4.00 (s, 3H), 2.83-2.88 (m, 1H), 0.71-0.74 (m, 2H), 0.53-0.56 (m, 2H) ppm. |
| Ik-235 | ¹H-NMR (400 MHz, d₆-DMSO): δ = 10.53 (s, 1H), 8.30 (d, 1H), 7.72 (m, 2H), 7.45 (d, 1H), 3.73 (s, 3H), 2.84 (m, 1 H), 1.85 (m, 1H), 0.92 (m, 2H), 0.80 (m, 2H), 0.69 (m, 2H), 0.53 (m, 2H) ppm. |
| Ik-236 | ¹H-NMR (400 MHz, d₆-DMSO): δ = 10.79 (s, 1H), 9.03 (m, 1H), 7.80 (m, 1H), 7.76 (m, 1H), 7.52 (m, 1H), 7.27 (t, 1H), 4.06 (m, 2H), 3.87 (s, 3H) ppm. |
| Ik-239 | ¹H-NMR (400 MHz, d₆-DMSO): δ = 10.84 (s, 1H), 9.02 (m, 1H), 7.78 (m, 1H), 7.76 (m, 1H), 7.51 (d, 1H), 7.27 (t, 1H), 4.06 (m, 2H), 3.88 (s 1H) ppm. |
| Ik-240 | ¹H-NMR (400 MHz, d₆-DMSO): δ = 10.65 (s, 1H), 8.32 (m, 1H), 7.73 (m, 1H), 7.69 (m, 1H), 7.47 (d, 1H), 7.24 (t, 1H), 3.87 (s, 3H), 2.83 (m, 1H), 0.69 (m, 2H), 0.53 (m, 2H) ppm. |
| Ik-243 | ¹H-NMR (400 MHz, d₆-DMSO): δ = 10.68 (s, 1H), 9.01 (m, 1H), 7.78 (m, 2H), 7.51 (d, 1H), 4.06 (m, 2H), 3.82 (s, 3H) ppm. |
| Ik-245 | ¹H-NMR (400 MHz, d₆-DMSO): δ = 10.60 (s, 1H), 8.32 (m, 1H), 7.72 (m, 1H), 7.68 (m, 1H), 7.45 (d, 1H), 4.85 (m, 2H), 3.81 (sm 3H), 2.84 (m, 1H), 0.70 (m, 2H), 0.53 (m, 2H) ppm. |
| Ik-246 | ¹H-NMR (400 MHz, d₆-DMSO): δ = 10.40 (s, 1H), 7.83 (m, 2H), 7.52 (m, 1H), 6.59 (s, 1H), 4.82 (m, 2H), 4.08 (m, 2H), 3.96 (s, 3H) ppm. |
| Ik-248 | ¹H-NMR (400 MHz, d₆-DMSO): δ = 10.94 (s, 1H), 8.51 (d, 1H), 7.87 (m, 2H), 7.74 (m, 2H), 7.50 (d, 1H), 7.34 (m, 2H), 3.98 (s, 3H), 2.83 (m, 1H), 0.70 (m, 2H), 0.52 (m, 2H) ppm. |
| Ik-249 | ¹H-NMR (400 MHz, d₆-DMF): δ = 10.89 (s, 1H), 8.42 (m, 1H), 8.16 (d, 2H), 7.97 (m, 1H), 7.94 (d, 2H), 7.89 (m, 1H), 7.55 (m, 1H), 4.14 (s, 4H), 2.97 (m, 1H), 0.78 (m, 2H), 0.53 (m, 2H) ppm. |
| Ik-25 | ¹H-NMR (400 MHz, d₃-Acetonitril): δ = 9.23 (bs, 1H), 7.92 (d, 1H), 7.76 (dd, 1H), 7.25 (d, 1H), 7.08 (bs, 1H), 6.78 (t, 1H), 3.98 (s, 3H), 2.81-2.86 (m, 1H), 0.74-0.79 (m, 2H), 0.55-0.60 (m, 2H) ppm. |
| Ik-253 | ¹H-NMR (400 MHz, d₆-DMF): δ = 10.92 (s, 1H), 9.16 (t, 1H), 8.16 (d, 2H), 8.04 (m, 1H), 7.95 (m, 3H), 7.61 (d, 1H), 4.26 (m, 2H), 4.14 (s, 3H) ppm. |
| Ik-257 | ¹H-NMR (400 MHz, d₆-DMSO): δ = 10.69 (s, 1H), 9.00 (m, 1H), 7.79 (m, 1H), 7.75 (m, 1H), 7.51 (d, 1H), 4.88 (m, 2H), 4.06 (m, 2H), 3.81 (s, 3H) ppm. |
| Ik-258 | ¹H-NMR (400 MHz, d₆-DMSO): δ = 11.24 (s, 1H), 9.03 (m, 1H), 8.23 (s, 1H), 8.17 (s, 2H), 7.77 (m, 2H), 7.55 (d, 1H), 4.08 (m, 2H), 4.01 (s, 3H) ppm. |
| Ik-259 | ¹H-NMR (400 MHz, d₆-DMSO): δ = 11.18 (s, 1H), 8.34 (m, 1H), 8.22 (s, 1H), 8.18 (s, 2H), 7.70 (m, 2H), 7.49 (d, 1H), 2.84 (m, 1H), 0.70 (m, 2H), 0.55 (m, 2H) ppm. |
| Ik-260 | ¹H-NMR (400 MHz, d₆-DMSO): δ = 10.39 (s, 1H), 8.28 (m, 1H), 7.70 (m, 2H), 7.44 (m, 1H), 4.87 (m, 2H), 3.53 (s, 3H), 2.82 (m, 1H), 0.70 (m,2H), 0.54 (m, 2H) ppm. |
| Ik-261 | ¹H-NMR (400 MHz, d₆-DMSO): δ = 10.97 (s, 1H), 8.31 (m, 1H), 7.67 (m, 2H), 7.47 (d, 1H), 3.76 (s, 3H), 2.82 (m, 1H), 1.92 (m, 1H), 0.94 (m, 2H), 0.85 (m, 2H), 0.69 (m, 2H), 0.54 (m, 2H) ppm. |
| Ik-262 | (d₃-Acetonitril) 0.52 (m, 2H), 0.75 (m, 2H), 2.83 (m, 2H), 4.04 (s, 3H), 4.46 (m, 2H), 5.20 (m, 1H), 5.90 (m, 2H), 7.3-7.5 (m, 2H), 7.67-7.76 (m, 1H), 9.0 (brs, 1H). |
| Ik-263 | (d₆-DMSO) 0.45 (m, 2H), 0.70 (m, 2H), 1.10 (t, 3H), 2.67 (m, 2H), 2.85 (m, 1H) 3.96 (s, 3H), 7.42 (m, 1H), 7.55 (m, 1H), 7.80 (m, 1H), 8.10 (s, 1H), 8.26 (br.s, 1H). |
| Ik-264 | (d₆-DMSO) 0.46 (m, 2H), 0.72 (m, 2H), 2.87 (m, 1H) 4.17 (s, 3H), 7.44 (m, 1H), 7.55 (m, 1H), 7.77 (m, 1H), 8.30 (br. s, 1H). |
| Ik-265 | (d₆-DMSO) 0.47 (m, 2H), 0.73 (m, 2H), 2.83 (m, 1H) 4.19 (s, 3H), 7.46 (m, 1H), 7.52 (s, 1H), 7.77 (m, 1H), 8.30 (br. s, 1H). |
| Ik-269 | (d₆-DMSO) 3.93 (m, 2H) 4.03 (s, 3H), 6.01 (m, 1H), 6.40 (m, 1H), 7.50 (m, 1H), 7.70 (m, 1H), 7.78 (m, 1H), 8.59 (m, 1H). |
| Ik-270 | ¹H-NMR (d₃-d₃-Acetonitril) 9.25 (s, 1H, br), 8.50 (s, 1H, br), 7.80 (m, 1H), 7.72 (d, 1H), 7.65 (m, 1H), 7.50 (m, 1H), 7.40-7.25 (m, 3H), 5.15 (m, 1H), 3.98 (s, 3H), 1.50 (d, 3H) ppm. |
| Ik-272 | ¹H-NMR (d₆-DMSO) 10.94 (s, 1H), 9.00 (t, 1H), 7.81 (d, 1H), 7.77 (dd, 1H), 7.52 (d, 1H), 7.30 (m, 4H), 7.25 (m, 1H), 4.46 (d, 2H), 3.96 (s, 3H), 2.65 (q, 2H), 1.09 (t, 3H) ppm. |
| Ik-273 | ¹H-NMR (d₆-DMSO) 11.46 (s, 1H), 9.52 (t, 1H), 8.46 (s, 1H), 7.73 (d, 1H), 7.70 (dd, 1H), 7.59 (d, 1H), 4.78 (d, 2H), 4.03 (s, 3H) ppm. |
| Ik-276 | ¹H-NMR (d₆-DMSO) 11.43 (s, 1H), 9.96 (d, 1H), 7.94 (s, 1H), 7.78 (dd, 1H), 7.69 (d, 1H), 7.55 (d, 1H), 6.40 (m, 1H), 4.03 (s, 3H), 3.76 (s, 3H) ppm. |
| Ik-278 | ¹H-NMR (d₆-DMSO) 11.44 (s, 1H), 9.23 (d, 1H), 7.79 (d, 1H), 7.70 (dd, 1H), 7.56 (d, 1H), 7.24 (dd, 1H), 6.90 (dd, 1H), 5.30 (m, 1H), 4.02 (s, 3H), 3.80 (dd, 1H), 3.15 (dd, 1H) ppm. |
| Ik-279 | ¹H-NMR (d₃-d₃-Acetonitril) 9.28 (s, 1H, br), 7.72 (d, 1H), 7.65 (dd, 1H), 7.46 (d, 1H), 6.82 (s, 1H, br), 3.98 (s, 3H), 2.84 (m, 1H), 1.09 (d, 3H), 1.05 (m, 1H), 0.95 (m, 1H), 0.25 (m, 1H) ppm. |
| Ik-280 | ¹H-NMR (d₃-d₃-Acetonitril) 9.27 (s, 1H, br), 7.68 (d, 1H), 7.64 (dd, 1H), 7.44 (d, 1H), 6.90 (s, 1H, br), 3.97 (s, 3H), 2.50 (m, 1H), 1.09 (d, 3H), 0.95 (m, 1H), 0.75 (m, 1H), 0.55 (m, 1H) ppm. |
| Ik-281 | ¹H-NMR (d₆-DMSO) 11.43 (s, 1H), 8.60 (t, 1H), 7.73 (d, 1H), 7.67 (dd, 1H), 7.54 (d, 1H), 4.10 (q, 2H, J(H,F)=9Hz), 4.02 (s, 3H), 3.70 (t, 2H), 3.40 (m, 2H) ppm. |
| Ik-285 | ¹H-NMR (d₆-DMSO) 11.40 (s, 1H), 8.49 (d, 1H), 7.70 (m, 2H), 7.52 (d, 1H), 4.02 (s, 3H), 3.95 (m, 1H), 3.82 (m, 4H), 1.75 (m, 2H), 1.50 (m, 2H) ppm. |
| Ik-287 | ¹H-NMR (d₆-DMSO) 11.39 (s, 1H), 8.66 (d, 1H), 7.70-7.50 (m, 2H), 7.52 (d, 1H), 5.70 (s, 2H), 4.50 (m, 1H), 4.02 (s, 3H), 2.70 (m, 2H), 2.28 (m, 2H) ppm. |
| Ik-288 | ¹H-NMR (d₆-DMSO) 11.41 (s, 1H), 8.62 (d, 1H), 7.72 (dd, 1H), 7.66 (d, 1H), 7.54 (d, 1H), 4.02 (s, 3H), 4.00 (m, 1H), 1.30 (d, 3H), 1.20 (m, 1H), 1.10-1.00 (m, 3H) ppm. |
| Ik-289 | ¹H-NMR (d₆-DMSO) 11.39 (s, 1H), 8.20 (s, 1H), 7.72 (dd, 1H), 7.64 (d, 1H), 7.50 (d, 1H), 4.03 (s, 3H), 2.12 (s, 2H), 1.36 (s, 3H) ppm. |
| Ik-293 | ¹H-NMR (d₃-d₃-Acetonitril) 9.30 (s, 1H, br), 7.72 (d, 1H), 7.65 (dd, 1H), 7.47 (d, 1H), 7.23 (d, 1H, br), 4.86 (m, 1H), 3.98 (s, 3H), 2.57 (s, 1H), 1.46 (d, 3H) ppm. |
| Ik-299 | ¹H-NMR (d₆-DMSO) 11.44 (s, 1H), 8.62 (d, 1H), 7.74 (dd, 1H), 7.66 (d, 1H), 7.55 (d, 1H), 4.30 (m, 1H), 4.02 (s, 3H), 3.40 (m, 2H), 1.24 (d, 3H) ppm. |
| Ik-300 | ¹H-NMR (d₆-DMSO) 11.39 (s, 1H), 8.40 (s, 1H), 7.73 (dd, 1H), 7.60 (d, 1H), 7.51 (d, 1H), 4.02 (s, 3H), 3.95 (q, 2H, J(H,F)= 13Hz), 1.42 (s, 6H) ppm. |
| Ik-311 | ¹H-NMR (d₆-DMSO) 8.49 (1H, d, J= 4 Hz, NH), 7.70 (1H, dd, J= 8, 2.5 Hz), 7.68 (1H, s), 7.50 (1H, d, J= 8 Hz), 4.7 (1H, ddd, J= 62, 4,4 Hz), 4.00 (3H, s), 2.85 (1H, m), 1.15 (1H, m), 1.05 (1H, m) ppm. |
| Ik-342 | ¹H-NMR (d₆-DMSO) 11.41 (s, 1H), 8.55 (d, 1H), 7.86 (d, 1H), 7.80 (m, 2H), 7.65 (m, 1H), 7.54 (d, 1H), 7.38 (d, 1H), 7.30 (t, 1h), 4.75 (s, 2H, br), 4.04 (s, 3H), 2.70 (m, 1H), 0.65 (m, 2H), 0.45 (m, 2H) ppm. |
| Ik-345 | ¹H-NMR (d₆-DMSO) 11.43 (s, 1H), 7.75-7.70 (m, 2H), 7.55 (m, 1H), 4.60 (m, 1H), 4.03 (s, 3H), 2.85 (m, 1H), 2.65 (m, 1H), 1.95 (m, 1H), 1.80 (m, 1H), 1.50-1.30 (m, 4H) ppm. |
| Ik-347 | ¹H-NMR (400 MHz, d₆-DMSO): δ = 8.17 (m, 1H), 7.28 (m, 1H), 7.04 (m, 2H), 4.15 (s, 3H), 2.77 (m, 1H), 0.67 (m 2H), 0.49 (m, 2H) ppm. |
| Ik-348 | ¹H-NMR (400 MHz, d₆-DMSO): δ = 8.40 (s, 1H), 8.10 (m, 1H), 7.42 (d, 1H), 7.15 (m, 1H), 7.11 (m, 1H), 4.18 (s, 3H), 3.48 (m, 1H), 1.17 (d, 3H), 0.90 (m, 1H), 0.43 (m, 1H), 0.33 (m, 2H), 0.22 (m, 1H) ppm. |
| Il-1 | 1H-NMR (400 MHz, d₆-DMSO): δ = 11.05 (br. m, 1H), 8.33 (br. d, 1H), 7.63-7.67 (m, 2H), 7.48 (d, 1H), 2.80-2.86 (m, 1H), 0.69-0.72 (m, 2H), 0.53-0.55 (m, 2H) ppm. |
| Ir-2 | ¹H-NMR (400 MHz, d₆-DMSO): δ = 10.60 (s, 1H), 8.29 (d, 1H), 8.14 (d, 1H), 7.83 (d, 1H), 7.75-7.70 (m, 2H), 7.44 (d, 1H), 2.86-2.81 (m, 1H), 2.63 (s, 3H), 0.72-0.67 (m, 2H), 0.55-0.52 (m, 2H) ppm. |
| Ir-10 | ¹H-NMR (400 MHz, d₆-DMSO): δ = 10.79 (s, 1H), 8.58-8.54 (m, 1H), 8.37 (d, 1H), 8.08 (d, 1H), 7.78-7.76 (m, 1H), 7.72-7.68 (m, 1H), 7.49 (d, 1H), 6.43-6.37 (m, 1H), 6.06-5.96 (m, 1H), 4.06-4.01 (m, 1H), 3.92-3.89 (m, 1H) ppm. |
| Ir-11 | ¹H-NMR (400 MHz, d₆-DMSO): δ = 10.66 (s, 1H), 8.99 (m, 1H), 8.15 (d, 1H), 7.85-7.77 (m, 3H), 7.50 (d, 1H), 4.10-4.01 (m, 1H), 2.64 (s, 3H) ppm. |

### Herstellung der Ausgangsverbindungen

### Ethyl-4-(difluormethyl)-2-(pentafluorethyl)pyrimidin-5-carboxylat

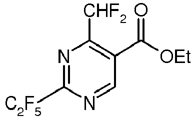

Eine Mischung von 1,620 g (10 mmol) 2,2,3,3,3-Pentafluorpropanimidamid (käuflich) und 2,222 g (10 mmol) Ethyl-(2Z)-2-(ethoxymethyliden)-4,4-difluor-3-oxobutanoat (Herstellung s. WO 2005/123690) in 10 ml absolutem Ethanol wird nach Zugabe von 0,680 g (10 mmol) Natriumethylat 4 Tage unter Rückfluss gerührt. Anschließend wird im Vakuum eingeengt und der Rückstand in 10 ml Wasser aufgenommen und zweimal mit 10 ml Essigsäureethylester extrahiert. Die organischen Phasen werden successiv mit 5 ml Wasser und 5 ml gesättigter Natriumchloridlösung gewaschen, über Natriumsulfat getrocknet, filtriert und im Vakuum eingeengt. Nach chromatographischer Reinigung mit einer Mischung von Cyclohexan und Essigsäureethylester erhält man 1,264 g (3.95 mM, 39,5 % der Theorie) Ethyl-4-(difluormethyl)-2-(pentafluorethyl)pyrimidin-5-carboxylat als weißen Feststoff.

¹H-NMR (400 MHz, d₆-DMSO): δ = 9.58 (s, 1H), 7.49 (t, 1H), 4.45 (q, 2H), 1.38 (t, 3H) ppm.

HPLC-MS^{a)}: logP = 3,42 Masse (m/z) = 321 [M+H]⁺.

Auf gleiche Weise wurden erhalten:

### Ethyl-2-(pentafluorethyl)-4-(trifluormethyl)pyrimidin-5-carboxylat aus Ethyl-2-(ethoxymethyliden)-4,4,4-trifluor-3-oxobutanoat (kommerziell verfügbar) und 2,2,3,3,3-Pentafluorpropanimidamid

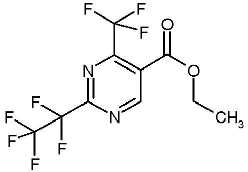

¹H-NMR (400 MHz, d₆-DMSO): δ = 9.66 (s, 1H), 4.45 (q, 2H), 1.36 (t, 3H) ppm.

HPLC-MS^{a)}: logP = 3,86 Masse (m/z) = 339 [M+H]⁺.

### Ethyl-2-(heptafluorpropyl)-4-(trifluormethyl)pyrimidin-5-carboxylat aus Ethyl-2-(ethoxymethyliden)-4,4,4-trifluor-3-oxobutanoat (kommerziell verfügbar) und 2,2,3,3,4,4,4-Heptafluorbutanimidamid (käuflich oder Herstellung s. Journal of Fluorine Chemistry, 2003, 122(2), 175-182)

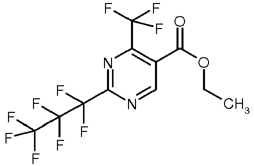

¹H-NMR (400 MHz, d₆-DMSO): δ = 9.68 (s, 1H), 4.46 (q, 2H), 1.36 (t, 3H) ppm.

HPLC-MS^{a)}: logP = 4,32 Masse (m/z) = 389 [M+H]⁺.

### Ethyl-4,6-dimethyl-2-(pentafluorethyl)pyrimidin-5-carboxylat aus Ethyl-(2E)-2-acetyl-3-ethoxybut-2-enoat (Herstellung s. J. Med. Chem. 2006, 49, 6351) und 2,2,3,3,3-Pentafluorpropanimidamid

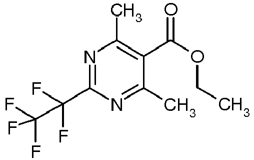

¹H-NMR (400 MHz, d₆-DMSO): δ = 4.46 (q, 2H), 3.1 (s, 6H), 1.36 (t, 3H) ppm.

HPLC-MS^{a)}: logP = 3,68 Masse (m/z) = 299 [M+H]⁺.

### Ethyl-4-methyl-2-(trifluormethyl)pyrimidin-5-carboxylat ist käuflich

Ethyl-4-methyl-2-(pentafluorethyl)pyrimidin-5-carboxylat kann analog zu dem Vorschrift Bioorg. Med. Chem. Letters, 2005, *15*, 4898 synthetisiert werden.

### 4-(Difluormethyl)-2-(pentafluorethyl)pyrimidin-5-carbonsäure

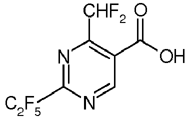

In 4 ml absolutem Ethanol werden 1,150 g (3.59 mM) Ethyl-4-(difluormethyl)-2-(pentafluorethyl)pyrimidin-5-carboxylat gelöst. Es werden 5,388 ml (10,77 mM) 2N Natronlauge zugegeben und das Reaktionsgemisch wird 4 h bei Raumtemperatur gerührt.

Es wird durch Zugabe von 2N Salzsäure auf pH 2-3 gestellt. Der entstandene Feststoff wird abgesaugt, mit wenig Wasser gewaschen und mit Cyclohexan verrieben. Man erhält 0,870 g (2,98 mM, 82,9% der Theorie) 4-(Difluormethyl)-2-(pentafluorethyl)pyrimidin-5-carbonsäure als weißen Feststoff.

¹H-NMR (400 MHz, d₆-DMSO): δ = 9.55 (s, 1H), 7.58 (t, 1H) ppm.

HPLC-MS^{a)}: logP = 1,80 Masse (m/z) = 293 [M+H]⁺.

Auf gleiche Weise wurden erhalten:

### 2-(Pentafluorethyl)-4-(trifluormethyl)pyrimidin-5-carbonsäure aus Ethyl-2-(pentafluorethyl)-4-(trifluormethyl)pyrimidin-5-carboxylat

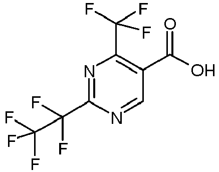

¹H-NMR (400 MHz, d₆-DMSO): δ = 9.40 (s, 1H) ppm.

HPLC-MS^{a)}: logP = 1,80 Masse (m/z) = 311 [M+H]⁺.

### 2-(Heptafluorpropyl)-4-(trifluormethyl)pyrimidin-5-carbonsäure aus Ethyl-2-(heptafluorpropyl)-4-(trifluormethyl)pyrimidin-5-carboxylat

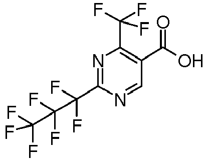

¹H-NMR (400 MHz, d₆-DMSO): δ = 9.50 (s, 1H) ppm.

HPLC-MS^{a)}: logP = 2,23 Masse (m/z) = 361 [M+H]⁺.

### 4-methyl-2-(trifluormethyl)pyrimidin-5-carbonsäure aus Ethyl-4-methyl-2-(trifluormethyl)pyrimidin-5-carboxylat

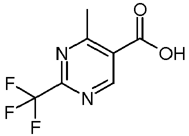

¹H-NMR (400 MHz, d₆-DMSO): δ = 9.19 (s, 1H) ppm.

HPLC-MS^{a)}: logP = 1,26 Masse (m/z) = 207 [M+H]⁺.

### 4-Methyl-2-(pentafluorethyl)pyrimidin-5-carbonsäure aus Ethyl-4-methyl-2-(pentafluorethyl)pyrimidin-5-carboxylat

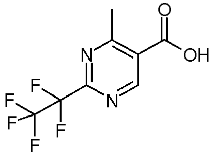

¹H-NMR (400 MHz, d₆-DMSO): δ = 9.25 (s, 1H) ppm.

HPLC-MS^{a)}: logP = 1,97 Masse (m/z) = 257 [M+H]⁺.

### 4,6-Dimethyl-2-(pentafluorethyl)pyrimidin-5-carbonsäure aus Ethyl-4,6-dimethyl-2-(pentafluorethyl)pyrimidin-5-carboxylat

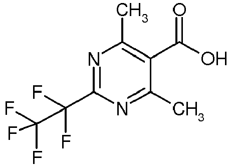

¹H-NMR (400 MHz, d₆-DMSO): δ = 2.58 (s, 6H) ppm.

HPLC-MS^{a)}: logP = 1,63 Masse (m/z) = 271 [M+H]⁺.

4-Chlor-3-(trifluormethyl)pyridin-2-carbonsäure aus 4-Chlor-3-(trifluormethyl)pyridine wurde analog zur Literaturstelle European Journal of Organic Chemistry 2004, 18, 3793 aus 4-Chlor-3-(trifluormethyl)pyridin hergestellt

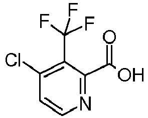

¹H-NMR (400 MHz, d₆-DMSO): δ = 9.13 (d, 1H), 9.07 (d, 1H) ppm.

HPLC-MS: logP = 1,16 Masse (m/z) = 226 [M+H]⁺.

### 5-Cyano-1-methyl-3-pentafluorethyl-4-trifluormethyl-1H-pyrazol

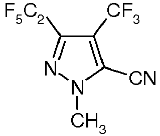

42,0 g (146,8 mM) 5-Fluor-1-methyl-3-pentafluorethyl-4-trifluormethyl-pyrazol [Darstellung s. Izvestiya Akademii Nauk SSSR, Seriya Khimicheskaya 1990, (11), 2583-9] und 11,5 g (234,9 mM) Natriumcyanid werden in 150 mL Acetonitril p.A. suspendiert und anschließend unter Schutzgasatmosphäre unter Rückflußtemperatur erhitzt. Nach dem Abkühlen wird das Reaktionsgemisch auf ein Gemisch aus 300 mL destilliertem Wasser und 300 mL Diethylether gegossen. Die wässrige Phase wird dreimal mit Diethylether extrahiert. Die vereinigten organischen Phasen werden zweimal mit Wasser und einmal mit gesättigter wässrigen Natriumchlorid-Lösung gewaschen. Die organische Phase wird über Natriumsulfat getrocknet und anschließend filtriert. Das Lösungsmittel wird am Rotationsverdampfer unter vermindertem Druck entfernt und der so erhaltene Rückstand im Vakuum fraktioniert destilliert.

Man erhält 37,0 g (119,9 mM, 82% der Theorie) 5-Cyano-1-methyl-3-pentafluorethyl-4-trifluormethyl-pyrazol als farblose Flüssigkeit (Sdp. 74° C / 10 mbar).

¹H-NMR (400 MHz, d₃-Acetonitril): δ = 4,11 (s, 3H, CH₃) ppm

GC-MS: Retentionszeit 2,67 min; Masse (m/z): 224 (M)⁺.

### 1-Methyl-3-pentafluorethyl-4-trifluormethyl-1H-pyrazol-5-carbonsäure

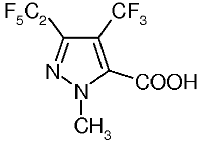

11,0 g (37,5 mM) 5-Cyano-1-methyl-3-pentafluorethyl-4-trifluormethyl-pyrazol, 22.0 mL 50%-ige Natronlauge und 7.0 mL destilliertes Wasser werden im Ölbad erhitzt bis der Feststoff geschmolzen ist. Das Reaktionsgemisch wird anschließend über Nacht gerührt (Ölbadtemperatur 100° C). Nach dem Abkühlen wird das Reaktionsgemisch auf ein Gemisch aus 150 mL konzentrierter Salzsäure und 150 mL Eis gegossen. Es wird 0,5 h nachgerührt und der Feststoff abfiltriert. Der Feststoff wird mit wenig Wasser gewaschen und dann im Ölpumpenvakuum getrocknet.

Man erhält 11.2 g (35.7 mM, 95 % der Theorie) 1-Methyl-3-pentafluorethyl-4-trifluormethylpyrazol-5-carbonsäure als weißen Feststoff.

¹H-NMR (400 MHz, d₃-Acetonitril) δ = 4.08 (s, 3H, CH₃) ppm;

HPLC-MS^{a)}: logP = 1,86; Masse (m/z): 313,0 (M+H)⁺.

### 1-Methyl-3-pentafluorethyl-1H-pyrazol

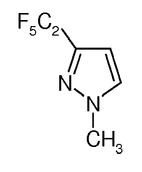

Zu einer Lösung von 30,90 g (141,67 mmol) (E)-5-Ethoxy-1,1,1,2,2-pentafluoropent-4-en-3-one (Herstellung : Synthesis 2000, 5, 738-42) in 56 ml Methanol werden 7.18 g (155.83 mmol) Methylhydrazin getropft und das Reaktionsgemisch 18 Stunden lang unter Rückfluß erhitzt. Das Methanol wird zum Großteil bei Normaldruck abdestilliert und der Rückstand auf Eis gegeben. Die wässrige Phase wird dreimal mit Dichlormethan extrahiert und die organische Phase anschließend dreimal mit gesättigter Kochsalzlösung gewaschen. Nach Trocknen über Natriumsulfat wird das Lösungsmittel am Rotationsverdampfer bei vermindertem Druck abdestilliert. Man erhält 15,81 g (52 % der Theorie) 1-Methyl-3-pentafluorethyl-1*H*-pyrazol als Öl.

¹H-NMR (400 MHz, d₃-Acetonitril) δ = 3.89 (s, 3H, CH₃), 6.57 (m, 1H, CH), 7.61(m, 1H, CH) ppm;

HPLC-MS^{a)}: logP = 2,29; Masse (m/z): 201 (M+H)⁺.

Auf gleiche Weise wurden erhalten:

### 1-Methyl-3-(1-chlor,1,2,2,2-tetrafluorethyl-1H-pyrazol aus (E)-5-Ethoxy-1-(1-chlor-1,1,2,2-tetrafluoropent-4-en-3-one

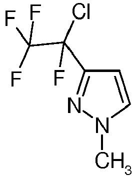

¹H-NMR (400 MHz, d₃-Acetonitril) δ = 3.89 (s, 3H, CH₃), 6.54 (m, 1H, CH), 7.58(m, 1H, CH) ppm;

HPLC-MS^{a)}: logP = 2,46; Masse (m/z): 217 (M+H)⁺.

### 1-Methyl-3-heptafluorpropyl-1H-pyrazol aus (E)-5-Ethoxy-1,1,1,2,2,3,3-heptafluorohex-4-en-3-one

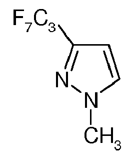

¹H-NMR (400 MHz, d₃-Acetonitril) δ = 3.94 (s, 3H, CH₃), 6.65 (m, 1H, CH), 7.91(m, 1H, CH) ppm;

HPLC-MS^{a)}: logP = 2,84; Masse (m/z): 251 (M+H)⁺.

### 1-Methyl-3-nonafluorbutyl-1H-pyrazol aus (E)-5-Ethoxy-1,1,1,2,2,3,3,4,4-nonafluorohept-4-en-3-one

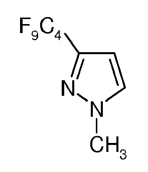

¹H-NMR (400 MHz, d₃-Acetonitril) δ = 3.97 (s, 3H, CH₃), 6.57 (m, 1H, CH), 7.61(m, 1H, CH) ppm;

HPLC-MS^{a)}: logP = 3,38; Masse (m/z): 301 (M+H)⁺.

### 3-{[difluoro(trifluoromethoxy)methoxy](difluoro)methyl}-1-methyl-1H-pyrazol aus (E)-5-Ethoxy-3-{[difluoro(trifluoromethoxy)methoxy](difluoro)methyl}-4-en-3-one

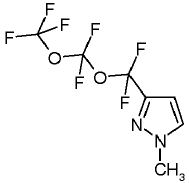

¹H-NMR (400 MHz, d₃-Acetonitril) δ = 3.90 (s, 3H, CH₃), 6.54 (m, 1H, CH), 7.58(m, 1H, CH) ppm;

HPLC-MS^{a)}: logP = 3,79; Masse (m/z): 333 (M+H)⁺.

### 4-Brom-1-Meth,3-heptafluorpropyl-1H-pyrazol

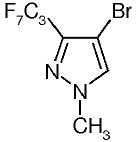

Zu einer Lösung von 4,65 g (18,59 mmol) 1-Methyl-3-heptafluorpropyl-1H-pyrazol in 18 ml Wasser werden bei 40°C 3,27 g (20,45 mmol) Brom getropft und das Reaktionsgemisch zunächst 1Stunde bei 60°C und dann 18 Stunden bei Raumtemperatur nachgerührt. Die wässrige Phase wird dreimal mit Dichlormethan extrahiert und die organische Phase über Natriumsulfat getrocknet. Das Dichlormethan wird am Rotationsverdampfer bei vermindertem Druck abdestilliert. Man erhält 5,75 g (77,85 % der Theorie) 1-Methyl-3-heptafluorpropyl-4-bromo-1H-pyrazol als Öl.

¹H-NMR (400 MHz, d₃-Acetonitril) δ = 3.90 (s, 3H, CH₃), 7.73(m, 1H, CH) ppm;

HPLC-MS^{a)}: logP = 3,53; Masse (m/z): 330 (M+H)⁺.

Auf gleiche Weise wurden erhalten:

### 4-Brom-1-Methyl-3-pentafluorethyl-1H-pyrazol aus 1-Methyl-3-pentafluorethyl-1H-pyrazol

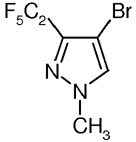

¹H-NMR (400 MHz, d₃-Acetonitril) δ = 3.90 (s, 3H, CH₃), 7.77(m, 1H, CH) ppm;

HPLC-MS^{a)}: logP = 2,99; Masse (m/z): 280 (M+H)⁺.

### 4-Brom-1-Methyl-3-(1-chlor-1,2,2,2-tetrafluorethyl-1H-pyrazol aus 1-Methyl-3-(1-chlor,1,2,2,2-tetrafluorethyl-1H-pyrazol

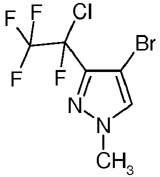

¹H-NMR (400 MHz, d₃-Acetonitril) δ = 3.89 (s, 3H, CH₃), 7.75(m, 1H, CH) ppm;

HPLC-MS^{a)}: logP = 3,17; Masse (m/z): 296 (M+H)⁺.

### 1-Methyl-3-pentafluorethyl-1H-pyrazol-5-carbonsäure

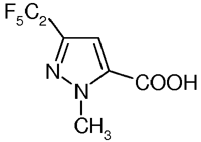

Unter Argonatmosphäre werden 5,00 g (24,99 mmol) 1-Methyl-3-pentafluorethyl-1*H*-pyrazol in Diethylether vorgelegt und die Lösung auf -78°C gekühlt. Man tropft 11,09 ml (27,73 mmol) 2M Lithiumdiisopropylamidlösung in THF/Heptan zu und gibt bei -30°C unter starkem Rühren 450 g zerstoßenes Trockeneis zu. Nach Beendigung der Gasentwicklung versetzt man das Reaktionsgemisch mit 235 ml Wasser und stellt mit 1N Natronlauge pH 11 ein. Die alkalische Lösung wird dreimal mit Ethylacetat extrahiert und daraufhin mit 1N Salzsäure auf pH 2 gestellt. Die wässrige Phase wird dreimal mit Ethylacetat extrahiert und die organische Phase über Natriumsulfat getrocknet. Nach Abdestillieren des Lösungsmittels am Rotationsverdampfer unter vermindertem Druck erhält man 1,20 g (17,75 % der Theorie) 1-Methyl-3-pentafluorethyl-1H-pyrazol-5-carbonsäure als Feststoff.

¹H-NMR (400 MHz, d₃-Acetonitril) δ = 4.16 (s, 3H, CH₃), 7.14(m, 1H, CH) ppm;

HPLC-MS^{a)}: logP = 2,08; Masse (m/z): 245 (M+H)⁺.

Auf gleiche Weise wurden erhalten:

### 4-Brom-1-Methyl-3-pentafluoroethyl-1H-pyrazol-5-carbonsäure aus 4-Brom-1-Methyl-3-pentafluorethyl-1H-pyrazol

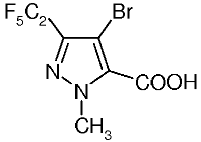

¹H-NMR (400 MHz, d₃-Acetonitril) δ = 4.15 (s, 3H, CH₃), ppm;

HPLC-MS^{a)}: logP = 4,69; Masse (m/z): 324 (M+H)⁺.

### 4-Brom-1-Methyl-3-heptafluorpropyl-1H-pyrazol-5-carbonsäure aus 4-Brom-1-Methyl-3-heptafluorpropyl-1H-pyrazol

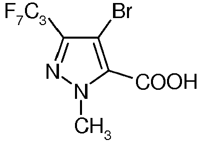

¹H-NMR (400 MHz, d₃-Acetonitril) δ = 4.15 (s, 3H, CH₃), ppm;

HPLC-MS^{a)}: logP = 2,26; Masse (m/z): 374 (M+H)⁺.

### 4-Brom-1-Methyl-3-(1-chloro-1,2,2,2-tetrafluoroethyl)-1H-pyrazol-5-carbonsäure aus 4-Brom-1-Methyl-3-(1-chlor-1,2,2,2-tetrafluorethyl-1H-pyrazol

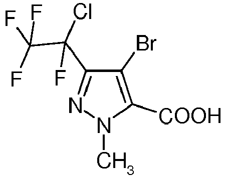

¹H-NMR (400 MHz, d₃-Acetonitril) δ = 4.14 (s, 3H, CH₃), ppm;

HPLC-MS^{a)}: logP = 2,43; Masse (m/z): 340 (M+H)⁺.

### 1-Methyl-3-nonafluorbutyl-1H-pyrazol-5-carbonsäure aus 1-Methyl-3-nonafluorbutyl-1H-pyrazol

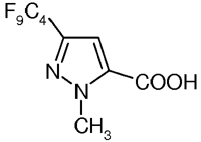

¹H-NMR (400 MHz, d₃-Acetonitril) δ = 4.17 (s, 3H, CH₃), 7.14(m, 1H, CH) ppm;

HPLC-MS^{a)}: logP = 3,01; Masse (m/z): 345 (M+H)⁺.

### 3-{[difluoro(trifluoromethoxy)methoxy](difluoro)methyl}-1-methyl-1H-pyrazol-5-carbonsäure aus 3-{[difluoro(trifluoromethoxy)methoxy](difluoro)methyl}-1-methyl-1H-pyrazol

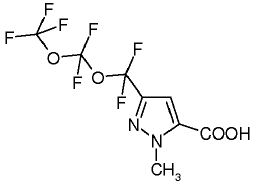

¹H-NMR (400 MHz, d₃-Acetonitril) δ = 4.16 (s, 3H, CH₃), 7.11(m, 1H, CH) ppm;

HPLC-MS^{a)}: logP = 3,38; Masse (m/z): 377 (M+H)⁺.

### 4-Brom-1-Methyl-3-nonafluorbutyl-1H-pyrazol-5-carbonsäure

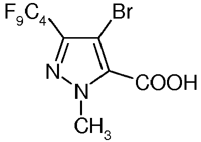

Zu einer Lösung von 0,50 g (1,45 mmol) 1-Methyl-3-nonafluorbutyl-1H-pyrazol in 3,5 ml Wasser werden bei 40°C 0,255 g (1,60 mmol) Brom getropft und das Reaktionsgemisch zunächst 1Stunde bei 60°C und dann 3 Tage bei Raumtemperatur nachgerührt. Die wässrige Phase wird dreimal mit Dichlormethan extrahiert und die organische Phase über Natriumsulfat getrocknet. Das Dichlormethan wird am Rotationsverdampfer bei vermindertem Druck abdestilliert. Man erhält 0,54 g (80,12 % der Theorie) 4-Brom-1-Methyl-3-nonafluorbutyl-1H-pyrazolcarbonsäure als Öl.

¹H-NMR (400 MHz, d₃-Acetonitril) δ = 4.16 (s, 3H, CH₃), ppm;

HPLC-MS^{a)}: logP = 3,17; Masse (m/z): 424 (M+H)⁺.

Auf gleiche Weise wurden erhalten:

### 4-Brom-3-{[difluoro(trifluoromethoxy)methoxy](difluoro)methyl}-1-methyl-1H-pyrazol-5-carbonsäure aus 3-{[difluoro(trifluoromethoxy)methoxy](difluoro)methyl}-1-methyl-1H-pyrazol-5-carbonsäure

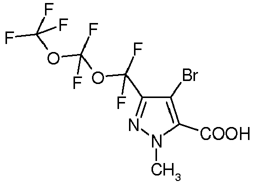

¹H-NMR (400 MHz, d₃-Acetonitril) δ = 4.14 (s, 3H, CH₃), ppm;

HPLC-MS^{a)}: logP = 3,56; Masse (m/z): 456 (M+H)⁺.

### 1-Methyl-3-pentafluorethyl-4-iodo-1H-pyrazol-5-carbonsäure

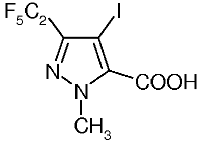

Zu einer Lösung von 1,20 g (4,91 mmol) 1-Methyl-3-pentafluorethyl-1H-pyrazol in 4,3 ml Acetonitril werden 1,34 g (2,46 mmol) Ammoniumcer(IV)nitrat und anschließend 0,75 g (2,95 mmol) Iod gegeben und das Reaktionsgemisch 18 Stunden unter Rückfluß erhitzt. Nach Zusatz von 20 ml Dichlormethan wäscht man zunächst mit Wasser, mit Natriumdisulfitlösung und schließlich mit gesättigter Kochsalzlösung. Die organische Phase wird über Natriumsulfat getrocknet und das Lösungsmittel wird am Rotationsverdampfer bei vermindertem Druck abdestilliert. Man erhält 1,28 g (47 % der Theorie) 4-Iod-1-Methyl-3-pentafluorethyl-1H-pyrazolcarbonsäure als Öl.

¹H-NMR (400 MHz, d₃-Acetonitril) δ = 4.16 (s, 3H, CH₃), ppm;

HPLC-MS: logP = 2,33; Masse (m/z): 371 (M+H)⁺.

### Biologische Beispiele

Die Wirksamkeit der erfindungsgemäßen Verbindungen gegenüber tierischen Schädlingen wird durch die folgenden biologischen Beispiele illustriert.

### Beispiel A

### Phaedon-Test (PHAECO Spritzbehandlung)

Lösungsmittel: 78,0 Gewichtsteile Aceton
   1,5 Gewichtsteile Dimethylformamid
Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis)* werden mit einer Wirkstoffzubereitung der gewünschten Konzentration gespritzt und nach dem Abtrocknen mit Larven des Meerrettichblattkäfers (*Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp Nr : lak-93,lak-90,lak-1,lak-92,lak-89,lak-88,lak-87,lak-86,lak-85,lak-84,lak-83,lak-82, lak-81,lak-79,lak-80,lak-74,lak-75,lak-76,lak-77,lak-73,lak-2,lai-1,lai-4,lak-69,lae-1,lae-4,
lak-35,lak-36,lak-37,lak-38,lak-39,lak-40,lak-41,lak-42,lak-43,lak-44,lak-45,lak-46,
lak-47,lak-48,lak-49,lak-50,lak-51,lak-52,lak-53,lak-54,lak-55,lak-57,lak-3,lak-59,lak-60
,lak-61,lak-62,lak-63,lak-64,lak-65,lak-66,lak-67,lak-68,lak-31,lak-30,lak-26,lak-27,
lak-28,lab-l,lar-9,lar-10,lak-25,lab-2,lak-24,lak-18,lak-19,lak-20,lak-21,lak-22,lak-23,
lak-16,lak-17,lak-15,lak-13,lak-14,laj-1,lab-3,lab-4,lab-5,lak-11,lak-12,lak-9,lak-7,
lak-102,lak-6,lak-4,lak-98,lak-99,lak-100,lak-95,lak-96,lak-117,lak-118,lak-119,lak-120,
lak-121,lak-122,lab-9,laj-3,lab-7,lab-10,laj-2,lak-5,lab-18,lab-15,lab-16,lab-17,lab-24,
lab-31,lab-27,lab-28,lab-29,lab-30

### Beispiel B

### Spodoptera frugiperda-Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays)* werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele eine Wirkung von ≥ 80 % bei einer Aufwandmenge von 500 g/ha:
Bsp Nr : lak-93,lak-90,lak-1,lak-87,lak-86,lak-84,lak-83,lak-80,lak-75,lak-73,lak-35,
lak-36,lak37,lak-38,lak-39,lak-40,lak-43,lak-44,lak-47,lak-49,lak-54,lak-57,lak-3,lak-59,
lak-61,lak-62,lak-63,lak-64,lak-65,lak-66,lak-67,lak-68,lak-31,lak-30,lak-26,lak-28,
lak-18,lak-19,lak-21,lak-22,lak-16,lak-13,lak-11,lak-9,lak-7,lak-6,lak-99,lak-95,lak-117,
lak-118,lak-119,lak-120,lak-121,lak-122,lab-9,lab-15,lab-16,lab-17,lab-31,lab-29,lab-30

### Beispiel C

### Myzus-Test (MYZUPE Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*)*,* die von allen Stadien der Grünen Pfirsichblattlaus *(Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele eine Wirkung von ≥ 80 % bei einer Aufwandmenge von 500 g/ha:
Bsp Nr: lak-93,lak-90,lak-1,lak-88,lak-87,lak-86,lak-85,lak-83,lak-82,lak-2,lak-66,lak-33,lak-32,lak-30,lak-24,lak-22,lak-104,lab-3,lak-9,lak-6,lab-15,lak-108,lab-19,lab-20,lab-23,lab-24,lab-27,lab-30,lak-113,lak-34

### Beispiel D

### Tetranychus-Test, OP-resistent (TETRUR Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (*Phaseolus vulgaris*), die von allen Stadien der Gemeinen Spinnmilbe (*Tetranychus urticae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von ≥ 80 % bei einer Aufwandmenge von 500 g/ha :
Bsp Nr : lak-93,lak-92,lak-89,lak-88,lak-87,lak-86,lak-85,lak-84,lak-83,lak-82,lak-81,
lak-78,lak-79,lak-80,lak-2,lak-36,lak-37,lak-38,lak-39,lak-40,lak-41,lak-42,lak-43,lak-44,
lak-45,lak-46,lak-47lak-48,lak-49,lak-50,lak-51,lak-52,lak-54,lak-53,lak-57,lak-3,lak-60,
lak-61,lak-62,lak-63,lak-64lak-66,lak-67,lak-68,lak-33,lak-32,lak-31,lak-30,lak-28,lab-2,
lak-24,lak-18,lak-19,lak-20,lak-21,lak-22,lak-23,lak-16,lak-14,lab-3,lab-4,lab-5,lab-6,
lak-12,lak-9,lak-7,lak-98,lak-117,lak-118,lak-119,lak-120,lak-122,lab-9,lab-7,lab-10,
lab-8,lab-18,lab-13,lab-14,lab-15,lab-16,lab-17,lab-22,lab-27,lab-28,lab-29
Bei diesem Test zeigen z. B. die folgenden Verbindungen der Herstellungsbeispiele eine Wirkung von ≥ 80 % bei einer Aufwandmenge von 100 g/ha :
   Bsp Nr : lak-90, lak-1

### Beispiel E

### Lucilia cuprina-Test (LUCICU)

### Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.
08-3068.doc

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigen z. B. die folgenden Verbindungen der Herstellungsbeispiele eine Wirkung von ≥ 80 % bei einer Aufwandmenge von 100 ppm :
Bsp Nr : lak-93,lak-90,lak-88,lak-87,lak-86,lak-85,lak-84,lak-83,lak-80,lak-37,lak-38,
lak-39,lak-40,lak-43,lak-47,lak-49,lak-51,lak-3,lak-62,lak-63,lak-64,lak-67,lak-68,lak-32,
lak-18,lak-19,lak-22

### Beispiel F

### Ctenocephalides felis; oral (CTECFE)

### Lösungsmittel: 1 Gewichtsteil Dimethylsulfoxid

Zwecks Herstellung einer zweckmäßigen Wirkstoffzubereitung vermischt man 2 Gewichtsteile Wirkstoff mit der angegebenen Menge Lösungsmittel. Ein Teil des Konzentrats wird mit citriertem Rinderblut verdünnt und die gewünschte Konzentration hergestellt.
20 nüchterne adulte Flöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach der gewünschten Zeit wird die Abtötung in % bestimmt. Dabei bedeutet 100 %, dass alle Flöhe abgetötet wurden; 0 % bedeutet, dass kein Floh abgetötet wurde.

Bei diesem Test zeigen z. B. die folgenden Verbindungen der Herstellungsbeispiele eine Wirkung von ≥ 80 % bei einer Aufwandmenge von 100 ppm :
Bsp Nr : lak-90,lak-88,lak-87,lak-86,lak-85,lak-83,lak-82,lak-39,lak-43,lak-47,lak-49,
lak-51,lak-3,lak-62,lak-63,lak-64,lak-68,lak-18,lak-22

### Beispiel G

### Musca domestica-Test (MUSCDO)

### Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die einen Schwamm enthalten, der mit der Wirkstoffzubereitung der gewünschten Konzentration behandelt wurde, werden mit *Musca domestica Adulten* besetzt.

Nach der gewünschten Zeit wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z. B. die folgenden Verbindungen der Herstellungsbeispiele eine Wirkung von ≥ 80 % bei einer Aufwandmenge von 100 ppm :
Bsp Nr : lak-90,lak-88,lak-87,lak-86,lak-39,lak-40,lak-51,lak-3,lak-62,lak-63,lak-64,lak-67

### Beispiel H

### Boophilus microplus -Test (BOOPMI Injektion)

### Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Die Wirkstofflösung wird in das Abdomen (*Boophilus microplus)* injiziert, die Tiere werden in Schalen überführt und in einem klimatisierten Raum aufbewahrt. Die Wirkungskontrolle erfolgt auf Ablage fertiler Eier.

Nach der gewünschten Zeit wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele gute Wirksamkeit bei einer Aufwandmenge von 20µg / Tier : siehe Tabelle
Bsp Nr : lak-93,lak-90,lak-88,lak-87,lak-86,lak-85,lak-84,lak-83,lak-82,lak-80,lak-37,
lak-38,lak-39,lak-40,lak-43,lak-47,lak-49,lak-51,lak-3,lak-64,lak-68,lak-18,lak-19,lak-22

### Beispiel I

### Boophilus microplus -Test (BOOPMI dip)

### Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Adulte Zeckenweibchen (*Boophilus microplus*) werden in perforierte Plastikbecher gesetzt und in der gewünschten Konzentration eine Minute getaucht. Die Zecken werden auf Filterpapier in Schalen überführt. Die Zecken werden 42 Tage unter klimatisierten Bedingungen gelagert und die Eiablage beobachtet.

Nach der gewünschten Zeit wird die Wirkung in % bestimmt. Dabei bedeutet 100%, dass keine Zecke fertile Eier gelegt hat.

Bei diesem Test zeigen z. B. die folgenden Verbindungen der Herstellungsbeispiele eine Wirkung von ≥ 80 % bei einer Aufwandmenge von 100 ppm:
Bsp Nr : lak-93,lak-88,lak-87,lak-86,lak-85,lak-83,lak-51,lak-3,lak-62,lak-63,lak-64,
lak-67,lak-19,lak-22

### Beispiel J

### Amblyomma hebaraeum -Test (AMBYHE)

### Lösungsmittel: Dimethylsulfoxid

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Zeckenymphen (*Amblyomma hebraeum*) werden in perforierte Plastikbecher gesetzt und in der gewünschten Konzentration eine Minute getaucht. Die Zecken werden auf Filterpapier in eine Petrischale überführt und in einem Klimaschrank 42 Tage gelagert.

Nach der gewünschten Zeit wird die Abtötung in % bestimmt. Dabei bedeutet 100 %, dass alle Zecken abgetötet wurden; 0 % bedeutet, dass keine Zecken abgetötet wurden.

Bei diesem Test zeigen z. B. die folgenden Verbindungen der Herstellungsbeispiele eine Wirkung von ≥ 80 % bei einer Aufwandmenge von 100 ppm:
Bsp Nr : lak-90,lak-83

## Patentansprüche

1. Verbindungen der allgemeinen Formel (Ik), 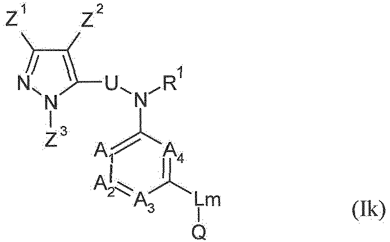 In welchen
Z¹ für 2,4-Dichlorphenyl, 2-Chlorphenyl, 3,5-Bis(trifluormethyl)phenyl, 4-(Trifluormethoxy)phenyl, 4-(Trifluormethyl)phenyl, 4-Fluorphenyl, C₂F₅, C₃F₇, C₄F₉, CF(CH₃)₂, CF₂CF₃, CF₃, CF₃CClF, CF₃CH₂O, CF₃OC₂F₄OCF₂, CHF₂, CHFCF₃, OCHF₂ steht,
Z² für H, 4-Fluor-phenyl, Cl, F, Br, I, CF₃,CH₃, Ethyl, Prop-2-enyl, Vinyl steht,
Z³ für H, Methyl, Ethyl, 1-Methylethyl, C(CH₃)₃, CH₂OCH₃, CF(CH₃)₂ steht,
R¹ für H, Methyl, Ethyl, 1-Methylethyl, CH₂OCH₃ steht,
A¹ für C-H, CCH₃, C-Cl, C-F, N steht,
A² für C-H, C-F, C-Cl, C-Br, C-OCH₃, CCONHcPr, C-Cyan, N steht,
A³ für Bindung zu Q, C-H, C-F, C-Cl, C-Br, C-I, C-NO₂, C-CH₃, C-OCH3, COCHF₂, C-SCH₃, C-SO₂CH₃, N steht,
A⁴ für C-H, C-F, C-Cl, N steht,
Lm für C(O)O, C(O)OCH₂C(O), C(O)OCH₂C(O)NH, C(O)OCH₂C(O)NHC(O), C(O)OCH₂C(O)NMe, CO, CON(CH₂CH₃), CON(CH₃), CON(Cyclopropyl), CONCH₃, CONH, steht und
Q für einen der Reste H, Methyl, Ethyl, NH₂, 3-Chlor-prop-2-enyl, (1R,2R)-2-Methylcyclopropyl, (1R,2S)-2-Methylcyclopropyl, (1S,2R)-2-Fluorcyclopropyl, (2R)-1,1,1-Trifluorpropan-2-yl, (2S)-1,1,1-Trifluor-3-methylbutan-2-yl, (2S)-1,1,1-Trifluorpropan-2-yl, (2Z)-3 -Chlorbut-2-en-1-yl, [4-(Trifluormethyl)-1,3-thiazol-2-yl]methyl, 1-(1-Chlorcyclopropyl)ethyl, 1-(2-Fluorphenyl)ethyl, 1-(3-Fluorphenyl)ethyl, 1-(Pyridin-3-yl)ethyl, 1-(Trifluormethyl)cyclopropyl, 1,1,1-Trifluorbutan-2-yl, 1,1,1-Trifluorpentan-2-yl, 1,1-Dimethylbut-2-inyl, 1,1-Dimethylethyl, 1,1-Dioxido-2,3-dihydrothiophen-3-yl,1,2,4-Triazol-3-ylmethyl, 1-CH₃-2-(ethylsulfanyl)ethyl, 1-Cyanethyl, 1-Cyclopropylethyl, 1-Fluorpropan-2-yl, 1-Methylethyl, 1-Methylpropyl, 1-Phenylethyl, 1-Pyridin-2-ylethyl, 1-Trifluormethylethyl, 2-(2,2,2-Trifluorethoxy)ethyl, 2-(Methylsulfanyl)ethyl, 2,2,2-Trifluor-1-(4-methyl-1,3thiazol-2-yl)ethyl, 2-Methyl-1-(methylsulfanyl)propan-2-yl 2,2,2-Trifluor-1-[1-methyl-4-(trifluormethyl)-1H-imidazol-2-yl]ethyl, 2,2,2-Trilfuorethyl, 2,2,3,3,3-Pentafluorpropyl, 2,2-Difluorcyclopropyl, 2,2-Difluormethyl, 2,2-Difluorpropyl, 2,2-Dimethyl-3-fluorpropyl, 2,2-Dimethylcyclopropyl-methyl, 2,4-Difluorbenzyl, 2,5-Difluorphenylmethyl, 2,6-Difluorphenylmethyl, 2-Chlorbenzyl, 2-Cyan-1-methoxypropan-2-yl, 2-Cyanoethyl, 2-Cyanpropan-2-yl, 2-Ethoxyethyl, 2-Ethylcyclopropyl, 2-Fluorcyclopropyl, 2-Fluorphenylmethyl, 2-Hydroxypropyl, 2-Methyl-1-(methylsulfanyl)propan-2-yl, 2-Methyl-1-(methylsulfonyl)propan-2-yl, 2-Methylbenzyl, 2-Methylbutyl, 2-Methylcyclopropyl, 2-Methylprop-2-enyl, 2-Methylpropyl, 2-Phenylcyclopropyl, 2-Trifluormethylphenylethyl, 3,3,3-Trifluorpropyl, 3,3-Dichlor-1,1-dimethylprop-2-enyl, 3,3-Dichlorprop-2-en-1-yl, 3,4-Difluorbenzyl, 3-Chlor-prop-2-enyl, 3-Fluorphenylmethyl, 3-Methylbenzyl, 3-Methyloxetan-3-ylmethyl, 4-(Trifluormethyl)piperidin-1-yl, 4,4,4-Trifluor-2-methylbutan-2-yl, 4,4,4-Trifluorbutan-2-yl, 4-Chlorbenzyl, 4-Chlorphenylethyl, 4-Fluorbenzyl, 4-Methylbenzyl, 4-Trifluormethylcyclohexyl, 5-Fluorpyridin-2-ylmethyl, Benzyl, But-3-in-2-yl, CH(CH₃)CF₃, -CH₂-[Bindung zu A³], cis-2-Fluorcyclopropyl, Cyanomethyl, Cyclobutyl, Cyclopent-3-en-1-yl, Cyclopentyl, Cyclopropyl, Cyclopropyl(tetrahydro-2H-pyran-4-yl)amino, Dicyclopropylmethyl, Hydroxymethyl, Isoxazol-3-ylmethyl, Methoxy, Methoxycarbonyl, Methoxycarbonylmethyl, Methylsulfonyl, N,N-Diethylamino, N,N-Dimethylamino, N-Allylamino, N-Ethylamino, Oxetan-3-yl, Prop-2-enyl, Prop-2-inyl, Propyl, Pyridin-2-ylmethyl, Pyridin-4-ylmethyl, Pyrimidin-2-ylmethyl, Tetrahydro-2H-pyran-4-yl, trans-2-Fluorcyclopropyl, trans-4-Hydroxycyclohexyl steht, und
U für C(=O) steht.

2. Verwendung von Verbindungen der allgemeinen Formel (Ik) gemäß Anspruch 1 zur Bekämpfung tierischer Schädlinge, die in der Landwirtschaft, in den Forsten, im Vorrats- und Materialschutz vorkommen.

3. Verwendung von Verbindungen der allgemeinen Formel (Ik) gemäß Anspruch 2 zur Bekämpfung von Arthropoden, Insekten, Spinnentieren Helminthen, Nematoden und Mollusken, die in der Landwirtschaft, in den Forsten, im Vorrats- und Materialschutz vorkommen.

4. Verwendung von Verbindungen der allgemeinen Formel (Ik) gemäß Anspruch 1 zur Herstellung pharmazeutischer Zusammensetzungen zur Bekämpfung von Parasiten auf Tieren.

5. Verbindungen der allgemeinen Formel (V-6) 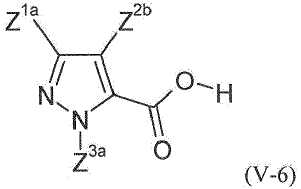 (V-6)
wobei unabhängig voneinander
Z^{1a} für 1,2,2,2-Tetrafluorethyl, 1-Chlor-1,2,2,2-tetrafluorethyl, Pentafluorethyl, Heptafluor-n-propyl, Heptafluor-isopropyl, Nonafluor-n-butyl, Difluormethoxy, Difluormethyl, ,
Z^{2b} für Fluor, Chlor, Brom, Iod, Methyl, Ethyl, Trifluormethyl, 2-Propenyl,,
Z^{3a} für Ethyl, Isopropyl, tert-Butyl, 1-Fluor-1-methylethyl,
stehen.

6. Verfahren zur Herstellung von Pflanzenschutzmitteln enthaltend Verbindungen der allgemeinen Formel (Ik) gemäß Anspruch 1 sowie übliche Streckmittel und/oder oberflächenaktive Substanzen.

7. Verwendung von Verbindungen der allgemeinen Formel (Ik) gemäß Anspruch 1 zum Schutz des Vermehrungsmaterials von Pflanzen, insbesondere von Saatgut.

## Claims

1. Compounds of the general formula (Ik), 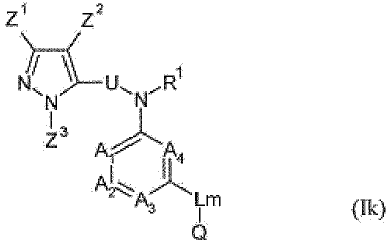 in which
Z¹ is 2,4-dichlorophenyl, 2-chlorophenyl, 3,5-bis(trifluoromethyl)phenyl, 4-(trifluoro-methoxy)phenyl, 4-(trifluoromethyl)phenyl, 4-fluorophenyl, C₂F₅, C₃F₇, C₄F₉, CF(CH₃)₂, CF₂CF₃, CF₃, CF₃CClF, CF₃CH₂O, CF₃OC₂F₄OCF₂, CHF₂, CHFCF₃, OCHF₂,
Z² is H, 4-fluorophenyl, Cl, F, Br, I, CF₃, CH₃, ethyl, prop-2-enyl, vinyl,
Z³ is H, methyl, ethyl, 1-methylethyl, C(CH₃)₃, CH₂OCH₃, CF(CH₃)₂,
R¹ is H, methyl, ethyl, 1-methylethyl, CH₂OCH₃,
A¹ is C-H, CCH₃, C-Cl, C-F, N,
A² is C-H, C-F, C-Cl, C-Br, C-OCH₃, CCONHcPr, C-cyano, N,
A³ is a bond to Q, C-H, C-F, C-Cl, C-Br, C-I, C-NO₂, C-CH₃, C-OCH₃, COCHF₂, C-SCH₃, C-SO₂CH₃, N,
A⁴ is C-H, C-F, C-Cl, N,
Lm is C(O)O, C(O)OCH₂C(O), C(O)OCH₂C(O)NH, C(O)OCH₂C(O)NHC(O), C(O)OCH₂C(O)NMe, CO, CON(CH₂CH₃), CON(CH₃), CON(cyclopropyl), CONCH₃, CONH, and
Q is one of the radicals H, methyl, ethyl, NH₂, 3-chloroprop-2-enyl, (1R,2R)-2-methylcyclo-propyl, (1R,2S)-2-methylcyclopropyl, (1S,2R)-2-fluorocyclopropyl, (2R)-1,1,1-trifluoro-propan-2-yl, (2S)-1,1,1-trifluoro-3-methyl-butan-2-yl, (2S)-1,1,1-trifluoropropan-2-yl, (2Z)-3-chlorobut-2-en-1-yl, [4-(trifluoro-methyl)-1,3-thiazol-2-yl]methyl, 1-(1-chloro-cyclopropyl)ethyl, 1-(2-fluorophenyl)ethyl, 1-(3-fluorophenyl)ethyl, 1-(pyridin-3-yl)-ethyl, 1-(trifluoromethyl)cyclopropyl, 1,1,1-trifluorobutan-2-yl, 1,1,1-trifluoropentan-2-yl, 1,1-dimethylbut-2-ynyl, 1,1-dimethyl-ethyl, 1,1-dioxido-2,3-dihydrothiophen-3-yl, 1,2,4-triazol-3-ylmethyl, 1-CH₃-2-(ethyl-sulphanyl)ethyl, 1-cyanoethyl, 1-cyclo-propylethyl, 1-fluoropropan-2-yl, 1-methyl-ethyl, 1-methylpropyl, 1-phenylethyl, 1-pyridin-2-ylethyl, 1-trifluoromethylethyl, 2-(2,2,2-trifluoroethoxy)ethyl, 2-(methyl-sulphanyl)ethyl, 2,2,2-trifluoro-1-(4-methyl-1,3-thiazol-2-yl)ethyl, 2-methyl-1-(methyl-sulfanyl)propan-2-yl, 2,2,2-trifluoro-1-[1-methyl-4-(trifluoromethyl)-1H-imidazol-2-yl]-ethyl, 2,2,2-trifluoroethyl, 2,2,3,3,3-penta-fluoropropyl, 2,2-difluorocyclopropyl, 2,2-difluoroethyl, 2,2-difluoropropyl, 2,2-dimethyl-3-fluoropropyl, 2,2-dimethylcyclo-propylmethyl, 2,4-difluorobenzyl, 2,5-difluorophenylmethyl, 2,6-difluorophenyl-methyl, 2-chlorobenzyl, 2-cyano-1-methoxy-propan-2-yl, 2-cyanoethyl, 2-cyanopropan-2-yl, 2-ethoxyethyl, 2-ethylcyclopropyl, 2-fluorocyclopropyl, 2-fluorophenylmethyl, 2-hydroxypropyl, 2-methyl-1-(methyl-sulphanyl)propan-2-yl, 2-methyl-1-(methylsulphonyl)propan-2-yl, 2-methylbenzyl, 2-methylbutyl, 2-methylcyclopropyl, 2-methyl-prop-2-enyl, 2-methylpropyl, 2-phenylcyclo-propyl, 2-trifluoromethylphenylethyl, 3,3,3-trifluoropropyl, 3,3-dichloro-1,1-dimethyl-prop-2-enyl, 3,3-dichloroprop-2-en-1-yl, 3,4-difluorobenzyl, 3-chloroprop-2-enyl, 3-fluro-phenylmethyl, 3-methylbenzyl, 3-methyloxetan-3-ylmethyl, 4-(trifluoromethyl)piperidin-1-yl, 4,4,4-trifluoro-2-methylbutan-2-yl, 4,4,4-trifluorobutan-2-yl, 4-chlorobenzyl, 4-chlorophenylethyl, 4-fluorobenzyl, 4-methylbenzyl, 4-trifluoromethylcyclohexyl, 5-fluoropyridin-2-ylmethyl, benzyl, but-3-yn-2-yl, CH(CH₃)CF₃, -CH₂-[bond to A³], cis-2-fluorocyclopropyl, cyanomethyl, cyclobutyl, cyclopent-3-en-1-yl, cyclopentyl, cyclo-propyl, cyclopropyl(tetrahydro-2H-pyran-4-yl)amino, dicyclopropylmethyl, hydroxymethyl, isoxazol-3-ylmethyl, methoxy, methoxy-carbonyl, methoxycarbonylmethyl, methyl-sulphonyl, N,N-diethylamino, N,N-dimethyl-amino, N-allylamino, N-ethylamino, oxetan-3-yl, prop-2-enyl, prop-2-ynyl, propyl, pyridin-2-ylmethyl, pyridin-4-ylmethyl, pyrimidin-2-ylmethyl, tetrahydro-2H-pyran-4-yl, trans-2-fluorocyclopropyl, trans-4-hydroxycyclohexyl, and
U is C(=O).

2. Use of compounds of the general formula (Ik) according to Claim 1 for controlling animal pests which occur in agriculture, in forests, in the protection of stored materials and the protection of other materials.

3. Use of compounds of the general formula (Ik) according to Claim 2 for controlling arthropods, insects, arachnids, helminths, nematodes and molluscs which occur in agriculture, in forests, in the protection of stored materials and the protection of other materials.

4. Use of compounds of the general formula (Ik) according to Claim 1 for preparing pharmaceutical compositions for controlling parasites on animals.

5. Compounds of the general formula (V-6) 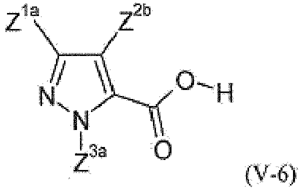 (V-6)
where, independently of one another,
Z^{1a} is 1,2,2,2-tetrafluoroethyl, 1-chloro-1,2,2,2-tetrafluoroethyl, pentafluoroethyl, heptafluoro-n-propyl, heptafluoroisopropyl, nonafluoro-n-butyl, difluoromethoxy, difluoromethyl,
Z^{2b} is fluorine, chlorine, bromine, iodine, methyl, ethyl, trifluoromethyl, 2-propenyl,
Z^{3a} is ethyl, isopropyl, tert-butyl, 1-fluoro-1-methylethyl.

6. Process for preparing crop protection compositions comprising compounds of the general formula (Ik) according to Claim 1 and customary extenders and/or surface-active substances.

7. Use of compounds of the general formula (Ik) according to Claim 1 for protecting the propagation material of plants, in particular of seed material.

## Revendications

1. Composés de formule générale (Ik), 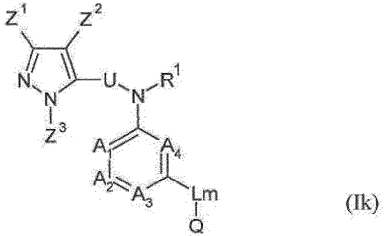 dans laquelle
Z¹ représente le groupe 2,4-dichlorophényle, 2-chlorophényle, 3,5-bis(trifluorométhyl)phényle, 4-(trifluorométhoxy)phényle, 4-(trifluorométhyl)phényle, 4-fluorophényle, C₂F₅, C₃F₇, C₄F₉, CF(CH₃)₂, CF₂CF₃, CF₃, CF₃CClF, CF₃CH₂O, CF₃OC₂F₄OCF₂, CHF₂, CHFCF3, OCHF₂,
Z² représente H, le groupe 4-fluoro-phényle, Cl, F, Br, I, le groupe CF₃, CH₃, éthyle, prop-2-ényle, vinyle,
Z³ représente H, le groupe méthyle, éthyle, 1-méthyl-éthyle, C(CH₃)₃, CH₂OCH₃, CF(CH₃)₂,
R¹ représente H, le groupe méthyle, éthyle, 1-méthyl-éthyle, CH₉OCH₃,
A¹ représente C-H, CCH₃, C-Cl, C-F, N,
A² représente C-H, C-F, C-Cl, C-Br, C-OCH₃, CCONHcPr, C-cyano, N,
A³ représente une liaison à Q, C-H, C-F, C-Cl, C-Br, C-I, C-NO₂, C-CH₃, C-OCH₃, COCHF₂, C-SCH₃, C-SO₂CH₃, N,
A⁴ représente C-H, C-F, C-Cl, N,
Lm représente C(O)O, C(O)OCH₂C(O), C(O)OCH₂C(O)NH, C(O)OCH₂C(O)NHC(O), C(O)OCH₂C(O)NMe, CO, CON(CH₂CH₃), CON(CH₃), CON(cyclopropyle), CONCH₃, CONH, et
Q représente l'un des radicaux H, méthyle, éthyle, NH₂, 3-chloro-prop-2-ényle, (1R,2R)-2-méthyl-cyclopropyle, (1R,2S)-2-méthylcyclopropyle, (1S,2R)-2-fluorocyclopropyle, (2R)-1,1,1-trifluoropropan-2-yle, (2S)-1,1,1-trifluoro-3-méthylbutan-2-yle, (2S)-1,1,1-trifluoropropan-2-yle, (2Z)-3-chlorobut-2-én-1-yle, [4-(trifluoro-méthyl)-1,3-thiazol-2-yl]méthyle, 1-(1-chloro-cyclopropyl)éthyle, 1-(2-fluorophényl)éthyle, 1-(3-fluorophényl)éthyle, 1-(pyridin-3-yl)éthyle, 1-(trifluorométhyl)cyclopropyle, 1,1,1-trifluoro-butan-2-yle, 1,1,1-trifluoropentan-2-yle, 1,1-diméthylbut-2-ynyle, 1,1-diméthyléthyle, 1,1-dioxydo-2,3-dihydrothiophén-3-yle, 1,2,4-triazol-3-ylméthyle, 1-CH₃-2-(éthylsulfanyl)éthyle, 1-cyanoéthyle, 1-cyclopropyléthyle, 1-fluoro-propan-2-yle, 1-méthyléthyle, 1-méthylpropyle, 1-phényléthyle, 1-pyridin-2-yléthyle, 1-trifluoro-méthyléthyle, 2-(2,2,2-trifluoroéthoxy)éthyle, 2-(méthylsulfanyl)éthyle, 2,2,2-trifluoro-1-(4-méthyl-1,3-thiazol-2-yl)éthyle, 2-méthyl-1-(méthylsulfanyl)propan-2-yle 2,2,2-trifluoro-1-[1-méthyl-4-(trifluorométhyl)-1H-imidazol-2-yl]éthyle, 2,2,2-trifluoroéthyle, 2,2,3,3,3-pentafluoropropyle, 2,2-difluoro-cyclopropyle, 2,2-difluoroéthyle, 2,2-difluoro-propyle, 2,2-diméthyl-3-fluoropropyle, 2,2-diméthylcyclopropyl-méthyle, 2,4-difluorobenzyle, 2,5-difluorophénylméthyle, 2,6-difluorophényl-méthyle, 2-chlorobenzyle, 2-cyano-1-méthoxypropan-2-yle, 2-cyanoéthyle, 2-cyanopropan-2-yle, 2-éthoxyéthyle, 2-éthylcyclopropyle, 2-fluoro-cyclopropyle, 2-fluorophénylméthyle, 2-hydroxy-propyle, 2-méthyl-1-(méthylsulfanyl)propan-2-yle, 2-méthyl-1-(méthylsulfonyl)propan-2-yle, 2-méthyl-benzyle, 2-méthylbutyle, 2-méthylcyclopropyle, 2-méthylprop-2-ényle, 2-méthylpropyle, 2-phényl-cyclopropyle, 2-trifluorométhylphényléthyle, 3,3,3-trifluoropropyle, 3,3-dichloro-1,1-diméthyl-prop-2-ényle, 3,3-dichloroprop-2-én-1-yle, 3,4-difluorobenzyle, 3-chloro-prop-2-ényle, 3-fluoro-phénylméthyle, 3-méthylbenzyle, 3-méthyloxétan-3-ylméthyle, 4-(trifluorométhyl)pipéridin-1-yle, 4,4,4-trifluoro-2-méthylbutan-2-yle, 4,4,4-trifluorobutan-2-yle, 4-chlorobenzyle, 4-chloro-phényléthyle, 4-fluorobenzyle, 4-méthylbenzyle, 4-trifluorométhylcyclohexyle, 5-fluoropyridin-2-ylméthyle, benzyle, but-3-yn-2-yle, CH(CH₃)CF₃, -CH₂-[liaison à A³], cis-2-fluorocyclopropyle, cyanométhyle, cyclobutyle, cyclopent-3-én-1-yle, cyclopentyle, cyclopropyle, cyclopropyl-(tétrahydro-2H-pyran-4-yl)amino, dicyclopropyl-méthyle, hydroxyméthyle, isoxazol-3-ylméthyle, méthoxy, méthoxycarbonyle, méthoxycarbonylméthyle, méthylsulfonyle, N,N-diéthylamino, N,N-diméthyl-amino, N-allylamino, N-éthylamino, oxétan-3-yle, prop-2-ényle, prop-2-ynyle, propyle, pyridin-2-ylméthyle, pyridin-4-ylméthyle, pyrimidin-2-ylméthyle, tétrahydro-2H-pyran-4-yle, trans-2-fluorocyclopropyle, trans-4-hydroxycyclohexyle, et
U représente C(=O).

2. Utilisation de composés de formule générale (Ik) selon la revendication 1, pour la lutte contre des ravageurs animaux qui apparaissent en agriculture, dans les forêts, dans la protection des stocks et des matériaux.

3. Utilisation de composés de formule générale (Ik) selon la revendication 2, pour la lutte contre des arthropodes, insectes, arachnides, helminthes, nématodes et mollusques, qui apparaissent en agriculture, dans les forêts, dans la protection des stocks et des matériaux.

4. Utilisation de composés de formule générale (Ik) selon la revendication 1, pour la préparation de compositions pharmaceutiques destinées à la lutte contre des parasites d'animaux.

5. Composés de formule générale (V-6) 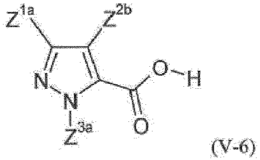 (V-6)
dans laquelle, indépendamment les uns des autres
Z^{1a} représente le groupe 1,2,2,2-tétrafluoroéthyle, 1-chloro-1,2,2,2-tétrafluoroéthyle, pentafluoro-éthyle, heptafluoro-n-propyle, heptafluoro-isopropyle, nonafluoro-n-butyle, difluorométhoxy, difluorométhyle,
Z^{2b} représente un atome de fluor, de chlore, de brome, d'iode, le groupe méthyle, éthyle, trifluoro-méthyle, 2-propényle,
Z^{3a} représente le groupe éthyle, isopropyle, tert-butyle, 1-fluoro-1-méthyléthyle.

6. Procédé pour la préparation de produits phytosanitaires contenant des composés de formule générale (Ik) selon la revendication 1 ainsi que des diluants usuels et/ou substances tensioactives usuelles.

7. Utilisation de composés de formule générale (Ik) selon la revendication 1, pour la protection du matériel de multiplication de plantes, en particulier de semences.
